# EUROPÄISCHE PATENTSCHRIFT

(11) **EP 2 683 688 B1**
(45) Veröffentlichungstag und Bekanntmachung des Hinweises auf die Patenterteilung: **06.05.2015**
(21) Anmeldenummer: 12706624.9
(22) Anmeldetag: 05.03.2012
(51) Int. Cl.: C07D 209/24, C07D 235/32, A01N 43/38, A01N 43/52

(54) **INDOL- UND BENZIMIDAZOLCARBONSÄUREAMIDE ALS INSEKTIZIDE UND AKARIZIDE**
INDOLECARBOXAMIDES AND BENZIMIDAZOLECARBOXAMIDES AS INSECTICIDES AND ACARICIDES
AMIDES D'ACIDE CARBOXYLIQUE D'INDOLE ET DE BENZIMIDAZOLE UTILISÉS COMME INSECTICIDES ET ACARICIDES

(30) Priorität: 09.03.2011 EP 11157401; 09.03.2011 US 201161450817 P
(43) Veröffentlichungstag der Anmeldung: 15.01.2014
(73) Patentinhaber: Bayer Intellectual Property GmbH, 40789 Monheim am Rhein (DE)
(72) Erfinder: HEIL, Markus, 42799 Leichlingen (DE); HEILMANN, Eike, Kevin, 40597 Düsseldorf (DE); HOLMWOOD, Graham, 51371 Leverkusen (DE); JESCHKE, Peter, 51467 Bergisch Gladbach (DE); MAUE, Michael, 40764 Langenfeld (DE); KAPFERER, Tobias, 4054 Basel (CH); RIEDRICH, Matthias, 50735 Köln (DE); BECKER, Angela, 40235 Düsseldorf (DE); MALSAM, Olga, 51503 Rösrath (DE); LÖSEL, Peter, 51371 Leverkusen (DE); VOERSTE, Arnd, 50674 Köln (DE); GÖRGENS, Ulrich, 40882 Ratingen (DE); ANDREE, Roland, 40764 Langenfeld (DE)
(74) Vertreter: BIP Patents
(86) Internationale Anmeldenummer: PCT/EP2012/053752
(87) Internationale Veröffentlichungsnummer: WO 2012/119984

(56) Entgegenhaltungen:
- EP-A1- 0 697 172
- EP-A1- 1 188 745
- EP-A1- 1 439 169
- US-A- 3 732 241
- US-A1- 2011 105 532

## Beschreibung

Die vorliegende Erfindung betrifft neue Schädlingsbekämpfungsmittel, ein Verfahren zu ihrer Herstellung und ihre Verwendung als Wirkstoffe, insbesondere ihre Verwendung als Insektizide und Akarizide.

In der Literatur sind Indol-2-carboxamide und Benzimidazol-2-carboxamide und ihre Verwendung als Arzneimittel beschrieben, siehe beispielsweise WO-A-2010/126164, WO-A-2010/054138, US 2009/0041722, WO-A-2007/115938, EP1460064, WO-A-2004-A-056768, WO-A-2004/032921, WO-A-20010/32622. Es wurde nun überraschend gefunden, dass bestimmte neue Indol- und Benzimidazolcarbonsäureamide starke insektizide und akarizide Eigenschaften bei gleichzeitig guter Pflanzenverträglichkeit, günstiger Warmblütertoxizität und guter Umweltverträglichkeit besitzen. Die erfindungsgemäßen neuen Verbindungen wurden bislang nicht offenbart.

Gegenstand der vorliegenden Erfindung sind daher Verbindungen der allgemeinen Formel (I) 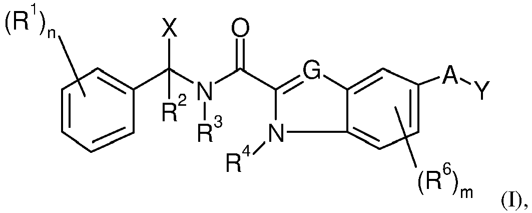 wobei
- R¹: für Halogen, Nitro, Cyano, für gegebenenfalls einfach oder mehrfach durch Halogen substituiertes C₁-C₆-Alkyl, C₁-C₆-Alkoxy, C₁-C₆-Alkylthio, C₁-C₆-Alkylsulfinyl oder C₁-C₆-Alkylsulfonyl steht,
- n: für 1,2, 3, 4 oder 5 steht,
oder
- R¹: für -OCF₂O-, -(CF₂)₂O-oder -O(CF₂)₂O- steht, und an zwei benachbarten Kohlenstoffatomen gebunden ist, wobei n dann für 1 steht,
- R²: für Wasserstoff, oder für gegebenenfalls einfach oder mehrfach, gleich oder verschieden substituiertes C₁-C₄-Alkyl steht,
wobei die Substituenten unabhängig voneinander ausgewählt sind aus Halogen oder C₁-C₄-Alkyl
- R³: für Wasserstoff, für gegebenenfalls einfach oder mehrfach, gleich oder verschieden substituiertes C₁-C₄-Alkyl, C₁-C₄-Alkylcarbonyl, oder C₁-C₄-Alkoxycarbonyl steht,
wobei die Substituenten unabhängig voneinander ausgewählt sind aus Cyano, Halogen, C₁-C₄-Alkyl oder C₁-C₄-Alkoxy
- R⁴: für gegebenenfalls einfach oder mehrfach, gleich oder verschieden substituiertes C₁-C₆-Alkyl, C₂-C₆-Alkenyl, C₃-C₆-Alkinyl, C₃-C₆-Cycloalkyl, C₃-C₆-Cycloalkyl-C₁-C₄-alkyl oder Aryl-C₁-C₄-alkyl steht,
wobei die Substituenten unabhängig voneinander ausgewählt sind aus Halogen, Cyano, C₁-C₄-Alkyl oder C₁-C₄-Alkoxy, C₁-C₄-Alkoxycarbonyl oder aus gegebenenfalls einfach oder mehrfach, gleich oder verschieden substituiertem Aryloxy oder Aryl-C₁-C₃-alkoxy
wobei die Substituenten unabhängig voneinander ausgewählt sind aus Halogen, Cyano, C₁-C₄-Alkyl, C₁-C₄-Alkoxy
- G: für C(R⁵) oder N steht,
- R⁵: für Wasserstoff, Halogen oder Cyano steht,
- R⁶: für Halogen, Nitro, Cyano, für gegebenenfalls einfach oder mehrfach, durch Halogen substituiertes C₁-C₆-Alkyl oder C₁-C₆-Alkoxy steht,
- m: für 0, 1, 2, 3 steht
- X: für C₁-C₆-Halogenalkyl steht, das gegebenenfalls zusätzlich einfach bis dreifach substituiert sein kann, wobei die Substituenten unabhängig voneinander ausgewählt sind aus Hydroxy, Cyano oder C₁-C₄-Alkoxy,
- A: für eine bivalente chemische Gruppierung steht, die ausgewählt ist aus den Gruppierungen -C(R¹¹)(R¹²)NR¹³C(=O)- oder -C(=O)NR¹³-, wobei die jeweils erstgenannte (linke) Anknüpfungsstelle am Ring und die jeweils zweitgenannte (rechte) Anknüpfungsstelle an Y anknüpft,
und wobei
- R¹¹ und R¹²: unabhängig voneinander für Wasserstoff oder für C₁-C₄-Alkyl steht,
- R¹³: für Wasserstoff, für C₁-C₄-Alkyl, C₃-C₆-Cycloalkyl, C₁-C₄-Alkylcarbonyl, C₁-C₄-Alkoxycarbonyl oder C₁-C₄-Alkenyl, steht,
- Y: für gegebenenfalls ein- oder mehrfach, gleich oder verschieden substituiertes C₁-C₆-Alkyl, C₂-C₆-Alkenyl, C₃-C₆-Alkinyl, C₃-C₆-Cycloalkyl, Aryl, Aryl-C₁-C₄-alkyl, Hetaryl oder Hetaryl-C₁-C₄-alkyl steht,
wobei die Substituenten ausgewählt sind aus Halogen, Nitro, Cyano, Hydroxy, Aminothiocarbonyl, Aminocarbonyl, C₁-C₄-Alkylaminocarbonyl, Di-(C₁-C₄-Alkyl)-aminocarbonyl, Hydroxycarbonyl, C₁-C₄-Alkyl, C₁-C₄-Halogenalkyl, C₃-C₆-Cycloalkyl, C₁-C₄-Alkyl-C₃-C₄-Cycloalkyl, C₂-C₆-Alkenyl, C₂-C₆-Alkinyl, C₁-C₆-Alkoxy, C₁-C₆-Alkoxycarbonyl, C₁-C₆-Alkylcarbonyl, C₁-C₄-Alkoxyimino-C₁-C₄-Alkyl, C₁-C₆-Alkylthio, C₁-C₆-Alkylsulfinyl oder C₁-C₆-Alkylsulfonyl,
wobei
- n: für 2, 3, 4 oder 5 steht, wenn
mindestens ein Substituent R¹ für Trifluormethyl steht, und gleichzeitig
- Y: für unsubstituiertes C₁-C₄-Alkyl, 2,2-Difluorethyl, unsubstituiertes C₂-C₆-Alkenyl, unsubstituiertes C₃-C₆-Alkinyl, für unsubstituiertes C₃-C₆-Cycloalkyl oder unsubstituiertes Hetaryl steht, und
- A: für -C(=O)NR¹³- steht,
und
- G: für C(R⁵) steht,

sowie Salze und N-Oxide von Verbindungen der Formel (I) und deren Anwendung zur Bekämpfung von tierischen Schädlingen

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls vorliegende E/Z-Isomere sowie Diastereomere oder Enantiomere.

Die substituierten Indol- und Benzimidazolcarbonsäureamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.

Die jeweilige Zahl der Substituenten n und m in der Formel (I) umfasst dabei nur die Substituenten, die von Wasserstoff verschieden sind. Aus diesem Grund ist Wasserstoff in der Definition von R¹ und R⁶ auch nicht enthalten. Natürlich steht als Substituent immer Wasserstoff, wenn kein Substituent R¹ oder R⁶ an der jeweiligen Stelle vorhanden ist.

Als bevorzugt, besonders bevorzugt und ganz besonders bevorzugt gelten Verbindungen der Formel (I), sowie ein Vefahren zur Bekämpfung von Schädlingen unter Anwendung der Verbindungen der Formel (I), wobei
- R¹: bevorzugt für Halogen, Nitro, Cyano, für gegebenenfalls einfach oder mehrfach durch Halogen substituiertes C₁-C₄-Alkyl, C₁-C₄-Alkoxy, C₁-C₄-Alkylthio, C₁-C₄-Alkylsulfinyl oder C₁-C₄-Alkylsulfonyl steht,
- n: bevorzugt für 1, 2, 3, 4 oder 5 steht
oder
- R¹: für -OCF₂O- oder -O(CF₂)₂O- steht, und an zwei benachbarten Kohlenstoffatomen gebunden ist, wobei n dann für 1 steht,
- R²: bevorzugt für Wasserstoff oder für gegebenenfalls einfach bis dreifach substituiertes C₁-C₄-Alkyl steht,
wobei die Substituenten unabhängig voneinander ausgewählt sind aus Halogen oder C₁-C₄-Alkyl,
- R³: bevorzugt für Wasserstoff, für gegebenenfalls einfach oder mehrfach, gleich oder verschieden substituiertes C₁-C₄-Alkyl, C₁-C₄-Alkylcarbonyl oder C₁-C₄-Alkoxycarbonyl steht,
wobei die Substituenten unabhängig voneinander ausgewählt sind aus Cyano, Halogen oder C₁-C₄-Alkoxy
- R⁴: bevorzugt für gegebenenfalls einfach oder mehrfach, gleich oder verschieden substituiertes C₁-C₄-Alkyl, C₂-C₆-Alkenyl, C₃-C₆-Alkinyl, C₃-C₆-Cycloalkyl, C₃-C₆-Cycloalkyl-C₁-C₃-alkyl oder Aryl-C₁-C₄-alkyl steht,
wobei die Substituenten unabhängig voneinander ausgewählt sind aus Halogen, Cyano, C₁-C₄-Alkyl, C₁-C₄-Alkoxy, C₁-C₄-Alkoxycarbonyl oder aus gegebenenfalls einfach oder mehrfach, gleich oder verschieden substituiertem Aryloxy oder Aryl-C₁-C₃-alkoxy,
wobei die Substituenten unabhängig voneinander ausgewählt sind aus Halogen, Cyano, C₁-C₄-Alkyl, C₁-C₄-Alkoxy
- G: bevorzugt für C(R⁵) oder N steht,
- R⁵: bevorzugt für Wasserstoff , Halogen oder Cyano steht,
- R⁶: bevorzugt für Halogen, Nitro, Cyano, für gegebenenfalls einfach oder mehrfach, durch Halogen substituiertes C₁-C₄-Alkyl oder C₁-C₄-Alkoxy steht,
- m: bevorzugt für 0, 1, 2 steht
- X: bevorzugt für C₁-C₄-Halogenalkyl steht, das gegebenenfalls zusätzlich einfach bis dreifach durch Hydroxy, Cyano oder C₁-C₄-Alkoxy substituiert sein kann,
- A: bevorzugt für eine bivalente chemische Gruppierung steht, die ausgewählt ist aus den Gruppierungen -C(R¹¹)(R¹²)NR¹³C(=O)- oder -C(=O)NR¹³-, wobei die jeweils erstgenannte (linke) Anknüpfungsstelle am Ring und die jeweils zweitgenannte (rechte) Anknüpfungsstelle an Y anknüpft,
- R¹¹ und R¹²: unabhängig voneinander bevorzugt für Wasserstoff oder für C₁-C₄-Alkyl steht,
- R¹³: bevorzugt für Wasserstoff, für C₁-C₄-Alkyl, C₃-C₆-Cycloalkyl, C₁-C₄-Alkylcarbonyl, C₁-C₄-Alkoxycarbonyl oder C₂-C₄-Alkenyl steht,
- Y: bevorzugt für gegebenenfalls ein- oder mehrfach, gleich oder verschieden substituiertes C₁-C₄-Alkyl, C₂-C₄-Alkenyl, C₂-C₄-Alkinyl, C₃-C₆-Cycloalkyl, Phenyl, Phenylmethyl, Pyridinyl, Pyridinylmethyl, Pyrimidinyl oder Pyrimidinylmethyl steht,
wobei die Substituenten ausgewählt sind aus Halogen, Nitro, Cyano, Hydroxy, Aminothiocarbonyl, Aminocarbonyl, C₁-C₄-Alkyl, C₁-C₄-Halogenalkyl, C₃-C₅-Cycloalkyl, C₁-C₄-Alkyl-C₃-C₄-Cycloalkyl, C₂-C₄-Alkenyl, C₂-C₄-Alkinyl, C₁-C₄-Alkoxy, C₁-C₄-Alkylthio, C₁-C₄-Alkylsulfinyl, C₁-C₄-Alkylsulfonyl, C₁-C₄-Alkoxycarbonyl, C₁-C₆-Alkylcarbonyl oder C₁-C₄-Alkoxyimino-C₁-C₄-Alkyl.
wobei
- n: für 2, 3, 4 oder 5 steht, wenn
mindestens ein Substituent R¹ für Trifluormethyl steht,
und gleichzeitig
- Y: für unsubstituiertes C₁-C₄-Alkyl, 2,2-Difluorethyl, unsubstituiertes C₂-C₆-Alkenyl, unsubstituiertes C₃-C₆-Alkinyl, für unsubstituiertes C₃-C₆-Cycloalkyl oder unsubstituiertes Hetaryl steht, und
- A: für -C(=O)NR¹³- steht,
und
G für C(R⁵) steht,
- R¹: besonders bevorzugt für Halogen, Nitro, Cyano, für gegebenenfalls einfach oder mehrfach, durch Fluor oder Chlor substituiertes C₁-C₄-Alkyl, C₁-C₄-Alkoxy, C₁-C₄-Alkylthio, C₁-C₄-Alkylsulfinyl, C₁-C₄-Alkylsulfonyl steht,
- n: besonders bevorzugt für 1, 2, 3, 4 oder 5 steht
oder
- R¹: für -OCF₂O- steht, und an zwei benachbarten Kohlenstoffatomen gebunden ist, wobei n dann für 1 steht,
- R²: besonders bevorzugt für Wasserstoff oder für Methyl steht,
- R³: besonders bevorzugt für Wasserstoff, Methyl, Ethyl, Methylcarbonyl, Ethylcarbonyl, Methoxycarbonyl oder Ethoxycarbonyl steht,
- R⁴: besonders bevorzugt für gegebenenfalls einfach bis dreifach substituiertes C₁-C₄-Alkyl, C₂-C₄-Alkenyl , C₃-C₄-Alkinyl, C₃-C₅-Cycloalkyl, C₃-C₅-Cycloalkyl-C₁-C₃-alkyl oder Phenylalkyl steht,
wobei die Substituenten unabhängig voneinander ausgewählt sind aus Fluor, Cyano, Methoxy, Ethoxy, Methyl, Ethyl, Methoxycarbonyl, Ethoxycarbonyl, Phenyloxy oder Phenyl-C₁-C₃-alkoxy.
- G: besonders bevorzugt für C(R⁵) oder N steht,
- R⁵: besonders bevorzugt für Wasserstoff, Fluor, Chlor, Brom oder Cyano steht,
- R⁶: besonders bevorzugt für Halogen, Nitro, Cyano, oder für gegebenenfalls einfach bis dreifach durch Halogen substituiertes C₁-C₄-Alkyl oder C₁-C₄-Alkoxy steht,
- m: besonders bevorzugt für 0, 1 oder 2 steht,
- X: besonders bevorzugt für C₁-C₄-Halogenalkyl steht,
- A: besonders bevorzugt für eine bivalente chemische Gruppierung steht, die ausgewählt ist aus den Gruppierungen -C(R¹¹)(R¹²)NR¹³C(=O)- oder -C(=O)NR¹³-, wobei die jeweils erstgenannte (linke) Anknüpfungsstelle am Ring und die jeweils zweitgenannte (rechte) Anknüpfungsstelle an Y anknüpft,
und wobei
- R¹¹ und R¹²: unabhängig voneinander besonders bevorzugt für Wasserstoff oder Methyl stehen,
und wobei
- R¹³: besonders bevorzugt für Wasserstoff, Methyl, Ethyl, Cyclopropyl, Methylcarbonyl, Ethylcarbonyl, Methoxycarbonyl, Ethoxycarbonyl oder Prop-2-en-1-yl steht,
- Y: besonders bevorzugt für gegebenenfalls ein- bis dreifach, gleich oder verschieden substituiertes C₁-C₄-Alkyl, C₂-C₄-Alkenyl, C₂-C₄-Alkinyl, C₃-C₆-Cycloalkyl, Phenyl, Phenylmethyl, Pyridin-2-yl, Pyridin-2-yl-methyl, 1,3-Pyrimidin-2-yl oder 1,3-Pyrimidin-2-yl-methyl steht,
wobei die Substituenten ausgewählt sind aus Fluor, Chlor, Nitro, Cyano, C₁-C₄-Alkyl, Trifluormethyl, C₃-C₄-Cycloalkyl, C₁-C₄-Alkyl-C₃-C₄-Cycloalkyl, C₂-C₄-Alkenyl, C₃-C₄-Alkinyl C₁-C₄-Alkoxy, C₁-C₄-Alkylthio, C₁-C₄-Alkylsulfinyl, C₁-C₆-Alkylsulfonyl, C₁-C₄-Alkoxycarbonyl oder Aminothiocarbonyl,
wobei
- n: für 2, 3, 4 oder 5 steht, wenn
mindestens ein Substituent R¹ für Trifluormethyl steht,
und gleichzeitig
- Y: für unsubstituiertes C₁-C₄-Alkyl, 2,2-Difluorethyl, unsubstituiertes C₂-C₆-Alkenyl, unsubstituiertes C₃-C₆-Alkinyl, für unsubstituiertes C₃-C₆-Cycloalkyl oder unsubstituiertes Hetaryl steht, und
- A: für -C(=O)NR¹³- steht,
und
- G: für C(R⁵) steht,
- R¹: ganz besonders bevorzugt für Cyano, Fluor, Chlor, Brom, Iod, Difluormethyl, Dichlorfluormethyl, Chlordifluormethyl, Trifluormethyl, Pentafluorethyl, Chlortetrafluorethyl, Difluormethoxy, Trifluormethoxy, Trifluormethylthio, Trifluormethylsulfinyl, Trifluormethylsulfonyl, steht,
- n: ganz besonders bevorzugt für 1, 2, 3, 4 oder 5 steht oder
- R¹: ganz besonders bevorzugt für -OCF₂O- steht, und an zwei benachbarten Kohlenstoffatomen gebunden ist, wobei n dann für 1 steht,
- R²: ganz besonders bevorzugt für Wasserstoff steht,
- R³: ganz besonders bevorzugt für Wasserstoff steht,
- R⁴: ganz besonders bevorzugt für Methyl, Ethyl, Prop-1-yl, Prop-2-en-1-yl, Prop-2-in-1-yl, Ethenyl, But-2-in-1-yl, Cyclopropyl, Cyclopropylmethyl, Cyclobutyl, Cyanomethyl, 2-Methylprop-1-yl, Ethoxymethyl, Methoxycarbonylmethyl, Phenylmethyl oder Benzyloxymethyl, steht,
- G: ganz besonders bevorzugt für C(R⁵) oder N steht,
- R⁵: ganz besonders bevorzugt für Wasserstoff, Chlor, Brom oder Cyano steht,
- R⁶: ganz besonders bevorzugt für Cyano, Fluor, Chlor, Brom, Methyl, Ethyl, Isopropyl oder Trifluormethyl steht,
- m: ganz besonders bevorzugt für 0 oder 1 steht,
- X: ganz besonders bevorzugt für Trifluormethyl steht,
- A: ganz besonders bevorzugt für eine bivalente chemische Gruppierung steht, die ausgewählt ist aus den Gruppierungen -CH₂NHC(=O)- oder -C(=O)NR¹³-, wobei die jeweils erstgenannte (linke) Anknüpfungsstelle am Ring und die jeweils zweitgenannte (rechte) Anknüpfungsstelle an Y anknüpft,
und wobei
- R¹³: ganz besonders bevorzugt für Wasserstoff, Methyl, Ethyl oder Prop-2-en-1-yl steht,
- Y: ganz besonders bevorzugt für Methyl, Ethyl, Propan-1-yl, Propan-2-yl, Butan-1-yl, Butan-2-yl, 2-Methylpropan-1-yl, 2-Methylpropan-2-yl, Cyclopropyl, Cyclobutyl, Cyanomethyl, 1-Cyanoethyl, 2-Cyanoethyl, 1-Cyanoprop-1-yl, 2-Cyanoprop-1-yl, 3-Cyanoprop-1-yl, 1-Cyanoprop-2-yl, 2-Cyanoprop-2-yl, 1-Cyanocyclopropyl, 2-Cyanoprop-2-en-1-yl, 2-Cyanocyclopropyl, 1-Cyanocyclobutyl, 2-Cyanocyclobutyl, 3-Cyanocyclobutyl, 2-Fluorethyl, 2,2-Difluorethyl, 2,2,2-Trifluorethyl, 1-Fluorpropan-2-yl, 2,2-Difluorprop-1-yl, 1,3-Difluorpropan-2-yl, 1-Methylcyclopropyl, 2-Methylcyclopropyl, 1-Ethylcyclopropyl, 1-Ethinylcyclopropyl, 1-Ethinylcyclobutyl, 1-Methoxycyclopropyl, 1-Ethoxycyclopropyl, 1-Methoxycarbonylcyclopropyl, 1-Ethoxycarbonyl-cyclopropyl, 1,1'-Bi(Cyclopropyl)-1-yl, Cyclopropylmethyl, 1-Trifluormethylcyclopropyl, Pyridin-2-yl, 5-Chloipyridin-2-yl, 5-Fluorpyridin-2-yl, 1-Cyano-1-phenylmethyl, 1,2-Dimethylcyclopropyl, 1-(Aminothiocarbonyl)cyclopropyl, 1-Cyano-2-methylpropan-1-yl, 1-Cyanobut-3-in-1-yl, 1-Cyano-2-methylpropan-1-yl, 1-Cyano-propan-2-yl, 1-Cyano-1-cyclopropylethyl, 1-Cyano-1-ethylprop-1-yl, 1-Cyano-1-methylcyclopropyl-methyl, (2-R)-1-(Methylsulfinyl)propan-2-yl oder 1,3-Dimethoxy-2-cyanopropan-2-yl steht, wenn A für die Gruppierung -C(=O)NR¹³- steht,
oder
- Y: ganz besonders bevorzugt für Methyl, Ethyl, Propan-1-yl, Propan-2-yl, Butan-1-yl, 2-Fluorethyl, 2,2-Difluorethyl, 2,2,2-Trifluorethyl, Cyclopropyl, Cyclobutyl oder Cyclopropylmethyl steht, wenn A für die Gruppierung -CH₂NHC(=O)- steht,
wobei
- n: für 2, 3, 4 oder 5 steht, wenn
mindestens ein Substituent R¹ für Trifluormethyl steht,
und gleichzeitig
- Y: für unsubstituiertes C₁-C₄-Alkyl, 2,2-Difluorethyl, unsubstituiertes C₂-C₆-Alkenyl, unsubstituiertes C₃-C₆-Alkinyl, für unsubstituiertes C₃-C₆-Cycloalkyl oder unsubstituiertes Hetaryl steht, und
- A: für -C(=O)NH- steht,
und
- G: für C(R⁵) steht.

Die oben angegebenen einzelnen allgemeinen, bevorzugten, besonders bevorzugten und ganz besonders bevorzugten Bedeutungen für die Substituenten R¹ bis R⁸, n, m, X, G, A und Y können erfindungsgemäß beliebig miteinander kombiniert werden.

In den bevorzugten Definitionen ist, sofern nichts anderes angegeben ist, Halogen ausgewählt aus der Reihe Fluor, Chlor, Brom und Iod, bevorzugt wiederum aus der Reihe Fluor, Chlor und Brom.

In den besonders bevorzugten Definitionen ist, sofern nichts anderes angegeben ist, Halogen ausgewählt aus der Reihe Fluor, Chlor, Brom und Iod, bevorzugt wiederum aus der Reihe Fluor, Chlor und Brom, In den ganz besonders bevorzugten Definitionen ist, sofern nichts anderes angegeben ist, Halogen ausgewählt aus der Reihe Fluor, Chlor, Brom und Iod, bevorzugt wiederum aus der Reihe Fluor, Chlor und Brom,

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Bevorzugt, besonders bevorzugt und ganz besonders bevorzugt sind Verbindungen, welche jeweils die unter bevorzugt, besonders bevorzugt und ganz besonders bevorzugt genannten Substituenten tragen.

Gesättigte oder ungesättigte Kohlenwasserstoffreste wie Alkyl, Alkenyl oder Alkinyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

In einer bevorzugten Ausführungsform betrifft die Erfindung die Verbindungen der Formel (I-1) 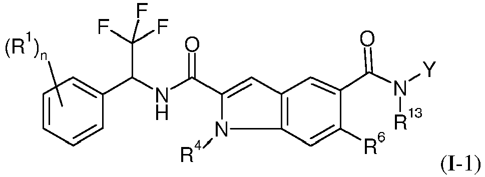 wobei R¹, R⁴, R⁶, R¹³, Y und n die oben beschriebenen Bedeutungen haben,

In einer weiteren bevorzugten Ausführungsform betrifft die Erfindung die Verbindungen der Formel (I-2) 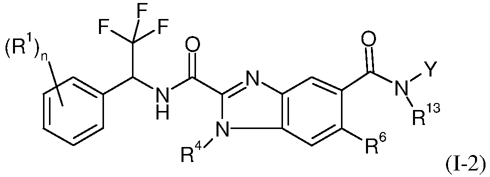 wobei R¹, R⁴, R⁶, R^{13,} Y und n die oben beschriebenen Bedeutungen haben,

In einer weiteren bevorzugten Ausführungsform betrifft die Erfindung die Verbindungen der Formel (I-3) 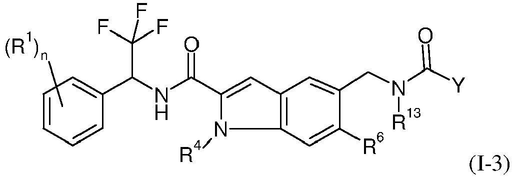 wobei R¹, R⁴, R⁶, R¹³, Y und n die oben beschriebenen Bedeutungen haben,

In einer weiteren bevorzugten Ausführungsform betrifft die Erfindung die Verbindungen der Formel (I-4) 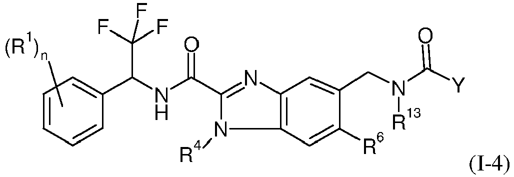 wobei R¹, R⁴, R⁶, Y, R¹³ und n die oben beschriebenen Bedeutungen haben,

Ebenfalls bevorzugte erfindungsgemäße Verbindungen sind die in Tabelle 1 gezeigten Verbindungen der allgemeinen Formel (I)

Die vorliegenden Verbindungen der allgemeinen Formel (I) können gegebenenfalls ein chirales Kohlenstoffatom aufweisen.

Gemäß den Regeln nach Cahn, Ingold und Prelog (CIP-Regeln) können diese Substituenten sowohl eine (R)- als auch eine (S)-Konfiguration aufweisen.

Von der vorliegenden Erfindung werden Verbindungen der allgemeinen Formel (I) sowohl mit (S)- als auch mit (R)-Konfiguration an den jeweiligen chiralen Kohlenstoffatomen umfasst, d.h., dass die vorliegende Erfindung die Verbindungen der allgemeinen Formel (I) erfasst, in welchen die betreffenden Kohlenstoffatome jeweils unabhängig voneinander
(1) eine (R)-Konfiguration; oder
(2) eine (S)-Konfiguration
   aufweisen. Wenn mehrere Chiralitätszentren in den Verbindungen der allgemeinen Formel (I) vorliegen, sind beliebige Kombinationen der Konfigurationen der Chiralitätszentren möglich, d.h. dass
(1) ein Chiralitätszentrum die (R)-Konfiguration und das andere Chiralitätszentrum die (S)-Konfiguration;
(2) ein Chiralitätszentrum die (R)-Konfiguration und das andere Chiralitätszentrum die (R)-Konfiguration; und
(3) ein Chiralitätszentrum die (S)-Konfiguration und das andere Chiralitätszentrum die (S)-Konfiguration
aufweisen kann.

Die Verbindungen der Formel (I) umfassen ebenfalls gegebenenfalls vorliegende Diastereomere oder Enantiomere sowie E/Z-Isomere sowie Salze und N-Oxide von Verbindungen der Formel (I) und deren Anwendung zur Bekämpfung von tierischen Schädlingen.

Die Erfindung betrifft auch die Verwendung der erfindungsgemäßen Verbindungen der allgemeinen Formel (I) zur Herstellung von Schädlingsbekämpfungsmitteln.

Die Erfindung betrifft auch Schädlingsbekämpfungsmittel enthaltend erfindungsgemäße Verbindungen der allgemeinen Formel (I) und / oder deren Salze in biologisch wirksamen Gehalten von > 0,00000001 Gew.-%, bevorzugt > 0,001 Gew.-% bis 95 Gew.-%, bezogen auf das Gewicht des Schädlingsbekämpfungsmittels.

Die Erfindung betrifft auch Verfahren zur Bekämpfung von tierischen Schädlingen, bei dem man erfindungsgemäße Verbindungen der allgemeinen Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Bevorzugt ist die Behandlung, insbesondere die therapeutische Behandlung, des menschlichen oder tierischen Körpers ausgeschlossen.

Die Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm: Arthropoda, insbesondere aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Centruroides spp., Chorioptes spp., Dermanyssus gallinae, Dermatophagoides pteronyssius, Dermatophagoides farinae, Dermacentor spp., Eotetranychus spp., Epitrimerus pyri, Eutetranychus spp., Eriophyes spp., Halotydeus destructor, Hemitarsonemus spp., Hyalomma spp., Ixodes spp., Latrodectus spp., Loxosceles spp., Metatetranychus spp., Nuphersa spp., Oligonychus spp., Ornithodorus spp., Ornithonyssus spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vaejovis spp., Vasates lycopersici.

Aus der Ordnung der Anoplura (Phthiraptera) z.B. Damalinia spp., Haematopinus spp., Linognathus spp., Pediculus spp., Ptirus pubis, Trichodectes spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Prostephanus truncatus, Psylliodes spp., Ptinus spp., Rhizobius ventralis, Rhizopertha dominica, Sitophilus spp., Sphenophorus spp., Stegobium paniceum, Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Chrysops spp., Cochliomyia spp., Contarinia spp., Cordylobia anthropophaga, Culex spp., Culicoides spp., Culiseta spp., Cuterebra spp., Dacus oleae, Dasyneura spp., Delia spp., Dermatobia hominis, Drosophila spp., Echinocnemus spp., Fannia spp., Gasterophilus spp., Glossina spp., Haematopota spp., Hydrellia spp., Hylemyia spp., Hyppobosca spp., Hypoderma spp., Liriomyza spp.. Lucilia spp., Lutzomia spp., Mansonia spp., Musca spp., Nezara spp., Oestrus spp., Oscinella frit, Pegomyia spp., Phlebotomus spp., Phorbia spp., Phormia spp., Prodiplosis spp., Psila rosae, Rhagoletis spp., Sarcophaga spp., Simulium spp, Stomoxys spp., Tabanus spp., Tannia spp., Tetanops spp., Tipula spp..

Aus der Ordnung der Heteroptera z.B. Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Acromyrmex spp., Athalia spp., Atta spp., Diprion spp., Hoplocampa spp., Lasius spp., Monomorium pharaonis, Solenopsis invicta, Tapinoma spp., Vespa spp..

Aus der Ordnung der Isopoda z.B. Armadillidium vulgare, Oniscus asellus, Porcellio scaber.

Aus der Ordnung der Isoptera z.B. Coptotermes spp., Cornitermes cumulans, Cryptotermes spp., Incisitermes spp., Microtermes obesi, Odontotermes spp., Reticulitermes spp.,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichophaga tapetzella, Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta spp., Pulex irritans, Schistocerca gregaria, Supella longipalpa.

Aus der Ordnung der Siphonaptera z.B. Ceratophyllus spp., Ctenocephalides spp., Tunga penetrans, Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Zygentoma (= Thysanura), for example, Lepisma saccharina, Thermobia domestica.

Schädlinge aus dem Stamm: Mollusca, insbesondere aus der Klasse der Bivalvia, z.B. Dreissena spp.

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Tierparasiten aus den Stämmen: Plathelminthes und Nematoda, insbesondere aus der Klasse der Helminthen z.B. Ancylostoma duodenale, Ancylostoma ceylanicum, Acylostoma braziliensis, Ancylostoma spp., Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Pflanzenschädlinge aus dem Stamm: Nematoda, d.h. pflanzenparasitäre Nematoden, insbesondere Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

### Subphylum: Protozoa

### Weiterhin lassen sich Protozoen, wie Eimeria, bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und

Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die Behandlung der Pflanzen und Pflanzenteile mit den erfindungsgemäßen Wirkstoffen erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Eine bevorzugte direkte Behandlung der Pflanzen ist die Blattapplikation, d.h. erfindungsgemäße Wirkstoffe werden auf das Blattwerk aufgebracht, wobei die Behandlungsfrequenz und die Aufwandmenge auf den Befallsdruck des jeweiligen Schädlings abgestimmt sein kann.

Bei systemisch wirksamen Verbindungen gelangen die erfindungsgemäßen Wirkstoffe über das Wurzelwerk in die Pflanzen. Die Behandlung der Pflanzen erfolgt dann durch Einwirkung der erfindungsgemäßen Wirkstoffe auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der erfindungsgemäßen Wirkstoffe getränkt, oder durch die Bodenapplikation, d.h. die erfindungsgemäßen Wirkstoffe werden in fester Form, (z.B. in Form eines Granulats) in den Standort der Pflanzen eingebracht. Bei Wasserreiskulturen kann das auch durch Zudosieren der erfindungsgemaäßen Wirkstoffen in einer festen Anwendungsform (z.B. als Granulat) in ein überflutetes Reisfeld sein.

Die erfindungsgemäßen Wirkstoffe können als solche oder in ihren Formulierungen auch in Mischungen mit bekannten Fungiziden, Bakteriziden, Akaraiziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischungen ist größer als die Summe der Wirksamkeit der Einzelverbindungen.

Als Mischpartner kommen zum Beispiel die folgenden Verbindungen in Betracht:

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chloipyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.
(2) GABA-gesteuerte Chlorid-Kanal-Antagonisten, wie beispielsweise
   Cyclodien-organochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise
   Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
   DDT; oder Methoxychlor.
(4) Nikotinerge Acetylcholin-Rezeptor (nAChR) Agonisten, wie beispielsweise
   Neonikotinoide, z.B. Acetamiprid, Clothianidin, Dinotefuran, Imidacloprid, Nitenpyram, Thiacloprid und Thiamethoxam; oder
   Nikotin.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise
   Avermectine/Milbemycine, z.B. Abamectin, Emamectin-benzoat, Lepimectin und Milbemectin.
(7) Juvenilhormon-Imitatoren, wie beispielsweise
   Juvenilhormon-Analoge, z.B. Hydroprene, Kinoprene und Methoprene; oder
   Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
   Etoxazole.
(11) Mikrobielle Dismptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin und Fenbutatin-oxid; oder
   Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartap-hydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
   METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
   Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid; oder Cyanid.
(25) Komplex-II-Elektronentransportinhibitoren, wie beispielsweise Cyenopyrafen.
(28) Ryanodimezeptor-Effektoren, wie beispielsweise
   Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Sulfoxaflor und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt ausWO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925) und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233).

### Fungizide

(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid, 5,8-Difluor-N-[2-(2-fluor-4-{[4-(trifluormethyl)pyridin-2-yl]oxy}phenyl)ethyl]quinazolin-4-amin, N-[9-(Dichlormethylen)-1,2,3,4-tetrahydro-1,4-methanonaphthalen-5-yl]-3-(difluormethyl)-1-methyl-1H-pyrazol-4-carboxamid, N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Coumethoxystrobin, Coumoxystrobin, Dimoxystrobin, Enestroburin, Famoxadon, Fenamidon, Fenoxystrobin, Fluoxastrobin, Kresoxim-Methyl, Metominostrobin, Orysastrobin, Picoxystrobin, Pyraclostrobin, Pyrametostrobin, Pyraoxystrobin, Pyribencarb, Triclopyricarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim, Pyrimethanil und 3-(5-Fluor-3,3,4,4-tetramethyl-3,4-dihydroisoquinolin-1-yl)quinolin.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon, Tricyclazol und 2,2,2-Trifluorethyl {3-methyl-1-[(4-methylbenzoyl)amino]butan-2-yl}carbamat.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl und Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Pyriofenon (Chlazafenon), Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthün, Pentachlorphenol und dessen Salze, Phenothrin, Phosphorsäure und deren Salze, Propamocarb-Fosetylat, Propanosin-Natrium, Proquinazid, Pyrimorph, (2E)-3-(4-Tert-butylphenyl)-3-(2-chlorpyridin-4-yl)-1-(morpholin-4-yl)prop-2-en-1-on, (2Z)-3-(4-Tert-butylphenyl)-3-(2-chlorpyridin-4-yl)-1-(morpholin-4-yl)prop-2-en-1-on, Pyrrolnitrin, Tebufloquin, Tecloftalam, Tolnifanid, Triazoxid, Trichlamid, Zarilamid, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2,6-Dimethyl-1H,5H-[1,4]dithiino[2,3-c:5,6-c']dipyrrol-1,3,5,7(2H,6H)-tetron, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3-(4,4,5-Trifluor-3,3-dimethyl-3,4-dihydroisoquinolin-1-yl)quinolin, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Fluor-2-[(4-fluorbenzyl)oxy]pyrimidin-4-amin, 5-Fluor-2-[(4-methylbenzyl)oxy]pyrimidin-4-amin, 5-Methyl-6-octyl[1,2,4]triazolo[1,5-a]pyrimidin-7-amin, Ethyl-(2Z)-3-amino-2-cyan-3-phenylprop-2-enoat, N'-(4-{[3-(4-Chlorbenzyl)-1,2,4-thiadiazol-5-yl]oxy}-2,5-dimethylphenyl)-N-ethyl-N-methylimidoformamid, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl}-2-phenylacetamid, N'-{4-[(3-Tert-butyl-4-cyano-1,2-thiazol-5-yl)oxy]-2-chlor-5-methylphenyl}-N-ethyl-N-methylimidoformamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol, Chinolin-8-olsulfat(2:1) und Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon, N-[2-(4-{[3-(4-Chlorphenyl)prop-2-in-1-yl]oxy}-3-methoxyphenyl)ethyl]-N2-(methylsulfonyl)valinamid, 4-Oxo-4-[(2-phenylethyl)amino]butansäure und But-3-yn-1-yl {6-[({[(Z)-(1-methyl-1H-tetrazol-5-yl)(phenyl)methylen]amino}oxy)methyl]pyridin-2-yl}carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren, Angießen und bei Vermehrungsmaterial, insbesondere bei Saatgut, weiterhin durch ein- oder mehrschichtiges Umhüllen.

Bevorzugt sind Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten die aus Teilen der Bäume hergestellt werden.

Der Begriff Nutzpflanzen, wie hier verwendet, bezeichnet Kulturpflanzen, die als Pflanzen für die Gewinnung von Nahrungsmitteln, Futtermitteln, Treibstoffen oder für technische Zwecke eingesetzt werden.

Zu den Nutzpflanzen, die mit dem erfindungsgemäßen Wirkstoffen behandelt werden können können, zählen z. B. folgende Pflanzenarten: Turf, Reben, Getreide, beispielsweise Weizen, Gerste, Roggen, Hafer, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung stellt keine Limitierung dar.

Als besonders geeignete Zielkulturen für die Behandlung mit den erfindungsgemäßen Wirkstoffen sind folgende Pflanzen anzusehen: Baumwolle, Aubergine, Turf, Kernobst, Steinobst, Beerenobst, Mais, Weizen, Gerste, Gurke, Tabak, Reben, Reis, Getreide, Birne, Bohnen, Sojabohnen, Raps, Tomate, Paprika, Melonen, Kohl, Kartoffel und Apfel.

Als Bäume, die entsprechend dem erfindungsgemäßen Verfahren verbessert werden können, seien beispielhaft genannt: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Als bevorzugte Bäume, die entsprechend dem erfindungsgemäßen Verfahren verbessert werden können, können genannt werden: Aus der Baumart Aesculus: A. hippocastanum, A. pariflora, A. carnea; aus der Baumart Platanus: P. aceriflora, P. occidentalis, P. racemosa; aus der Baumart Picea: P. abies; aus der Baumart Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; aus der Baumart Eucalyptus: E. grandis, E. globulus, E. camadentis, E. nitens, E. obliqua, E. regnans, E. pilularus.

Als besonders bevorzugte Bäume, die entsprechend dem erfindungsgemäßen Verfahren verbessert werden können, können genannt werden: Aus der Baumart Pinus: P. radiate, P. ponderosa, P. contorta, P. sylvestre, P. strobes; aus der Baumart Eucalyptus: E. grandis, E. globulus, E. camadentis.

Als ganz besonders bevorzugte Bäume, die entsprechend dem erfindungsgemäßen Verfahren verbessert werden können, können genannt werden: Rosskastanie, Platanengewächse, Linde, Ahornbaum.

Die vorliegende Erfindung kann auch an beliebigen Rasenarten ("turfgrasses") durchgeführt werden, einschließlich "cool season turfgrasses" und "warm season turfgrasses".

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Darüber hinaus können die erfindungsgemäßen Verbindungen zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von Schädlingen ein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Kozzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Zu diesen Parasiten gehören:
Aus der Ordnung der Anoplurida z.B. Haematopinus spp., Linognathus spp., Pediculus spp., Phtirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;
Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;
Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp. (z.B. Suppella longipalpa);
Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Die erfindungsgemäßen Wirkstoffe eignen sich auch zur Bekämpfung von Arthropoden, Helminthen und Protozoen, die Tiere befallen. Zu den Tieren zählen landwirtschaftliche Nutztiere wie z.B. Rinder, Schafe, Ziegen, Pferde, Schweine, Esel, Kamele, Büffel, Kaninchen, Hühner, Puten, Enten, Gänse, Zuchtfische, Honigbienen. Zu den Tieren zählen außerdem Haustiere - die auch als Heimtiere bezeichnet werden - wie z.B. Hunde, Katzen, Stubenvögel, Aquarienfische sowie sogenannte Versuchstiere wie z.B. Hamster, Meerschweinchen, Ratten und Mäuse.

Durch die Bekämpfung dieser Arthropoden, Helminthen und/oder Protozoen sollen Todesfälle vermindert und die Leistung (bei Fleisch, Milch, Wolle, Häuten, Eiern, Honig usw.) und die Gesundheit des Wirtstieres verbessert werden, so dass durch den Einsatz der erfindungsgemäßen Wirkstoffe eine wirtschaftlichere und einfachere Tierhaltung möglich ist.

So ist es beispielsweise wünschenswert, die Aufnahme von Blut des Wirts durch die Parasiten (falls zutreffend) zu verhindern oder zu unterbrechen. Eine Bekämpfung der Parasiten kann außerdem dazu beitragen, die Übertragung infektiöser Substanzen zu verhindern.

Der Begriff "Bekämpfung", so wie er hier bezogen auf den Bereich Tiergesundheit verwendet wird, bedeutet, dass die Wirkstoffe wirken, indem sie das Vorkommen des betreffenden Parasiten in einem mit solchen Parasiten befallenen Tier auf unschädliche Niveaus reduzieren. Genauer gesagt bedeutet "Bekämpfung", wie hier verwendet, dass der Wirkstoff den betreffenden Parasiten tötet, sein Wachstum hemmt oder seine Proliferation inhibiert.

Im Allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100- bis 10000facher Verdünnung) anwenden oder sie als chemisches Bad verwenden.

Beim Einsatz im Bereich Tiergesundheit können die erfindungsgemäßen Wirkstoffe in Kombination mit geeigneten Synergisten oder anderen Wirkstoffen wie beispielsweise Akariziden, Insektiziden, Anthelmintika, Mittel gegen Protozoen, verwendet werden.

Außerdem wurde gefunden, daß die erfindungsgemäßen Verbindungen eine hohe insektizide Wirkung gegen Insekten zeigen, die technische Materialien zerstören. Entsprechend bezieht sich die vorliegende Erfindung auch auf die Verwendung der erfindungsgemäßen Verbindungen zum Schutz von technischen Materialien gegen Befall oder Zerstörung durch Insekten.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirexjuvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene-und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

### (A) Die Verbindungen der allgemeinen Formel (I)

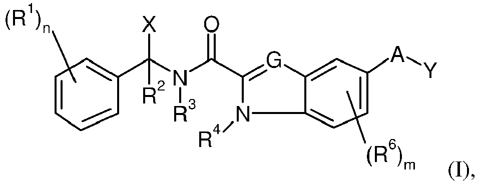

wobei R¹ bis R¹³, A, X, Y, m und n die oben beschriebenen Bedeutungen haben, können erhalten werden, indem man zunächst Carbonsäuren der allgemeinen Formel (II) 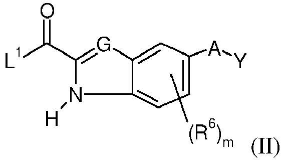 wobei
- L¹: für Hydroxy oder Halogen steht,
mit Aminen der Formel (III) 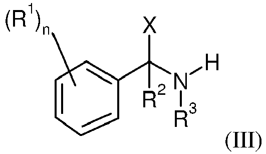 umsetzt. Hierbei kann für (II) zum einen ein Säurehalogenid (z.B. L¹ = Chlor) in Gegenwart einer Base, wie z.B. Triethylamin oder Natriumhydroxid eingesetzt werden. Zum anderen kann die Carbonsäure (L'= OH) aber auch unter der Verwendung von Kupplungsreagenzien wie z.B Dicyclohexylcarbodiimid und Additiven wie 1-Hydroxybenzotriazol hergestellt werden [Chem. Ber. 1970, 788].

Verwendbar sind ferner Kupplungsreagenzien wie 1-Ethyl-3-(3-dimethylaminopropyl)carbodiimid, 1,1'-Carbonyl-1H-imidazol, N-[(1H-Benzotriazol-1-yloxy)(dimethylamino)methylen]-N-methylmethanaminiumhexafluorophosphat, und ähnliche Verbindungen. Als Kupplungsreagenzien zur Durchführung des Dartellungsverfahrens finden alle, die zur Herstellung einer Ester- oder Amidbindung geeignet sind (vgl. z. B. Bodansky et al., Peptide Synthesis, 2nd ed., Wiley & Sons, New York, 1976; Gross, Meienhofer, The Peptide: Analysis, Synthesis, Biology (Academic Press, New York, 1979), Verwendung. Des Weiteren können auch gemischte Anhydride zur Darstellung von (I) verwendet werden. [J. Am. Chem. Soc 1967, 5012] Bei diesem Verfahren können verschiedene Chlorameisensäureester zum Einsatz kommen, wie z.B. Chlorameisensäureisobutylester, Chlorameisensäureisopropylester. Ebenfalls können dafür Diethylacetylchlorid, Trimethylacetylchlorid und ähnliche verwendet werden. Die so erhaltenen Verbindungen der Formel (IVa) 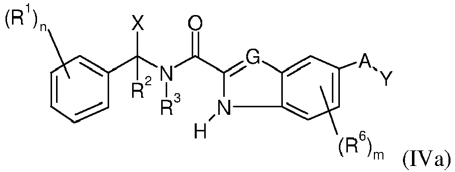 werden anschließend mit Alkylierungsmitteln der Formel (V)

R⁴-L² (V)

wobei
- L²: für Halogen, für die Mesyl- oder für die Tosylgruppe steht und R⁴ die oben angegebene Bedeutung hat,

in Gegenwart von Basen wie z.B. Natriumhydrid zu Verbindungen der Formel (I) umgesetzt.

### (B) Verbindungen der allgemeinen Formel (Ia)

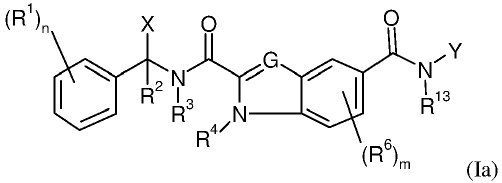

können erhalten werden, in dem man Carbonsäurederivate der allgemeinen Formel (II-1b) oder (II-2b) 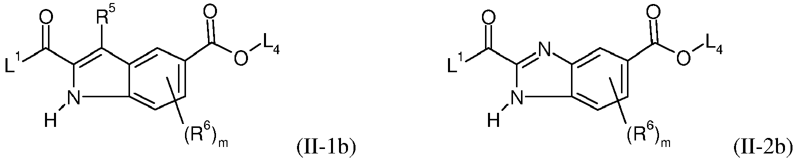 wobei
- L¹: für Hydroxy oder Halogen und
- L⁴: für C₁-C₄-Alkyl steht,
zunächst nach dem unter (A) beschriebenen Verfahren mit Aminen der Formel (III) 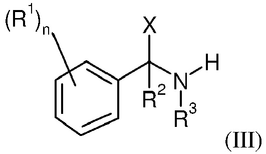 umsetzt, und die so erhaltenen Carbonsäureester der Formel (IVb) 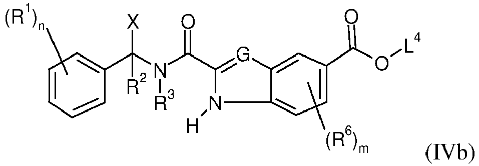 mit Alkylierungsmitteln der Formel (V)

R4-L² (V)

in Gegenwart von Basen wie z.B. Natriumhydrid zu Verbindungen der Formel (VIa) 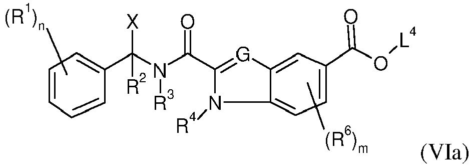 umsetzt und anschließend mit Aminen der allgemeinen Formel (VII) 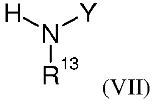 umsetzt, wobei die Umsetzung
a) direkt mit Estern der Formel (VIa) in Gegenwart eines Aktivierungsreagenzes wie z.B. Trimethylaluminium erfolgt
   oder
b) die Ester der Formel (VIa) zunächst unter sauren oder alkalischen Bedingungen zu Carbonsäuren der Formel (VIb) 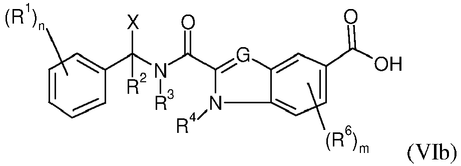 hydrolysiert werden und diese anschließend mit Aminen der Formel (VII) in Gegenwart eines Kondensationsreagenzes umgesetzt werden.

### (C) Verbindungen der allgemeinen Formel (Ib)

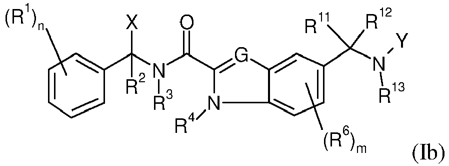

können erhalten werden, indem man Carbonsäuren der allgemeinen Formel (II-1c) oder (II-2c) 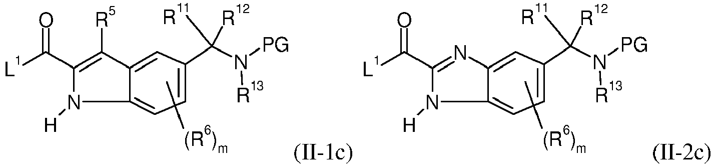 wobei
- L¹: für Halogen oder für eine Hydroxygruppe und
- PG: für eine Aminschutzgruppe, wie z.B die tert.Butyloxycarbonyl (Boc)-Schutzgruppe
steht, zunächst nach dem unter (A) beschriebenen Verfahren mit Aminen der allgemeinen Formel (III) 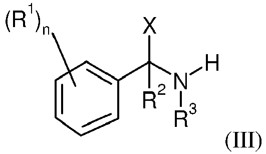 umsetzt, die so erhaltenen Verbindungen der Formel (IVc) 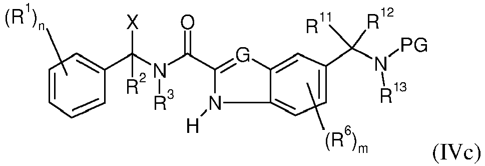 anschließend mit Alkylierungsmitteln der Formel (V)

R⁴-L² (V)

in Gegenwart einer Base, wie z.B. Natriumhydrid zu Verbindungen der Formel (VIc) 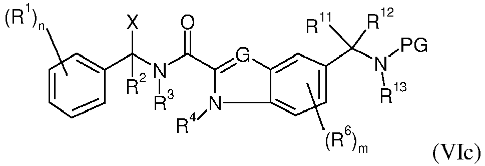 umsetzt, anschließend die Schutzgruppe PG entfernt, wobei Amine der Formel (VId) 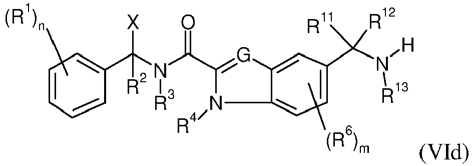 erhalten werden. Die Umsetzung der Verbindung der Formel (VIc) zu der ungeschützten Verbindung der Formel (VId) kann nach allgemein bekannten Methoden durchgeführt werden (vgl. Greene's protective groups in organic synthesis, 4th ed., P.G.M. Wuts, T.W. Greene, John Wiley & Sons, Inc., Hoboken, New Jersey, 2007); beispielsweise kann (VIc, PG = Boc) mit Trifluoressigsäure in Dichlormethan zu Verbindung der Formel (VId) umgesetzt werden. Verbindungen der Formel (VId) können schließlich mit Carbonsäurederivaten der Formel (VIII) 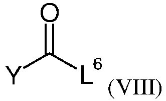 wobei
- L⁶: für Chlor, Hydroxy oder (unter Bildung eines Anhydrids) für Y-C(=O)-O- steht,
in Gegenwart von Basen (L⁶= Cl) oder Kondensationsmitteln (L⁶= OH) zu Verbindungen der Formel (Ib) umgesetzt werden.

Indolcarbonsäuren der Formel (II, L₁=OH) sind neu. Sie können in Analogie zu bekannten Verfahren nach den in Schema 1 bis 4 beschriebenen Methoden erhalten werden.

Indolcarbonsäuren der Formeln (II-1a) und (II-1b) können nach Schema 1 erhalten werden.

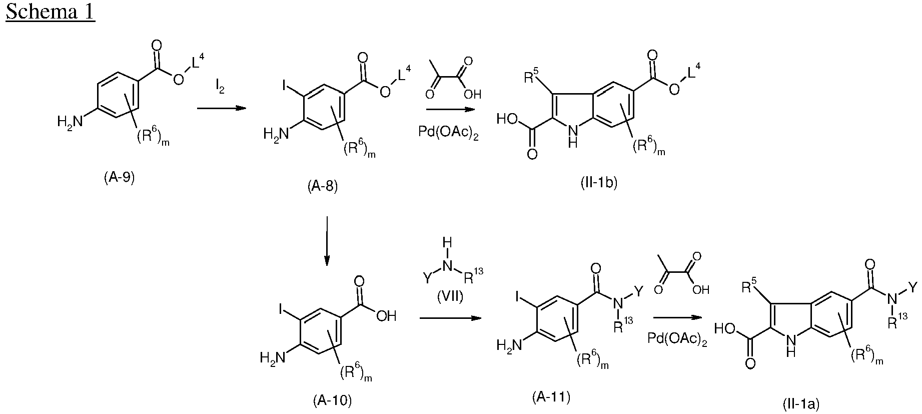

Verbindungen der Formel (II-1b) werden hierbei in Analogie zu bekannten Verfahren aus Verbindungen der Formel (A-8) durch Umsetzung mit Brenztraubensäure in Gegenwart eines Palladiumkatalysators, wie z.B. Palladiumacetat erhalten (vgl. z.B. Bioorganic & Medicinal Chemistry Letters, 20(9), 2010, 2722-2725), wobei Verbindungen (II-1b, R⁵= H) erhalten werden, die gegebenenfalls durch Umsetzung mit einem Halogenierungsreagenz wie z.B. Chlor- oder Bromsuccinimid in Verbindungen (II-1b) mit R⁵= Hal überführt werden können (vgl. z.B. WO-A- 2009/023179). Verbindungen der Formel (A-8) sind bekannt oder können durch Iodierung aus Anilinen der Formel (A-9) nach bekannten Verfahren erhalten werden (vgl. z.B. Bioorganic & Medicinal Chemistry Letters, 20(9), 2010, 2722-2725). Aniline der Formel (A-9) sind kommerziell verfügbar oder können nach bekannten Verfahren erhalten werden. Ester der Formel (A-8) können nach allgemein bekannten Methoden auch zu Carbonsäuren der Formel (A-10) hydrolysiert werden (vgl. Greene's protective groups in organic synthesis, 4th ed., P.G.M. Wuts, T.W. Greene, John Wiley & Sons, Inc., Hoboken, New Jersey, 2007**))** und anschließend, gegebenenfalls über ein intermediär gebildetes Säurechlorid, mit Aminen der Formel (VII) zu Amiden der Formel (A-11) umgesetzt werden (vgl. **z.B.** die unter (A) zur Synthese von Verbindungen der Formel (I) angegebenen Methoden) Verbindungen der Formel (A11) können dann wie oben beschrieben mit Brenztraubensäure zu Indolcarbonsäuren der Formel **(II-**1a**)** umgesetzt werden.

Die Erfindung betrifft auch die Carbonsäuren der allgemeinen Formel (II-1aa) 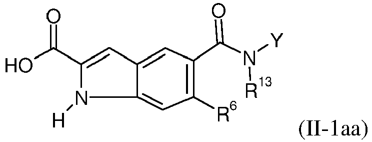 bei denen R6, Y und R13 die oben angegebene Bedeutung haben und die entsprechend Schema 1 hergestellt werden können.

Die Erfindung betrifft ferner auch die Carbonsäuren der allgemeinen Formel (II-1ba) 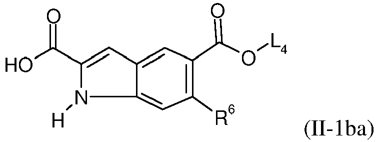 bei denen L⁴ für für C₁-C₄-Alkyl steht und R⁶ die oben angegebene Bedeutung hat, wobei die Verbindung 6-Chlor-5-(ethoxycarbonyl)-1H-indol-2-carbonsäure ausgenommen ist, und die entsprechend Schema 1 hergestellt werden können.

Neue Benzimidazolcarbonsäuren der Formeln (II-2a), (II-2b) und (II-2c) können z.B. nach Schema 2 in Analogie zu bekannten Verfahren erhalten werden.

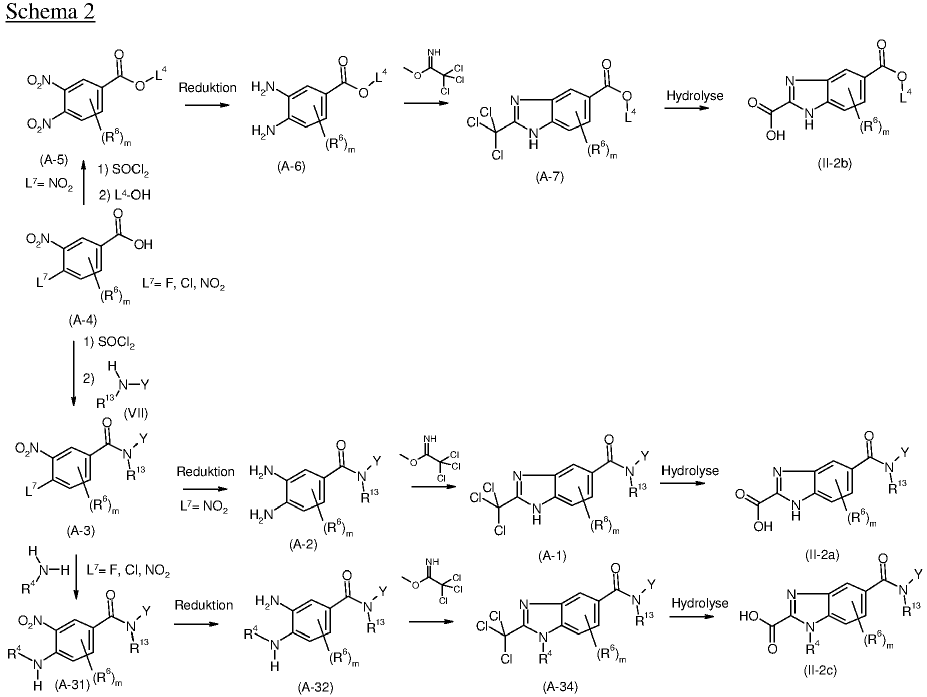

Benzimidazolderivate der Formel (II-2a) werden aus Verbindungen der Formel (A-1) durch Hydrolyse z.B. mit Methanol erhalten (vgl. z.B. Bioorganic & Medicinal Chemistry Letters, 20(2), 2010, 586-590). Auf gleiche Weise können Verbindungen der Formel (II-2c), die am Imidazolstickstoff einen Substituenten R⁴ tragen, aus Verbindungen der Formel (A-34) erhalten werden. Verbindungen der Formel (A-1) können nach bekannten Verfahren durch Umsetzung von 1,2-Diaminophenylderivaten der Formel (A-2) mit 2,2,2-Trichloracetimidat erhalten werden (vgl. z.B. Bioorganic & Medicinal Chemistry Letters, 20(2), 2010, 586-590). Ebenso werden Verbindungen der Formel (A-34) aus Verbindungen der Formel (A-32) erhalten. 1,2-Diaminophenylderivaten der Formel (A-2) sind bekannt oder können nach bekannten Verfahren aus Verbindungen der Formel (A-3, L⁷= NO₂) erhalten werden (vgl. z.B. European Journal of Medicinal Chemistry, 44(5), 2009, 2002-2008). Die Reduktion von Nitro Derivaten der Formel (A-31) nach allgemein bekannten Verfahren ergibt Verbindungen der Formel (A-32). Mono Nitro Derivate der Formel (A-31) können durch Umsetzung von Verbindungen der Formel (A-3, L⁷= NO₂, F, Cl) mit primären Aminen erhalten werden. Amide der Formel (A-3) können nach allgemein bekannten Verfahren durch Umsetzung von Carbonsäuren der Formel (A-4, L⁷= NO₂, Cl, F) mit Aminen der Formel (VII) erhalten werden (vgl. hierzu die Methoden für die Synthese von Verbindungen der Formel (IVa) unter (A) angegebenen Bedingungen). Dinitrocarbonsäuren der Formel (A-4, L⁷= NO₂,) sind bekannt (siehe z.B. WO2009/47558A1), Carbonsäuren der Formel (A-4, L⁷= F, Cl) sind kommerziell verfügbar.

Nach dem oben beschriebenen Verfahren für Verbindungen der Formel (II-2a) können ausgehend von Estern der Formel (A-5) über Verbindungen der Formel (A-6) und (A-7) auch Benzimidazolcarbonsäuren der Formel (II-2b) erhalten werden. Ester der Formel (A-5) können nach allgemein bekannten Verfahren aus Carbonsäuren der Formel (A-4) erhalten werden (vgl. z.B. Organikum, Wiley-VCH, 22. Auflage).

Die Erfindung betrifft auch die Carbonsäuren der allgemeinen Formel (II-2aa) 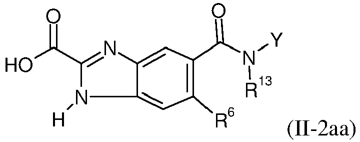 bei denen R⁶, Y und R¹³ die oben angegebene Bedeutung haben und die entsprechend Schema 2 hergestellt werden können.

Die Erfindung betrifft auch die Carbonsäuren der allgemeinen Formel (II-2ba) 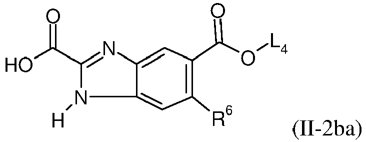 bei denen L⁴ für für C₁-C₄-Alkyl steht und R⁶ die oben angegebene Bedeutung hat und die entsprechend Schema 2 hergestellt werden können.

Neue Indolcarbonsäuren der Formel (II-1c) und **(II-**1d**)** können z.B. nach Schema 3 erhalten werden.

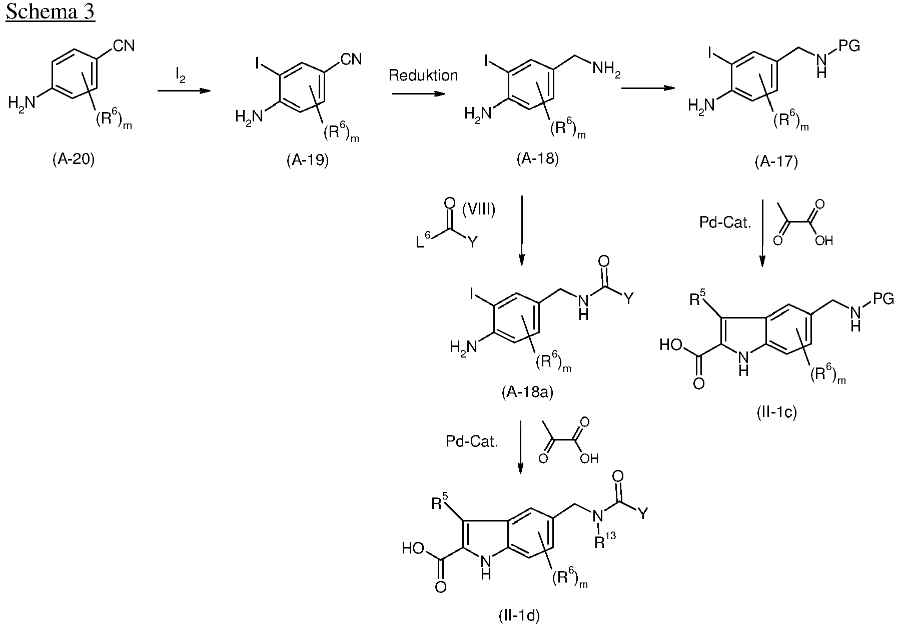

Hierbei werden Verbindungen der Formel (A-17) mit Brenztraubensäure in Gegenwart eines Palladiumkatalysators, wie z.B. Palladiumacetat umgesetzt (vgl. z.B. Bioorganic & Medicinal Chemistry Letters, 20(9), 2010, 2722-2725), wobei Verbindungen (II-1c, R⁵= H) erhalten werden, die gegebenenfalls durch Umsetzung mit einem Halogenierungsreagenz wie z.B. Chlor- oder Bromsuccinimid in Verbindungen (II-1c) mit R5= Hal überführt werden können (vgl. z.B. WO-A-2009/023179). Verbindungen der Formel (A-17) sind bekannt oder können in Analogie zu bekannten Verfahren aus Benzylaminen der Formel (A-18) durch Umsetzung mit Reagenzien, die zur Einführung einer Schutzgruppe (PG) geeignet sind z.B. mit Di-tert.-butyldicarbonat, erhalten werden (vgl. z.B. WO-A-2006/101321). Benzylamine der Formel (A-18) sind bekannt oder können nach allgemein bekannten Verfahren oder in Analogie zu bekannten Verbindungen durch Reduktion von Nitrilen der Formel (A-19) erhalten werden (vgl. z.B. WO-A-2006/101321). Nitrile der Formel (A-19) sind bekannt oder können in Analogie zu bekannten Verbindungen durch Umsetzung von Aminobenzonitrilen der Formel (A-20) mit einem Iodierungsreagenz wie z.B. Iod erhalten werden (vgl. z.B. Journal of Medicinal Chemistry (2001), 44(23), 3856-3871). Aminonitrile der Formel (A-20) sind kommerziell verfügbar oder können nach bekannten Verfahren erhalten werden.

Verbindungen der Formel (II-1d) können erhalten werden, indem man Verbindungen der Formel (A-18) mit Carbonsäurederivaten der Formel (VIII) zunächst zu Amiden der Formel (A-18a) umsetzt und diese anschließend nach dem oben für Verbindungen der Formel (II-1c) beschriebenen Verfahren mit Brenztraubensäure zu Verbindungen der Formel (II-1d, R⁵= H) umsetzt, die gegebenenfalls durch Umsetzung mit einem Halogenierungsreagenz wie z.B. Chlor- oder Bromsuccinimid, in Verbindungen (II-1d) mit R⁵= Hal überführt werden können (vgl. z.B. WO-A- 2009/023179).

Neue Benzimidazolcarbonsäuren der Formel (II-2c) und (II-2d) können z.B. nach Schema 4 erhalten werden.

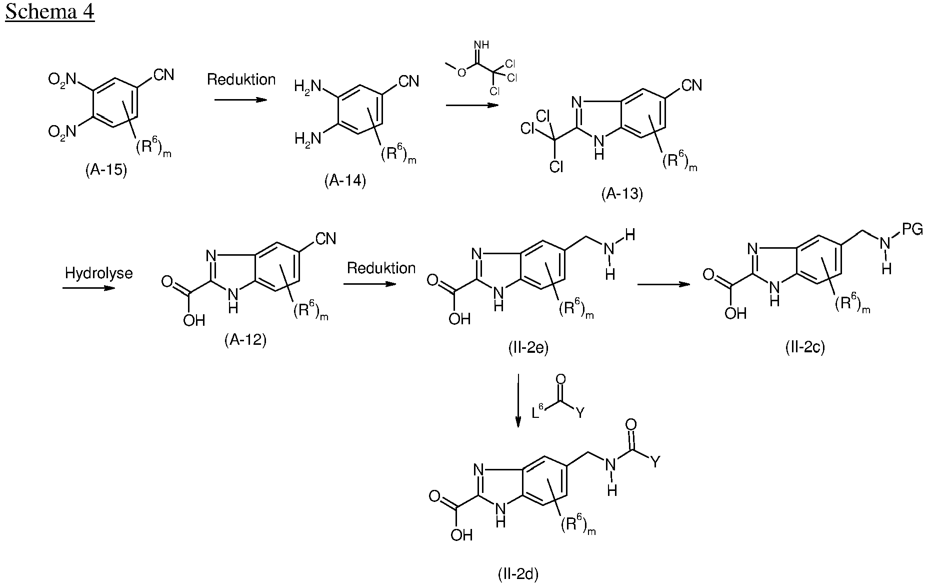

Benzimidazolderivate der Formel (II-2c) werden durch Umsetzung von Benzylaminen der Formel (II-2e) in Analogie zu bekannten Verfahren mit Reagenzien, die zur Einführung einer Schutzgruppe (PG) geeignet sind z.B. mit Di-tert.-butyldicarbonat, erhalten (vgl. Greene's protective groups in organic synthesis, 4th ed., P.G.M. Wuts, T.W. Greene, John Wiley & Sons, Inc., Hoboken, New Jersey, 2007). Alternativ können Benzylamine der Formel (A-11) auch nach allgemein bekannten Verfahren (vgl. z.B. die unter (A) zur Synthese von Verbindungen der Formel (I) angegebenen Methoden) mit Carbonsäurederivaten der Formel (VIII) umgesetzt werden, wobei man Benzimidazole der Formel (II-2d) erhält.
Amine der Formel (II-2e) können nach allgemein bekannten Methoden aus den entsprechenden Nitrilen der Formel (A-12) erhalten werden (vgl. z.B. WO-A-2008/075196). Benzimidazolderivate der Formel (A-12) werden z.B. aus Verbindungen der Formel (A-13) durch Hydrolyse z.B. mit Methanol erhalten (vgl. z.B. Bioorganic & Medicinal Chemistry Letters, 20(2), 2010, 586-590). Verbindungen der Formel (A-13) können nach bekannten Verfahren durch Umsetzung von 1,2-Diaminophenylderivaten der Formel (A-14) mit 2,2,2-Trichloracetimidat erhalten werden (vgl. z.B. Bioorganic & Medicinal Chemistry Letters, 20(2), 2010, 586-590). 1,2-Diaminophenylderivate der Formel (A-14) sind bekannt oder können nach bekannten Verfahren aus Dinitrophenylverbindungen der Formel (A-15) erhalten werden (vgl. z.B. European Journal of Medicinal Chemistry, 44(5), 2009, 2002-2008). Dinitroverbindungen der Formel (A-15) sind bekannt oder können aus den entsprechenden Carbonsäuren der Formel (A-4) nach allgemein bekannten Methoden erhalten werden. (siehe z.B. WO2009/47558A1, US 5591378, Helvetica Chimica Acta (1943), 26, 1125). Carbonsäurehalogenide, besonders bevorzugt Carbonsäurechloride, wie sie ebenfalls durch die allgemeinen Strukturen (II) repräsentiert werden (L¹= Halogen), können durch die Umsetzung einer Carbonsäure (L=OH) mit Halogenierungsreagenzien wie Thionylchlorid, Thionylbromid, Phosphorylchlorid, Oxalylchlorid, Phosphortrichlorid, etc. hergestellt werden. [Houben-Weyl, 1952, Bd. VIII, S.463 ff.].

Halogenalkyl substituierte Amine der allgemeine Formel (III) sind kommerziell erhältlich, literaturbekannt oder können anhand literaturbekannter Verfahren synthetisiert werden. Beispielsweise können Arylhalogenide in Gegenwart von Magnesium in einer Grignard-Reaktion mit Halogenalkylcarbonsäureestern umgesetzt werden. Die so entstandenen Ketone lassen sich dann durch eine reduktive Aminierung in die entsprechenden Amine überführen (vgl. DE-A-2723464).

Neue Halogenalkyl substituierte Amine der allgemeinen Formel (III; R₂= H, R₃=H) können z.B. nach Schema 5 erhalten werden, 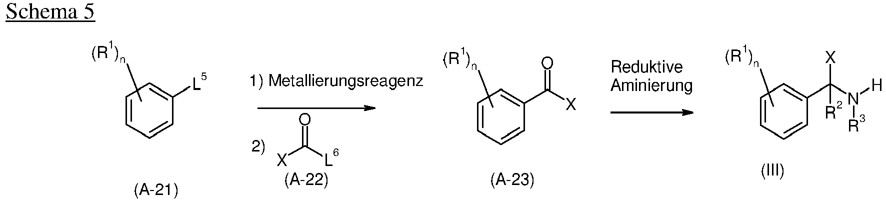 wobei
- L⁶: für -C₁-C₄-Alkoxy oder für -N(CH₃)-O-C₁-C₄-Alkyl steht,
indem käufliche oder literaturbekannte Verbindungen der Formel (A-21) zunächst mit einem Metallierungsreagenz, wie z.B. n-Butyllithium zu einem metallorganischen Intermediat umgesetzt, welches anschließend mit einer Verbindung der Formel (A-22) umgesetzt wird, wobei Ketone der Formel (A-23) erhalten werden (vgl. z.B. Chem. Med.Chem., 4(7), 2009, 1182-1188). Diese können dann in Analogie zu allgemein bekannten Vorschriften durch reduktive Aminierung zu Aminen der Formel (III) umgesetzt werden (vgl. z.B. DE-A-2723464).

Verbindungen der Formeln (A-21), (A-22), (V), (VII), (VIII) sind literaturbekannte Substanzen oder kommerziell erhältlich.

Die erfindungsgemäßen Verfahren zur Herstellung der neuen Verbindungen der Formel (I) werden vorzugsweise unter Verwendung eines Verdünnungsmittels durchgeführt. Als Verdünnungsmittel zur Durchführung der erfindungsgemäßen Verfahren kommen neben Wasser alle inerten Lösungsmittel in Frage. Als Beispiele sind zu nennen:. Halogenkohlenwasserstoffe (z.B. Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, 1,4-Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol); Nitrile (wie z.B. Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, N,N-Dimethyl-formamid, N,N-Dipropyl-formamid, N,N-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-Diformyl-piperazin) und Ketone (z.B. Aceton, Acetophenon, Methylethylketon, Methylbutylketon).

Selbstverständlich kann man das erfindungsgemäße Verfahren auch in Gemischen der genannten Lösungs- und Verdünnungsmittel durchführen.

Die Reaktionstemperaturen können bei der Durchführung des erfindungsgemäßen Verfahrens in einem größeren Bereich variiert werden. Im Allgemeinen arbeitet man bei Temperaturen zwischen -30°C und +150°C, vorzugsweise zwischen -10°C und +100°C.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im Allgemeinen bei absoluten Drücken zwischen 0,1 bar und 15 bar - durchzuführen.

Zur Durchführung des erfindungsgemäßen Verfahrens werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels, gegebenenfalls auch unter einer Schutzgas-Atmosphäre (z.B. unter Stickstoff, Argon oder Helium) durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), N,N,N,N-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrazol, N-Methyl-morpholin, N-Methylhexamethylendiamin, Pyridin, 4-Pyrrolidinopyridin, 4-Dimethylamino-pyridin, Chinolin, α-Picolin, β-Picolin, Pyrimidin, Acridin, N,N,N',N'-Tetramethylendiamin, N,N,N',N'-Tetraethylendiamin, Chinoxalin, N-Propyl-diisopropylamin, N-Ethyl-diisopropylamin, N,N'-Dimethyl-cyclohexylamin, 2,6-Lutidin, 2,4-Lutidin oder Triethylendiamin).

Als saure Reaktionshilfsmittel zur Durchführung der erfindungsgemäßen Verfahren können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)-chlorid, Bortrifluorid oder sein Etherat, Titan(IV)chlorid, Zinn(IV)-chlorid) und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure) eingesetzt werden.

Die folgenden Herstellungs- und Verwendungsbeispiele illustrieren die Erfindung ohne sie zu beschränken.

### Herstellungsbeispiele

In den folgenden Beispielen bedeuten RT Raumtemperatur, d.h. 20°C, und der Begriff 1 eq bedeutet 1 Äquivalent.

### Synthesebeispiel 1

### 6-Chlor-N⁵-(2,2-difluorethyl)-1-ethyl-N²-{2,2,2-trifluor-1-[4-fluor-3-(trifluormethyl)phenyl]ethyl}-1H-indol-2,5-dicarboxamid (Verbindung Nr. 19 in Tabelle 1)

### Stufe 1: Ethyl-4-amino-2-chlor-5-iodbenzoat

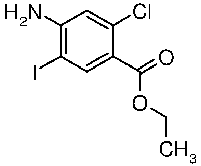

Eine Iodlösung in Ethanol wurde mit Silber(I)sulfat und Ethyl-4-amino-2-chlorbenzoat versetzt und dann für 45 min bei Raumtemperatur gerührt. Das Reaktionsgemisch wurde über einer Fritte filtriert und das Filtrat im Vakuum eingeengt. Der Rückstand wurde in EtOAc aufgeschlämmt und mit verdünnter Natriumhydrogencarbonatlösung versetzt. Nachdem alles in Lösung gegangen ist, wurde die wässrige Phase abgetrennt und Natriumthiosulfat darin gelöst. Die organische Phase wurde erneut mit der wässrigen Phase gewaschen und die wässrige Phase mit Ethylacetat extrahiert. Die vereinigten organischen Phasen wurden über Natriumsulfat getrocknet, filtriert und im Vakuum eingeengt. Säulenchromatographische Reinigung an Kieselgel mit Cyclohexan / Ethylacetat als Laufmittel (Gradient von 10 % Ethylacetat auf 33 % Ethylacetat).ergab 1,85 g (74 % der Theorie) Ethyl-4-amino-2-chlor-5-iodbenzoat
HPLC-MS: logP = 2,95; Masse (m/z): 326,0 (M+H)⁺; ¹H-NMR (CD₃CN) 1.32 (t, 3H), 4.27 (q, 2H), 5.01 (br. s, 2H), 6.80 (s, 1H), 8.16 (s, 1H).
Analog wurden erhalten:
*Methyl-4-amino-2-methyl-5-iodbenzoat*
*aus Methyl-4-amino-2-methylbenzoat*
HPLC-MS: logP = 2,57; Masse (m/z): 292,0 (M+H)⁺; ¹H-NMR (D₆-DMSO) 2.37 (s, 3H), 3.72 (s, 3H), 5.91 (br. s, 2H), 6.57 (s, 1H), 8.08 (s, 1H).
*Ethyl-4-amino-2-ethyl-5-iodbenzoat*
*aus Ethyl-4-amino-2-ethylbenzoat*
HPLC-MS: logP = 3,50; Masse (m/z): 320,0 (M+H)⁺; ¹H-NMR (D₆-DMSO) 1.10 (t, 3 H), 1.27 (t, 3H), 2.79 (q, 2H), 4.19 (q, 2H), 5.89 (br. s, 2H), 6.60 (s, 1H), 8.06 (s, 1H).
*Methyl-4-amino-2-isopropyl-5-iodbenzoat*
*aus Methyl-4-amino-2-isopropylbenzoat*
HPLC-MS: logP = 3,30; Masse (m/z): 320,0 (M+H)⁺; ¹H-NMR (D₆-DMSO) 1.34 (d, 6H), 3.70 (s, 3H), 3.80 (m, 1H), 5.87 (br. s, 2H), 6.78 (s, 1H), 8.01 (s, 1H).

### Stufe 2: 6-Chlor-5-(ethoxycarbonyl)-1H-indol-2-carbonsäure

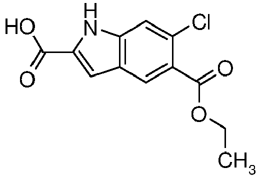

Eine Lösung von Ethyl-4-amino-2-chlor-5-iodbenzoat (1,82 g, 5,59 mmol) in N,N-Dimethylformamid (18 ml) unter Argon wurde mit Brenztraubensäure (1,27 ml, 18,2 mmol) und 1,4-Diazabicyclo[2.2.2]octan versetzt, evakuiert und mit Argon geflutet. Dann wurde für 5 min Argon durch die Lösung geleitet und anschließend Palladium(II)acetat (68 mg, 0,30 mmol) zugegeben und für 2 h auf 100 °C erhitzt. Die abgekühlte Lösung wurde durch Celite filtriert und der Filterkuchen mit Ethylacetat (100ml) gespült. Das Filtrat (Suspension) wurde mit Salzsäure (2 M; 2 x 25 ml) und mit Wasser (2 x 25 ml) gewaschen, über Natriumsulfat getrocknet und filtriert. Das Filtrat wurde im Vakuum bis zur Trockne eingeengt und lieferte einen rotbraunen Feststoff (1,93 g ca. 51 % Produkt), der ohne weitere Reinigung für den nächsten Schritt eingesetzt wurde.
Analog wurden erhalten:
*6-Methyl-5-(methoxycarbonyl)-1H-indol-2-carbonsäure*
aus Methyl-4-amino-2-methyl-5-iodbenzoat
HPLC-MS: logP = 2,57; Masse (m/z): 234,1 (M+H)⁺; ¹H-NMR (D₆-DMSO) 2.37 (s, 3H), 3.2 - 3.4 (br. s, 1H), 3.72 (s, 3H), 7.18 (s, 1H), 7.30 (s, 1H), 8.28 (s, 1H), 11.93 (s, 1H).
*6-Ethyl-5-(ethoxycarbonyl)-1H-indol-2-carbonsäure*
aus Ethyl-4-amino-2-ethyl-5-iodbenzoat,
HPLC-MS: logP = 2,27; Masse (m/z): 262,2 (M+H)⁺; ¹H-NMR (D₆-DMSO) 1.19 (t, 3H), 1.34 (t, 3H), 3.00 (q, 2H) 3.2 - 3.4 (br. s, 1H), 4.29 (q, 2H), 7.18 (s, 1H), 7.31 (s, 1H), 8.22 (s, 1H), 11.91 (s, 1H).
*6-Isopropyl-5-(methoxycarbonyl)-1H-indol-2-carbonsäure*
aus Methyl-4-amino-2-isopropyl-5-iodbenzoat
HPLC-MS: logP = 2,20; Masse (m/z): 262,1 (M+H)⁺; ¹H-NMR (D₆-DMSO) 1.18 (d, 6H), 3.2 - 3.4 (br. s, 1H), 3.80 (m, 1H), 7.14 (s, 1H), 7.42 (s, 1H), 8.12 (s, 1H), 11.85 (s, 1H).

### Stufe 3: Ethyl-6-chlor-2-({2,2,2-trifluor-1-[4-fluor-3-(trifluormethyl)phenyl]ethyl}carbamoyl)-1H-indol-5-carboxylat

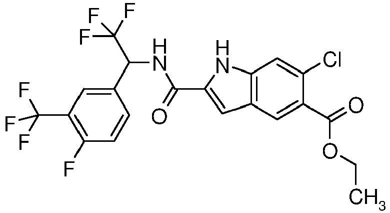

2,2,2-Trifluor-1-[4-fluor-3-(trifluormethyl)phenyl]ethanamin (0,82 g, 3,05 mmol) wurde in N,N-Dimethylformamid (6 mL) gelöst und 6-Chlor-5-(ethoxycarbonyl)-1H-indol-2-carbonsäure (0,82 g, 3,05 mmol), N-[(1H-Benzotriazol-1-yloxy)(dimethylamino)methylen]-N-methylmethanaminium-hexafluorophosphat (1,16 g, 3,05 mmol) und 4-Methylmorpholin (0,92 g, 9,10 mmol) zugegeben. Das Reaktionsgemisch wurde 16 h bei Raumtemperatur gerührt und anschließend Wasser zugegeben. Die wässrige Phase wurde dreimal mit Ethylacetat extrahiert, über Natriumsulfat getrocknet, auf Kieselgel adsorbiert und mit Cyclohexan/Ethylacetat (4/1) chromatographiert. Es wurden 1,29 g (65 % d.Th.) Ethyl-6-chlor-2-({2,2,2-trifluor-1-[4-fluor-3-(trifluormethyl)phenyl]ethyl}carbamoyl)-1H-indol-5-carboxylat erhalten.
HPLC-MS: logP = 4,23; Masse (m/z): 511,0(M+H)+; ¹HNMR (D₆-DMSO): δ 1.34 (t, 3H), 4.33 (q, 2H), 6.37-6.40 (m, 1H), 7.54 (s, 1H), 7.60 (s, 1H), 7.65-7.68 (m, 1H), 8.17-8.19 (m, 1H), 8.27 (s, 1H), 8.30-8.31 (m, 1H), 9.72-9,74 (m, 1H), 12.16 (s, 1H).

### Analog wurden erhalten:

### Ethyl-6-chlor-2-({2,2,2-trifluor-1-[3-chlorphenyl]ethyl}carbamoyl)-1H-indol-5-carboxylat

HPLC-MS: logP = 4,01; Masse (m/z): 458,9 (M+H)⁺; ¹HNMR (D₆-DMSO): δ 1.35 (t, 3H), 4.33 (q, 2H), 6.16-6.25 (m, 1H), 7.50-7.55 (m, 3H), 7.62 (s, 1H), 7.71-7.72 (m, 1H), 7.90 (s, 1H), 8.26 (s, 1H), 9.65 (d, 1H), 12.13 (s, 1H).

### Ethyl-6-chlor--2-({2,2,2-trifluor-1-[3-bromphenyl]ethyl}carbamoyl)-1H-indol-5-carboxylat

HPLC-MS: logP = 4,09; Masse (m/z): 502,9 (M+H)⁺; ¹HNMR (D₆-DMSO): δ 1.35 (t, 3H), 4.33 (q, 2H), 6.16-6.24 (m, 1H), 7.42-7.76 (m, 5H), 8.03 (s, 1H), 8.26 (s, 1H), 9.65 (d, 1H), 12.13 (s, 1H).

### Ethyl-6-chlor-2-({2,2,2-trifluor-1-[3,5-dichlorphenyl]ethyl}carbamoyl)-1H-indol-5-carboxylat

HPLC-MS: logP = 4,54; Masse (m/z): 493,0 (M+H)⁺; ¹HNMR (CD₃CN): δ 1.38 (t, 3H), 4.36 (q, 2H), 6.03-6.06 (m, 1H), 7.35 (s, 1H), 7.55 (s, 1H), 7.59 (s, 1H), 7.62 (s, 2H), 8.00 (s, 1H), 8.26 (s, 1H), 10.22 (s, 1H).

### Ethyl-6-chlor-2-({2,2,2-trifluor-1-[3-chlor-4-fluorphenyl]ethyl}carbamoyl)-1H-indol-5-carboxylat

HPLC-MS: logP = 4,08; Masse (m/z): 477,0 (M+H)⁺; ¹HNMR (D₆-DMSO): δ 1.35 (t, 3H), 4.34 (q, 2H), 6.21-6.30 (m, 1H), 7.53-7.62 (m, 3H), 7.77-7.83 (m, 1H), 8.09 (d, 1H), 8.27 (s, 1H), 9.63 (d, 1H), 12.15 (s, 1H).

### Ethyl-6-chlor-2-({2,2,2-trifluor-1-[3-brom-4-fluorphenyl]ethyl}carbamoyl)-1H-indol-5-carboxylat

HPLC-MS: logP = 4,13; Masse (m/z): 521,0 (M+H)⁺; ¹HNMR (D₆-DMSO): δ 1.35 (t, 3H), 4.33 (q, 2H), 6.21-6.28 (m, 1H), 7.51-7.61 (m, 3H), 7.82-7.85 (m, 1H), 8.19-8.21 (m, 1H), 8.26 (s, 1H), 9.62 (d, 1H), 12.14 (s, 1H).

### Ethyl-6-chlor-2-({2,2,2-trifluor-1-[3,4-dichlorphenyl]ethyl}carbamoyl)-1H-indol-5-carboxylat

HPLC-MS: logP = 4,43; Masse (m/z): 493,1 (M+H)⁺.

### Ethyl-6-chlor-2-({2,2,2-trifluor-1-[3,5-dichlor-4-fluorphenyl]ethyl}carbamoyl)-1H-indol-5-carboxylat

HPLC-MS: logP = 4,52; Masse (m/z): 511,0 (M+H)⁺; ¹HNMR (D₆-DMSO): δ 1.35 (t, 3H), 4.34 (q, 2H), 6.29-6.33 (m, 1H), 7.55 (s, 1H), 7.59 (s, 1H), 8.11-8.13 (m, 2H), 8.26 (s, 1H), 9.60 (d, 1H), 12.15 (s, 1H).

### Ethyl-6-chlor-2-({2,2,2-trifluor-1-[3,5-dichlor-2,4-difluorphenyl]ethyl}carbamoyl)-1H-indol-5-carboxylat

HPLC-MS: logP = 4,68; Masse (m/z): 528,9 (M+H)⁺; ¹HNMR (D₆-DMSO): δ 1.34 (t, 3H), 4.34 (q, 2H), 6.35-6.40 (m, 1H), 7.55 (s, 1H), 7.58 (s, 1H), 8.24-8.27 (m, 2H), 9.76 (d, 1H), 12.19 (s, 1H).

### Ethyl-6-chlor-2-({2,2,2-trifluor-1-[3,4-difluorphenyl]ethyl}carbamoyl)-1H-indol-5-carboxylat

HPLC-MS: logP = 3,80; Masse (m/z): 461,0 (M+H)⁺; ¹HNMR (D₆-DMSO): δ 1.34 (t, 3H), 4.34 (q, 2H), 6.20-6.26 (m, 1H), 7.54-7.65 (m, 4H), 7.91-7.94 (m, 1H), 8.26 (s, 1H), 9.61 (d, 1H), 12.15 (s, 1H).

### Ethyl-6-chlor-2-({2,2,2-trifluor-1-[3,4,5-trichlorphenyl]ethyl}carbamoyl)-1H-indol-5-carboxylat

HPLC-MS: logP = 4,85; Masse (m/z): 526,9 (M+H)⁺; ¹HNMR (D₆-DMSO): δ 1.34 (t, 3H), 4.33 (q, 2H), 6.29-6.38 (m, 1H), 7.54 (s, 1H), 7.59 (s, 1H), 8.16 (s, 2H), 8.26 (s, 1H), 9.63 (d, 1H), 12.15 (s, 1H).

### Methyl-6-methyl-2-({2,2,2-trifluor-1-[4-fluor-3-(trifluormethyl)phenyl]ethyl}carbamoyl)-1H-indol-5-carboxylat

HPLC-MS: logP = 4,01; Masse (m/z): 477,0(M+H)+; 1HNMR (D₆-DMSO): δ 1.99 (s, 3H), 3.83 (s, 3H), 6.40-6.44 (m, 1H), 7.31 (s, 1H), 7.54 (s, 1H), 7.80-7.83 (m, 1H), 8.06-8.08 (m, 1H), 8.14 (s, 1H), 8.31 (s, 1H), 9.59-9.62 (m, 1H), 11.93 (s, 1H).

### Methyl-6-methyl-2-({2,2,2-trifluor-1-[3-(trifluormethyl)phenyl]ethyl}carbamoyl)-1H-indol-5-carboxylat

HPLC-MS: logP = 3,85; Masse (m/z): 459,10 (M+H)+; 1HNMR (D₆-DMSO): δ 1.99 (s, 3H), 3.83 (s, 3H), 6.30-6.35 (m, 1H), 7.31 (s, 1H), 7.55 (s, 1H), 7.61-7.63 (m, 1H), 7.70-7.74 (m, 1H), 8.06-8.08 (m, 1H), 8.21 (s, 1H), 8.30 (s, 1H), 9.60-9.63 (m, 1H), 11.91 (s, 1H).

### Methyl-6-methyl-2-({2,2,2-trifluor-1-[3-chlor-4-fluorphenyl]ethyl}carbamoyl)-1H-indol-5-carboxylat

HPLC-MS: logP = 3,81; Masse (m/z): 443,0(M+H)+; 1HNMR (D₆-DMSO): δ 1.99 (s, 3H), 3.82 (s, 3H), 6.21-6.25 (m, 1H), 7.31 (s, 1H), 7.52-7.56 (m, 2H), 7.78-7.82 (m, 1H), 8.07-8.09 (m, 1H), 8.29 (s, 1H), 9.59-9.62 (m, 1H), 11.93 (s, 1H).

### Methyl-6-methyl-2-({2,2,2-trifluor-1-[3-brom-4-fluorphenyl]ethyl}carbamoyl)-1H-indol-5-carboxylat

HPLC-MS: logP = 3,86; Masse (m/z): 487,0(M+H)+.

### Methyl-6-methyl-2-({2,2,2-trifluor-1-[3-chlorphenyl]ethyl}carbamoyl)-1H-indol-5-carboxylat

HPLC-MS: logP = 3,70; Masse (m/z): 425,0(M+H)+.

### Methyl-6-methyl-2-({2,2,2-trifluor-1-[3,5-dichlorphenyl]ethyl}carbamoyl)-1H-indol-5-carboxylat

HPLC-MS: logP = 4,24; Masse (m/z): 459,0(M+H)+.

### Methyl-6-methyl-2-({2,2,2-trifluor-1-[3-chlor-5-fluorphenyl]ethyl}carbamoyl)-1H-indol-5-carboxylat

HPLC-MS: logP = 3,83; Masse (m/z): 443,0(M+H)+.

### Methyl-6-methyl-2-({2,2,2-trifluor-1-[3,4-dichlorphenyl]ethyl}carbamoyl)-1H-indol-5-carboxylat

HPLC-MS: logP = 4,14; Masse (m/z): 459,0(M+H)+.

### Methyl-6-methyl-2-({2,2,2-trifluor-1-[3,5-dichlor-4-fluorphenyl]ethyl}carbamoyl)-1H-indol-5-carboxylat

HPLC-MS: logP = 4,31; Masse (m/z): 477,0(M+H)+; 1HNMR (D₆-DMSO): δ 1.99 (s, 3H), 3.83 (s, 3H), 6.28-6.32 (m, 1H), 7.31 (s, 1H), 7.53 (s, 2H), 8.11 (d, 1H), 8.29 (s, 1H), 9.47-9.49 (m, 1H), 11.92 (s, 1H).

### Methyl -6-methyl-2-({2,2,2-trifluor-1-[3,4-difluorphenyl]ethyl}carbamoyl)-1H-indol-5-carboxylat

HPLC-MS: logP = 3,47; Masse (m/z): 427,0(M+H)+.

### Methyl -6-methyl-2-({2,2,2-trifluor-1-[3,4,5-trichlorphenyl]ethyl}carbamoyl)-1H-indol-5-carboxylat

HPLC-MS: logP = 4,57; Masse (m/z): 493,0(M+H)+; 1HNMR (D₆-DMSO): δ 1.99 (s, 3H), 3.83 (s, 3H), 6.30-6.34 (m, 1H), 7.32 (s, 1H), 7.53 (s, 2H), 8.16 (s, 1H), 8.30 (s, 1H), 9.59-9.62 (m, 1H), 11.93 (s, 1H).

### Methyl -6-methyl-2-({2,2,2-trifluor-1-[2,2-difluor-1,3-benzodioxol-5-yl]ethyl}carbamoyl)-1H-indol-5-carboxylat

HPLC-MS: logP = 3,89; Masse (m/z): 471,0(M+H)+; 1HNMR (D₆-DMSO): δ 1.99 (s, 3H), 3.34 (s, 3H), 6.20-6.24 (m, 1H), 7.31 (s, 1H), 7.52-7.56 (m, 2H), 7.78-7.82 (m, 1H), 8.07-8.09 (m, 1H), 8.29 (s, 1H), 9.59-9.62 (m, 1H), 11.92 (s, 1H).

### Ethyl-6-ethyl-2-({2,2,2-trifluor-1-[3-(trifluor-methyl)phenyl]ethyl}carbamoyl)-1H-indol-5-carboxylat

HPLC-MS: logP = 4,37; Masse (m/z): 487,0(M+H)+; 1HNMR (D₆-DMSO): δ 1.18 (t, 3H), 1.34 (t, 3H), 3.01 (q, 2H), 4.31 (q, 2H), 6.30-6.35 (m, 1H), 7.32 (s, 1H), 7.54 (s, 1H), 7.70-7.74 (m, 1H), 7.81-7.94 (m, 1H), 8.06-8.09 (m, 1H), 8.21 (s, 1H), 9.60-9.68 (m, 1H), 11.92 (s, 1H).

### Methyl -6-isopropyl-2-({2,2,2-trifluor-1-[3-(trifluormethyl)phenyl]ethyl}carbamoyl)-1H-indol-5-carboxylat

HPLC-MS: logP = 4,32; Masse (m/z): 487,1 (M+H)+; 1HNMR (CD3CN): δ 1.26 (d, 6H), 3.78-3.83 (m, 1H), 3.86 (s, 3H), 6.13-6.18 (m, 1H), 7.32 (s, 1H), 7.50 (s, 1H), 7.64-7.68 (m, 1H), 7.76-7.78 (m, 1H), 7.88-7.90 (m, 1H), 7.98 (s, 1H), 8.08-8.11 (s, 1H), 8.16 (s, 1H), 10.081 (s, 1H).

### Stufe 4: Ethyl-6-chlor-1-ethyl-2-({2,2,2-trifluor-1-[4-fluor-3-(trifluormethyl)phenyl]ethyl}carbamoyl)-1H-indol-5-carboxylat

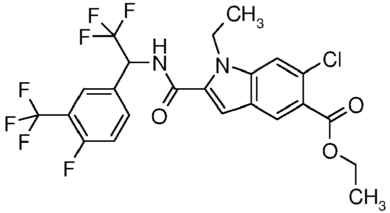

Ethyl-6-chlor-2-({2,2,2-trifluor-1-[4-fluor-3-(trifluormethyl)phenyl]ethyl}carbamoyl)-1H-indol-5-carboxylat (0,42 g, 0,82 mmol) wurde unter Argon bei 0 °C in N,N-Dimethylformamid (6 mL) gelöst. Es wurde Natriumhydrid (60%ig; 0,028 g, 0,72 mmol) zugegeben und 2 h unter Eiskühlung nachgerührt. Iodethan (0,10 g, 0,65 mmol) wurde zugegeben. Das Reaktionsgemisch wurde innerhalb von 36 h unter Rühren aufgetaut. Es wurden Wasser und Ethylacetat zugegeben und die Phasen getrennt. Die organische Phase wurde mit gesättigter Natriumchloridlösung gewaschen, über Magnesiumsulfat getrocknet und das Lösemittel im Vakuum entfernt. Der Rückstand wurde mit Cyclohexan/Ethylacetat (4/1) chromatographiert und ergab 0,23 g (53 % d.Th.) Ethyl-6-chlor-1-ethyl-2-({2,2,2-trifluor-1-[4-fluor-3-(trifluormethyl)phenyl]ethyl}carbamoyl)-1H-indol-5-carboxylat.
HPLC-MS: logP = 5,16; Masse (m/z): 539,0 (M+H)⁺; ¹HNMR (CD₃CN): δ 1.32 (t, 3H), 1.41 (t, 3H), 4.39 (q, 2H), 4.51 (q, 2H), 6.13-6.18 (m, 1H), 7.30 (s, 1H), 7.44-7.49 (m, 1H), 7.69 (s, 1H), 7.93-7.97 (m, 1H), 8.01-8.02 (m, 1H), 8.18-8.20 (m, 1H), 8.26 (s, 1H).

### Analog wurden erhalten:

*Ethyl-6-chlor-1-ethyl-2-({2,2,2-trifluor-1-[3-chlorphenyl]ethyl}carbamoyl)-1H-indol-5-carboxylat*

HPLC-MS: logP = 5,02; Masse (m/z): 487,1 (M+H)⁺; ¹HNMR (D₆-DMSO): δ 1.23 (t, 3H), 1.35 (t, 3H), 4.34 (q, 2H), 4.52 (q, 2H), 6.12-6.21 (m, 1H), 7.43 (s, 1H), 7.50-7.54 (m, 2H), 7.70-7.71 (m, 1H), 7.88-7.90 (m, 2H), 8.25 (s, 1H), 9.79 (d, 1H).

### Ethyl-6-chlor-1-ethyl-2-({2,2,2-trifluor-1-[3-bromphenyl]ethyl}carbamoyl)-1H-indol-5-carboxylat

HPLC-MS: logP = 5,15; Masse (m/z): 531,1 (M+H)⁺; ¹HNMR (D₆-DMSO): δ 1.23 (t, 3H), 1.35 (t, 3H), 4.33 (q, 2H), 4.52 (q, 2H), 6.11-6.20 (m, 1H), 7.42-7.46 (m, 2H), 7.64-7.76 (m, 2H), 8.03 (s, 1H), 8.25 (s, 1H), 9.78 (d, 1H).

### Ethyl-6-chlor-1-ethyl-2-({2,2,2-trifluor-1-[3,5-dichlorphenyl]ethyl}carbamoyl)-1H-indol-5-carboxylat

HPLC-MS: logP= 5,68; Masse (m/z): 521,1 (M+H)⁺; ¹HNMR (D₆-DMSO): δ 1.24 (t, 3H), 1.35 (t, 3H), 4.34 (q, 2H), 4.52 (q, 2H), 6.22-6.31 (m, 1H), 7.44 (s, 1H), 7.73 (s, 1H), 7.88 (s, 1H), 7.92 (s, 2H), 8.26 (s, 1H), 9.75 (d, 1H).

### Ethyl-6-chlor-1-ethyl-2-({2,2,2-trifluor-1-[3-chlor-4-fluorphenyl]ethyl}carbamoyl)-1H-indol-5-carboxylat

HPLC-MS: logP = 4,99; Masse (m/z): 505,1 (M+H)⁺; ¹HNMR (CD₃CN): δ 1.33 (t, 3H), 1.41 (t, 3H), 4.39 (q, 2H), 4.51 (q, 2H), 6.01-6.10 (m, 1H), 7.29 (s, 1H), 7.35-7.39 (m, 1H), 7.59-7.63 (m, 1H), 7.69 (s, 1H), 7.79-7.81 (m, 1H), 8.07-8.10 (m, 1H), 8.26 (s, 1H).

### Ethyl-1-ethyl-6-chlor-2-({2,2,2-trifluor-1-[3-brom-4-fluorphenyl]ethyl}carbamoyl)-1H-indol-5-carboxylat

HPLC-MS: logP= 5,13; Masse (m/z): 549,1 (M+H)⁺; ¹HNMR (D₆-DMSO): δ 1.23 (t, 3H), 1.34 (t, 3H), 4.33 (q, 2H), 4.51 (q, 2H), 6.16-6.25 (m, 1H), 7.42 (s, 1H), 7.48-7.52 (m, 1H), 7.80-7.84 (m, 1H), 7.88 (s, 1H), 8.18-8.20 (m, 1H), 8.25 (s, 1H), 9.76 (d, 1H).

### Ethyl-6-chlor-1-ethyl-2-({2,2,2-trifluor-1-[3,4-dichlorphenyl]ethyl}carbamoyl)-1H-indol-5-carboxylat

HPLC-MS: logP = 5,49; Masse (m/z): 520,9 (M+H)⁺; ¹HNMR (D₆-DMSO): δ 1.23 (t, 3H), 1.34 (t, 3H), 4.33 (q, 2H), 4.51 (q, 2H), 6.18-6.27 (m, 1H), 7.43 (s, 1H), 7.76 (s, 2H), 7.88 (s, 1H), 8.11 (s, 1H), 8.25 (s, 1H), 9.78 (d, 1H).

### Ethyl-6-chlor-1-ethyl-2-({2,2,2-trifluor-1-[3,5-dichlor-4-fluorphenyl]ethyl}carbamoyl)-1H-indol-5-carboxylat

HPLC-MS: logP = 5,47; Masse (m/z): 539,0 (M+H)⁺; ¹HNMR (D₆-DMSO): δ 1.24 (t, 3H), 1.34 (t, 3H), 4.34 (q, 2H), 4.52 (q, 2H), 6.23-6.32 (m, 1H), 7.43 (s, 1H), 7.89 (s, 1H), 8.10-8.12 (m, 2H), 8.26 (s, 1H), 9.72 (d, 1H).

### Ethyl-6-chlor-1-ethyl-2-({2,2,2-trifluor-1-[3,5-dichlor-2,4-difluorphenyl]ethyl}carbamoyl)-1H-indol-5-carboxylat

HPLC-MS: logP = 4,93; Masse (m/z): 557,0 (M+H)⁺; ¹HNMR (D₆-DMSO): δ 1.24 (t, 3H), 1.35 (t, 3H), 4.34 (q, 2H), 4.52 (q, 2H), 6.30-6.39 (m, 1H), 7.46 (s, 1H), 7.89 (s, 1H), 8.25-8.28 (m, 2H), 9.87 (d, 1H).

### Ethyl-6-chlor-1-ethyl-2-({2,2,2-trifluor-1-[3,4-difluorphenyl]ethyl}carbamoyl)-1H-indol-5-carboxylat

HPLC-MS: logP = 4,79; Masse (m/z): 489,1 (M+H)⁺; ¹HNMR (D₆-DMSO): δ 1.23 (t, 3H), 1.35 (t, 3H), 4.34 (q, 2H), 4.52 (q, 2H), 6.15-6.24 (m, 1H), 7.42 (s, 1H), 7.55-7.65 (m, 2H), 7.88-7.95 (m, 2H), 8.25 (s, 1H), 9.75 (d, 1H).

### Ethyl-6-chlor-1-ethyl-2-({2,2,2-trifluor-1-[3,4,5-trichlorphenyl]ethyl}carbamoyl)-1H-indol-5-carboxylat

HPLC-MS: logP = 5,91; Masse (m/z): 554,9 (M+H)⁺; ¹HNMR (D₆-DMSO): δ 1.24 (t, 3H), 1.34 (t, 3H), 4.33 (q, 2H), 4.51 (q, 2H), 6.25-6.34 (m, 1H), 7.43 (s, 1H), 7.89 (s, 1H), 8.15 (s, 2H), 8.26 (s, 1H), 9.74 (d, 1H).

### Ethyl-6-methyl-1-ethyl-2-({2,2,2-trifluor-1-[3-(trifluomethyl)phenyl]ethyl}carbamoyl)-1H-indol-5-carboxylat

HPLC-MS: logP = 4,88; Masse (m/z): 487,1(M+H)⁺.

### Ethyl-6-methyl-1-ethyl-2-({2,2,2-trifluor-1-[3,4-dichlorphenyl]ethyl}carbamoyl)-1H-indol-5-carboxylat

HPLC-MS: logP = 4,13; Masse (m/z): 487,0(M+H)⁺.

### Ethyl-6-methyl-1-ethyl-2-({2,2,2-trifluor-1-[3,4-difluorphenyl]ethyl}carbamoyl)-1H-indol-5-carboxylat

HPLC-MS: logP = 4,44; Masse (m/z): 455,1(M+H)⁺.

### Ethyl-6-methyl-1-ethyl-2-({2,2,2-trifluor-1-[3,4,5-trichlorphenyl]ethyl}carbamoyl)-1H-indol-5-carboxylat

HPLC-MS: logP = 5,84; Masse (m/z): 521,1(M+H)⁺.

### Methyl-1-ethyl-6-methyl-2-({2,2,2-trifluor-1-[4-fluor-3-(trifluormethyl)phenyl]ethyl}carbamoyl)-1H-indol-5-carboxylat

HPLC-MS: logP = 4,89; Masse (m/z): 505,0(M+H)⁺.

### Methyl-1-ethyl-6-methyl-2-({2,2,2-trifluor-1-[3-chlorphenyl]ethyl}carbamoyl)-1H-indol-5-carboxylat

HPLC-MS: logP = 4,78; Masse (m/z): 453,1(M+H)⁺.

### Methyl-1-ethyl-6-methyl-2-({2,2,2-trifluor-1-[3,5-dichlorphenyl]ethyl}carbamoyl)-1H-indol-5-carboxylat

HPLC-MS: logP = 5,42; Masse (m/z): 487,1(M+H)⁺.

### Methyl-1-ethyl-6-methyl-2-({2,2,2-trifluor-1-[3-chlor-5-fluorphenyl]ethyl)carbamoyl)-1H-indol-5-carboxylat

HPLC-MS: logP = 4,89; Masse (m/z): 471,1(M+H)⁺.

### Methyl-1-ethyl-6-methyl-2-({2,2,2-trifluor-1-[3,5-dichlor-4-fluorphenyl]ethyl}carbamoyl)-1H-indol-5-carboxylat

HPLC-MS: logP = 4,89; Masse (m/z): 505,0(M+H)⁺.

### Methyl -1-ethyl-6-methyl-2-({2,2,2-trifluor-1-[3-Chlor-4-fluorphenyl]ethyl}carbamoyl)-1H-indol-5-carboxylat

HPLC-MS: logP = 4,81; Masse (m/z): 471,1(M+H)⁺.

### Methyl-1-ethyl-6-methyl-2-({2,2,2-trifluor-1-[3-Brom-4-fluorphenyl]ethyl}carbamoyl)-1H-indol-5-carboxylat

HPLC-MS: logP = 4,87; Masse (m/z): 515,1(M+H)⁺.

### Methyl -1-ethyl-6-methyl-2-({2,2,2-trifluor-1-[2,2-difluor-1,3-benzodioxol-5-yl]ethyl}carbamoyl)-1H-indol-5-carboxylat

HPLC-MS: logP = 4,90; Masse (m/z): 499,0(M+H)⁺.

### Ethyl-1,6-diethyl-2-({2,2,2-trifluor-1-[3-(trifluormethyl)phenyl]ethyl}carbamoyl)-1H-indol-5-carboxylat

HPLC-MS: logP = 5,45; Masse (m/z): 515,1(M+H)⁺.

### Methyl -1-ethyl-6-isopropyl-2-({2,2,2-trifluor-1-[3-(trifluormethyl)phenyl]ethyl}carbamoyl)-1H-indol-5-carboxylat

HPLC-MS: logP = 5,28; Masse (m/z): 515,0(M+H)⁺.

### Stufe 5: 6-Chlor-N⁵-(2,2-difluorethyl)-1-ethyl-N²-{2,2,2-trifluor-1-[4-fluor-3-(trifluormethyl)phenyl]ethyl}-1H-indol-2,5-dicarboxamid

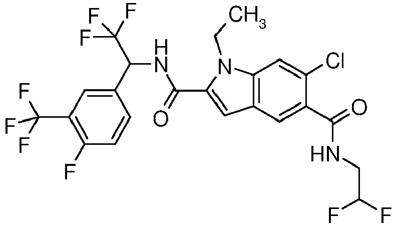

2,2-Difluorethanamin (0,12 g, 1,42 mmol) wurde unter Argon in Dichlormethan (2 mL) gelöst. Bei Raumtemperatur wurde eine Lösung von Trimethylaluminium in Dichlormethan (0,71 mL, 1,42 mmol) zugetropft. Das Reaktionsgemisch wurde 30 min bei Raumtemperatur gerührt und anschließend eine Lösung von Ethyl-6-chlor-1-ethyl-2-({2,2,2-trifluor-1-[4-fluor-3-(trifluormethyl)phenyl]ethyl}carbamoyl)-1H-indol-5-carboxylat (0,078 g, 0,14 mmol) in Dichlormethan (2 mL) zugetropft. Das Reaktionsgemisch wurde 16 h unter Rückfluss erhitzt und nach Abkühlen Wasser zugegeben. Die wässrige Phase wurde dreimal mit Ethylacetat extrahiert. Die vereinigten organischen Phasen wurden über Natriumsulfat getrocknet und das Lösemittel im Vakuum entfernt. Der Rückstand wurde mit Cyclohexan/Ethylacetat (4/1) chromatographiert und ergab 0,065 g (81 % d.Th.) 6-Chlor-N⁵-(2,2-difluorethyl)-1-ethyl-N²-{2,2,2-trifluor-1-[4-fluor-3-(trifluormethyl)phenyl]ethyl}-1H-indol-2,5-dicarboxamid.
HPLC-MS: logP = 5,16; Masse (m/z): 574,0(M+H)⁺; ¹HNMR (CD₃CN): δ 1.32 (t, 3H), 3.74-3.84 (m, 2H), 4.51 (q, 2H), 5.93-6.23 (m, 2H), 7.15 (bs, 1H), 7.25 (s, 1H), 7.44-7.48 (m, 1H), 7.65 (s, 1H), 7.86 (s, 1H), 7.94-7.97 (m, 1H), 8.01-8.02 (m, 1H), 8.16-8.19 (m, 1H).

### Synthesebeispiel 2

### 6-Chlor-N⁵-(1-cyancyclopropyl)-1-ethyl-N²-{2,2,2-trifluor-1-[3-(trifluormethyl)phenyl]ethyl}-1H-indol-2,5-dicarboxamid (Verbindung Nr. 7 in Tabelle 1)

### Stufe 1: Ethyl-6-chlor-2-({2,2,2-trifluor-1-[3-(trifluormethyl)phenyl]ethyl}carbamoyl)-1H-indol-5-carboxylat

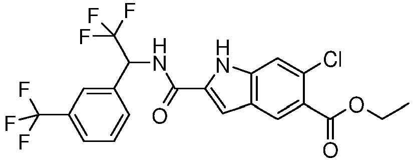

Eine Lösung des Rohprodukts von 6-Chlor-5-(ethoxycarbonyl)-1H-indol-2-carbonsäure (1,9 g) aus Synthesebeispiel 1, Stufe 2 und 2,2,2-Trifluor-1-[3-trifluormethylphenyl]ethanamin (1,12 g, 4,60 mmol) (Lit. DE 2723464) in N,N-Dimethylformamid (15 ml) wurde mit 4-(4,6-Dimethoxy[1.3.5]triazin-2-yl)-4-methylmorhpoliniumchloridhydrat (953 mg, 4,60 mmol) versetzt und 4 Tage bei Raumtemperatur gerührt. Die Reaktionslösung wurde mit Salzsäure (1 M) versetzt und mit Ethylacetat extrahiert. Die organische Phase wurde mit ges. Natriumhydrogencarbonatlösung und gesättigter Natriumchloridlösung gewaschen, über Natriumsulfat getrocknet, filtriert und im Vakuum zur Trockne eingeengt. Säulenchromatographische Reinigung an Kieselgel mit Cyclohexan / Ethylacetat als Laufmittel (Gradient von 10 % Ethylacetat auf 25 % Ethylacetat) ergab 603 mg (26 % der Theorie über 2 Stufen) Ethyl-6-chlor-2-({2,2,2-trifluor-1-[3-(trifluormethyl)phenyl]ethyl}carbamoyl)-1H-indol-5-carboxylat.
HPLC-MS: logP = 4,12; Masse (m/z): 493,1 (M+H)⁺; ¹H-NMR (CD₃CN) 1.38 (t, 3H), 4.35 (q, 2H), 6.16 (quint, 1H), 7.36 (s, 1H), 7.58 (s, 1H), 7.65 - 7.69 (m, 1H), 7.76 - 7.79 (m, 1H), 7.87 - 7.89 (m, 1H), 7.98 (s, 1H), 8.07 - 8.09 (m, 1H), 8.25 (s, 1H), 10.22 (s, 1H).

### Stufe 2: Ethyl-6-chlor-1-ethyl-2-({2,2,2-trifluor-1-[3-(trifluormethyl)phenyl]ethyl}carbamoyl)-1H-indol-5-carboxylat

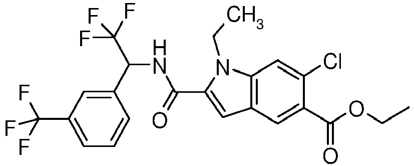

Ethyl-6-chlor-2-({2,2,2-trifluor-1-[3-(trifluormethyl)phenyl]ethyl}carbamoyl)-1H-indol-5-carboxylat (1,00 g, 2,03 mmol) wurde unter Argon bei 0 °C in N,N-Dimethylformamid (20 mL) gelöst. Es wurde Natriumhydrid (60 %ig; 0,079 g, 1,97 mmol) zugegeben und 2 h unter Eiskühlung nachgerührt. Anschließend wurde Iodethan (0,15 mL, 1,93 mmol) zugetropft. Das Reaktionsgemisch wurde innerhalb von 36 h unter Rühren aufgetaut. Es wurden Wasser und Ethylacetat zugegeben und die Phasen getrennt. Die organische Phase wurde mit gesättigter Natriumchloridlösung gewaschen, über Magnesiumsulfat getrocknet und das Lösemittel im Vakuum entfernt. Der Rückstand wurde mit Cyclohexan/Ethylacetat (4/1) chromatographiert und ergab 0,65 g (64 % d.Th.) Ethyl-6-chlor-1-ethyl-2-({2,2,2-trifluor-1-[3-(trifluormethyl)phenyl]ethyl}carbamoyl)-1H-indol-5-carboxylat.
HPLC-MS: logP = 5,00; Masse (m/z): 521,1(M+H)⁺; ¹HNMR (CD₃CN): δ 1.29 (t, 3H), 1.38 (t, 3H), 4.36 (q, 2H), 4.48 (q, 2H), 6.12-6.15 (m, 1H), 7.27 (s, 1H), 7.66-7.69 (m, 2H), 7.77-7.79 (m, 1H), 7.88-7.89 (m, 1H), 7.97 (s, 1H), 8.08-8.10 (m, 1H), 8.24 (s, 1H).

### Stufe 3: 6-Chlor-1-ethyl-2-({2,2,2-trifluor-1-[3-(trifluormethyl)phenyl]ethyl}carbamoyl)-1H-indol-5-carbonsäure

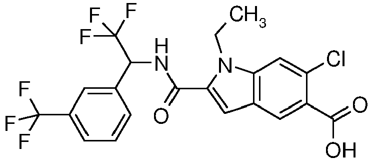

Ethyl-6-chlor-1-ethyl-2-({2,2,2-trifluor-1-[3-(trifluormethyl)phenyl]ethyl}carbamoyl)-1H-indol-5-carboxylat (0,10 g, 0,19 mmol) wurde in 5 mL Dichlormethan gelöst und bei -10 °C eine Lösung von Bortribromid in Dichlormethan (0,96 mL, 0,96 mmol) zugetropft. Das Reaktionsgemisch wurde 1 h bei - 10 °C und anschließend 2 h bei Raumtemperatur nachgerührt. Es wurde Wasser zugegeben und der ausgefallene Feststoff abgesaugt und getrocknet. Es wurden 0,077 g (71 % d.Th.) 6-Chlor-1-ethyl-2-({2,2,2-trifluor-1-[3-(trifluormethyl)phenyl]ethyl}carbamoyl)-1H-indol-5-carbonsäure erhalten. HPLC-MS: logP = 3,71; Masse (m/z): 493,3(M+H)+; 1HNMR (D₆-DMSO): δ 1.22 (t, 3H), 4.50 (q, 2H), 6.24-6.31 (m, 1H), 7.42 (s, 1H), 7.70-7.74 (m, 1H), 7.81-7.83 (m, 2H), 8.05 (d, 1H), 8.19 (s, 1H), 8.26 (s, 1H), 9.85 (d, 1H).

### Analog wurden erhalten:

### 6-Chlor-1-ethyl-2-({2,2,2-trifluor-1-[3-chlorphenyl]ethyl}carbamoyl)-1H-indol-5-carbonsäure

HPLC-MS: logP = 3,65; Masse (m/z): 459,0 (M+H)⁺; ¹HNMR (D₆-DMSO): δ 1.23 (t, 3H), 4.51 (q, 2H), 6.12-6.21 (m, 1H), 7.43 (s, 1H), 7.50-7.53 (m, 2H), 7.68-7.71 (m, 1H), 7.84 (s, 1H), 7.90 (s, 1H), 8.26 (s, 1H), 9.76 (d, 1H), 13.00 (s, 1H).

### 6-Chlor-1-ethyl-2-({2,2,2-trifluor-1-[3-bromphenyl)ethyl}carbamoyl)-1H-indol-5-carbonsäure

HPLC-MS: logP = 3,65; Masse (m/z): 503,0 (M+H)⁺; ¹HNMR (D₆-DMSO): δ 1.23 (t, 3H), 4.51 (q, 2H), 6.11-6.20 (m, 1H), 7.41-7.45 (m, 2H), 7.64-7.66 (m, 1H), 7.73-7.75 (m, 1H), 7.84 (s, 1H), 8.03 (s, 1H), 8.26 (s, 1H), 9.73 (d, 1H), 13.01 (s, 1H).

### 6-Chlor-1-ethyl-2-({2,2,2-trifluor-1-[3,5-dichlorphenyl]ethyl}carbamoyl)-1H-indol-5-carbonsäure

HPLC-MS: logP = 4,21; Masse (m/z): 493,0 (M+H)⁺; ¹HNMR (D₆-DMSO): δ 1.23 (t, 3H), 4.51 (q, 2H), 6.21-6.30 (m, 1H), 7.43 (s, 1H), 7.73 (s, 1H), 7.84 (s, 1H), 7.92 (s, 1H), 8.27 (s, 1H), 9.73 (d, 1H), 13.01 (s, 1H).

### 6-Chlor-1-ethyl-2-({2,2,2-trifluor-1-[3-chlor-4-fluorphenyl]ethyl}carbamoyl)-1H-indol-5-carbonsäure

HPLC-MS: logP = 3,68; Masse (m/z): 477,0 (M+H)⁺; ¹HNMR (D₆-DMSO): δ 1.23 (t, 3H), 4.51 (q, 2H), 6.16-6.25 (m, 1H), 7.42 (s, 1H), 7.52-7.57 (m, 1H), 7.78-7.81 (m, 1H), 7.84 (s, 1H), 8.07-8.09 (m, 1H), 8.27 (s, 1H), 9.74 (d, 1H).

### 6-Chlor-1-ethyl-2-({2,2,2-trifluor-1-[3-brom-4-fluorphenyl]ethyl}carbamoyl)-1H-indol-5-carbonsäure

HPLC-MS: logP = 3,70; Masse (m/z): 521,0 (M+H)⁺; ¹HNMR (D₆-DMSO): δ 1.23 (t, 3H), 4.51 (q, 2H), 6.16-6.24 (m, 1H), 7.42 (s, 1H), 7.48-7.52 (m, 1H), 7.80-7.84 (m, 2H), 8.18-8.20 (m, 1H), 8.27 (s, 1H), 9.73 (d, 1H), 13.00 (s, 1H).

### 6-Chlor-1-ethyl-2-({2,2,2-trifluor-1-[4-fluor-3-(trifluormethyl)phenyl]ethyl}carbamoyl)-1H-indol-5-carbonsäure

HPLC-MS: logP = 3,81; Masse (m/z): 510,9 (M+H)⁺; ¹HNMR (D₆-DMSO): δ 1.23 (t, 3H), 4.56 (q, 2H), 6.30-6.39 (m, 1H), 7.42 (s, 1H), 7.64-7.69 (m, 1H), 7.84 (s, 1H), 8.15-8.18 (m, 1H), 8.27-8.30 (m, 2H), 9.84 (d, 1H).

### 6-Chlor-1-ethyl-2-({2,2,2-trifluor-1-[3,4-dichlorphenyl]ethyl}carbamoyl)-1H-indol-5-carbonsäure

HPLC-MS: logP = 3,90; Masse (m/z): 493,0 (M+H)⁺; ¹HNMR (D₆-DMSO): δ 1.23 (t, 3H), 4.51 (q, 2H), 6.18-6.27 (m, 1H), 7.42 (s, 1H), 7.76 (s, 2H), 7.84 (s, 1H), 8.11 (s, 1H), 8.27 (s, 1H), 9.76 (d, 1H), 13.00 (s, 1H).

### 6-Chlor-1-ethyl-2-({2,2,2-trifluor-1-[3,5-dichlor-4-fluorphenyl]ethyl}carbamoyl)-1H-indol-5-carbonsäure

HPLC-MS: logP = 4,23; Masse (m/z): 510,9 (M+H)⁺; ¹HNMR (D₆-DMSO): δ 1.24 (t, 3H), 4.51 (q, 2H), 6.24-6.32 (m, 1H), 7.44 (s, 1H), 7.83 (s, 1H), 8.11 (s, 1H), 8.12 (s, 1H), 8.28 (s, 1H), 9.73 (d, 1H).

### 6-Chlor-1-ethyl-2-({2,2,2-trifluor-1-[3,5-dichlor-2,4-difluorphenyl]ethyl}carbamoyl)-1H-indol-5-carbonsäure

HPLC-MS: logP = 4,44; Masse (m/z): 529,0 (M+H)⁺; ¹HNMR (D₆-DMSO): δ 1.24 (t, 3H), 4.51 (q, 2H), 6.31-6.39 (m, 1H), 7.47 (s, 1H), 7.85 (s, 1H), 8.25-8.29 (m, 2H), 9.85 (d, 1H).

### 6-Chlor-1-ethyl-2-({2,2,2-trifluor-1-[3,4-difluorphenyl]ethyl}carbamoyl)-1H-indol-5-carbonsäure

HPLC-MS: logP = 3,46; Masse (m/z): 461,1 (M+H)⁺; ¹HNMR (D₆-DMSO): δ 1.23 (t, 3H), 4.51 (q, 2H), 6.14-6.23 (m, 1H), 7.42 (s, 1H), 7.53-7.66 (m, 2H), 7.84 (s, 1H), 7.90-7.95 (m, 1H), 8.26 (s, 1H), 9.73 (d, 1H).

### 6-Chlor-1-ethyl-2-({2,2,2-trifluor-1-[3,4,5-trichlorphenyl]ethyl}carbamoyl)-1H-indol-5-carbonsäure

HPLC-MS: logP = 4,55; Masse (m/z): 526,9 (M+H)⁺; ¹HNMR (D₆-DMSO): δ 1.23 (t, 3H), 4.51 (q, 2H), 6.25-6.34 (m, 1H), 7.43 (s, 1H), 7.84 (s, 1H), 8.15 (s, 2H), 8.28 (s, 1H), 9.72 (d, 1H), 13.0 (s, 1H).

### 1-Ethyl-6-methyl-2-({2,2,2-trifluor-1-[3-(trifluormethyl)phenyl]ethyl}carbamoyl)-1H-indol-5-carbonsäure

HPLC-MS: logP = 3,79; Masse (m/z): 473,1(M+H)⁺.

### 1-Ethyl-6-methyl-2-({2,2,2-trifluor-1-[3,4-dichlorphenyl]ethyl}carbamoyl)-1H-indol-5-carbonsäure

HPLC-MS: logP = 4,16; Masse (m/z): 473,0(M+H)⁺.

### 1-Ethyl-6-methyl-2-({2,2,2-trifluor-1-[3,4-difluorphenyl]ethyl}carbamoyl)-1H-indol-5-carbonsäure

HPLC-MS: logP = 3,45; Masse (m/z): 441,1,0(M+H)⁺.

### 1-Ethyl-6-methyl-2-({2,2,2-trifluor-1-[3,4,5-trichlorphenyl]ethyl}carbamoyl)-1H-indol-5-carbonsäure

HPLC-MS: logP = 4,69; Masse (m/z): 507,0(M+H)⁺.

### 1-Ethyl-6-methyl-2-({2,2,2-trifluor-1-[4-fluor-3-(trifluormethyl)phenyl]ethyl}carbamoyl)-1H-indol-5-carbonsäure

HPLC-MS: logP = 3,92; Masse (m/z): 491,0(M+H)⁺.

### 1-Ethyl-6-methyl-2-({2,2,2-trifluor-1-[3-chlorphenyl]ethyl}carbamoyl)-1H-indol-5-carbonsäure

HPLC-MS: logP = 4,72; Masse (m/z): 439,1(M+H)⁺.

### 1-Ethyl-6-methyl-2-({2,2,2-trifluor-1-[3,5-dichlorphenyl]ethyl}carbamoyl)-1H-indol-5-carbonsäure

HPLC-MS: logP = 4,19; Masse (m/z): 473,0(M+H)⁺.

### 1-Ethyl-6-methyl-2-({2,2,2-trifluor-1-[3-chlor-5-fluorphenyl]ethyl}carbamoyl)-1H-indol-5-carbonsäure

HPLC-MS: logP = 4,83; Masse (m/z): 457,1(M+H)⁺.

### 1-Ethyl-6-methyl-2-({2,2,2-trifluor-1-[3,5-dichlor-4-fluorphenyl]ethyl}carbamoyl)-1H-indol-5-carbonsäure

HPLC-MS: logP = 4,36; Masse (m/z): 491,1(M+H)⁺.

### 1-Ethyl-6-methyl-2-({2,2,2-trifluor-1-[3-Chlor-4-fluorphenyl]ethyl}carbamoyl)-1H-indol-5-carbonsäure

HPLC-MS: logP = 3,80; Masse (m/z): 457,1(M+H)⁺.

### 1-Ethyl-6-methyl-2-({2,2,2-trifluor-1-[3-Brom-4-fluorphenyl]ethyl}carbamoyl)-1H-indol-5-carbonsäure

HPLC-MS: logP = 3,86; Masse (m/z): 501,0(M+H)⁺.

### 1-Ethyl-6-methyl-2-({2,2,2-trifluor-1-[2,2-difluor-1,3-benzodioxol-5-yl]ethyl}carbamoyl)-1H-indol-5-carbonsäure

HPLC-MS: logP = 3,85; Masse (m/z): 485,0(M+H)⁺.

### 1,6-Diethyl-2-({2,2,2-trifluor-1-[3-(trifluormethyl)phenyl]ethyl}carbamoyl)-1H-indol-5-carbonsäure

HPLC-MS: logP = 4,09; Masse (m/z): 515,1,0(M+H)⁺.

### 1-Ethyl-6-isopropyl-2-({2,2,2-trifluor-1-[3-(trifluormethyl)phenyl]ethyl}carbamoyl)-1H-indol-5-carbonsäure

HPLC-MS: logP = 4,23; Masse (m/z): 487,0(M+H)⁺.

### Stufe 4: 6-Chlor-N⁵-(1-cyancyclopropyl)-1-ethyl-N²-{2,2,2-trifluor-1-[3-(trifluormethyl)phenyl]ethyl}-1H-indol-2,5-dicarboxamid

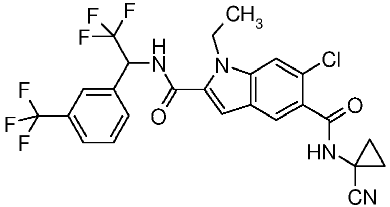

6-Chlor-1-ethyl-2-({2,2,2-trifluor-1-[3-(trifluormethyl)phenyl]ethyl}carbamoyl)-1H-indol-5-carbonsäure (93 %ig; 0,116 g, 0,22 mmol) wurde in Dichlormethan (2,5 mL) gelöst. Es wurden nacheinander N,N-Dimethylformamid (1 Tropfen) und Oxalylchlorid (0,058 mL, 0,66 mmol) zugetropft. Das Reaktionsgemisch wurde 30 min bei Raumtemperatur und 30 min bei 40 °C gerührt und anschließend das Lösemittel im Vakuum entfernt. Der Rückstand wurde in Dichlormethan (2,5 mL) gelöst und bei Raumtemperatur zu einer Lösung von 1-Aminocyclopropancarbonitril Hydrochlorid (0,052 g, 0,44 mmol) und Diisopropylethylamin (0,085 g, 0,66 mmol) in Dichlormethan (2,5 mL) getropft. Das Reaktionsgemisch wurde 16 h bei Raumtemperatur nachgerührt und anschließend Ethylacetat zugegeben. Die organische Phase wurde mit gesättigter Ammoniumchloridlösung gewaschen und die wässrige Phase zweimal mit Ethylacetat extrahiert. Die vereinigten organischen Phasen wurden über Natriumsulfat getrocknet und das Lösemittel im Vakuum entfernt. Der Rückstand wurde mit Wasser und Acetonitril über RP-Kieselgel chromatographiert und ergab 0,033 g (24 % d.Th.) 6-Chlor-N⁵-(1-cyancyclopropyl)-1-ethyl-N²-{2,2,2-trifluor-1-[3-(trifluormethyl)phenyl]ethyl}-1H-indol-2,5-dicarboxamid.
HPLC-MS: logP = 3,80; Masse (m/z): 557,1(M+H)⁺; ¹HNMR (CD3CN): δ 1.31 (t, 3H), 1.33-1.39 (m, 2H), 1.58-1.62 (m, 2H), 4.50 (q, 2H), 6.11-6.20 (m, 1H), 7.26 (s, 1H), 7.58 (s, 1H), 7.66 (s, 1H), 7.69-7.72 (m, 1H), 7.80-7.82 (m, 1H), 7.85 (s, 1H), 7.90-7.92 (m, 1H), 7.99 (s, 1H), 8.15-8.19 (m, 1H).

### Synthesebeispiel 3

### 5-(Acetamidomethyl)-6-chlor-1-ethyl-N-{2,2,2-tritluor-1-[3-(trifluormethyl)phenyl]ethyl}-1H-indol-2-carboxamid (Verbindung Nr. 26 in Tabelle 1)

### Stufe 1: 5-{[(tert-Butoxycarbonyl)amino]methyl}-6-chlor-1H-indol-2-carbonsäure

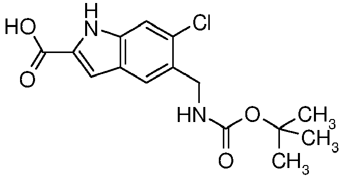

*tert*-Butyl-(4-amino-2-chlor-5-iodbenzyl)carbamat (1,18 g, 3,08 mmol) (bekannt aus WO-A-2006/101321) wurde unter Argon in N,N-Dimethylformamid (12 mL) vorgelegt. 2-Oxopropionsäure (0,88 g, 10,02 mmol), 1,4-Diazabicyclo[2.2.2]octan (1,12 g, 10,02 mmol) und Palladiumacetat (0,034 g, 0,15 mmol) wurden zugegeben. Das Reaktionsgemisch wurde 5 h bei 100 °C gerührt. Nach Abkühlen wurde die Lösung über Celite filtriert und der Filterkuchen mit Ethylacetat gewaschen. Das Filtrat wurde mit Salzsäure (0,1 M) und gesättigter Natriumchloridlösung gewaschen, die organische Phase über Natriumsulfat getrocknet und das Lösemittel im Vakuum entfernt. Es wurden 1,99 g Rohprodukt erhalten, das ohne weitere Reinigung weiter umgesetzt wurde.
¹HNMR (CD₃CN): δ 1.42 (s, 9H), 4.36 (d, 2H), 5.76 (bs, 1H), 7.16 (s, 1H), 7.54 (s, 1H), 7.64 (s, 1H), 9.95 (s, 1H).

### Stufe 2: tert-Butyl-{[6-chlor-2-({2,2,2-trifluor-1-[3-(trifluormethyl)phenyl]ethyl}carbamoyl)-1H-indol-5-yl]methyl}carbamat

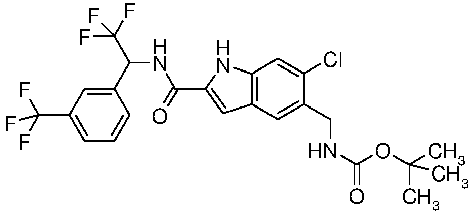

2,2,2-Trifluor-1-[3-(trifluormethyl)phenyl]ethanamin (0,75 g, 3,08 mmol) wurde in N,N-Dimethylformamid (52 mL) gelöst und 5-{[(tert-Butoxycarbonyl)amino]methyl}-6-chlor-1H-indol-2-carbonsäure (1,00 g, 3,08 mmol), N-[(1H-Benzotriazol-1-yloxy)(dimethylamino)methylen]-N-methylmethanaminiumhexafluorophosphat (1,17 g, 3,08 mmol) und 4-Methylmorpholin (0,93 g, 9,24 mmol) zugegeben. Das Reaktionsgemisch wurde 16 h bei Raumtemperatur gerührt und anschließend Wasser und Ethylacetat zugegeben. Nach Phasentrennung wurde die organische Phase mit verdünnter Natriumhydrogencarbonatlösung und gesättigter Natriumchloridlösung gewaschen, über Natriumsulfat getrocknet und das Lösemittel im Vakuum entfernt. Der Rückstand wurde mit Cyclohexan/Ethylacetat (5/1) chromatographiert. Es wurden 0,75 g (40 % d.Th.) tert-Butyl-{[6-chlor-2-({2,2,2-trifluor-1-[3-(trifluormethyl)phenyl]ethyl}carbamoyl)-1H-indol-5-yl]methyl}carbamat erhalten. HPLC-MS: logP = 4,37; Masse (m/z): 548,1(M-H)⁻; ¹HNMR (CD₃CN): δ 1.43 (s, 9H), 4.36 (d, 2H), 5.76 (bs, 1H), 6.14-6.17 (m, 1H), 7.25 (s, 1H), 7.51 (s, 1H), 7.65-7.68 (m, 2H), 7.76-7.78 (m, 1H), 7.87-7.89 (m, 1H), 7.97 (s, 1H), 8.02 (d, 1H), 10.01 (s, 1H).

### Stufe 3: tert-Butyl-{[6-chlor-1-ethyl-2-({2,2,2-trifluor-1-[3-(trifluormethyl)phenyl]-ethyl}carbamoyl)-1H-indol-5-yl]methyl}carbamat

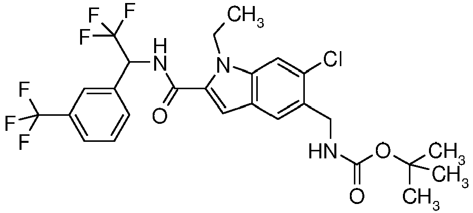

*tert*-Butyl-{[6-chlor-2-({2,2,2-trifluor-1-[3-(trifluormethyl)phenyl]ethyl}carbamoyl)-1H-indol-5-yl]methyl}carbamat (90 %ig; 0,74 g, 1,21 mmol) wurde unter Argon bei 0 °C in N,N-Dimethylformamid (12,5 mL) gelöst. Es wurde Natriumhydrid (60 %ig, 0,053 g, 1,33 mmol) zugegeben und 2 h unter Eiskühlung nachgerührt. Anschließend wurde Iodethan (0,19 g, 1,21 mmol) zugetropft. Das Reaktionsgemisch wurde innerhalb von 16 h unter Rühren aufgetaut. Es wurden Wasser und Ethylacetat zugegeben und die Phasen getrennt. Die organische Phase wurde mit gesättigter Natriumchloridlösung gewaschen, über Magnesiumsulfat getrocknet und das Lösemittel im Vakuum entfernt. Der Rückstand wurde mit Cyclohexan/Ethylacetat (4/1) chromatographiert und lieferte 0,42 g (60 % d.Th.) tert-Butyl-{[6-chlor-1-ethyl-2-({2,2,2-trifluor-1-[3-(trifluormethyl)phenyl]ethyl}-carbamoyl)-1 H-indol-5-yl]methyl}carbamat.
HPLC-MS: logP = 5,14; Masse (m/z): 576,1(M-H)⁻; ¹HNMR (CD₃CN): δ 1.30 (t, 3H), 1.45 (s, 9H), 4.39 (d, 2H), 4.49 (q, 2H), 5.78 (bs, 1H), 6.14-6.18 (m, 1H), 7.20 (s, 1H), 7.61 (s, 1H), 7.68-7.72 (m, 2H), 7.79-7.81 (m, 1H), 7.90-7.92 (m, 1H), 7.99-8.04 (m, 2H).

### Stufe 4: [6-Chlor-1-ethyl-2-({2,2,2-trifluor-1-[3-(trifluormethyl)phenyl]ethyl]carbamoyl)-1H-indol-5-yl]methanaminiumtrifluoraceta

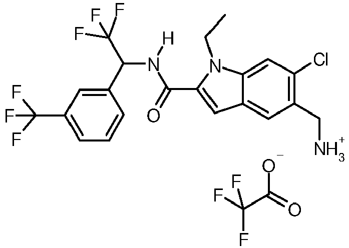

*tert*-Butyl-{[6-chlor-1-ethyl-2-({2,2,2-trifluor-1-[3-(trifluormethyl)phenyl]ethyl}carbamoyl)-1H-indol-5-yl]methyl}carbamat (0,42 g, 0,72 mmol) wurde in Dichlormethan (5 mL) gelöst und Trifluoressigsäure (0,82 g, 7,18 mmol) zugegeben. Das Reaktionsgemisch wurde 2 h bei Raumtemperatur gerührt und anschließend das Lösemittel im Vakuum entfernt. Es wurden 0,42 g (97 % d.Th.) [6-Chlor-1-ethyl-2-({2,2,2-trifluor-1-[3-(trifluormethyl)phenyl]ethyl}carbamoyl)-1H-indol-5-yl]methanaminiumtrifluoracetat erhalten.
HPLC-MS: logP = 2,15; Masse (m/z): 476,0(M-H-TFA)⁻; ¹HNMR (CD₃CN): δ 1.31 (t, 3H), 4.37 (s, 2H), 4.50 (q, 2H), 6.12-6.20 (m, 1H), 7.25 (s, 1H), 7.58 (bs, 3H), 7.68-7.72 (m, 2H), 7.80-7.82 (m, 1H), 7.88 (s, 1H), 7.90-7.92 (m, 1H), 8.00 (s, 1H), 8.19 (d, 1H).

### Stufe 5: 5-(Acetamidomethyl)-6-chlor-1-ethyl-N-{f2,2,2-trifluor-1-[3-(trifluormethyl)phenyl]ethyl}-1H-indol-2-carboxamid

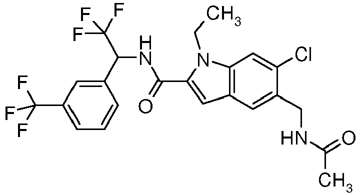

Bei 0 °C wurden [6-Chlor-1-ethyl-2-({2,2,2-trifluor-1-[3-(trifluormethyl)phenyl]ethyl}carbamoyl)-1H-indol-5-yl]methanaminiumtrifluoracetat (0,07 g, 0,15 mmol), Triethylamin (0,036 g, 0,36 mmol) und N,N-Dimethylpyridin-4-amin (0,002 g, 0,12 mmol) in Dichlormethan (1 mL) vorgelegt. Essigsäureanhydrid (0,021 g, 0,21 mmol) wurde zugetropft und das Reaktionsgemisch 16 h bei Raumtemperatur nachgerührt. Es wurde Ethylacetat zugegeben und die organische Phase nacheinander mit Wasser, gesättigter Ammoniumchloridlösung und gesättigter Natriumhydrogencarbonatlösung gewaschen. Die organische Phase wurde über Natriumsulfat getrocknet und das Lösemittel im Vakuum entfernt. Es wurden 0,06 g (98 % d.Th.) 5-(Acetamidomethyl)-6-chlor-1-ethyl-N-{2,2,2-trifluor-1-[3-(trifluormethyl)phenyl]ethyl}-1H-indol-2-carboxamid erhalten.
HPLC-MS: logP = 3,77; Masse (m/z): 518,1(M-H)⁻; ¹HNMR (D₆-DMSO): δ 1.20 (t, 3H), 3.33 (s, 3H), 4.37 (d, 2H), 4.49 (q, 2H), 6.27-6.31 (m, 1H), 7.33 (s, 1H), 7.67 (s, 1H), 7.72-7.74 (m, 1H), 7.77 (s, 1H), 7.81-7.83 (m, 1H), 8.06 (d, 1H), 8.20 (s, 1H), 8.29 (t, 1H), 9.70 (d, 1H).

### Synthese beispiel 4

### 6-Chlor-N⁵-cyclopropyl-1-ethyl-N²-{2,2,2-trifluor-1-[3-(trifluormethyl)phenyl]ethyl}-1H-benzimidazol-2,5-dicarboxamid (Verbindung Nr. 4 in Tabelle 1)

### Stufe 1: 2-Chlor-N-cyclopropyl-4,5-dinitrobenzamid

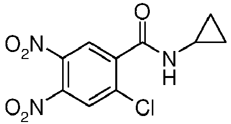

2-Chlor-4,5-dinitrobenzoesäure (bekannt aus WO-A-2009/47558) (8,0 g, 32,5 mmol) wurde in 1,2-Dichlorethan (80 mL) gelöst und unter Stickstoff Thionylchlorid (20 mL) zugegeben. Das Reaktionsgemisch wurde 4 h unter Rückfluss erhitzt und anschließend das Lösemittel im Vakuum entfernt. Der Rückstand wurde in Dichlormethan (80 mL) gelöst und bei 0 °C Cyclopropylamin (2,4 mL, 39 mmol) zugegeben. Das Reaktionsgemisch wurde 2 h bei Raumtemperatur gerührt und anschließend das Lösemittel im Vakuum entfernt. Der Rückstand wurde 30 min mit Diethylether (50 mL) verrührt und anschließend abgesaugt. Der Rückstand wurde mit Wasser (100 mL) gewaschen und getrocknet. 7,5 g (81 % d.Th.) 2-Chlor-N-cyclopropyl-4,5-dinitrobenzamid wurden so erhalten.
HPLC-MS: Masse (m/z): 286,0(M+H)⁺; ¹HNMR (D₆-DMSO) δ 0.53-0.57 (m, 2H), 0.71-0.76 (m, 2H), 2.80-2.84 (m, 1H), 8.41 (s, 1H), 8.55 (s, 1H), 8.84-8.85 (m, 1H).

### Analog wurden erhalten:

### 2-Brom-N-cyclopropyl-4,5-dinitrobenzamid

HPLC-MS: logP = 1,92; Masse (m/z): 329,9 (M+H)⁺; ¹HNMR (D₆-DMSO): δ 0.52-0.6 (m, 2H), 0.72-0,77 (m, 2H), 2,68-2,85 (m, 1H), 8.35 (s, 1H), 8.64 (s, 1H), 8.81-8.83 (d, 1H).

### 2-chloro-N-ethyl-4,5-dinitrobenzamide

HPLC-MS: logP = 1,84; Masse (m/z): 274,0 (M+H)⁺; ¹HNMR (D₆-DMSO): δ 1.11-1-19 (t, 3H), 3.25-3.35 (m, 2H), 8.40 (s, 1H), 8.55 (s, 1H), 8.78-8.80 (t broad, 1H).

### 2-chloro-4,5-dinitro-N-(2,2,2-trifluoroethyl)benzamide

HPLC-MS: logP = 2,35; Masse (m/z): 328.0 (M+H)⁺; ¹HNMR (D₆-DMSO): δ 4.14-4.20 (m, 2H), 8.44 (s, 1H), 8.60 (s, 1H), 9.51-9.54 (t, 1H).

### 2-chloro-N-cyclobutyl-4,5-dinitrobenzamide

HPLC-MS: logP = 2,36; Masse (m/z): 300.0(M+H)⁺; ¹HNMR (D₆-DMSO): δ 1.68-1.75 (m, 2H), 1.95-2.05 (m, 2H), 2.22-2.30 (m, 2H), 4.32-4.40 (m, 1H), 8.41 (s, 1H), 8.57 (s, 1H), 9.03-9.07 (d, 1H).

### Stufe 2 : 4,5-Diamino-2-chlor-N-cyclopropylbenzamid

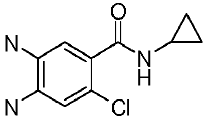

2-Chlor-N-cyclopropyl-4,5-dinitrobenzamid (7,5 g, 26,3 mmol) wurde in Ethanol (200 mL) und Wasser (40 mL) vorgelegt. Bei Raumtemperatur wurde Ammoniumchlorid (2,53 g, 47,4 mmol) zugegeben und das Reaktionsgemisch auf 60 °C erwärmt. Bei dieser Temperatur wurde Eisenpulver (14,7 g, 263 mmol) portionsweise zugegeben und das Reaktionsgemisch anschließend 4 h unter Rückfluss erhitzt. Das Ethanol wurde im Vakuum entfernt und die verbleibende wässrige Suspension über Celite filtriert. Das Filtrat wurde dreimal mit Ethylacetat extrahiert, die vereinigten organischen Phasen mit gesättigter Natriumchloridlösung gewaschen und über Natriumsulfat getrocknet. Nach Entfernen des Lösemittels im Vakuum wurden 4 g (68 % d.Th.) 4,5-Diamino-2-chlor-N-cyclopropylbenzamid erhalten.
¹HNMR (D₆-DMSO): δ 0.44-0.48 (m, 2H), 0.61-0.64 (m, 2H), 2.71-2.75 (m, 1H), 4.68 (bs, 2H), 4.98 (bs, 2H), 6.47 (s, 1H), 6.55 (s, 1H), 7.95-7.97 (d, 1H).

### Analog wurde erhalten:

### 4,5-diamino-2-chloro-N-ethylbenzamide

HPLC-MS: logP = 0,27; Masse (m/z): 214,2 (M+H)⁺; ¹HNMR (D₆-DMSO): δ 1.03-1.14 (t, 3H), 3.14-3.20 (m, 2H), 4.60-4.75 (broad, 2H), 4.9-5.1 (broad, 2H), 6.49 (s, 1H), 6.61 (s, 1H), 7.86-7.90 (t broad, 1H).

### 4,5-diamino-2-chloro-N-(2,2,2-trifluoroethyl)benzamide

HPLC-MS: logP = 0,93; Masse (m/z): 268,1 (M+H)⁺; ¹HNMR (D₆-DMSO): δ 3.95-4.07 (m, 2H), 4.72-4.78 (broad, 2H), 5.08-5.12 (broad, 2H), 6.52 (s, 1H), 6.64 (s, 1H), 8.52-8.58 (broad, 1H).

### 4,5-diamino-2-chloro-N-cyclobutylbenzamide

HPLC-MS: logP = 0.98; Masse (m/z): 240,0 (M+H)⁺; ¹HNMR (D₆-DMSO): δ 1.57-1.66 (m, 2H), 1.92-2.02 (m, 2H), 2.12-2.20 (m, 2H), 4.24-4.35 (m, 1H), 4.6-4.75 (broad, 2H), 4.95-5.5 (broad, 2H), 6.49 (s, 1H), 6.58 (s, 1H), 8.13-8.15 (d broad, 1H).

### Stufe 3: 6-Chlor-N-cyclopropyl-2-(trichlormethyl)-1H-benzimidazol-5-carboxamid

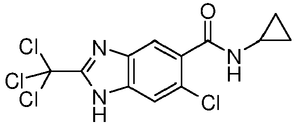

Zu einer Lösung von 4,5-Diamino-2-chlor-N-cyclopropylbenzamid (4,1 g, 18,2 mmol) in Eisessig (50 mL) wurde bei 0 °C Methyl-2,2,2-trichloracetimidat (2,21 mL, 18,2 mmol) zugegeben. Das Reaktionsgemisch wurde 16 h bei Raumtemperatur gerührt und anschließend das Lösemittel im Vakuum entfernt. Das 6-Chlor-N-cyclopropyl-2-(trichlormethyl)-1H-benzimidazol-5-carboxamid wurde für Stufe 4 direkt ohne Reinigung eingesetzt.

### Stufe 4: Methyl-6-chlor-5-(cyclopropylcarbamoyl)-1H-benzimidazol-2-carboxylat

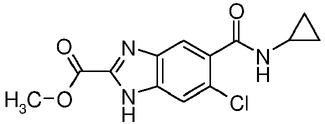

Das 6-Chlor-N-cyclopropyl-2-(trichlormethyl)-1H-benzimidazol-5-carboxamid aus Stufe 3 wurde in Methanol (200 mL) gelöst und 4 h unter Rückfluss erhitzt. Nach Entfernen des Lösemittels im Vakuum wurde der Rückstand in Ethylacetat aufgenommen und mit gesättigter Natriumhydrogencarbonatlösung gewaschen. Die organische Phase wurde über Natriumsulfat getrocknet und das Lösemittel im Vakuum entfernt. Der Rückstand wurde mit Dichlormethan/Methanol (95/5) chromatographiert. Es wurden 2,1 g (53 % d.Th.) Methyl-6-chlor-5-(cyclopropylcarbamoyl)-1H-benzimidazol-2-carboxylat erhalten. HPLC-MS: Masse (m/z): 293,9(M+H)⁺; ¹HNMR (D₆-DMSO): δ 0.54 (m, 2H), 0.67-0.72 (m, 2H), 2.80-2.85 (m 1H), 3.95 (s, 3H), 7.51-7.59 (m, 1H), 7.78-7.90 (m, 1H), 8.49-8.51 (d, 1H), 13.76-13.85 (m, 1H).

### Analog wurden erhalten:

### Methyl 6-chloro-5-(ethylcarbamoyl)-1H-benzimidazole-2-carboxylate

HPLC-MS: logP = 0.87; Masse (m/z): 282.0 (M+H)⁺;

### Methyl 6-chloro-5-(cyclobutylcarbamoyl)-1H-benzimidazole-2-carboxylate

HPLC-MS: logP = 1.34; Masse (m/z): 308.1 (M+H)⁺;

### Methyl 6-chloro-5-[(2,2,2-trifluoroethyl)carbamoyl]-1H-benzimidazole-2-carboxylate

HPLC-MS: logP = 1.37; Masse (m/z): 336.0 (M+H)⁺;

### Stufe 5: 6-Chlor-5-(cyclopropylcarbamoyl)-1H-benzimidazol-2-carbonsäure

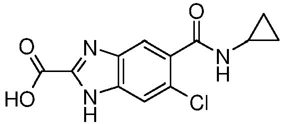

Zu einer Lösung von Methyl-6-chlor-5-(cyclopropylcarbamoyl)-1H-benzimidazol-2-carboxylat (2,1 g, 7,2 mmol) in Tetrahydrofuran (50 mL) wurde bei 0 °C eine Lösung von Lithiumhydroxid Monohydrat (0,6 g, 14,3 mmol) in Wasser (25 mL) getropft. Das Reaktionsgemisch wurde 14 h bei dieser Temperatur gerührt. Nach Entfernen des Tetrahydrofurans im Vakuum wurde die wässrige Lösung mit Salzsäure (2 M) angesäuert. Der ausgefallene Feststoff wurde abgesaugt. Es wurden 1,7 g (81 % d.Th.) 6-Chlor-5-(cyclopropylcarbamoyl)-1H-benzimidazol-2-carbonsäure erhalten.
HPLC-MS: Masse (m/z): 290,9(M+H)⁺; ¹HNMR (D₆-DMSO): δ 0.48-0.5 (m, 2H), 0.64-0.67 (m, 2H), 2.77-2.80 (m, 1H), 7.59 (s, 1H), 7.69 (s, 1H), 8.47-8.48 (d, 1H).

### Analog wurden erhalten:

### 6-chloro-5-(ethylcarbamoyl)-1H-benzimidazole-2-carboxylic acid

HPLC-MS: logP = 0.07; Masse (m/z): 268.1 (M+H)⁺;

### 6-chloro-5-(cyclobutylcarbamoyl)-1H-benzimidazole-2-carboxylic acid

HPLC-MS: logP = 0.80; Masse (m/z): 294.1 (M+H)⁺;

### Stufe 6: 6-Chlor-N⁵-cyclopropyl-N'-{2,2,2-trifluor-1-[3-(trifluormethyl)phenyl]ethyl}-1H-benziminidazol-2,5-dicarboxamid

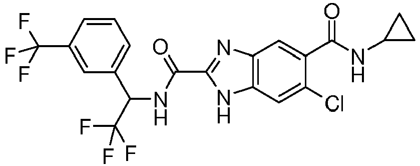

2,2,2-Trifluor-1-[3-(trifluormethyl)phenyl]ethanamin (0,23 g, 0,82 mmol) wurde in N,N-Dimethylformamid (4 mL) gelöst und 6-Chlor-5-(cyclopropylcarbamoyl)-1H-benzimidazol-2-carbonsäure (0,23 g, 0,82 mmol), N-[(1H-Benzotriazol-1-yloxy)(dimethylamino)methylen]-N-methylmethanaminiumhexafluorophosphat (0,31 g, 0,82 mmol) und 4-Methylmorpholin (0,25 g, 2,47 mmol) zugegeben. Das Reaktionsgemisch wurde 16 h bei Raumtemperatur gerührt und anschließend Ethylacetat zugegeben. Die organische Phase wurde mit gesättigter Natriumhydrogencarbonatlösung und gesättigter Natriumchloridlösung gewaschen, über Natriumsulfat getrocknet und das Lösemittel im Vakuum entfernt. Der Rückstand wurde mit Cyclohexan/Ethylacetat (1/1) chromatographiert und ergab 0,24 g (57 % d.Th.) 6-Chlor-N⁵-cyclopropyl-N²-{2,2,2-trifluor-1-[3-(trifluormethyl)phenyl]ethyl}-1H-benzimidazol-2,5-dicarboxamid.
HPLC-MS: logP = 2,97; Masse (m/z): 505,1(M+H)⁺; ¹HNMR (CD₃CN): δ 0.58-0.65 (m, 2H), 0.72-0.79 (m, 2H), 2.82-2.89 (m, 1H), 6.06-6.15 (m, 1H), 6.92 (bs, 1H), 7.63-7.69 (m, 2H), 7.77-7.85 (m, 2H), 7.89 (d, 1H), 7.98 (s, 1H), 8.65 (d, 1H), 11.44-11.53 (m, 1H).

### Analog wurden erhalten:

### 6-chloro-N⁵-cyclobutyl-N²-{2,2,2-trifluoro-1-[4-fluoro-3-(trifluoromethyl)phenyl]ethyl}-1H-benzimidazole-2,5-dicarboxamide

HPLC-MS: logP = 3.38; Masse (m/z): 537.1 (M+H)⁺; ¹HNMR (D₆-DMSO): δ 1.64-1.72 (m, 2H), 1.97-2.06 (m, 2H), 2.12-2.28 (m, 2H), 4.35-4.41 (m, 1H), 6.32-6.39 (m, 1H), 7.52-7.89 (m, 3H), 8.20-8.26 (broad, 1H), 8.49-8.51 (broad, 1H), 8.56-8.75 (dd, 1H), 10.6 (s, 1H), 13.67-13.78 (d, 1H).

### 6-chloro-N⁵-cyclobutyl-N²-{2,2,2-trifluoro-1-[3-(trifluoromethyl)phenyl]ethyl]-1H-benzimidazole-2,5-dicarboxamide

HPLC-MS: logP = 3.33; Masse (m/z): 519.1 (M+H)⁺; ¹HNMR (D₆-DMSO): δ 1.62-1.73 (m, 2H), 1.95-2.08 (m, 2H), 2.20-2.29 (m, 2H), 4.32-4.44 (m, 1H), 6.28-6.38 (m, 1H), 7.50-7.95 (m, 4H), 8.12-8.17 (d, 1H), 8.41 (s, 1H), 8.66-8.77 (d broad, 1H), 10.5 (s, 1H), 13.5-13.9 (d broad, 1H).

### N²-[1-(3-bromo-4-fluorophenyl)-2,2,2-trifluoroethyl]-6-chloro-N⁵-cyclopropyl-1H-benzimidazole-2,5-dicarboxamide

HPLC-MS: logP = 2.93; Masse (m/z): 533.0 (M+H)⁺; ¹HNMR (D₆-DMSO): δ 0.50-058 (m, 2H), 0.68-0.75 (m, 2H), 2.80-2.88 (m, 1H), 6.15-6.27 (q, 1H), 7.38-7.95 (m, 4H), 8.35-8.40 (d, 1H), 8.48-8.55 (dd, 1H), 10.4 (s broad, 1H), 13.6-13.8 (d , 1H).

### 6-chloro-N⁵-(2,2,2-trifluroroethyl)-N²-{2,2,2-trifluoro-1-[3-(trifluoromethyl)phenyl]ethyl}-1H-benzimidazole-2,5-dicarboxamide

HPLC-MS: logP = 3.30; Masse (m/z): 547.0 (M+H)⁺; ¹HNMR (D₆-DMSO): δ 4.05-4.15 (m, 2H), 6.28-6.38 (m, 1H), 7.55-7.86 (m, 4H), 8.12-8.17 (d, 1H), 8.39-8.43 (s broad, 1H), 9.13-9.25 (m, 1H), 10.5 (s, 1H), 13.7-13.9 (d , 1H).

### Stufe 7: 5-Chlor-N⁶-cyclopropyl-1-ethyl-N'-{2,2,2-trifluor-1-[3-(trifluormethyl)phenyl]ethyl}-1H-benzimidazol-2,6-dicarboxamid und 6-Chlor-N⁵-cyclopropyl-1-ethyl-N²-{2,2,2-trifluor-1-[3-(trifluormethyl)phenyl]ethyl}-1H-benzimidazol-2,5-dicarboxamid

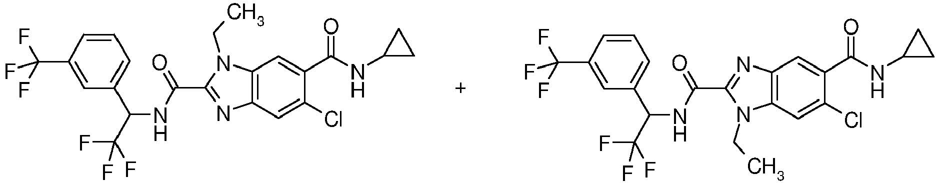

6-Chlor-N⁵-cyclopropyl-N²-{2,2,2-trifluor-1-[3-(trifluormethyl)phenyl]ethyl}-1H-benzimidazol-2,5-dicarboxamid (0,1 g, 0,22 mmol) wurde unter Argon bei 0 °C in N,N-Dimethylformamid (2 mL) gelöst. Es wurde Natriumhydrid (60 %ig, 0,0082 g, 0,22 mmol) zugegeben und 1 h unter Eiskühlung nachgerührt. Anschließend wurde Iodethan (0,034 g, 0,22 mmol) zugetropft. Das Reaktionsgemisch wurde innerhalb von 16 h unter Rühren aufgetaut. Es wurden Wasser und Ethylacetat zugegeben und die Phasen getrennt. Die organische Phase wurde mit gesättigter Natriumchloridlösung gewaschen, über Magnesiumsulfat getrocknet und das Lösemittel im Vakuum entfernt. Der Rückstand wurde mit Cyclohexan/Ethylacetat (4/1) chromatographiert und ergab 0,012 g (12 % d.Th.) 5-Chlor-N⁶-cyclopropyl-1-ethyl-N²-{2,2,2-trifluor-1-[3-(trifluormethyl)phenyl]-ethyl}-1H-benzimidazol-2,6-*dicarboxamide* und 0,018 g (18% d.Th.) 6-Chlor-N⁵-cyclopropyl-1-ethyl-N²-{2,2,2-trifluor-1-[3-(trifluormethyl)phenyl]ethyl}-1H-benzimidazol-2,5-dicarboxamid.
HPLC-MS: logP = 3,67; Masse (m/z): 533,2(M+H)⁺; ¹HNMR (CD₃CN): δ 0.59-0.62 (m, 2H), 0.76-0.79 (m, 2H), 1.38 (t, 3H), 2.83-2.89 (m, 1H), 4.61-4.64 (m, 2H), 6.08-6.13 (m, 1H), 6.92-6.93 (m, 1H), 7.68 (t, 1H), 7.71 (s, 1H), 7.77-7.79 (m, 2H), 7.90 (d, 1H), 7.98 (s, 1H), 8.76 (d, 1H).

HPLC-MS: logP = 3,71; Masse (m/z): 533,1(M+H)⁺; ¹HNMR (CD₃CN): δ 0.60-0.63 (m, 2H), 0.76-0.79 (m, 2H), 1.39 (t, 3H), 2.84-2.88 (m, 1H), 4.62-4.66 (m, 2H), 6.08-6.14 (m, 1H), 6.97-6.98 (m, 1H), 7.66-7.69 (m, 2H), 7.78-7.79 (m, 2H), 7.90 (d, 1H), 7.98 (s, 1H), 8.81 (d, 1H).

### Synthesebesispiel 5

### 6-chloro-N⁵,1-dicyclopropyl-N²-{2,2,2-tritluoro-1-[3-(tritluoromethyl)phenyl]ethyl}-1H-benzimidazole-2,5-dicarboxamide (Verbindung Nr. 312 in Tabelle 1)

### Stufe 1: 2-chloro-N-cyclopropyl-4-(cyclopropylamino)-5-nitrobenzamide

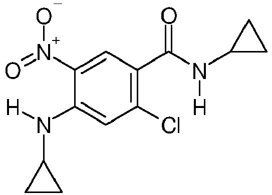

2.00 g (5.95 mmol) 2-chloro-N-cyclopropyl-4,5-dinitrobenzamide (siehe Synthesebeispiel 4, Stufe 1) wurden in 150 ml 1,2.Dichlorethan vorgelegt, 0.85 g (14.88 mmol) Cyclopropylamin zugegeben und drei Stunden unter Rückfluß erwärmt. Nach dem Abkühlen wurde mit Wasser versetzt und das 2-chloro-N-cyclopropyl-4-(cyclopropylamino)-5-nitrobenzamide abgesaugt und getrocknet.
Ausbeute 1.16 g (66 % d.Th.)

HPLC/MS: logP = 2,33; Masse (m/z):296,1 (M+1); ¹HNMR (D₆-DMSO): δ 0.50-0.58 (m, 2H), 0.61-0.71 (m, 4H), 0.88-0.94 (m, 2H), 2.64-2.72 (m, 1H), 2.75-2.83 (m, 1H), 7.39 (s, 1H), 8.11 (s broad, 1H), 8.45-8.50 (d broad, 1H).

In analoger Weise wurden hergestellt:

### 2-bromo-N-cyclopropyl-4-(ethylamino)-5-nitrobenzamide

HPLC/MS: logP = 2,06; Masse (m/z): 328 (M+1); ¹HNMR (D₆-DMSO): δ 0.50-0.60 (m, 2H), 0.62-0.74 (m, 2H), 1.18-1.24 (t, 3H), 2.72-2.82 (m, 1H), 3.36-3.46 (m, 2H), 7.28 (s, 1H), 8.03 (s, 1H), 8.21-8.27 (t broad, 1H), 8.42-8.47 (d broad, 1H).

### 2-chloro-N-cyclopropyl-4-(isopropylamino)-5-nitrobenzamide

HPLC/MS: logP = 2,39 ; Masse (m/z): 298,1 (M+1); ); ¹HNMR (D₆-DMSO): δ 0.48-0.55 (m, 2H), 0.66-0.72 (m, 2H), 1.24-1.28 (d, 2H), 2.75-2.82 (m, 1H), 3.98-4.08 (m, 1H), 7.18 (s, 1H), 7.95-7.99 (d, 1H), 8.11 (s, 1H), 8.45-8.48 (d , 1H).

Am Beispiel der Herstellung von 2-chloro-N-cyclopropyl-4-(ethylamino)-5-nitrobenzamide wurde die Herstellbarkeit von entsprechenden Intermediaten ausgehend von 3-Nitro-4-F-benzoesäuren gezeigt:

### A) 2-chloro-N-cyclopropyl-4-fluoro-5-nitrobenzamide

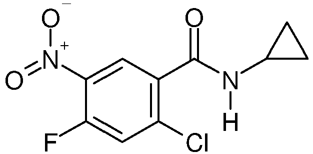

2 g (9.11 mmol 2-chloro-4-fluoro-5-nitrobenzoic acid wurden in 91 g Dichlorethan gelöst, mit 22,66 g Thionylchlorid versetzt und bis zum Ende der Gasentwicklung gekocht. Die flüchtigen Bestandteile wurden abdestilliert und das so erhaltene Säurechlorid direkt im Folgeschritt eingesetzt. 2.166 g (9.1 mmol) 2-chloro-4-fluoro-5-nitrobenzoyl chloride wurden in 100 ml Dichlormethan gelöst, diese Lösung auf 0°C gekühlt und mit 0.52 g (9.1 mmol) Cyclopropylamin sowie 1.38 g (13.65 mmol) Triethylamin zusammen vorgelöst in 5 ml Dichlormethan versetzt. Nach zweistündigen Rühren bei Raumtemperatur wurden die flüchtigen Bestandteile abdestilliert, der erhaltene Rückstand mit wenig Diethylether angeschlämmt, die Diethyletherphase abdekantiert und der Rückstand mit Wasser verrührt.
Nach dem Absaugen und Trocknen wurde das 2-chloro-N-cyclopropyl-4-fluoro-5-nitrobenzamide erhalten.
Ausbeute: 2.0 g (93% d.Th.)
HPLC/MS: logP = 1,59; Masse (m/z): 259,0 (M+1); ¹HNMR (D₆-DMSO): δ 0.50-0.59 (m, 2H), 0.68-0.77 (m, 2H), 2.75-2.86 (m, 1H), 7.96-8.01 (d, 1H), 8.20-8.27 (d, 1H), 8.68-8.79 (d broad, 1H).

### B) 2-chloro-N-cyclopropyl-4-(ethylamino)-5-nitrobenzamide

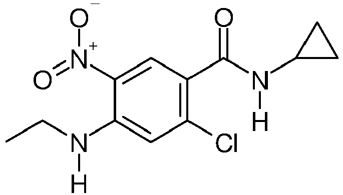

2.17 g (8.40 mmol) 2-chloro-N-cyclopropyl-4-fluoro-5-nitrobenzamide wurden in 100 ml THF vorgelegt und mit 12 ml einer 2 molaren Lösung von Ethylamin inTHF (16.79 mmol) versetzt und 18 Stunden in einer verschlossenen Ampulle bei Raumtemperatur gerührt. Das Reaktionsgemisch wurde auf Wasser gegossen, die organischen Lösungsmittel abdestilliert und der anfallende Feststoff abgesaugt.
Ausbeute: 2.10 g (81 % d.Th.)
HPLC/MS: logP = 2,03; Masse (m/z): 284,0 (M+1); ¹HNMR (D₆-DMSO): δ 0.48-0.58 (m, 2H), 0.64-0.72 (m, 2H), 1.17-1.23 (t, 3H), 2.74-2.83 (m, 1H), 3.4 (m, 2H), 7.13 (s, 1H), 8.11 (s, 1H), 8.26-8.31 (t broad, 1H), 8.44-8.50 (d broad, 1H).

### Stufe 2: 5-amino-2-chloro-N-cyclopropyl-4-(cyclopropylamino)benzamide

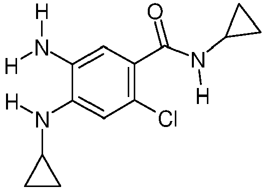

5.50 g (18.6 mmol) 2-chloro-N-cyclopropyl-4-(cyclopropylamino)-5-nitrobenzamide wurden in einer Mischung aus 550 g Ethanol und 110 g Wasser vorgelegt und 1.79 g (33.48 mmol) Ammoniumchlorid zugegeben. Diese Mischung wurde auf 60°C erwärmt 10,39 g (185.98 mmol) Eisenpulver (325 mesh) zugefügt und dann fünf Stunden unter Rückfluß gerührt.
Zur Aufarbeitung wurden die flüchtigen Bestandteile abdestilliert mit 250 ml Wasser verdünnt und über Kieselgur filtriert. Die wässrige Phase wurde ausgiebig mit Essigsäure-ethylester (3 x 100 ml) extrahiert, die organische Phase mit gesättigter Natriumchloridlösung gewaschen, über Natriumsulfat getrocknet und 5-amino-2-chloro-N-cyclopropyl-4-(cyclopropylamino)benzamide zu erhalten.

### Ausbeute: 4.40 g (89 % d.Th)

HPLC/MS: logP = 1,61; Masse (m/z): 266,1 (M+1); ¹HNMR (D₆-DMSO): δ 0.38-0.54 (m, 4H), 0.58-0.75 (m, 4H), 2.30-2.39 (m, 1H), 2.70-2.79 (m, 1H), 4.70-4.79 (broad, 2H), 6.55 (s, 1H), 6.65 (s, 1H), 7.98-8.02 (d, 1H).

In analoger Weise wurden hergestellt:

### 5-amino-2-chloro-N-cyclopropyl-4-(ethylamino)benzamide

HPLC/MS: logP = 1,42; Masse (m/z): 254,2 (M+1); ¹HNMR (D₆-DMSO): δ 0.42-0.50 (m, 2H), 0.60-0.67 (m, 2H), 1.16-1.22 (t, 3H), 2.70-2.78 (m, 1H), 3.00-3.09 (m, 1H),4.75-4.82 (s broad, 2H), 4.86-4.92 (t broad, 1H), 6.29 (s, 1H), 6.58 (s, 1H), 7.96-8.62 (d broad, 1H).

### 5-amino-2-bromo-N-cyclopropyl-4-(cyclopropylamino)benzamide

HPLC/MS: logP = 1,61; Masse (m/z): 310 (M+1); ¹HNMR (D₆-DMSO): δ 0.38-0.42 (m, 2H), 0.45-0.52 (m, 2H), 0.60-0.65 (m, 2H), 0.69-0.78 (m, 2H), 2.30-2.39 (m, 1H), 2.70-2.78 (m, 1H), 4.72-4.81 (broad, 2H), 5.47-5.49 (s, 1H), 6.53 (s, 1H), 6.80 (s, 1H), 7.98-8.04 (d, 1H).

### Stufe 3: Methyl 6-chloro-1-cyclopropyl-5-(cyclopropylcarbamoyl)-1H-benzimidazole-2-carboxylate

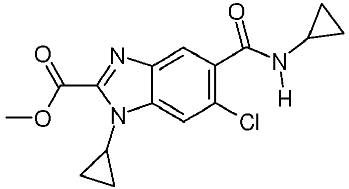

0,30 g (1.13 mmol) 5-amino-2-chloro-N-cyclopropyl-4-(cyclopropylamino) benzamide wurden in 30 ml Essigsäure gelöst, auf 0°C gekühlt und 0.20 g (1.13 mmol) Methyl-2,2,2-trichloracetamidat zugegeben. Diese Lösung wurde 18 Stunden bei Raumtemperatur gerührt, um nach dem Abdestillieren der flüchtigen Bestandteile das Intermediat 6-chloro-N,1-dicyclopropyl-2-(trichloromethyl)-1H-benzimidazole-5-carboxamide zu erhalten.
Dieses wurde in 20 ml Methanol gelöst und vier Stunden (DC.Kontrolle) unter Rückfluß gerührt. Der nach Abdestillieren der flüchtigen Bestandteile erhaltene Rückstand wurde mittels Kieselgelchromatographie Cyclohexan/Essigester (0% Essigsäureethylester auf 60 %) gereinigt.

### Ausbeute: 0.10 g (24 % d.Th.)

HPLC/MS: logP = 1,63; Masse (m/z): 334,1 (M+1); ¹HNMR (D₆-DMSO): δ 0.52-0.58 (m, 2H), 0.66-0.74 (m, 2H), 0.92-0.98 (m, 2H), 1.18-1.26 (m, 2H), 2.79-2.88 (m, 1H), 3.57-3.64 (m, 1H), 3.93 (s, 3H), 7.78 (s, 1H), 7.79 (s, 1H), 8.46-8.51 (d broad, 1H).

In analoger Weise wurde hergestellt:

### Methyl 6-bromo-1-cyclopropyl-5-(cyclopropylcarbamoyl)-1H-benzimidazole-2-carboxylate

HPLC/MS: logP = 1.68; Masse (m/z): 378 (M+1); ¹HNMR (D₆-DMSO): δ 0.50-0.59 (m, 2H), 0.66-0.73 (m, 2H), 0.92-0.98 (m, 2H), 1.20-1.28 (m, 2H), 2.79-2.87 (m, 1H), 3.54-3.64 (m, 1H), 3.96 (s, 3H), 7.75 (s, 1H), 7.94 (s, 1H), 8.47-8.50 (d broad, 1H).

### Stufe 4: 6-chloro-1-cyclopropyl-5-(cyclopropylcarbamoyl)-1H-benzimidazole-2-carboxylic acid

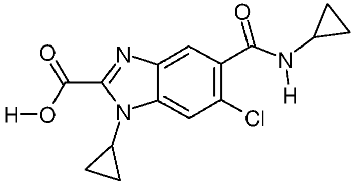

1.65 g (4.9mmol) Methyl 6-chloro-1-cyclopropyl-5-(cyclopropylcarbamoyl)-1H-benzimidazole-2-carboxylate wurden in 82 ml THF gelöst und bei 0°C mit 0,24g (9.89 mmol) Lithiumhydroxyd vorgelöst in 18 ml Wasser versetzt, das Reaktionsgemisch 18 Stunden bei Raumtemperatur nachgerührt. Das THF wurde abdestilliert und die wässrige Phase dreimal mit Essigsäureethylester extrahiert, die wässrige Phase mit HCl auf pH = 3 eingestellt. Der ausfallende Feststoff wurde abgesaugt. Die Säure wurde wegen der relativen Instabilität ohne weitere Reinigung für Folgereaktionen eingesetzt.
Ausbeute: 600 mg (38 % d. Th.).

In analoger Weise wurde hergestellt:

### 6-bromo-1-cyclopropyl-5-(cyclopropylcarbamoyl)-1H-benzimidazole-2-carboxylic acid

### Stufe 5: 6-chlor-o-N⁵,1-dicyclopropyl-N²-{2,2,2-trifluoro-1-[3-(trifluoromethyl)phenyl]ethyl}-1H-benzimidazole-2,5-dicarboxamide

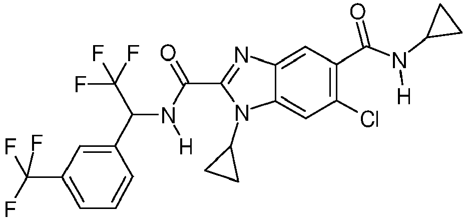

0.063 g (0.20 mmol) 6-chloro-1-cyclopropyl-5-(cyclopropylcarbamoyl)-1H-benzimidazole-2-carboxylic acid wurden unter Argon in 2 ml DMF gelöst, 0,048 g (0.20 mmol) 2,2,2-Trifluor-3-trifluormethyl-phenethylamin, 0.075 g (0.20 mmol) HBTU und 0,060 g (0.59 mmol) 4-Methylmorpholin zugefügt und das Reaktionsgemisch über 18 Stunden bei Raumtemperatur gerührt. Anschliessend wurde das Reaktionsgemisch auf Wasser gegeben, mit Essigsäuremethylester ergiebig extrahiert, die organische Phase mit gesättigter Natriumchloridlösung gewaschen, über Natriumsulfat getrocknet und mittels Kieselgelchromatographie Cyclohexan/Essigester (0% Essigsäureethylester auf 60 %) das Zielprodukt isoliert.
Ausbeute: 13 mg (12 % d.Th.)
HPLC/MS: logP = 3,48; Masse (m/z): 545,0 (M+1); ¹HNMR (D₆-DMSO): δ 0.52-0.60 (m, 2H), 0.69-0.74 (m, 2H), 0.74-0.88 (m, 2H), 1.00-1.14 (m, 2H), 2.80-2.89 (m, 1H), 3.50-3.59 (m, 1H), 6.28-6.38 (m, 1H), 7.70-7.88 (m, 4H), 8.06-8.10 (d, 1H), 8.25 (s, 1H), 8.44-8.49 (d, 1H), 10.44 (s, 1H).

### Synthesebeispiel 6

### 6-Chlor-N⁵-(1-cyancyclopropyl)-1-(prop-2-in-1-yl)-N²-{2,2,2-trifluor-1-[3-(tritluormethyl)phenyl]ethyl}-1H-indol-2,5-dicarboxamid (Verbindung Nr. 57 in Tabelle1) und 6-Chlor-N⁵-(1-cyancyclopropyl)-1-cyclopropyl-N²-{2,2,2-trifluor-1-[3-(trifluormethyl)phenyl]ethyl}-1H-indol-2,5-dicarboxamid (Verbindung Nr. 302 in Tabelle 1)

### Stufe 1: 6-Chlor-2-({2,2,2-trifluor-1-[3-(trifluormethyl)phenyl]ethyl}carbamoyl)-1H-indol-5-carbonsäure

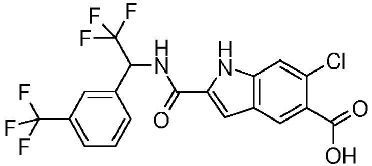

Ethyl-6-chlor-2-({2,2,2-trifluor-1-[3-(trifluormethyl)phenyl]ethyl}carbamoyl)-1H-indol-5-carboxylat (2,90 g, 5,0 mmol, 85%ig) wurde in 135 mL Dichlormethan gelöst und bei -10 °C eine Lösung von Bortribromid in Dichlormethan (27,5 mL, 27,5 mmol) zugetropft. Das Reaktionsgemisch wurde 1 h bei-10 °C und anschließend 2 h bei Raumtemperatur nachgerührt. Es wurde Wasser zugegeben und der ausgefallene Feststoff abgesaugt und getrocknet. Es wurden 1,85 g (79 % d.Th.) 6-Chlor-2-({2,2,2-trifluor-1-[3-(trifluormethyl)phenyl]ethyl}carbamoyl)-1H-indol-5-carbonsäure erhalten. HPLC-MS: logP = 3,02; Masse (m/z): 465,0(M+H)⁺; 1HNMR (CD₃CN): δ 6.11-6.19 (m, 1H), 7.38 (s, 1H), 7.59 (s, 1H), 7.64-7.69 (m, 1H), 7.76-7.78 (m, 1H), 7.88-7.90 (m, 1H), 7.98 (s, 1H), 8.24 (d, 1H), 8.33 (s, 1H), 10.39 (s, 1H).

### Analog wurden erhalten:

### 6-Chlor-2-{[1-(3-chlor-4-fluorphenyl)-2,2,2-trifluorethyl]carbamoyl}-1H-indol-5-carbonsäure

HPLC-MS: logP = 2,93; Masse (m/z): 448,9 (M+H)⁺; ¹HNMR (D₆-DMSO): δ 6.20-6.28 (m, 1H), 7.52-7.57 (m, 2H), 7.60 (s, 1H), 7.78-7.82 (m, 1H), 8.07-8.09 (m, 1H), 8.28 (s, 1H), 9.59 (d, 1H), 12.08 (s, 1H), 12.97 (s, 1H).

### 6-Chlor-2-({2,2,2-trifluor-1-[4-fluor-3-(trifluormethyl)phenyl]ethyl}carbamoyl)-1H-indol-5-carbonsäure

HPLC-MS: logP = 3,08; Masse (m/z): 483,0 (M+H)⁺; ¹HNMR (D₆-DMSO): δ 6.33-6.42 (m, 1H), 7.52 (s, 1H), 7.59-7.60 (m, 1H), 7.64-7.69 (m, 1H), 8.16-8.19 (m, 1H), 8.26-8.30 (m, 2H), 9.70 (d, 1H), 12.09 (s, 1H).

### 6-Chlor-2-{[1-(3-chlorphenyl)-2,2,2-trifluorethyl]carbamoyl}-1H-indol-5-carbonsäure

HPLC-MS: logP = 2,90; Masse (m/z): 431,0 (M+H)⁺; ¹HNMR (D₆-DMSO): δ 6.15-6.24 (m, 1H), 7.48-7.54 (m, 3H), 7.62 (s, 1H), 7.70-7.72 (m, 1H), 7.90 (s, 1H), 8.28 (s, 1H), 9.63 (d, 1H), 12.08 (s, 1H).

### Stufe 2: 6-Chlor-N⁵-(1-cyancyclopropyl)-N²-{2,2,2-trifluor-1-[3-(trifluormethyl)phenyl]ethyl}-1H-indol-2,5-dicarboxamid

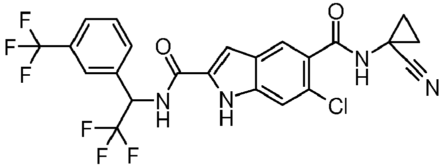

6-Chlor-2-({2,2,2-trifluor-1-[3-(trifluormethyl)phenyl]ethyl}carbamoyl)-1H-indol-5-carbonsäure (2,74 g, 5,07 mmol, 86%ig) wurde in N,N-Dimethylformamid (14 mL) gelöst und 1-Aminocyclopropancarbonitrilhydrochlorid (0,78 g, 6,59 mmol), N-[(1H-Benzotriazol-1-yloxy)(dimethylamino)methylen]-N-methylmethanaminiumhexafluorophosphat (2,12 g, 5,07 mmol) und 4-Methylmorpholin (1,54 g, 15,2 mmol) zugegeben. Das Reaktionsgemisch wurde 16 h bei Raumtemperatur gerührt und anschließend Ethylacetat zugegeben. Die organische Phase wurde mit Salzsäure (1 mol/L) gewaschen und die wässrige Phase nochmals mit Ethylacetat extrahiert. Die vereinigten organischen Phasen wurden mit gesättigter Natriumchloridlösung gewaschen und der in beiden Phasen nicht lösliche Teil abfiltriert. Der Filterkuchen wird mit siedendem Ethylacetat (15 mL) trituiert und der Niederschlag mit warmem Ethylacetat gewaschen. Es verblieben 2,01 g (74 % d.Th.) 6-Chlor-N⁵-(1-cyancyclopropyl)-N²-{2,2,2-trifluor-1-[3-(trifluormethyl)phenyl]ethyl}-1H-indol-2,5-dicarboxamid.
HPLC-MS: logP = 3,12; Masse (m/z): 529,0 (M+H)⁺; ¹HNMR (D₆-DMSO): δ 1.25-1.29 (m, 2H), 1.56-1.60 (m, 2H), 6.30-6.38 (m, 1H), 7.50 (s, 1H), 7.57 (s, 1H), 7.70-7.74 (m, 1H), 7.82-7.86 (m, 2H), 8.07-8.09 (m, 1H), 8.21 (s, 1H), 9.30 (s, 1H), 9.73 (d, 1H), 12.07 (s, 1H).

### Analog wurden erhalten:

### 6-Chlor-N²-[1-(3-chlor-4-fluorphenyl)-2,2,2-trifluorethyl]-N⁵-(1-cyancyclopropyl)-1H-indol-2,5-dicarboxamid.

HPLC-MS: logP = 2,98; Masse (m/z): 513,0 (M+H)⁺; ¹HNMR (D₆-DMSO): δ 1.26-1.28 (m, 2H), 1.56-1.58 (m, 2H), 6.19-6.28 (m, 1H), 7.50-7.56 (m, 3H), 7.77-7.82 (m, 1H), 7.85 (s, 1H), 8.07-8.09 (m, 1H), 9.29 (s, 1H), 9.58 (d, 1H), 12.00 (s, 1H).

### 6-Chlor-N²-[1-(3-chlorphenyl)-2,2,2-trifluorethyl]-N⁵-(1-cyancyclopropyl)-1H-indol-2,5-dicarboxamid

HPLC-MS: logP = 2,88; Masse (m/z): 495,1 (M+H)⁺; ¹HNMR (D₆-DMSO): δ 1.26-1.28 (m, 2H), 1.56-1.58 (m, 2H), 6.15-6.24 (m, 1H), 7.50-7.57 (m, 3H), 7.69-7.73 (m, 1H), 7.85 (s, 1H), 7.89 (s, 2H), 9.29 (s, 1H), 9.60 (d, 1H), 11.98 (s, 1H).

### 6-Chlor-N⁵-(1-cyancyclopropyl)-N²-[2,2,2-trifluor-1-[4-fluor-3-(trifluormethyl)phenyl]ethyl}-1H-indol-2,5-dicarboxamid

HPLC-MS: logP = 3,10; Masse (m/z): 547,1 (M+H)⁺; ¹HNMR (D₆-DMSO): δ 1.25-1.29 (m, 2H), 1.56-1.60 (m, 2H), 6.33-6.42 (m, 1H), 7.50 (s, 1H), 7.55 (s, 1H), 7.64-7.69 (m, 1H), 7.86 (s, 1H), 8.17 (bs, 1H), 8.28-8.30 (m, 1H), 9.28 (s, 1H), 9.67 (d, 1H), 12.03 (s, 1H).

### 6-Chlor-N⁵-(1-cyancyclopropyl)-N²-[2,2,2-trifluor-1-(3,4,5-trichlorphenyl)ethyl]-1H-indol-2,5-dicarboxamide

HPLC-MS: logP = 3,71; Masse (m/z): 562,9 (M+H)⁺; ¹HNMR (D₆-DMSO): δ 1.26-1.28 (m, 2H), 1.57-1.59 (m, 2H), 6.30-6.34 (m, 1H), 7.50 (s, 1H), 7.54 (s, 1H), 7.86 (s, 1H), 8.15 (s, 2H), 9.28 (s, 1H), 9.58 (d, 1H), 12.05 (s, 1H).

### 6-Chlor-N⁵-(1-cyancyclopropyl)-N²-[1-(3,5-dichlorphenyl)-2,2,2-trifluorethyl]-1H-indol-2,5-dicarboxamide

HPLC-MS: logP = 3,38; Masse (m/z): 529,0 (M+H)⁺; ¹HNMR (D₆-DMSO): δ 1.23-1.27 (m, 2H), 1.56-1.59 (m, 2H), 6.25-6.34 (m, 1H), 7.50 (s, 1H), 7.55 (s, 1H), 7.86 (s, 1H), 7.92 (s, 1H), 9.28 (s, 1H), 9.57 (d, 1H), 12.03 (s, 1H).

### N²-[1-(3-Brom-4-fluorphenyl)-2,2,2-trifluorethyl]-6-chlor-N⁵-(1-cyancyclopropyl)-1H-indol-2,5-dicarboxamide

### HPLC-MS: logP = 2,98; Masse (m/z): 556,9 (M+H)⁺.

### N²-[1-(3-Bromphenyl)-2,2,2-trifluorethyl]-6-chlor-N⁵-(1-cyancyclopropyl)-1H-indol-2,5-dicarboxamid

HPLC-MS: logP = 2,96; Masse (m/z): 539,0 (M+H)⁺; ¹HNMR (D₆-DMSO): δ 1.24-1.27 (m, 2H), 1.56-1.61 (m, 2H), 6.12-6.23 (m, 1H), 7.42-7.46 (m, 1H), 7.50 (s, 1H), 7.56 (s, 1H), 7.64-7.66 (m, 1H), 7.69-7.73 (m, 1H), 7.85 (s, 1H), 8.03 (s, 1H), 9.28 (s, 1H), 9.59 (d, 1H), 12.02 (s, 1H).

Stufe 3 (Variante A): *6-Chlor-N⁵-(1-cyancyclopropyl)-1-(prop-2-in-1-yl)-N²-{2,2,2-trifluor-1-[3-(trifluormethyl)phenyl]ethyl}-1H-indol-2,5-dicarboxamid (Verbindung Nr*. *57 in Tabelle1)*

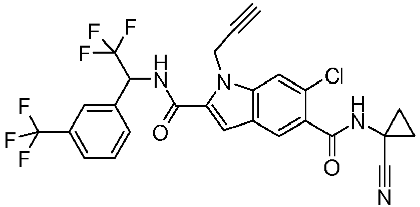

6-Chlor-N⁵-(1-cyancyclopropyl)-N²-{2,2,2-trifluor-1-[3-(trifluormethyl)phenyl]ethyl}-1H-indol-2,5-dicarboxamid (0,14 g, 0,26 mmol) wurde unter Argon bei 0 °C in N,N-Dimethylformamid (1 mL) gelöst. Es wurde Natriumhydrid (60 %ig; 0,079 g, 1,97 mmol) zugegeben und 1,5 h unter Eiskühlung nachgerührt. Anschließend wurde Propargylbromid (0,04 mg, 0,26 mmol) zugetropft. Das Reaktionsgemisch wurde innerhalb von 16 h unter Rühren aufgetaut. Es wurden Wasser und Ethylacetat zugegeben und die Phasen getrennt. Die organische Phase wurde mit gesättigter Natriumchloridlösung gewaschen, über Magnesiumsulfat getrocknet und das Lösemittel im Vakuum entfernt. Der Rückstand wird mittels präparativer HPLC gereinigt (Kromasil100, C18 250x20; Laufmittel Acetonitril in Wasser Gradient: 10-100%) und es wurden 0,075 g (50 % d.Th.) 6-Chlor-N⁵-(1-cyancyclopropyl)-1-(prop-2-in-1-yl)-N²-{2,2,2-trifluor-1-[3-(trifluormethyl)phenyl]ethyl}-1H-indol-2,5-dicarboxamid erhalten.
HPLC-MS: logP = 3,49; Masse (m/z): 567,1 (M+H)⁺; ¹HNMR (D₆-DMSO): δ 1.26-1.33 (m, 2H), 1.57-1.61 (m, 2H), 3.21 (t, 1H), 5.45 (s, 2H), 6.27-6.35 (m, 1H), 7.50 (s, 1H), 7.70-7.74 (m, 1H), 7.82-7.84 (m, 1H), 7.88-7.89 (m, 2H), 8.05-8.07 (m, 1H), 8.21 (s, 1H), 9.33 (s, 1H), 9.88 (d, 1H).

### Stufe 3 (Variante B): 6-Chlor-N⁵-(1-cyancyclopropyl)-1-cyclopropyl-N²-{2,2,2-trifluor-1-[3-(trifluormethyl)phenyl]ethyl)-1H-indol-2,5-dicarboxamid (Verbindung Nr. 302 in Tabelle 1)

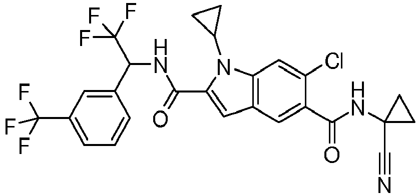

6-Chlor-N⁵-(1-cyancyclopropyl)-N²-{2,2,2-trifluor-1-[3-(trifluormethyl)phenyl]ethyl}-1H-indol-2,5-dicarboxamid (0,20 g, 0,34 mmol), Cyclopropylboronsäure (0,063 g, 0,69 mmol) und Natriumcarbonat (0,074 g, 0,69mmol) wurden als Suspension in N,N-Dimethylformamid (1 mL) vorgelegt und mit einer heißen Lösung von Kupfer(II)-acetat (0,064 g, 0,34 mmol) und 2,2'-Bipyridyl (0,054 g, 0,34 mmol) versetzt. Das Reaktionsgemisch wurde bei 70°C über 16 h gerührt. Nach dem Abkühlen wurde Salzsäure (10 mL, 1 mol/L) zugegeben und die organische Phase abgetrennt. Die wässrige Phase wurde mit Ethylacetat extrahiert (3 x) und die vereinigten organischen Phasen mit gesättigter Natriumchloridlösung gewaschen, über Magnesiumsulfat getrocknet und das Lösemittel im Vakuum entfernt. Der Rückstand wird mittels präparativer HPLC gereinigt (Kromasil100, C18 250x20; Laufmittel Acetonitril in Wasser Gradient: 10-100%) und es wurden 0,013 g (6 % d.Th.) 6-Chlor-N⁵-(1-cyancyclopropyl)-1-cyclopropyl-N'-{ 2,2,2-trifluor-1-[3-(trifluormethyl)phenyl]ethyl}-1H-indol-2,5-dicarboxamid erhalten.
HPLC-MS: logP = 3,59; Masse (m/z): 569,1 (M+H)⁺; ¹HNMR (D₆-DMSO): δ 0.58-0.72 (m, 2H), 0.99-1.04 (m, 2H), 1.25-1.29 (m, 2H), 1.56-1.60 (m, 2H), 3.47-.53 (m, 1H), 6.24-6.32 (m, 1H), 7.02 (s, 1H), 7.68 (s, 1H), 7.71-7.75 (m, 1H), 7.80-7.84 (m, 2H), 8.04-8.06 (m, 1H), 8.18 (s, 1H), 9.29 (s, 1H), 9.91 (d, 1H).

### Synthesebeispiel 7

### 3,6-Dichlor-N⁵-(1-cyancyclopropyl)-1-ethyl-N²-{2,2,2-trifluor-1-[3-(trifluormethyl)phenyl]ethyl}-1H-indol-2,5-dicarboxamid (Verbindung Nr. 271 in Tabelle 1)

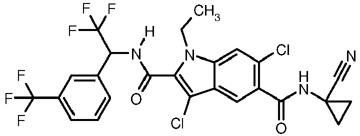

6-Chlor-N⁵-(1-cyancyclopropyl)-1-ethyl-N²-{2,2,2-trifluor-1-[3-(trifluormethyl)phenyl]ethyl}-1H-indol-2,5-dicarboxamid (0,100 g, 0,18 mmol) wird in Tetrahydrofuran (2 mL) angelöst, mit N-Chlorsuccinimid (0,024 g, 0,18 mmol) versetzt und für 48 h bei Raumtemperatur gerührt. Das Reaktionsgemisch wird dann im Vakuum eingedampft und der Rückstand mit Cyclohexan/Ethylacetat (1/1) chromatographiert und ergibt 0,104 g (98 % d.Th.) 3,6-Dichlor-N5-(1-cyancyclopropyl)-1-ethyl-N2- {2,2,2-trifluor-1-[3-(trifluormethyl)phenyl]ethyl}-1H-indol-2,5-dicarboxamid.
HPLC-MS: logP = 3,95; Masse (m/z): 591,0 (M+H)⁺; ¹HNMR (D₆-DMSO): δ 1.16 (t, 3H), 1.30-1.34 (m, 2H), 1.56-1.59 (m, 2H), 4.29 (q, 2H), 6.28-6.36 (m, 1H), 7.72-7.75 (m, 2H), 7.83-7.85 (m, 1H), 7.97 (s, 1H), 8.03-8.05 (m, 1H), 8.18 (s, 1H), 9.36 (s, 1H), 10.30 (d, 1H).

### Synthesebeispiel 8

### 6-Chlor-3-cyan-N5-(1-cyancyclopropyl)-1-ethyl-N2-12,2,2-trifluor-1-[3-(trifluormethyl)phenyl]ethyl}-1H-indol-2,5-dicarboxamid (Verbindung Nr. 279 in Tabelle 1)

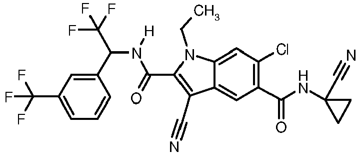

6-Chlor-N⁵-(1-cyancyclopropyl)-1-ethyl-N²-{2,2,2-trifluor-1-[3-(trifluormethyl)phenyl]ethyl}-1H-indol-2,5-dicarboxamid (0,100 g, 0,18 mmol) wird in trockenem Acetonitril (2 mL) unter Argon vorgelegt, auf 0 °C gekühlt und tropfenweise mit Chlorsulfonylisocyanat (0,028 g, 0,19 mmol) versetzt. Nach 30 min wird N,N-Dimethylformamid (0,015 g, 0,19 mmol) zugegeben und und für weitere 30 min bei 0 °C und über Nacht bei Raumtemperatur gerührt. Dann wird erneut auf 0 °C gekühlt und tropfenweise Chlorsulfonylisocyanat (0,028 g, 0,19 mmol) zugegeben, nach 30 min mit N,N-Dimethylformamid (0,015 g, 0,19 mmol) versetzt und weitere 48 h bei Raumtemperatur gerührt. Das Reaktionsgemisch wird dann im Vakuum eingedampft, der Rückstand mit Natriumhydrogencarbonat versetzt und mit Dichlormethan extrahiert. Die organische Phase wird mit Natriumsulfat getrocknet, filtriert und im Vakuum eingeengt. Der Rückstand wird mittels präparativer HPLC gereinigt (Kromasil100, C18 250x20; Laufmittel Acetonitril in Wasser Gradient: 10-100%) und es werden 0,006 g (5 % d.Th.) 6-Chlor-3-cyan-N5-(1-cyancyclopropyl)-1-ethyl-N2-{2,2,2-trifluor-1-[3-(trifluormethyl)phenyl]ethyl}-1H-indol-2,5-dicarboxamid erhalten.
HPLC-MS: logP = 3,45; Masse (m/z): 582,1 (M+H)⁺; ¹HNMR (D₆-DMSO): δ 1.22 (t, 3H), 1.33-1.36 (m, 2H), 1.56-1.60 (m, 2H), 4.38 (q, 2H), 6.31-6.40 (m, 1H), 7.71-7.75 (m, 1H), 7.83-7.85 (m, 1H), 7.92 (s, 1H), 8.03-8.05 (m, 1H), 8.13 (s, 1H), 8.20 (s, 1H), 9.40 (s, 1H), 10.64 (d, 1H).

### Synthesebeispiel 9

### N⁵-(1-Carbamothioylcyclopropyl)-6-chlor-1-ethyl-N²-{2,2,2-trifluor-1-[3-(trifluormethyl)phenyl]ethyl}-1H-indol-2,5-dicarboxamid (Verbindung Nr. 273 in Tabelle1)

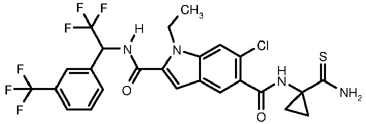

6-Chlor-N⁵-(1-cyancyclopropyl)-1-ethyl-N²-{2,2,2-trifluor-1-[3-(trifluormethyl)phenyl]ethyl}-1H-indol-2,5-dicarboxamid (0,200 g, 0,35 mmol) wird in Tetrahydrofuran (5,5 mL) unter Argon vorgelegt, mit 2,4-Bis(4-methoxyphenyl)-1,3,2,4-dithiadiphosphetan-2,4-disulfid (0,305 g, 0,75 mmol) versetzt und 48 h bei Raumtemperatur gerührt. Dann wird erneut mit 2,4-Bis(4-methoxyphenyl)-1,3,2,4-dithiadiphosphetan-2,4-disulfid (0,150 g, 0,38 mmol) versetzt und weitere 12 h bei Raumtemperatur gerührt. Das Reaktionsgemisch wird dann mit Ethylacetat verdünnt und mit einer Natriumhydrogencarbonatlösung gewaschen. Die organische Phase wird mit Magnesiumsulfat getrocknet, filtriert und im Vakuum eingeengt. Der Rückstand wird mittels präparativer HPLC gereinigt (Kromasil100, C18 250x20; Laufmittel Acetonitril in Wasser Gradient: 10-100%) und es werden 0,025 g (11 % d.Th) N⁵-(1-Carbamothioylcyclopropyl)-6-chlor-1-ethyl-N²-{2,2,2-trifluor-1-[3-(trifluormethyl)phenyl]ethyl}-1H-indol-2,5-dicarboxamid erhalten.
HPLC-MS: logP = 3,56; Masse (m/z): 591,1 (M+H)⁺; ¹HNMR (D₆-DMSO): δ 1.20 (t, 3H), 1.24-1.27 (m, 2H), 1.83-1.86 (m, 2H), 4.52 (q, 2H), 6.26-6.35 (m, 1H), 7.39 (s, 1H), 7.70-7.74 (m, 1H), 7.80-7.84 (m, 2H), 8.05-8.07 (m, 1H), 8.12 (s, 1H), 8.21 (s, 1H), 8.78 (s, 1H), 8.87 (s, 1H), 9.77-9.80 (m, 2H).

### Synthese von Aminen der Formel (III)

### 2,2,2-Trifluor-1-[4-ftuor-3-(trifluormethyl)phenyl]ethanamin

### Stufe 1: 2,2,2-Trifluor-1-[4-fluor-3-(trifluormethyl)phenyl]ethanon

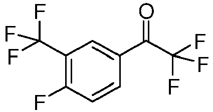

n-Butyllithium (2,5 M in Hexan, 46 mL) wurde bei -90 °C in Tetrahydrofuran (250 mL) vorgelegt. Bei - 95 °C wurde 4-Brom-1-fluor-2-(trifluormethyl)benzol (25 g, 103 mmol) zugetropft. Das Reaktionsgemisch wurde 40 min. bei -78 °C gerührt, dann auf -100 °C abgekühlt und Ethyltrifluoracetat (16,1 g, 113 mmol) zugetropft, wobei die Temperatur zwischen -90 °C und -80 °C gehalten wurde. Das Reaktionsgemisch wurde langsam auf -20 °C erwärmt und anschließend auf -80 °C abgekühlt. Es wurden 10 %ige Salzsäure und gesättigte Natriumchloridlösung zugetropft. Das Reaktionsgemisch wurde innerhalb 16 h aufgetaut und anschließend mit Diethylether extrahiert. Die organische Phase wurde mit Wasser und gesättigter Natriumchloridlösung gewaschen, über Natriumsulfat getrocknet und das Lösemittel im Vakuum entfernt. Der Rückstand wurde in Toluol aufgenommen und zunächst bei Normaldruck, dann unter Vakuum destilliert. Es wurden 22,5 g (86 % d.Th.) 2,2,2-Trifluor-1-[4-fluor-3-(trifluormethyl)phenyl]ethanon erhalten.
Kp: 99-101 °C (100 Torr)
¹H (CDCl₃): δ 7.42 (m, 1H); 8.20-8.37 (m, 2H).

### Stufe 2: 2,2,2-Trifluor-1-[4-fluor-3-(trifluormethyl)phenyl]ethanamin

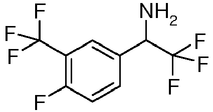

2,2,2-Trifluor-1-[4-fluor-3-(trifluormethyl)phenyl]ethanon (21,0 g, 81 mmol) wurde in Diethylether (200 mL) vorgelegt und bei 0 °C Benzylamin (9,1 g, 85 mmol) und Triethylamin (16,4 g, 162 mmol) zugegeben. Anschließend wurde eine Lösung von Titantetrachlorid (7,8 g, 41 mmol) in Hexan (100 mL) zugetropft. Das Reaktionsgemisch wurde 3 h bei Raumtemperatur nachgerührt. Die entstandene Suspension wurde filtriert, der Feststoff mit Diethylether gewaschen und verworfen. Das Lösemittel des Filtrats wurde im Vakuum entfernt und der Rückstand in Triethylamin (100 mL) aufgenommen. Die Lösung wurde 16 h bei Raumtemperatur stehen gelassen. Das Triethylamin wurde im Vakuum entfernt, der Rückstand in Dichlormethan aufgenommen und mit 10 %iger Salzsäure angesäuert. Die Mischung wurde 16 h bei Raumtemperatur gerührt und anschließend die Phasen getrennt. Die organische Phase wurde mit Wasser gewaschen und die vereinigten wässrigen Phasen mit 33 %iger Natronlauge unter Eiskühlung auf pH 12 gestellt. Die wässrige Phase wurde dreimal mit Dichlormethan extrahiert und die vereinigten organischen Phasen über Magnesiumsulfat getrocknet. Das Lösemittel wurde im Vakuum entfernt. Es wurden 6,5 g (31% d.Th.) 2,2,2-Trifluor-1-[4-fluor-3-(trifluormethyl)phenyl]ethanamin erhalten.
¹H (CDCl₃): δ 1.8 (br.s, 2H); 4.46 (q, J = 7.0 Hz, 1H); 7.22 (m, 1H); 7.64-7.72 (m, 2H).

### Analog erhalten wurden :

1-(3-Brom-4-fluorphenyl)-2,2,2-trifluorethanamin,
   ¹H (CDCl₃): δ 1.76 (br.s, 2H); 4.38 (q, 1H); 7.14 - 7.26 (m, 1H); 7.38 (m, 1H); 7.68 (m, 1H);
1-(3-Chlorphenyl)-2,2,2-trifluorethanamin,
   (CDCl₃): δ 1.75 (br.s, 2H); 4.40 (q, 1H); 7.31-7.38 (m, 3H); 7.45 (s, 1H).
1-(3,5-Dichlorphenyl)-2,2,2-trifluorethanamin,
   (CDCl₃): δ 1.77 (br.s, 2H); 4.36 (q, 1H); 7.35 - 7.39 (m, 3H).
1-(3-Chlor-5-fluorphenyl)-2,2,2-trifluorethanamin,
   ¹H (CDCl₃): δ 1.77 (br.s, 2H); 4.38 (q, 1H); 7.10 (m, 2H); 7.26 (s, 1H).
1-(3,4-Dichlorphenyl)-2,2,2-trifluorethanamin,
   ¹H (CDCl₃): δ 1.76 (br.s, 2H); 4.38 (q, 1H); 7.14 - 7.26 (m, 1H); 7.38 (m, 1H); 7.68 (m, 1H);
1-(3,5-Dichlor-4-Fluorphenyl)-2,2,2-trifluorethanamin,
   ¹H (CDCl₃): δ 1.75 (br.s, 2H); 4.36 (q, 1H); 7.50 (s, 2H).
1-(3,5-Dichlor-2,4-Difluorphenyl)-2,2,2-trifluorethanamin,
   ¹H (CDCl₃): δ 1.84 (br.s, 2H); 4.76 (q, 1H); 7.53 (m, 1H).
1-(3,4-Difluorphenyl)-2,2,2-trifluorethanamin,
   ¹H (CDCl₃): δ 1.61 (br.s, 2H); 4.40 (q, 1H); 7.18 - 7.34 (m, 3H).
1-(3,4,5-Trichlorphenyl)-2,2,2-trifluorethanamin,
   ¹H (CDCl₃): δ 1.76 (br.s, 2H); 4.36 (q, 1H); 7.50 (s, 2H).
1-(2,2-Difluor-1,3-benzodioxol-5-yl)-2,2,2-trifluorethanamin,
   ¹H (CDCl₃): δ 1.75 (br.s, 2H); 4.43 (q, 1H); 7.07 - 7.26 (m, 3H).

### Synthese von Aminen der Formel (A-9)

### Methyl-4-amino-2-ethylbenzoat und Methyl-4-amino-2-isopropylbenzoat

### Stufe1: Methyl-4-nitro-2-vinylbenzoat

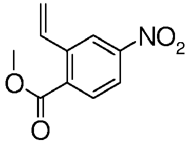

12,0 g (46,1 mmol) Methyl-2-Brom-4-nitrobenzoat wurden in 240 ml 1,2-Dimethoxyethan vorgelegt , mit 2,66 g (2,30 mmol) Tetrakis(triphenylphosphin)palladium versetzt und 20 min gerührt. Anschließend wurde eine Lösung aus 6.38g (46,1 mmol) Kaliumcarbonat in 80 ml Wasser zugesetzt und 11,11 g (46,1 mmol) 2,4,6-Trivinylcyclotriboroxan zugegeben. Das Reaktionsgemisch wurde 20 Stunden bei Rückflußtemperatur gerührt. Nach Abkühlen wurde auf Wasser gegeben, mit Ethylacetat extrahiert, die organischen Phasen mit Magnesiumsulfat getrocknet und das Lösungsmittel im Vakuum abdestilliert. Der Rückstand wurde über Kieselgel mit Cyclohexan / Ethylacetat (Verhältnis 6:1) als Laufmittel chromatographiert. Es wurden 4,5 g (46 % d. Th.) Methyl-4-nitro-2-vinylbenzoat erhalten.
HPLC/MS: logP = 2,74; Masse (m/z): 208,0 (M+1); ¹HNMR (d₆-DMSO): δ 5.75 (d, 1H), 6.01 (d, 1H), 7.2-7.3 (m, 1H), 8.00 (d, 1H), 8.20 (d, 1H), 8.42 (s, 1H).

### Auf analoge Weise erhalten wurde:

### Methyl-4-nitro-2-(prop-1-en-2-yl)benzoat

### aus Methyl-2-Brom-4-nitrobenzoat und 4,4,5,5-Tetramethyl-2-(prop-1-en-2-yl)-1,3,2-dioxaborolan

HPLC/MS: logP = 2,99; Masse (m/z): 222,1 (M+1); ¹HNMR (d₆-DMSO): δ 2.07 (s, 3H), 3.83 (s, 3H), 4.92 (s, 1H), 5.23 (m, 2H), 7.91 (d, 1H), 8.10 (s, 1H), 8,23 (d, 1H).

### Stufe 2: Methyl-4-amino-2-ethylbenzoat

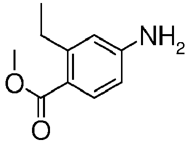

15,0 g (72,3 mmol) Methyl-4-nitro-2-vinylbenzoat in 150 ml Methanol wurden im Autoklaven bei 5 bar 15 Stunden hydriert. Die Lösung wurde über Kieselgur filtriert, das Lösungsmittel im Vakuum abdestilliert und der Rückstand über Kieselgel mit Cyclohexan / Ethylacetat (Verhältnis 3:1) als Laufmittel chromatographiert. Es wurden 2,4 g (24 % d. Th.) Methyl-4-amino-2-ethylbenzoat erhalten. HPLC/MS: logP = 1,84; Masse (m/z): 180,2 (M+1); ¹HNMR (CD₃CN): δ 1.14 (t, 3H), 2.88 (q, 2H), 3.75 (s, 3H), 4.60 (br.s, 2H), 6.45-6.50 (m, 2H), 7.68 (d, 1H).
Auf analoge Weise erhalten wurde:

### Methyl-4-amino-2-isopropylbenzoat

### durch Hydrierung von Methyl-4-nitro-2-(prop-1-en-2-yl)benzoat

HPLC/MS: logP = 2,14; Masse (m/z): 194,2 (M+1); ¹HNMR (CD₃CN): δ 1.18 (d, 6H), 3.76 (s, 3H), 3.85-3.91 (m, 1H), 4.59 (br.s, 2H), 6.44 (d, 1H), 6.67 (s, 1H), 7.61 (d, 1H).

Die in der folgenden Tabelle 1 beschriebenen erfindungsgemäßen Verbindungen der Formel (I) sind ebenfalls bevorzugte erfindungsgemäße Verbindungen, die man gemäß oder analog zu den oben beschriebenen Synthesebeispielen erhält.

**Tabelle 1**

| | | | | | | | | | | | | | |
|---|---|---|---|---|---|---|---|---|---|---|---|---|---|
| 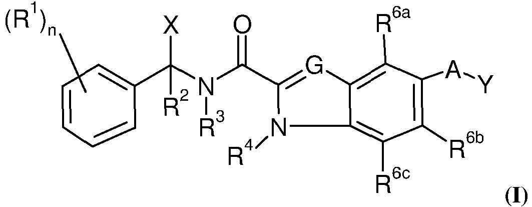 | | | | | | | | | | | | | |

| **Nr.** | **(R¹)ₙ** | **X** | **R²** | **R³** | **R⁴** | **G** | **R^{6a}** | **R^{6b}** | **R^{6c}** | **A** | **Y** | **(M+H)^{+a)}** | **log p^{a)}** |
|---|---|---|---|---|---|---|---|---|---|---|---|---|---|
| 1 | 3-CF₃ | CF₃ | H | H | CH₃ | N | H | Cl | H | CONH | Cyclopropyl | 519,1 | 3,4 |
| 2 | 3-Cl | CF₃ | H | H | Et | CH | H | Cl | H | CONH | Cyclopropyl | 498,1 | 3,65 |
| 3 | 3-CF₃;4-F | CF₃ | H | H | Et | CH | H | Cl | H | CONH | Cyclopropyl | 550,1 | 3,93 |
| 4 (Synthese -Bspl. 4) | 3-CF₃ | CF₃ | H | H | Et | N | H | Cl | H | CONH | Cyclopropyl | 533,2 | 3,67 |
| 5 | 3-Cl | CF₃ | H | H | Prop-2-in-1-yl | CH | H | Cl | H | CONH | Cyclopropyl | 508,0 | 3,45 |
| 6 | 3-CF₃;4-F | CF₃ | H | H | Prop-2-in-1-yl | CH | H | Cl | H | CONH | Cyclopropyl | 560,1 | 3,59 |
| 7 (Synthese -Bspl. 2) | 3-CF₃ | CF₃ | H | H | Et | CH | H | Cl | H | CONH | 1-Cyanocyclopropyl | 557,2 | 3,78 |
| 8 | 3-CF₃ | CF₃ | H | H | Et | CH | H | Cl | H | CONH | 1-Fluorpropan-2-yl | 552,1 | 3,88 |
| 9 | 3-CF₃ | CF₃ | H | H | Et | CH | H | Cl | H | CONH | 1-Cyanoethyl | 545,1 | 3,72 |
| 10 | 3-CF₃ | CF₃ | H | H | Et | CH | H | Cl | H | CONH | 2,2-Difluorpropan-1-yl | 570,1 | 4,06 |
| 11 | 3-CF₃ | CF₃ | H | H | Et | CH | H | Cl | H | CONH | 1,3-Difluorpropan-2-yl | 570,1 | 3,86 |
| 12 | 3-Br;4-F | CF₃ | H | H | Et | CH | H | Cl | H | CONH | Cyclopropyl | 560,1 | 3,82 |
| 13 | 3-Cl;5-Cl | CF₃ | H | H | Et | CH | H | Cl | H | CONH | Cyclopropyl | 532,0 | 4,27 |
| 14 | 3-Br;4-F | CF₃ | H | H | Prop-2-in-1-yl | CH | H | Cl | H | CONH | Cyclopropyl | 570,0 | 3,52 |
| 15 | 3-CF₃ | CF₃ | H | H | Et | CH | H | Cl | H | CONH | 2-Cyanopropan-2-yl | 559,1 | 3,89 |
| 16 | 3-CF₃;4-F | CF₃ | H | H | Et | CH | H | Cl | H | CONH | 1-Cyanocyclopropyl | 575,1 | 3,88 |
| 17 | 3-CF₃;4-F | CF₃ | H | H | Et | CH | H | Cl | H | CONH | Et | 538,1 | 3,91 |
| 18 | 3-Cl;4-F | CF₃ | H | H | Et | CH | H | Cl | H | CONH | Cyclopropyl | 516,1 | 3,82 |
| 19 (Synthese -Bspl. 1) | 3-CF₃;4-F | CF₃ | H | H | Et | CH | H | Cl | H | CONH | 2,2-Difluorethyl | 574,0 | 4,03 |
| 20 | 3-Cl;4-F | CF₃ | H | H | Et | CH | H | Cl | H | CONH | 1-Cyanocyclopropyl | 541,1 | 3,79 |
| 21 | 3-CF₃ | CF₃ | H | H | Et | CH | H | Cl | H | CONH | Cyanomethyl | 531,0 | 3,64 |
| 22 | 3-CF₃ | CF₃ | H | H | Et | CH | H | Cl | H | CONH | 1-Methylcyclopropyl | 546,2 | 4,16 |
| 23 | 3-CF₃ | CF₃ | H | H | Et | CH | H | CF₃ | H | CH₂NH CO | Cyclopropyl | 580,3 | 4,37 |
| 24 | 3-CF₃ | CF₃ | H | H | Et | CH | H | Cl | H | CH₂NH CO | Cyclopropyl | 546,2 | 4,19 |
| 25 | 3-CF₃ | CF₃ | H | H | Et | CH | H | Cl | H | CH₂NH CO | Et | 534,1 | 4,06 |
| 26 (Synthese -Bspl. 3) | 3-CF₃ | CF₃ | H | H | Et | CH | H | Cl | H | CH₂NH CO | CH₃ | 520,2 | 3,77 |
| 27 | 3-CF₃;4-F | CF₃ | H | H | Et | CH | H | H | H | CONH | Cyclopropyl | 550,2 | 3,74 |
| 28 | 3-CF₃ | CF₃ | H | H | Et | CH | H | Cl | H | CONH | 1-(Methoxycarbonyl)-cyclopropyl | 590,2 | 3,86 |
| 29 | 3-CF₃ | CF₃ | H | H | Et | CH | H | Cl | H | CONH | 1,1'-Bi(Cyclopropyl)-1-yl | 572,2 | 4,45 |
| 30 | 3-CF₃ | CF₃ | H | H | Et | CH | H | Cl | H | CONH | 1-Ethylcyclopropyl | 560,1 | 4,45 |
| 31 | 3-CF₃ | CF₃ | H | H | Et | CH | H | Cl | H | CONH | 1-Ethoxycyclopropyl | 576,2 | 4,19 |
| 32 | 3-CF₃ | CF₃ | H | H | Et | CH | H | Cl | H | CONH | 1-Ethinylcyclopropyl | 556,4 | 3,86 |
| 33 | 3-CF₃ | CF₃ | H | H | Et | CH | H | Cl | H | CONH | 1-Ethinylcyclobutyl | 570,4 | 4,20 |
| 34 | 3-CF₃ | CF₃ | H | H | Et | CH | H | Cl | H | CONH | 1-(Ethoxycarbonyl) cyclopropyl | 604,4 | 3,99 |
| 35 | 3-CF₃ | CF₃ | H | H | Et | CH | H | Cl | H | CONH | 3-Cyanopropan-1-yl | | |
| 36 | 3-CF₃ | CF₃ | H | H | Et | CH | H | Cl | H | CONH | 1-Cyanocyclobutyl | 571,2 | 4,09 |
| 37 | 3-Cl;4-F | CF₃ | H | H | Et | N | H | Cl | H | CONH | Cyclopropyl | 517,1 | 3,59 |
| 38 | 3-CF₃;4-F | CF₃ | H | H | Et | N | H | Cl | H | CONH | Cyclopropyl | 551,1 | 3,71 |
| 39 | 3-Cl | CF₃ | H | H | Et | CH | H | Cl | H | CONH | 1-Cyanocyclopropyl | 523,1 | 3,67 |
| 40 | 3-Br | CF₃ | H | H | Et | CH | H | Cl | H | CONH | Cyclopropyl | 542,0 | 3,71 |
| 41 | 3-Br | CF₃ | H | H | Et | CH | H | Cl | H | CONH | 1-Cyanocyclopropyl | 567,0 | 3,75 |
| 42 | 3-Br;4-F | CF₃ | H | H | Et | CH | H | Cl | H | CONH | 1-Cyanocyclopropyl | 585,0 | 3,76 |
| 43 | 3-Cl;5-Cl | CF₃ | H | H | Et | CH | H | Cl | H | CONH | 1-Cyanocyclopropyl | 557,0 | 4,14 |
| 44 | 3-CF₃ | CF₃ | H | H | Me | CH | H | Cl | H | CONH | 1-Cyanocyclopropyl | 543,1 | 3,55 |
| 45 | 3-Cl | CF₃ | H | H | Me | CH | H | Cl | H | CONH | Cyclopropyl | 484,1 | 3,33 |
| 46 | 3-Cl | CF₃ | H | H | Me | CH | H | Cl | H | CONH | 1-Cyanocyclopropyl | 509,0 | 3,31 |
| 47 | 3-Br | CF₃ | H | H | Me | CH | H | Cl | H | CONH | Cyclopropyl | 528,0 | 3,42 |
| 48 | 3-Br | CF₃ | H | H | Me | CH | H | Cl | H | CONH | 1-Cyanocyclopropyl | 553,0 | 3,39 |
| 49 | 3-CF₃;4-F | CF₃ | H | H | Me | CH | H | Cl | H | CONH | Cyclopropyl | 536,1 | 3,70 |
| 50 | 3-CF₃;4-F | CF₃ | H | H | Me | CH | H | Cl | H | CONH | 1-Cyanocyclopropyl | 561,1 | 3,52 |
| 51 | 3-Br;4-F | CF₃ | H | H | Me | CH | H | Cl | H | CONH | Cyclopropyl | 546,0 | 3,61 |
| 52 | 3-Br;4-F | CF₃ | H | H | Me | CH | H | Cl | H | CONH | 1-Cyanocyclopropyl | 570,9 | 3,44 |
| 53 | 3-Cl;4-F | CF₃ | H | H | Me | CH | H | Cl | H | CONH | Cyclopropyl | 502,1 | 3,42 |
| 54 | 3-Cl;4-F | CF₃ | H | H | Me | CH | H | Cl | H | CONH | 1-Cyanocyclopropyl | 527,0 | 3,52 |
| 55 | 3-Cl;5-Cl | CF₃ | H | H | Me | CH | H | Cl | H | CONH | Cyclopropyl | | |
| 56 | 3-Cl;5-Cl | CF₃ | H | H | Me | CH | H | Cl | H | CONH | 1-Cyanocyclopropyl | | |
| 57 (Synthese Bspl. 6) | 3-CF₃ | CF₃ | H | H | Prop-2-in-1-yl | CH | H | Cl | H | CONH | 1-Cyanocyclopropyl | 567,1 | 3,49 |
| 58 | 3-Cl | CF₃ | H | H | Prop-2-in-1-yl | CH | H | Cl | H | CONH | 1-Cyanocyclopropyl | 533,0 | 3,42 |
| 59 | 3-Br | CF₃ | H | H | Prop-2-in-1-yl | CH | H | Cl | H | CONH | Cyclopropyl | 552,0 | 3,44 |
| 60 | 3-Br | CF₃ | H | H | Prop-2-in-1-yl | CH | H | Cl | H | CONH | 1-Cyanocyclopropyl | 577,0 | 3,48 |
| 61 | 3-CF₃;4-F | CF₃ | H | H | Prop-2-in-1-yl | CH | H | Cl | H | CONH | 1-Cyanocyclopropyl | 585,1 | 3,65 |
| 62 | 3-Br;4-F | CF₃ | H | H | Prop-2-in-1-yl | CH | H | Cl | H | CONH | 1-Cyanocyclopropyl | 595,0 | 3,57 |
| 63 | 3-Cl;4-F | CF₃ | H | H | Prop-2-in-1-yl | CH | H | Cl | H | CONH | Cyclopropyl | 526,1 | 3,57 |
| 64 | 3-Cl;4-F | CF₃ | H | H | Prop-2-in-1-yl | CH | H | Cl | H | CONH | 1-Cyanocyclopropyl | 551,1 | 3,41 |
| 65 | 3-Cl;5-Cl | CF₃ | H | H | Prop-2-in-1-yl | CH | H | Cl | H | CONH | Cyclopropyl | 542,0 | 3,90 |
| 66 | 3-Cl;5-Cl | CF₃ | H | H | Prop-2-in-1-yl | CH | H | Cl | H | CONH | 1-Cyanocyclopropyl | 567,0 | 3,87 |
| 67 | 3-CF₃ | CF₃ | H | H | Et | N | H | Cl | H | CONH | 1-Cyanocyclopropyl | | |
| 68 | 3-Cl | CF₃ | H | H | Et | N | H | Cl | H | CONH | Cyclopropyl | | |
| 69 | 3-Cl | CF₃ | H | H | Et | N | H | Cl | H | CONH | 1-Cyanocyclopropyl | | |
| 70 | 3-Br | CF₃ | H | H | Et | N | H | Cl | H | CONH | Cyclopropyl | | |
| 71 | 3-Br | CF₃ | H | H | Et | N | H | Cl | H | CONH | 1-Cyanocyclopropyl | | |
| 72 | 3-CF₃;4-F | CF₃ | H | H | Et | N | H | Cl | H | CONH | 1-Cyanocyclopropyl | | |
| 73 | 3-Br;4-F | CF₃ | H | H | Et | N | H | Cl | H | CONH | Cyclopropyl | | |
| 74 | 3-Br;4-F | CF₃ | H | H | Et | N | H | Cl | H | CONH | 1-Cyanocyclopropyl | | |
| 75 | 3-Cl;4-F | CF₃ | H | H | Et | N | H | Cl | H | CONH | 1-Cyanocyclopropyl | | |
| 76 | 3-Cl;5-Cl | CF₃ | H | H | Et | N | H | Cl | H | CONH | Cyclopropyl | | |
| 77 | 3-Cl;5-Cl | CF₃ | H | H | Et | N | H | Cl | H | CONH | 1-Cyanocyclopropyl | | |
| 78 | 3-CF₃ | CF₃ | H | H | Me | N | H | Cl | H | CONH | 1-Cyanocyclopropyl | | |
| 79 | 3-Cl | CF₃ | H | H | Me | N | H | Cl | H | CONH | Cyclopropyl | | |
| 80 | 3-Cl | CF₃ | H | H | Me | N | H | Cl | H | CONH | 1-Cyanocyclopropyl | | |
| 81 | 3-Br | CF₃ | H | H | Me | N | H | Cl | H | CONH | Cyclopropyl | | |
| 82 | 3-Br | CF₃ | H | H | Me | N | H | Cl | H | CONH | 1-Cyanocyclopropyl | | |
| 83 | 3-CF₃;4-F | CF₃ | H | H | Me | N | H | Cl | H | CONH | Cyclopropyl | | |
| 84 | 3-CF₃;4-F | CF₃ | H | H | Me | N | H | Cl | H | CONH | 1-Cyanocyclopropyl | | |
| 85 | 3-Br;4-F | CF₃ | H | H | Me | N | H | Cl | H | CONH | Cyclopropyl | | |
| 86 | 3-Br;4-F | CF₃ | H | H | Me | N | H | Cl | H | CONH | 1-Cyanocyclopropyl | | |
| 87 | 3-Cl;4-F | CF₃ | H | H | Me | N | H | Cl | H | CONH | Cyclopropyl | | |
| 88 | 3-Cl;4-F | CF₃ | H | H | Me | N | H | Cl | H | CONH | 1-Cyanocyclopropyl | | |
| 89 | 3-Cl;5-Cl | CF₃ | H | H | Me | N | H | Cl | H | CONH | Cyclopropyl | | |
| 90 | 3-Cl;5-Cl | CF₃ | H | H | Me | N | H | Cl | H | CONH | 1-Cyanocyclopropyl | | |
| 91 | 3-CF₃ | CF₃ | H | H | Prop-2-in-1-yl | N | H | Cl | H | CONH | Cyclopropyl | 543,1 | 3,55 |
| 92 | 3-CF₃ | CF₃ | H | H | Prop-2-in-1-yl | N | H | Cl | H | CONH | 1-Cyanocyclopropyl | | |
| 93 | 3-Cl | CF₃ | H | H | Prop-2-in-1-yl | N | H | Cl | H | CONH | Cyclopropyl | | |
| 94 | 3-Cl | CF₃ | H | H | Prop-2-in-1-yl | N | H | Cl | H | CONH | 1-Cyanocyclopropyl | | |
| 95 | 3-Br | CF₃ | H | H | Prop-2-in-1-yl | N | H | Cl | H | CONH | Cyclopropyl | | |
| 96 | 3-Br | CF₃ | H | H | Prop-2-in-1-yl | N | H | Cl | H | CONH | 1-Cyanocyclopropyl | | |
| 97 | 3-CF₃;4-F | CF₃ | H | H | Prop-2-in-1-yl | N | H | Cl | H | CONH | Cyclopropyl | 561,0 | 3,62 |
| 98 | 3-CF₃;4-F | CF₃ | H | H | Prop-2-in-1-yl | N | H | Cl | H | CONH | 1-Cyanocyclopropyl | | |
| 99 | 3-Br;4-F | CF₃ | H | H | Prop-2-in-1-yl | N | H | Cl | H | CONH | Cyclopropyl | | |
| 100 | 3-Br;4-F | CF₃ | H | H | Prop-2-in-1-yl | N | H | Cl | H | CONH | 1-Cyanocyclopropyl | | |
| 101 | 3-Cl;4-F | CF₃ | H | H | Prop-2-in-1-yl | N | H | Cl | H | CONH | Cyclopropyl | 527,0 | 3,50 |
| 102 | 3-Cl;4-F | CF₃ | H | H | Prop-2-in-1-yl | N | H | Cl | H | CONH | 1-Cyanocyclopropyl | | |
| 103 | 3-Cl;5-Cl | CF₃ | H | H | Prop-2-in-1-yl | N | H | Cl | H | CONH | Cyclopropyl | | |
| 104 | 3-Cl;5-Cl | CF₃ | H | H | Prop-2-in-1-yl | N | H | Cl | H | CONH | 1-Cyanocyclopropyl | | |
| 105 | 3-CF₃ | CF₃ | H | H | Et | CH | H | F | H | CONH | 1-Cyanocyclopropyl | | |
| 106 | 3-Cl | CF₃ | H | H | Et | CH | H | F | H | CONH | Cyclopropyl | | |
| 107 | 3-Cl | CF₃ | H | H | Et | CH | H | F | H | CONH | 1-Cyanocyclopropyl | | |
| 108 | 3-Br | CF₃ | H | H | Et | CH | H | F | H | CONH | Cyclopropyl | | |
| 109 | 3-Br | CF₃ | H | H | Et | CH | H | F | H | CONH | 1-Cyanocyclopropyl | | |
| 110 | 3-CF₃;4-F | CF₃ | H | H | Et | CH | H | F | H | CONH | Cyclopropyl | | |
| 111 | 3-CF₃;4-F | CF₃ | H | H | Et | CH | H | F | H | CONH | 1-Cyanocyclopropyl | | |
| 112 | 3-Br;4-F | CF₃ | H | H | Et | CH | H | F | H | CONH | Cyclopropyl | | |
| 113 | 3-Br;4-F | CF₃ | H | H | Et | CH | H | F | H | CONH | 1-Cyanocyclopropyl | | |
| 114 | 3-Cl;4-F | CF₃ | H | H | Et | CH | H | F | H | CONH | Cyclopropyl | | |
| 115 | 3-Cl;4-F | CF₃ | H | H | Et | CH | H | F | H | CONH | 1-Cyanocyclopropyl | | |
| 116 | 3-Cl;5-Cl | CF₃ | H | H | Et | CH | H | F | H | CONH | Cyclopropyl | | |
| 117 | 3-Cl;5-Cl | CF₃ | H | H | Et | CH | H | F | H | CONH | 1-Cyanocyclopropyl | | |
| 118 | 3-CF₃ | CF₃ | H | H | Me | CH | H | F | H | CONH | 1-Cyanocyclopropyl | | |
| 119 | 3-Cl | CF₃ | H | H | Me | CH | H | F | H | CONH | Cyclopropyl | | |
| 120 | 3-Cl | CF₃ | H | H | Me | CH | H | F | H | CONH | 1-Cyanocyclopropyl | | |
| 121 | 3-Br | CF₃ | H | H | Me | CH | H | F | H | CONH | Cyclopropyl | | |
| 122 | 3-Br | CF₃ | H | H | Me | CH | H | F | H | CONH | 1-Cyanocyclopropyl | | |
| 123 | 3-CF₃;4-F | CF₃ | H | H | Me | CH | H | F | H | CONH | Cyclopropyl | | |
| 124 | 3-CF₃;4-F | CF₃ | H | H | Me | CH | H | F | H | CONH | 1-Cyanocyclopropyl | | |
| 125 | 3-Br;4-F | CF₃ | H | H | Me | CH | H | F | H | CONH | Cyclopropyl | | |
| 126 | 3-Br;4-F | CF₃ | H | H | Me | CH | H | F | H | CONH | 1-Cyanocyclopropyl | | |
| 127 | 3-Cl;4-F | CF₃ | H | H | Me | CH | H | F | H | CONH | Cyclopropyl | | |
| 128 | 3-Cl;4-F | CF₃ | H | H | Me | CH | H | F | H | CONH | 1-Cyanocyclopropyl | | |
| 129 | 3-Cl;5-Cl | CF₃ | H | H | Me | CH | H | F | H | CONH | Cyclopropyl | | |
| 130 | 3-Cl;5-Cl | CF₃ | H | H | Me | CH | H | F | H | CONH | 1-Cyanocyclopropyl | | |
| 131 | 3-CF₃ | CF₃ | H | H | Prop-2-in-1-yl | CH | H | F | H | CONH | 1-Cyanocyclopropyl | | |
| 132 | 3-Cl | CF₃ | H | H | Prop-2-in-1-yl | CH | H | F | H | CONH | Cyclopropyl | | |
| 133 | 3-Cl | CF₃ | H | H | Prop-2-in-1-yl | CH | H | F | H | CONH | 1-Cyanocyclopropyl | | |
| 134 | 3-Br | CF₃ | H | H | Prop-2-in-1-yl | CH | H | F | H | CONH | Cyclopropyl | | |
| 135 | 3-Br | CF₃ | H | H | Prop-2-in-1-yl | CH | H | F | H | CONH | 1-Cyanocyclopropyl | | |
| 136 | 3-CF₃;4-F | CF₃ | H | H | Prop-2-in-1-yl | CH | H | F | H | CONH | Cyclopropyl | | |
| 137 | 3-CF₃;4-F | CF₃ | H | H | Prop-2-in-1-yl | CH | H | F | H | CONH | 1-Cyanocyclopropyl | | |
| 138 | 3-Br;4-F | CF₃ | H | H | Prop-2-in-1-yl | CH | H | F | H | CONH | Cyclopropyl | | |
| 139 | 3-Br;4-F | CF₃ | H | H | Prop-2-in-1-yl | CH | H | F | H | CONH | 1-Cyanocyclopropyl | | |
| 140 | 3-Cl;4-F | CF₃ | H | H | Prop-2-in-1-yl | CH | H | F | H | CONH | Cyclopropyl | | |
| 141 | 3-Cl;4-F | CF₃ | H | H | Prop-2-in-1-yl | CH | H | F | H | CONH | 1-Cyanocyclopropyl | | |
| 142 | 3-Cl;5-Cl | CF₃ | H | H | Prop-2-in-1-yl | CH | H | F | H | CONH | Cyclopropyl | | |
| 143 | 3-Cl;5-Cl | CF₃ | H | H | Prop-2-in-1-yl | CH | H | F | H | CONH | 1-Cyanocyclopropyl | | |
| 144 | 3-CF₃ | CF₃ | H | H | Et | CH | H | Br | H | CONH | 1-Cyanocyclopropyl | | |
| 145 | 3-Cl | CF₃ | H | H | Et | CH | H | Br | H | CONH | Cyclopropyl | | |
| 146 | 3-Cl | CF₃ | H | H | Et | CH | H | Br | H | CONH | 1-Cyanocyclopropyl | | |
| 147 | 3-Br | CF₃ | H | H | Et | CH | H | Br | H | CONH | Cyclopropyl | | |
| 148 | 3-Br | CF₃ | H | H | Et | CH | H | Br | H | CONH | 1-Cyanocyclopropyl | | |
| 149 | 3-CF₃;4-F | CF₃ | H | H | Et | CH | H | Br | H | CONH | Cyclopropyl | | |
| 150 | 3-CF₃;4-F | CF₃ | H | H | Et | CH | H | Br | H | CONH | 1-Cyanocyclopropyl | | |
| 151 | 3-Br;4-F | CF₃ | H | H | Et | CH | H | Br | H | CONH | Cyclopropyl | | |
| 152 | 3-Br;4-F | CF₃ | H | H | Et | CH | H | Br | H | CONH | 1-Cyanocyclopropyl | | |
| 153 | 3-Cl;4-F | CF₃ | H | H | Et | CH | H | Br | H | CONH | Cyclopropyl | | |
| 154 | 3-Cl;4-F | CF₃ | H | H | Et | CH | H | Br | H | CONH | 1-Cyanocyclopropyl | | |
| 155 | 3-Cl;5-Cl | CF₃ | H | H | Et | CH | H | Br | H | CONH | Cyclopropyl | | |
| 156 | 3-Cl;5-Cl | CF₃ | H | H | Et | CH | H | Br | H | CONH | 1-Cyanocyclopropyl | | |
| 157 | 3-CF₃ | CF₃ | H | H | Me | CH | H | Br | H | CONH | 1-Cyanocyclopropyl | | |
| 158 | 3-Cl | CF₃ | H | H | Me | CH | H | Br | H | CONH | Cyclopropyl | | |
| 159 | 3-Cl | CF₃ | H | H | Me | CH | H | Br | H | CONH | 1-Cyanocyclopropyl | | |
| 160 | 3-Br | CF₃ | H | H | Me | CH | H | Br | H | CONH | Cyclopropyl | | |
| 161 | 3-Br | CF₃ | H | H | Me | CH | H | Br | H | CONH | 1-Cyanocyclopropyl | | |
| 162 | 3-CF₃;4-F | CF₃ | H | H | Me | CH | H | Br | H | CONH | Cyclopropyl | | |
| 163 | 3-CF₃;4-F | CF₃ | H | H | Me | CH | H | Br | H | CONH | 1-Cyanocyclopropyl | | |
| 164 | 3-Br;4-F | CF₃ | H | H | Me | CH | H | Br | H | CONH | Cyclopropyl | | |
| 165 | 3-Br;4-F | CF₃ | H | H | Me | CH | H | Br | H | CONH | 1-Cyanocyclopropyl | | |
| 166 | 3-Cl;4-F | CF₃ | H | H | Me | CH | H | Br | H | CONH | Cyclopropyl | | |
| 167 | 3-Cl;4-F | CF₃ | H | H | Me | CH | H | Br | H | CONH | 1-Cyanocyclopropyl | | |
| 168 | 3-Cl;5-Cl | CF₃ | H | H | Me | CH | H | Br | H | CONH | Cyclopropyl | | |
| 169 | 3-Cl;5-Cl | CF₃ | H | H | Me | CH | H | Br | H | CONH | 1-Cyanocyclopropyl | | |
| 170 | 3-CF₃ | CF₃ | H | H | Prop-2-in-1-yl | CH | H | Br | H | CONH | 1-Cyanocyclopropyl | | |
| 171 | 3-Cl | CF₃ | H | H | Prop-2-in-1-yl | CH | H | Br | H | CONH | Cyclopropyl | | |
| 172 | 3-Cl | CF₃ | H | H | Prop-2-in-1-yl | CH | H | Br | H | CONH | 1-Cyanocyclopropyl | | |
| 173 | 3-Br | CF₃ | H | H | Prop-2-in-1-yl | CH | H | Br | H | CONH | Cyclopropyl | | |
| 174 | 3-Br | CF₃ | H | H | Prop-2-in-1-yl | CH | H | Br | H | CONH | 1-Cyanocyclopropyl | | |
| 175 | 3-CF₃;4-F | CF₃ | H | H | Prop-2-in-1-yl | CH | H | Br | H | CONH | Cyclopropyl | | |
| 176 | 3-CF₃;4-F | CF₃ | H | H | Prop-2-in-1-yl | CH | H | Br | H | CONH | 1-Cyanocyclopropyl | | |
| 177 | 3-Br;4-F | CF₃ | H | H | Prop-2-in-1-yl | CH | H | Br | H | CONH | Cyclopropyl | | |
| 178 | 3-Br;4-F | CF₃ | H | H | Prop-2-in-1-yl | CH | H | Br | H | CONH | 1-Cyanocyclopropyl | | |
| 179 | 3-Cl;4-F | CF₃ | H | H | Prop-2-in-1-yl | CH | H | Br | H | CONH | Cyclopropyl | | |
| 180 | 3-Cl;4-F | CF₃ | H | H | Prop-2-in-1-yl | CH | H | Br | H | CONH | 1-Cyanocyclopropyl | | |
| 181 | 3-Cl;5-Cl | CF₃ | H | H | Prop-2-in-1-yl | CH | H | Br | H | CONH | Cyclopropyl | | |
| 182 | 3-Cl;5-Cl | CF₃ | H | H | Prop-2-in-1-yl | CH | H | Br | H | CONH | 1-Cyanocyclopropyl | | |
| 183 | 3-CF₃ | CF₃ | H | H | Et | CH | H | Me | H | CONH | 1-Cyanocyclopropyl | 537,1 | 3,75 |
| 184 | 3-Cl | CF₃ | H | H | Et | CH | H | Me | H | CONH | Cyclopropyl | 478,1 | 3,57 |
| 185 | 3-Cl | CF₃ | H | H | Et | CH | H | Me | H | CONH | 1-Cyanocyclopropyl | 503,1 | 3,58 |
| 186 | 3-Br | CF₃ | H | H | Et | CH | H | Me | H | CONH | Cyclopropyl | | |
| 187 | 3-Br | CF₃ | H | H | Et | CH | H | Me | H | CONH | 1-Cyanocyclopropyl | | |
| 188 | 3-CF₃;4-F | CF₃ | H | H | Et | CH | H | Me | H | CONH | Cyclopropyl | 530,1 | 3,76 |
| 189 | 3-CF₃;4-F | CF₃ | H | H | Et | CH | H | Me | H | CONH | 1-Cyanocyclopropyl | 555,1 | 3,77 |
| 190 | 3-Br;4-F | CF₃ | H | H | Et | CH | H | Me | H | CONH | Cyclopropyl | 540,1 | 3,70 |
| 191 | 3-Br;4-F | CF₃ | H | H | Et | CH | H | Me | H | CONH | 1-Cyanocyclopropyl | 565,1 | 3,71 |
| 192 | 3-Cl;4-F | CF₃ | H | H | Et | CH | H | Me | H | CONH | Cyclopropyl | 496,1 | 3,65 |
| 193 | 3-Cl;4-F | CF₃ | H | H | Et | CH | H | Me | H | CONH | 1-Cyanocyclopropyl | 521,1 | 3,66 |
| 194 | 3-Cl;5-Cl | CF₃ | H | H | Et | CH | H | Me | H | CONH | Cyclopropyl | | |
| 195 | 3-Cl;5-Cl | CF₃ | H | H | Et | CH | H | Me | H | CONH | 1-Cyanocyclopropyl | 537,1 | 4,09 |
| 196 | 3-CF₃ | CF₃ | H | H | Me | CH | H | Me | H | CONH | 1-Cyanocyclopropyl | 523,1 | 3,49 |
| 197 | 3-Cl | CF₃ | H | H | Me | CH | H | Me | H | CONH | Cyclopropyl | | |
| 198 | 3-Cl | CF₃ | H | H | Me | CH | H | Me | H | CONH | 1-Cyanocyclopropyl | | |
| 199 | 3-Br | CF₃ | H | H | Me | CH | H | Me | H | CONH | Cyclopropyl | | |
| 200 | 3-Br | CF₃ | H | H | Me | CH | H | Me | H | CONH | 1-Cyanocyclopropyl | | |
| 201 | 3-CF₃;4-F | CF₃ | H | H | Me | CH | H | Me | H | CONH | Cyclopropyl | | |
| 202 | 3-CF₃;4-F | CF₃ | H | H | Me | CH | H | Me | H | CONH | 1-Cyanocyclopropyl | | |
| 203 | 3-Br;4-F | CF₃ | H | H | Me | CH | H | Me | H | CONH | Cyclopropyl | | |
| 204 | 3-Br;4-F | CF₃ | H | H | Me | CH | H | Me | H | CONH | 1-Cyanocyclopropyl | | |
| 205 | 3-Cl;4-F | CF₃ | H | H | Me | CH | H | Me | H | CONH | Cyclopropyl | | |
| 206 | 3-Cl;4-F | CF₃ | H | H | Me | CH | H | Me | H | CONH | 1-Cyanocyclopropyl | | |
| 207 | 3-Cl;5-Cl | CF₃ | H | H | Me | CH | H | Me | H | CONH | Cyclopropyl | | |
| 208 | 3-Cl;5-Cl | CF₃ | H | H | Me | CH | H | Me | H | CONH | 1-Cyanocyclopropyl | | |
| 209 | 3-CF₃ | CF₃ | H | H | Prop-2-in-1-yl | CH | H | Me | H | CONH | 1-Cyanocyclopropyl | 547,1 | 3,48 |
| 210 | 3-Cl | CF₃ | H | H | Prop-2-in-1-yl | CH | H | Me | H | CONH | Cyclopropyl | | |
| 211 | 3-Cl | CF₃ | H | H | Prop-2-in-1-yl | CH | H | Me | H | CONH | 1-Cyanocyclopropyl | | |
| 212 | 3-Br | CF₃ | H | H | Prop-2-in-1-yl | CH | H | Me | H | CONH | Cyclopropyl | | |
| 213 | 3-Br | CF₃ | H | H | Prop-2-in-1-yl | CH | H | Me | H | CONH | 1-Cyanocyclopropyl | | |
| 214 | 3-CF₃;4-F | CF₃ | H | H | Prop-2-in-1-yl | CH | H | Me | H | CONH | Cyclopropyl | | |
| 215 | 3-CF₃;4-F | CF₃ | H | H | Prop-2-in-1-yl | CH | H | Me | H | CONH | 1-Cyanocyclopropyl | | |
| 216 | 3-Br;4-F | CF₃ | H | H | Prop-2-in-1-yl | CH | H | Me | H | CONH | Cyclopropyl | | |
| 217 | 3-Br;4-F | CF₃ | H | H | Prop-2-in-1-yl | CH | H | Me | H | CONH | 1-Cyanocyclopropyl | | |
| 218 | 3-Cl;4-F | CF₃ | H | H | Prop-2-in-1-yl | CH | H | Me | H | CONH | Cyclopropyl | | |
| 219 | 3-Cl;4-F | CF₃ | H | H | Prop-2-in-1-yl | CH | H | Me | H | CONH | 1-Cyanocyclopropyl | | |
| 220 | 3-Cl;5-Cl | CF₃ | H | H | Prop-2-in-1-yl | CH | H | Me | H | CONH | Cyclopropyl | | |
| 221 | 3-Cl;5-Cl | CF₃ | H | H | Prop-2-in-1-yl | CH | H | Me | H | CONH | 1-Cyanocyclopropyl | | |
| 222 | 3-CF₃ | CF₃ | H | H | Et | CH | H | CF₃ | H | CONH | 1-Cyanocyclopropyl | | |
| 223 | 3-Cl | CF₃ | H | H | Et | CH | H | CF₃ | H | CONH | Cyclopropyl | | |
| 224 | 3-Cl | CF₃ | H | H | Et | CH | H | CF₃ | H | CONH | 1-Cyanocyclopropyl | | |
| 225 | 3-Br | CF₃ | H | H | Et | CH | H | CF₃ | H | CONH | Cyclopropyl | | |
| 226 | 3-Br | CF₃ | H | H | Et | CH | H | CF₃ | H | CONH | 1-Cyanocyclopropyl | | |
| 227 | 3-CF₃;4-F | CF₃ | H | H | Et | CH | H | CF₃ | H | CONH | Cyclopropyl | | |
| 228 | 3-CF₃;4-F | CF₃ | H | H | Et | CH | H | CF₃ | H | CONH | 1-Cyanocyclopropyl | | |
| 229 | 3-Br;4-F | CF₃ | H | H | Et | CH | H | CF₃ | H | CONH | Cyclopropyl | | |
| 230 | 3-Br;4-F | CF₃ | H | H | Et | CH | H | CF₃ | H | CONH | 1-Cyanocyclopropyl | | |
| 231 | 3-Cl;4-F | CF₃ | H | H | Et | CH | H | CF₃ | H | CONH | Cyclopropyl | | |
| 232 | 3-Cl;4-F | CF₃ | H | H | Et | CH | H | CF₃ | H | CONH | 1-Cyanocyclopropyl | | |
| 233 | 3-Cl;5-Cl | CF₃ | H | H | Et | CH | H | CF₃ | H | CONH | Cyclopropyl | | |
| 234 | 3-Cl;5-Cl | CF₃ | H | H | Et | CH | H | CF₃ | H | CONH | 1-Cyanocyclopropyl | | |
| 235 | 3-CF₃ | CF₃ | H | H | Me | CH | H | CF₃ | H | CONH | 1-Cyanocyclopropyl | | |
| 236 | 3-Cl | CF₃ | H | H | Me | CH | H | CF₃ | H | CONH | Cyclopropyl | | |
| 237 | 3-Cl | CF₃ | H | H | Me | CH | H | CF₃ | H | CONH | 1-Cyanocyclopropyl | | |
| 238 | 3-Br | CF₃ | H | H | Me | CH | H | CF₃ | H | CONH | Cyclopropyl | | |
| 239 | 3-Br | CF₃ | H | H | Me | CH | H | CF₃ | H | CONH | 1-Cyanocyclopropyl | | |
| 240 | 3-CF₃;4-F | CF₃ | H | H | Me | CH | H | CF₃ | H | CONH | Cyclopropyl | | |
| 241 | 3-CF₃;4-F | CF₃ | H | H | Me | CH | H | CF₃ | H | CONH | 1-Cyanocyclopropyl | | |
| 242 | 3-Br;4-F | CF₃ | H | H | Me | CH | H | CF₃ | H | CONH | Cyclopropyl | | |
| 243 | 3-Br;4-F | CF₃ | H | H | Me | CH | H | CF₃ | H | CONH | 1-Cyanocyclopropyl | | |
| 244 | 3-Cl;4-F | CF₃ | H | H | Me | CH | H | CF₃ | H | CONH | Cyclopropyl | | |
| 245 | 3-Cl;4-F | CF₃ | H | H | Me | CH | H | CF₃ | H | CONH | 1-Cyanocyclopropyl | | |
| 246 | 3-Cl;5-Cl | CF₃ | H | H | Me | CH | H | CF₃ | H | CONH | Cyclopropyl | | |
| 247 | 3-Cl;5-Cl | CF₃ | H | H | Me | CH | H | CF₃ | H | CONH | 1-Cyanocyclopropyl | | |
| 248 | 3-CF₃ | CF₃ | H | H | Prop-2-in-1-yl | CH | H | CF₃ | H | CONH | 1-Cyanocyclopropyl | | |
| 249 | 3-Cl | CF₃ | H | H | Prop-2-in-1-yl | CH | H | CF₃ | H | CONH | Cyclopropyl | | |
| 250 | 3-Cl | CF₃ | H | H | Prop-2-in-1-yl | CH | H | CF₃ | H | CONH | 1-Cyanocyclopropyl | | |
| 251 | 3-Br | CF₃ | H | H | Prop-2-in-1-yl | CH | H | CF₃ | H | CONH | Cyclopropyl | | |
| 252 | 3-Br | CF₃ | H | H | Prop-2-in-1-yl | CH | H | CF₃ | H | CONH | 1-Cyanocyclopropyl | | |
| 253 | 3-CF₃;4-F | CF₃ | H | H | Prop-2-in-1-yl | CH | H | CF₃ | H | CONH | Cyclopropyl | | |
| 254 | 3-CF₃;4-F | CF₃ | H | H | Prop-2-in-1-yl | CH | H | CF₃ | H | CONH | 1-Cyanocyclopropyl | | |
| 255 | 3-Br;4-F | CF₃ | H | H | Prop-2-in-1-yl | CH | H | CF₃ | H | CONH | Cyclopropyl | | |
| 256 | 3-Br;4-F | CF₃ | H | H | Prop-2-in-1-yl | CH | H | CF₃ | H | CONH | 1-Cyanocyclopropyl | | |
| 257 | 3-Cl;4-F | CF₃ | H | H | Prop-2-in-1-yl | CH | H | CF₃ | H | CONH | Cyclopropyl | | |
| 258 | 3-Cl;4-F | CF₃ | H | H | Prop-2-in-1-yl | CH | H | CF₃ | H | CONH | 1-Cyanocyclopropyl | | |
| 259 | 3-Cl;5-Cl | CF₃ | H | H | Prop-2-in-1-yl | CH | H | CF₃ | H | CONH | Cyclopropyl | | |
| 260 | 3-Cl;5-Cl | CF₃ | H | H | Prop-2-in-1-yl | CH | H | CF₃ | H | CONH | 1-Cyanocyclopropyl | | |
| 262 | 3-CF3 | CF₃ | H | H | Et | CH | H | Cl | H | CONH | 1-Cyano-1-methylcyclopropylmethyl | 585,2 | 4,28 |
| 263 | 3-CF₃ | CF₃ | H | H | Et | CH | H | Cl | H | CONH | 2-Cyanoethyl | 545,2 | 3,53 |
| 264 | 3-CF₃ | CF₃ | H | H | Et | CH | H | Cl | H | CONH | 1-Cyano-1-ethylpropan-1-yl | 587,2 | 4,41 |
| 266 | 3-CF₃ | CF₃ | H | H | Et | CH | H | Cl | H | CONH | 1,2-Dimethylcyclopropyl | 560,1 | 4,41 |
| 267 | 3-CF₃ | CF₃ | H | H | Et | CH | H | Cl | H | CONH | 1-Trifluormethylcyclopropyl | 600,0 | 4,30 |
| 268 | 3-CF₃ | CF₃ | H | H | Et | CH | H | Cl | H | CONH | 1-Cyanobut-3-in-1-yl | 569,1 | 3,91 |
| 269 | 3-CF₃ | CF₃ | H | H | Et | CH | H | Cl | H | CONH | 3-Cyanopropan-2-yl | 559,2 | 3,78 |
| 270 | 3-CF₃ | CF₃ | H | H | Et | CH | H | Cl | H | CONH | 1-Cyano-2-methylpropan-1-yl | 573,2 | 4,37 |
| 271 (Synthese Bspl. 7) | 3-CF₃ | CF₃ | H | H | Et | CCl | H | Cl | H | CONH | 1-Cyanocyclopropyl | 591,0 | 3,95 |
| 272 | 3-CF₃ | CF₃ | H | H | Cycloprop ylmethyl | CH | H | Cl | H | CONH | 1-Cyanocyclopropyl | 583,2 | 4,03 |
| 273 (Synthese Bspl. 9) | 3-CF₃ | CF₃ | H | H | Et | CH | H | Cl | H | CONH | 1-Aminothiocarbonylcyclopropyl | 591,0 | 3,56 |
| 274 | 3-CF₃ | CF₃ | H | H | Cyclobutyl | CH | H | Cl | H | CONH | 1-Cyanocyclopropyl | 583,2 | 4,03 |
| 275 | 3-Cl;4-Cl;5-Cl | CF₃ | H | H | Et | CH | H | Cl | H | CONH | 1-Cyanocyclopropyl | 590,9 | 4,43 |
| 276 | 3-OCHF₂ | CF₃ | H | H | Et | CH | H | Cl | H | CONH | Cyclopropyl | 530,1 | 3,48 |
| 277 | 3-Cl;4-Cl;5-Cl | CF₃ | H | H | Prop-2-in-1-yl | CH | H | Cl | H | CONH | 1-Cyanocyclopropyl | 601,0 | 4,24 |
| 278 | 3-OCHF₂ | CF₃ | H | H | Prop-2-in-1-yl | CH | H | Cl | H | CONH | Cyclopropyl | 540,0 | 3,32 |
| 279 (Synthese Bspl. 8) | 3-CF₃ | CF₃ | H | H | Et | C-CN | H | Cl | H | CONH | 1-Cyanocyclopropyl | 582,1 | 3,45 |
| 281 | 3-CF₃;4-F | CF₃ | H | H | Prop-2-en-1-yl | N | H | Cl | H | CONH | Cyclopropyl | 563,1 | 3,79 |
| 282 | 3-CF₃ | CF₃ | H | H | Et | CH | H | Et | H | CONH | 1-Cyanocyclopropyl | 551,1 | 3,98 |
| 283 | 3-CF₃ | CF₃ | H | H | Prop-2-en-1-yl | CH | H | Cl | H | CONH | 1-Cyanocyclopropyl | 569,1 | 3,74 |
| 284 | 3-CF₃ | CF₃ | H | H | Phenylmethyl | CH | H | Cl | H | CONH | 1-Cyanocyclopropyl | 619,1 | 4,07 |
| 285 | 3-CF₃ | CF₃ | H | H | But-2-in-1-yl | CH | H | Cl | H | CONH | 1-Cyanocyclopropyl | 581,1 | 3,87 |
| 286 | 3-CF₃ | CF₃ | H | H | Benzyloxymethyl | CH | H | Cl | H | CONH | 1-Cyanocyclopropyl | 649,1 | 4,17 |
| 287 | 3-CF₃ | CF₃ | H | H | Ethoxymethyl | CH | H | Cl | H | CONH | 1-Cyanocyclopropyl | 587,1 | 3,70 |
| 288 | 3-CF₃ | CF₃ | H | H | Prop-1-yl | CH | H | Cl | H | CONH | 1-Cyanocyclopropyl | 571,1 | 4,03 |
| 289 | 3-Cl;4-Cl;5-Cl | CF₃ | H | H | Et | CH | H | Me | H | CONH | 1-Cyanocyclopropyl | 571,1 | 4,43 |
| 290 | 3-Cl;5-Cl;4-F | CF₃ | H | H | Et | CH | H | Me | H | CONH | 1-Cyanocyclopropyl | 555,0 | 4,13 |
| 291 | 3-Cl;5-Cl | CF₃ | H | H | Et | CH | H | Me | H | CONH | 1-Cyanocyclopropyl | 537,1 | 4,07 |
| 292 | 3-F;4-F | CF₃ | H | H | Et | CH | H | Me | H | CONH | 1-Cyanocyclopropyl | 505,1 | 3,48 |
| 293 | 3-Cl;5-F | CF₃ | H | H | Et | CH | H | Me | H | CONH | 1-Cyanocyclopropyl | 521,1 | 3,85 |
| 294 | 3-CF₃ | CF₃ | H | H | Cyanomethyl | CH | H | Cl | H | CONH | 1-Cyanocyclopropyl | 568,1 | 3,30 |
| 295 | 3-F;4-F | CF₃ | H | H | Et | CH | H | Me | H | CONH | Cyclopropyl | 480,1 | 3,40 |
| 296 | 3-Cl;5-Cl;4-F | CF₃ | H | H | Et | CH | H | Me | H | CONH | Cyclopropyl | 530,1 | 4,16 |
| 297 | 3-Cl;4-C1 | CF₃ | H | H | Et | CH | H | Me | H | CONH | Cyclopropyl | 512,1 | 3,99 |
| 298 | 3-Cl;5-F | CF₃ | H | H | Et | CH | H | Me | H | CONH | Cyclopropyl | 496,1 | 3,77 |
| 299 | 3-CF₃ | CF₃ | H | H | 2-Methylpropan-1-yl | CH | H | Cl | H | CONH | 1-Cyanocyclopropyl | 585,1 | 4,13 |
| 300 | 3-CF₃;4-F | CF₃ | H | H | Cyclopropyl | N | H | Br | H | CONH | Cyclopropyl | 607,0 | 3,59 |
| 301 | 3-CF₃;4-F | CF₃ | H | H | Cyanomethyl | N | H | Cl | H | CONH | Cyclopropyl | 562,0 | 3,28/ 3,43 |
| 302 (Synthese Bspl. 6) | 3-CF₃ | CF₃ | H | H | Cyclopropyl | CH | H | Cl | H | CONH | 1-Cyanocyclopropyl | 569,1 | 3,60 |
| 303 | 3-Cl;4-F | CF₃ | H | H | Prop-2-en-1-yl | N | H | Cl | H | CON(C H2CH= CH2) | Cyclopropyl | 569,1 | 4,75 |
| 304 | 3-CF₃;4-F | CF₃ | H | H | Et | CH | H | Me | H | CONH | Cyclobutyl | 544,1 | 4,24 |
| 305 | 3-CF₃;4-F | CF₃ | H | H | Et | CH | H | Me | H | CONH | Propan-2-yl | 532,2 | 4,16 |
| 306 | 3-CF₃;4-F | CF₃ | H | H | Et | CH | H | Me | H | CONH | 1,3-Difluorpropan-2-yl | 568,1 | 4,03 |
| 307 | 3,4-(-OCF₂O-) | CF₃ | H | H | Et | CH | H | Me | H | CONH | Cyclopropyl | 524,1 | 3,81 |
| 308 | 3,4-(-OCF₂O-) | CF₃ | H | H | Et | CH | H | Me | H | CONH | 1-Cyanocyclopropyl | 549,1 | 3,80 |
| 309 | 3-CF₃ | CF₃ | H | H | Prop-2-en-1-yl | N | H | Cl | H | CON(C H₂CH=C H₂) | Cyclopropyl | 585,1 | 4,74 |
| 310 | 3-Cl;5-Cl;2-F;5-F | CF₃ | H | H | Et | CH | H | Cl | H | CONH | 1-Cyanocyclopropyl | 593,0 | 4,25 |
| 311 | 3-Cl;5-Cl;4-F | CF₃ | H | H | Et | CH | H | Cl | H | CONH | 1-Cyanocyclopropyl | 575.0 | 4,19 |
| 312 (Synthese Bspl. 5) | 3-CF₃ | CF₃ | H | H | Cyclopropyl | N | H | Cl | H | CONH | Cyclopropyl | 545,0 | 3,48 |
| 313 | 3-CF₃;4-F | CF₃ | H | H | Cyclopropyl | N | H | Cl | H | CONH | Cyclopropyl | 563,1 | 3,38 |
| 314 | 3-Br | CF₃ | H | H | Cyclopropyl | N | H | Cl | H | CONH | Cyclopropyl | 555,0 | 3,43 |
| 315 | 3-F;4-F | CF₃ | H | H | Et | CH | H | Cl | H | CONH | 1-Cyanocyclopropyl | 525,1 | 3,47 |
| 316 | 3-Cl;4-Cl | CF₃ | H | H | Et | CH | H | Cl | H | CONH | 1-Cyanocyclopropyl | 557,0 | 4,06 |
| 317 | 3-Cl;5-Cl | CF₃ | H | H | Et | N | H | Cl | H | CONH | Cyclopropyl | 533,0 | 4,07/ 4,15 |
| 318 | 3-CF₃ | CF₃ | H | H | Et | CH | H | Cl | H | CONH | 1-Cyano-1-phenylmethyl | 607,1 | 4,37 |
| 319 | 3-CF₃ | CF₃ | H | H | Et | CH | H | iPr | H | CONH | 1-Cyanocyclopropyl | 565,1 | 4,08 |
| 320 | 3-Cl:4-F | CF₃ | H | H | Et | CH | H | Me | H | CON(Et) | Et | 512,1 | 4,35 |
| 321 | 3-F;4-F | CF₃ | H | H | Et | CH | H | Me | H | CONH | 1-(Methylsulfinyl)propan-2-yl | 544,1 | 2,76 |
| 322 | 3-CF₃ | CF₃ | H | H | Cyclopropylmethyl | N | H | Cl | H | CONH | Cyclopropyl | 559,1 | 4,04 /4,04 |
| 323 | 3-F | CF₃ | H | H | Et | N | H | Cl | H | CON(Et) | Et | 499,1 | 3,95 / 4,00 |
| 324 | 3-CF₃ | CF₃ | H | H | Ethenyl | CH | H | H | H | CONH | 1-Cyanocyclopropyl | 521,1 | 3,48 |
| 325 | 3-CF₃ | CF₃ | H | H | Et | CH | H | Cl | H | CONH | 2-Cyano-1,3-dimethoxypropan-2-yl | 619,1 | 4,16 |
| 326 | 3-CF₃ | CF₃ | H | H | Methoxycarbonylmethyl | CH | H | Cl | H | CONH | 1-Cyanocyclopropyl | 601,1 | 3,36 |
| 327 | 3-Cl;5-Cl;4-F | CF₃ | H | H | Et | CH | H | Cl | H | CONH | Cyclopropyl | 550,0 | 4,29 |
| 328 | 3-Cl;5-Cl;2-F;4-F; | CF₃ | H | H | Et | CH | H | Cl | H | CONH | Cyclopropyl | 568,0 | 4,46 |
| 329 | 3-F;4-F; | CF₃ | H | H | Et | CH | H | Cl | H | CONH | Cyclopropyl | 500,1 | 3,52 |
| 330 | 3-Cl;5-Cl;2-F;4-F; | CF₃ | H | H | Et | CH | H | Me | H | CONH | 1-Cyanocyclopropyl | 573,1 | 4,34 |
| 331 | 3-CF₃;5-F | CF₃ | H | H | Et | CH | H | Me | H | CONH | 1-Cyanocyclopropyl | 555,1 | 4,02 |
| 332 | 3-CF₃;4-F | CF₃ | H | H | Et | N | H | Br | H | CONH | Cyclopropyl | 597,0 | 3.76 |

| | | | | | | | | | | | | | |
|---|---|---|---|---|---|---|---|---|---|---|---|---|---|
| Abkürzungen: Et = Ethyl, Me = Methyl; | | | | | | | | | | | | | |

Die in der Tabelle aufgeführten Benzimidazolderivate (G=N) liegen teilweise als Regioisosomerengemische der Substituenten -R^{6b} und -A-Y vor. In diesem Fall sind die log p Werte und NMR-Daten für beide Isomeren angegeben.
**1H-NMR Daten ^{b)}**

| |
|---|
| Verbindung Nr. 1 [CD₃CN] 8,74 0,59;8,73 0,59;7,98 1,91;7,90 1,03;7,89 1,17;7,81 2,24;7,80 2,16;7,79 1,02;7,78 1,32;7,77 4,03;7,69 1,00;7,68 1,74;7,67 4,03;7,66 0,88;7,66 2,56;6,91 0,50;6,11 0,64;6,10 0,81;6,08 0,65;5,45 7,05;4,11 0,38;4,10 9,61:4,09 0,34;4,08 16,00;4,06 0,41;4,05 0,39;2,88 0,67;2,88 0,39;2,87 0,71;2,87 0,76;2,86 1,07;2,86 1,38;2,85 1,25;2,85 0,79;2,84 0,52;2,17 6,43;1,97 1,74;1,97 0,87;1,96 1,15:1,95 0,96;1,95 9,78;1,95 18,25:1,94 25,81;1,94 17,31;1,93 8,94;1,27 0,37;1,27 0,46:1,22 0,52;1,20 1,03:1,19 0,48;0,79 0,34:0,79 0,64:0,78 0,86;0,78 1,25;0,78 1,52;0,77 2,06;0,77 1,47;0,77 1,05;0,77 2,04;0,76 1,53;0,76 1,05;0,75 0,70;0,62 0,42;0,62 0,72;0,61 0,92;0,61 1,06;0,61 2,19;0,61 2,23;0,60 2,10;0,60 1,53;0,59 0,42;0,59 0,53;0,01 0,64;0,00 21,51;-0,01 0,58 |
| Verbindung Nr. 2 [CD₃CN] 8,20 1,56:8,19 1,60;7,72 10,66;7,70 4,31;7,58 1,96;7,56 2,30;7,55 8,41;7,51 0,32;7,51 0,38;7,49 0,55;7,48 1,02:7,48 0,78;7,47 4,48;7,47 9,40;7,47 3,10;7,46 3,95;7,45 0,97;7,16 7,89;6,89 1,56;6,05 0,40;6,04 1,42;6,02 2,03;6,01 1,49;5,99 0,44;4,47 1,97;4,46 6,18;4,45 6,23;4,43 1,99;4,06 0,50;4,05 0,50;2,88 0,39;2,88 1,13;2,87 1,55;2,86 2,49;2,86 2,46;2,85 1,66;2,85 1,22;2,84 0,44;2,28 0,35;2,27 0,46:2,25 0,65;2,21 502,49;2,05 0,45;2,05 0,48:1,97 2,50;1,97 23,78;1,96 2,48:1,96 3,35;1,95 30,00;1,95 56,18;1,94 79,93;1,94 52,48:1,93 26,64;1,83 0,43:1,32 0,47:1,31 0,51;1,30 0,51;1,29 7,47:1,28 16,00;1,27 8,13:1,22 0,70;1,20 1,27:1,19 0,66;0,91 0,48;0,79 1,46;0,78 4,14;0,77 5,29;0,77 5,30;0,76 4,01;0,75 1,68;0,60 1,73;0,60 4,22;0,60 4,23;0,59 4,33;0,59 3,97;0,59 4,05;0,58 1,26;0,00 0,59 |
| Verbindung Nr. 3 [CD₃CN] 8,17 1,45;8,16 1,52;8,00 2,07;7,99 2,00;7,95 1,08;7,94 1,28;7,93 1,47;7,93 1,32;7,92 1,35;7,74 10,98;7,57 8,39;7,45 1,88;7,43 2,50;7,42 1,77;7,19 8,10;7,19 7,92;6,86 1,70;6,16 0,36;6,14 1,31;6,13 1,87;6,11 1,34;6,10 0,38;4,48 2,08;4,47 6,40;4,45 6,43;4,44 2,02;2,89 0,89;2,88 0,38;2,87 1,14;2,86 1,60;2,86 2,48;2,85 2,52;2,84 1,60;2,84 1,16;2,83 0,40;2,77 0,75;2,18 491,55;2,16 0,71;2,16 0,69;2,06 0,34;2,06 0,61;2,05 0,87;2,05 0,60;1,97 5,10;1,96 4,52;1,96 7,56;1,95 58,87;1,95 107,34;1,94 152,89;1,94 107,55;1,94 55,32;1,84 0,35;1,83 0,63;1,83 0,90;1,82 0,62;1,82 0,33;1,44 0,41;1,30 0,43;1,29 7,24;1,28 16,00;1,27 7,58;0,78 1,43;0,77 4,21;0,77 5,36;0,76 5,34;0,76 4,17;0,75 1,71;0,60 1,71;0,59 4,31;0,59 4,52;0,59 4,64;0,59 4,34;0,58 4,35;0,57 1,35;0,01 0,57;0,00 12,97;-0,01 0,50 |
| Verbindung Nr. 4 [CD₃CN] vgl. Synthesebeispiel 4 |
| Verbindung Nr. 5 [CD₃CN] 9,20 0,40;8,15 2,63;8,14 1,17;8,13 2,75;7,84 0,55;7,76 16,00;7,72 0,35;7,69 7,16;7,67 0,44;7,62 12,62;7,58 0,36;7,58 0,35;7,57 3,16;7,56 3,89;7,55 0,59;7,54 0,73;7,48 1,91;7,48 1,63;7,47 8,50;7,47 14,44;7,47 14,33;7,47 5,60;7,46 6,33;7,45 1,52;7,42 0,35;7,26 12,22;7,26 11,69;6,86 2,74;6,86 2,74;6,05 0,60;6,04 2,20;6,02 3,14;6,01 2,29;6,00 0,69;5,34 0,35;5,34 0,39;5,32 15,34;5,32 15,59;5,00 0,44;4,99 0,45;4,98 0,35;4,97 0,36;3,79 0,34;3,79 0,39;3,79 0,39;3,78 0,33;2,88 0,67;2,87 1,78;2,87 2,48;2,86 3,89;2,85 3,93;2,85 2,62;2,84 1,96;2,84 0,80;2,53 4,69;2,52 10,42;2,52 4,69;2,51 0,40;2,26 0,60;2,25 1,06;2,24 0,64;2,15 77,87;2,06 0,39;2,06 0,73;2,05 1,11;2,05 0,80;2,04 0,37;1,97 5,16;1,96 5,34;1,95 8,34;1,95 69,92;1,95 132,87;1,94 192,91;1,94 134,93;1,93 68,76;1,84 0,43;1,83 0,78;1,83 1,12;1,82 0,79;1,82 0,43;1,54 0,37;1,47 0,69;1,42 0,33;1,32 0,45;1,31 0,51;1,31 0,80;1,31 0,67;1,30 0,74;1,28 1,76;1,27 11,23;0,91 0,36;0,90 0,38;0,89 0,87;0,88 2,07;0,87 1,09;0,79 2,41;0,78 6,66;0,77 8,61;0,76 8,44;0,76 7,02;0,75 3,09;0,74 0,43;0,73 0,36;0,63 0,38;0,62 0,43;0,61 0,43;0,60 2,71;0,59 6,68;0,59 7,06;0,59 7,53;0,59 7,22;0,58 7,26;0,57 2,45;0,10 0,43;0,01 3,93;0,00 115,18;-0,01 5,12;-0,10 0,44 |
| Verbindung Nr. 6 [CD₃CN] 8,28 2,57;8,26 2,64;8,01 2,97;8,00 2,82;8,00 2,91;7,96 1,55;7,95 1,65;7,95 1,80;7,94 2,04;7,94 1,78;7,93 1,81;7,93 1,49;7,81 0,52;7,75 16,00;7,72 0,39;7,61 12,15;7,49 0,49;7,45 2,91;7,43 3,70;7,42 2,66;7,26 11,64;7,26 11,39;7,25 0,41;6,87 2,47;6,87 2,48;6,16 0,58;6,14 2,02;6,13 2,80;6,11 2,04;6,10 0,61;5,33 0,34;5,33 0,36;5,31 12,81;5,31 12,94;4,97 0,42;4,96 0,42;3,78 0,38;3,78 0,39;2,88 0,62;2,88 0,33;2,87 1,75;2,87 2,41;2,86 3,84;2,86 3,80;2,85 2,41;2,84 1,80;2,84 0,66;2,52 4,57;2,52 10,28;2,52 4,46;2,50 0,32;2,15 45,05;2,06 0,46;2,05 0,68;2,05 0,46;1,97 3,45;1,96 3,58;1,95 4,97;1,95 43,63;1,95 82,51;1,94 120,35;1,94 82,65;1,93 41,76;1,93 0,87;1,83 0,46;1,83 0,66;1,82 0,46;1,29 0,59;1,28 0,42;1,27 1,01;1,01 0,48;0,79 2,29;0,78 6,09;0,77 8,00;0,76 7,69;0,76 6,29;0,75 2,62;0,74 0,40;0,73 0,33;0,63 0,33;0,62 0,39;0,62 0,38;0,61 0,53;0,61 0,56;0,60 2,64;0,59 5,96;0,59 6,30;0,59 6,54;0,59 6,13;0,58 6,26;0,57 2,06;0,01 2,51;0,00 73,98;-0,01 2,61 |
| Verbindung Nr. 7 vgl. Synthesebeispiel 2 |
| Verbindung Nr. 8 [DMSO-D₆] 11,19 0,47;9,80 3,66;9,78 3,87;8,41 3,59;8,39 3,58;8,19 5,48;8,06 3,01;8,04 3,36;7,83 2,88;7,81 4,14;7,80 10,53;7,77 13,82;7,74 2,89;7,72 4,42;7,70 1,89;7,38 9,74;6,33 0,40;6,31 1,52;6,29 2,28;6,27 1,67;6,24 0,43;4,54 1,79;4,52 5,20;4,50 5,36;4,49 2,38;4,48 2,96;4,47 3,10;4,46 3,28;4,45 2,81;4,44 0,73;4,43 0,62;4,38 0,56;4,37 0,80;4,36 2,48;4,35 3,55;4,34 2,91;4,330,65;4,31 0,66;4,29 0,45;4,28 0,83;4,26 1,10;4,25 1,07;4,23 1,08;4,22 0,98;4,20 0,72;4,18 0,33;3,96 0,74;3,79 0,37;3,50 0,37;3,48 0,34;3,45 0,45;3,43 0,70;3,41 0,80;3,40 0,85;3,31 1512,34;3,28 18,26;3,22 0,34;2,67 1,02;2,67 1,37;2,66 1,02;2,59 0,40;2,57 0,51;2,54 1,82;2,51 79,38;2,50 147,87;2,50 192,53;2,50 132,91;2,49 63,33;2,33 0,95;2,33 1,25;2,32 0,88;1,99 0,57;1,40 1,04;1,38 0,33;1,36 0,34;1,32 0,33;1,30 0,36;1,28 0,54;1,24 2,53;1,22 7,34;1,21 15,56;1,19 16,00;1,18 12,44;1,17 11,62;1,13 0,38;1,03 0,80;1,01 0,79;0,87 0,35;0,85 0,40;0,01 1,08;0,00 25,51;-0,01 1,06 |
| Verbindung Nr. 9 [CD₃CN] 8,18 1,42;8,16 1,45;7,98 3,46;7,91 1,85;7,89 2,06;7,83 10,49;7,79 1,79;7,77 2,28;7,69 1,89;7,68 3,09;7,67 1,32;7,61 7,34;7,59 0,36;7,56 0,52;7,36 1,32;7,35 1,30;7,22 6,92;7,21 6,90;6,17 0,35;6,15 1,26;6,14 1,75;6,13 1,31;6,11 0,40;4,97 0,63;4,96 2,72;4,95 4,18;4,93 2,75;4,92 0,64;4,48 1,79;4,47 5,75;4,46 5,80;4,45 1,83;3,28 5,15;3,27 5,27;3,06 2,00;2,79 0,69;2,19 0,33;2,18 0,91;2,17 1,08;2,16 29,01;2,08 0,34;1,97 0,45;1,97 59,93;1,96 1,43;1,95 1,57;1,95 17,56;1,95 32,81;1,94 48,55;1,94 33,14;1,93 16,55;1,93 0,49;1,85 0,34;1,61 15,77;1,60 15,78;1,30 6,92;1,29 16,00;1,27 6,99;1,11 0,48;0,01 0,66;0,00 24,15;-0,01 0,73 |
| Verbindung Nr. 10 [CD₃CN] 8,15 1,40;8,14 1,44;7,98 4,36;7,90 2,31;7,88 2,59;7,81 11,55;7,79 2,21;7,77 2,80;7,69 2,26;7,68 3,73;7,66 1,60;7,62 9,13;7,22 9,07;7,14 0,86;7,13 1,53;7,12 0,88;6,17 0,37;6,15 1,37;6,14 1,99;6,12 1,41;6,11 0,40;4,49 2,03;4,48 6,49;4,47 6,55;4,46 2,12;3,82 1,98;3,81 2,02;3,80 4,21;3,79 4,14;3,77 2,13;3,76 2,07;2,18 202,01;2,06 0,40;2,05 0,57;2,05 0,39;1,97 12,34;1,96 2,76;1,95 3,48;1,95 33,73;1,95 62,60;1,94 92,49;1,94 63,69;1,93 32,30;1,83 0,39;1,83 0,55;1,82 0,38;1,73 6,45;1,70 13,71;1,67 6,87;1,30 7,30;1,29 16,00;1,28 7,47;0,00 7,31 |
| Verbindung Nr. 11 [CD₃CN] 8,13 1,63;8,10 1,66;7,97 3,77;7,90 1,99;7,88 2,35;7,82 10,28;7,79 1,81;7,77 2,57;7,69 2,19;7,67 3,20;7,65 1,28;7,61 7,88;7,22 7,76;7,22 7,61;7,09 1,17;7,07 1,15;6,18 0,45;6,16 1,55;6,13 2,08;6,11 1,53;6,09 0,46;4,73 0,40;4,72 0,41;4,71 0,86;4,70 2,27;4,69 5,08;4,68 3,86;4,67 0,48;4,67 0,50;4,66 0,97;4,65 0,46;4,64 0,36;4,64 0,44;4,63 0,43;4,63 0,39;4,62 0,53;4,60 0,72;4,60 1,32;4,59 1,21;4,58 3,19;4,57 7,14;4,56 3,10;4,55 1,09;4,54 0,84;4,54 0,79;4,53 0,44;4,52 0,47;4,50 2,11;4,49 6,29;4,47 6,32;4,45 2,01;2,15 292,31;2,12 0,82;2,11 0,81;2,11 0,82;2,10 0,59;2,09 0,40;1,97 0,91;1,96 4,51;1,96 4,96;1,95 31,38;1,95 58,32;1,94 81,47;1,93 55,88;1,93 28,58;1,77 0,37;1,77 0,49;1,76 0,33;1,44 11,91;1,31 7,09;1,29 16,00;1,27 7,18;0,01 0,36;0,00 7,84 |
| Verbindung Nr. 13 [DMSO-D₆] 9,69 3,84;9,67 4,02;8,41 4,28;8,41 4,40;7,92 12,71;7,92 12,80;7,80 10,36;7,77 15,06;7,74 4,91;7,73 9,01;7,73 4,52;7,66 0,33;7,38 9,85;6,28 0,33;6,27 1,19;6,26 1,80;6,24 1,31;6,23 0,38;5,76 0,33;4,53 1,65;4,51 5,04;4,50 5,03;4,49 1,65;3,52 0,35;3,38 0,62;3,35 1337,04;3,33 9,64;2,85 0,48;2,85 1,18;2,84 1,48;2,84 2,51;2,83 2,56;2,82 1,49;2,82 1,27;2,81 0,55;2,62 0,97;2,62 1,36;2,61 0,95;2,61 0,44;2,54 0,61;2,52 1,74;2,52 2,28;2,52 2,46;2,51 72,44;2,51 157,19;2,50 215,78;2,50 156,91;2,50 71,56;2,39 0,97;2,39 1,36;2,38 0,95;2,08 1,34;1,38 0,51;1,26 0,42;1,24 0,87;1,23 2,94;1,22 7,55;1,21 16,00;1,19 7,49;1,18 0,78;1,17 1,27;1,15 0,59;1,13 0,34;1,12 0,41;0,85 0,48;0,71 1,81;0,70 4,65;0,70 6,42;0,69 5,91;0,69 5,21;0,68 2,04;0,55 2,06;0,54 5,83;0,54 5,43;0,54 5,15;0,53 5,35;0,52 1,67;0,01 1,33;0,00 42,82;-0,01 1,25 |
| Verbindung Nr. 14 [CD₃CN] 8,09 1,05;8,07 1,07;7,93 1,48;7,92 1,52;7,92 1,52;7,91 1,47;7,77 6,96;7,64 6,28;7,62 0,93;7,62 0,93;7,62 0,82;7,52 0,39;7,42 0,50;7,33 1,93;7,31 3,67;7,30 1,86;7,27 5,84;6,85 1,08;6,04 0,92;6,03 1,30;6,01 0,92;5,33 6,44;5,32 6,47;4,06 0,38;4,05 0,38;2,87 0,63;2,87 1,00;2,86 1,47;2,85 1,49;2,85 1,02;2,84 0,71;2,53 1,63;2,52 3,46;2,52 1,64;2,15 128,09;2,06 0,45;2,05 0,67;2,05 0,46;1,97 1,73;1,97 4,22;1,96 3,29;1,95 4,63;1,95 42,72;1,95 78,84;1,94 115,42;1,94 79,69;1,93 40,87;1,92 1,05;1,83 0,46;1,83 0,65;1,82 0,44;1,47 1,52;1,44 16,00;1,27 0,56;1,22 0,45;1,20 0,85;1,19 0,45;1,11 0,50;0,79 0,87;0,78 2,61;0,77 3,25;0,77 3,62;0,76 2,53;0,75 1,27;0,60 1,06;0,59 2,84;0,59 3,03;0,59 2,77;0,58 2,90;0,57 0,90;0,01 1,11;0,00 36,58;-0,01 1,28 |
| Verbindung Nr. 15 [CD₃CN] 8,12 0,47;8,11 0,48;7,97 1,13;7,90 0,58;7,89 0,67;7,83 2,73;7,79 0,60;7,77 0,77;7,69 0,61;7,68 1,00;7,67 0,43;7,62 2,24;7,23 2,18;7,16 0,78;6,15 0,38;6,14 0,55;6,13 0,40;4,50 0,51;4,48 1,60;4,47 1,62;4,46 0,53;2,16 4,22;1,97 0,88;1.97 0,77;1,96 0,69;1,95 0,84;1,95 8,36;1,95 15,52;1,94 22,59:1,94 15,46;1,93 7,98;1,74 16,00;1,44 0,57;1,30 1,84;1,29 4,00;1,28 1,89;1,20 0,46;0,01 0,34;0,00 11,62;-0,01 0,40 |
| Verbindung Nr. 16 [CD₃CN] 8,31 0,58;8,11 1,76;8,10 1,80;7,99 2,10;7,98 1,94;7,98 2,00;7,95 1,07;7,94 1,24;7,93 1,42;7,93 1,27;7,92 1,35;7,81 10,66;7,66 0,46;7,61 7,99;7,50 2,97;7,45 2,02;7,44 2,58;7,42 1,94;7,27 0.45;7,21 7,59;6,16 0,40;6,14 1,44;6,13 2,01;6,12 1,45;6,10 0,44;5,45 1,09;4,49 1,90;4,47 5,91;4,46 5,93;4,45 1,89;3,82 0,36;3,81 0,40;2,89 0,89;2,77 0,79;2,77 0,75;2,15 3,45;2,06 0,36;2,05 0,53;2,05 0,38;1,97 0,50;1,97 0,70;1,97 47,78;1,96 2,45;1,95 3,14;1,95 33,04;1,95 62,70;1,94 91,90;1,94 62,55;1,93 31,53;1,93 0,55;1,83 0,38;1,83 0,54;1,82 0,38;1,59 2,55;1,58 6,23;1,57 6,32;1,56 3,04;1,54 0,41;1,42 0,34;1,38 0,41;1,37 0,37;1,36 3,36;1,35 6,35;1,34 6,80;1,33 2,88;1,32 0,62;1,31 1,04;1,30 1,30;1,30 7,34;1,29 2,62;1,29 16,00;1,27 8,13;1,22 0,33;1,21 0,41;1,20 0,46;1,19 0,53;1,19 0,34;1,09 0,67;1,08 0,76;1,08 0,82;1,07 0,73;0,91 1,36;0,90 1,33;0,89 0,51;0,88 0,91;0,87 0,53;0,01 1,50;0,00 48,78;-0,01 1,52 |
| Verbindung Nr. 17 [CD₃CN] 8,14 0,49;8,12 0,49;8,00 0,62;7,99 0,58;7,93 0,33;7,93 0,36;7,92 0,41;7,92 0,34;7,92 0,35;7,77 3,80;7,59 2,73:7,45 0,60;7,43 0,74;7,42 0,56;7,21 2,58;7,21 2,55;6,79 0,36;6,15 0,44;6,13 0,60;6,12 0,44;4,49 0,67;4,48 2,15;4,47 2,15;4,45 0,66;4,06 0,47:4,05 0,47;3,40 0,50;3,39 0,62;3,39 1,66;3,38 1,71;3,38 1,77;3,37 1,68;3,36 0,66;3,35 0,53;3,28 1,85;3,27 1,89;2,20 0,46;2,19 0,66;2,17 389,10;2,06 0,48;2,05 0,72;2,05 0,48;1,97 2,18:1,97 5,41;1,96 2,44;1,95 3,22;1,95 45,19;1,95 87,72;1,94 128,83;1,94 88,19;1,93 44,11;1,93 1,32;1,93 0,59;1,83 0,49;1,83 0,73;1,82 0,50;1,44 16,00;1,30 2,42;1,29 5,59;1,28 2,47;1,22 0,61;1,21 3,51;1,20 1,94;1,19 7,21;1.18 3,37;1,13 0,40:1,11 0,79;1,10 0,40;0,00 11,09;-0,01 0,33 |
| Verbindung Nr. 18 [CD₃CN] 8,28 0,50;8,15 1,11;8,13 1,07;7,83 1.69;7,82 1,88;7,81 1,87;7,81 1,88;7,76 10,36;7,64 0,92;7,63 0,96;7,63 0,95:7,62 1,23;7,61 1,58;7,61 1,22;7,60 1,18;7,59 7,27;7,39 3,80:7,38 0,55;7,37 4,93;7,35 3,02;7,21 6,94;7,20 7,32;6,89 1,23;6,09 0,39;6,07 1,35;6,05 1,80:6,03 1,38;6,01 0,47;4,52 1,76;4,51 0,38;4,50 5,87;4,48 5,91;4,46 1,79;4,39 0,74;4,38 0,76;4,36 0,35;4,10 0,89;4,08 0,93;2,92 0,36:2,91 1,11;2,90 1,55;2,89 2,47;2,88 2,56;2,87 1,48;2,86 1,19;2,85 0,41;2,26 0,41;2,25 0,60;2,25 0,65;2,25 0,67;2,24 0,67;2.24 0,80;2,24 0,81;2,24 0,77;2,24 0,78;2,24 0,85;2,24 0,81;2,24 0,89;2,24 0,90;2,24 0,91;2,23 1,03;2,23 1,26;2,23 1,29;2,23 1,43;2,23 1,55;2,23 1,73;2,23 2,08;2,23 2,23;2,23 2,45;2,23 2,55:2,23 2,59:2,23 2,83;2,22 2,99;2,22 3,21;2,22 3,50;2,22 3,90;2,21 667,68;2,21 569,48;2,20 4,79:2,20 3,26;2,19 2,52;2,19 1,75;2,19 1,56;2,19 1,24;2,19 0,89;2,19 0,94;2,19 0,95;2,18 0,99;2,18 0,93;2,18 0,79;2,18 0,61;2,15 0,54;2.14 0,94;2,14 1,22;2,13 0,81;2,12 0,44;2,00 4,26;1,99 14,57;1,99 7,14;1,98 73,25;1,98 138,96;1,97 199,67;1,96 135,48;1,96 68,26;1,95 1,31;1,94 0,50;1,81 0,34;1,80 0,72;1,80 1,09;1,79 0,74;1,79 0,38;1,47 5,71;1,42 0,80;1,41 1,61;1,39 0,79;1,36 0,38;1,33 6,86;1,31 16,00;1,29 6,79;1,25 1,18;1,23 2,27;1,22 0,40;1,22 1,16;1,17 0,46;1,16 0,45;1,14 0,91;1,13 0,44;0,82 1,22;0,81 3,58;0,80 4,61;0,79 4,98;0,79 3,22;0,77 1,66;0,64 1,74;0,63 3,70;0,63 3,58;0,62 3,70;0,62 3,28;0,61 3,40;0,60 1,12 |
| Verbindung Nr. 19 vgl. Synthesebeispiel 1 |
| Verbindung Nr. 20 [CD₃CN] 10,05 0,35:8,08 1,73;8,07 1,78;7,92 1,11;7,80 11,27;7,79 2,42;7,79 2,41;7,78 2,38;7,77 2,33;7,77 0,32;7,65 0,34;7,61 1,14;7,61 1,23;7,61 1,36;7,60 9,54;7,59 1,73;7,59 1,48;7,56 0,40;7,51 3,11;7,36 3,51;7,35 5,57;7,33 3,16;7,19 7,87;6,06 0,40;6,04 1,48;6,03 2,08;6,01 1,52;6,00 0,45;5,45 8,25;4,48 2,15;4,47 6,36;4,46 6,25;4,45 1,94;3,28 0,84;3,27 0,85;3,07 1,33;2,89 12,88;2,79 0,45;2,77 11,04;2,77 10,95;2,18 0,66;2,16 15,18;2,06 0,37;2,05 0,59;2,05 0,59;2,05 0,40;1,97 0,47;1,97 1,48;1,96 2,62;1,95 2,54;1,95 33,61;1,95 64,49;1,94 95,43;1,94 64,69;1,93 32,56;1,93 0,45;1,83 0,37;1,83 0,55;1,82 0,38;1,59 2,67;1,58 6,55;1,58 6,50;1,57 3,11;1,54 0,39;1,39 0,35;1,36 3,36;1,35 6,55;1,35 6,90;1,34 2,99;1,32 0,56;1,32 0,49;1,31 0,60;1,31 0,89;1,30 7,09;1,29 2,16;1,29 16,00;1,28 7,40;1,20 0,41;0,01 1,36;0,00 47,99;0,01 1,45 |
| Verbindung Nr. 21 [CD₃CN] 12,98 0,36:10,12 0,47;8,42 0,42;8,32 0,55;8,18 1,61;8,15 1,59;8,13 0,41;8,00 5,20;7,97 0,37;7,97 0,40;7,92 2,76;7,90 13,49;7,86 0,34;7,83 0,47;7,82 2,46;7,80 3,74;7,75 0,35;7,72 2,77;7,70 3,99;7,68 8,79;7,38 1,74;7,37 1,74;7,36 0,96;7,29 0,69;7,27 8,33;6,20 0,58;6,19 1,84;6,16 2,63;6,14 1,80;6,12 0,62;4,54 2,18;4,52 6,42;4,50 6,42;4,49 2,04;4,30 12,44;4,29 11,85;3,91 0,32:2,83 0,41;2,37 0,40;2,36 0,51;2,23 737,55;2,16 2,08;2,15 1,39;2,14 1,64;2,14 1,89;2,13 1,46;2,13 1,06;2,06 0,39;2,05 0,39;2,05 0,35;2,04 0,39;1,99 144,56;1,99 18,81;1,98 80,99;1,98 153,45;1,97 214,98;1,96 148,87;1,96 75,24;1,94 1,18;1,89 0,32;1,82 0,76;1,81 0,54;1,80 0,90;1,80 1,13;1,79 1,03;1,79 0,56;1,39 0,34;1,37 0,46;1,35 0,55;1,34 7,34;1,32 16,00;1,30 7,59;1,29 0,33;1,16 0,44;1,14 0,97;1,13 0,36;0,94 0,44 |
| Verbindung Nr. 22 [CD₃CN] 10,04 0,44;8,25 0,85;8,22 0,88;8,15 0,35;7,98 2,26;7,92 1,16;7,90 1,35;7,79 1,08;7,77 1,60;7,69 7,18;7,67 2,02;7,65 0,78;7,54 4,90;7,17 4,85;7,17 4,70;7,02 1,22;6,15 0,93;6,13 1,22;6,11 0,93;4,48 1,16;4,46 3,81;4,44 3,82;4,43 1,18;3,60 0,50;2,18 0,34;2,16 224,20;2,14 0,43;2,11 0,34;1,96 3,08;1,96 2,40;1,95 21,84;1,95 41,43;1,94 58,70;1,93 39,49;1,93 19,74;1,77 0,32;1,49 0,80;1,46 16,00;1,32 0,50;1,29 4,32;1,27 10,04;1,26 4,19;1,11 0,35;0,84 1,05;0,83 3,37:0,82 3,41;0,81 1,38;0,67 1,85;0,66 4,08;0,66 4,19;0,65 1,32;0,00 1,40 |
| Verbindung Nr. 23 [DMSO-D₆] 16,04 0,54;12,04 0,82;9,82 4,25;9,79 4,29;8,60 2,17;8,58 4,26;8,57 2,41;8,24 0,63;8,20 6,47;8,08 3,64;8,06 3,77;8,00 8,99;7,84 3,46;7,82 4,79;7,79 9,51;7,74 3,42;7,72 5,22;7,70 2,17;7,39 11,10;6,36 0,77;6,34 1,92;6,32 2,68;6,29 2,05;6,27 0,56;4,61 2,01;4,60 5,79;4,58 5,74;4,56 2,00;4,53 0,96;4,51 6,97;4,50 6,99;3,47 0,57;3,32 906,68;3,30 14,04;3,29 1,07;3,27 0,75:2,67 3,22;2,50 481,75;2,46 0,65;2,33 2,75;2,22 0,60;2,07 1,88;1,81 0,78;1,80 1,72;1,79 1,97;1,78 2,79;1,77 1,90;1,76 1,97;1,75 1,02;1,73 1,01;1,72 1,79;1,71 2,01;1,70 3,41;1,69 1,98;1,68 1,81;1,67 0,95;1,52 0,57;1,50 0,69;1,49 0,93;1,47 0,63;1,30 0,57;1,28 0,72;1,24 8,40;1,22 16,00;1,21 7,30;1,17 0,55;1,14 0,59;1,12 0,72;1,10 1,63;1,09 1,71;1,09 3,82;1,08 6,74;1,07 3,80;1,06 6,19;1,05 2,84;1,04 1,82;1,04 1,40;1,02 3,19;1,02 6,25;1,00 6,06;1,00 3,95;0,98 1,55;0,87 0,61;0,85 0,67;0,81 1,49;0,79 1,64;0,78 1,47;0,77 1,82;0,76 1,25;0,74 1,42;0,72 8,94;0,71 12,85;0,69 7,49;0,63 0,75;0,15 0,95;0,00 178,64;-0,15 0,82 |
| Verbindung Nr. 24 [DMSO-D₆] 9,70 3,97;9,67 4,09;8,52 1,93;8,51 3,95;8,50 1,89;8,20 5,68;8,07 3,10;8,05 3,38;7,83 2,97;7,81 4,14;7,78 10,87;7,74 3,12;7,72 4,83;7,70 2,03;7,66 11,30;7,34 10,44;6,34 0,44;6,31 1,61;6,29 2,34;6,27 1,64;6,25 0,52;4,52 1,87;4,50 5,32;4,49 5,36;4,47 1,78;4,41 8,23;4,40 8,08;4,02 0,38;3,33 277,41;3,30 3,78;2,68 0,77;2,67 0,96;2,67 0,73;2,51 114,66;2,50 149,84;2,50 104,34;2,33 0,69;2,33 0,96;2,32 0,65;2,08 0,56;1,99 1,72;1,78 0,37;1,71 0,79;1,70 1,72;1,69 1,73;1,69 1,49;1,68 3,20;1,67 1,82;1,66 1,76:1,65 0,86;1,23 1,39;1,22 7,31;1,20 16,00;1,18 7,00;1,17 1,59;1,16 0,61;1,09 0,63;1,08 0,84;1,07 0,51;1,06 0,84;1,05 0,38;1,02 0,50;1,02 0,83;1,01 0,63;1,00 0,71;1,00 0,50;0,77 0,38;0,75 0,33;0,73 1,06;0,71 3,87:0,71 8,37;0,70 7,10;0,69 9,87;0,69 10,14;0,68 3,51;0.67 4,08;0,67 7,14;0,66 2,85;0,65 0,83;0,01 3,05;0,00 70,59;-0,01 2,33;-0,15 0,36 |
| Verbindung Nr. 25 [DMSO-D₆] 11,97 0,37:9,70 2,29:9,68 2,38;8,25 1,21;8,23 2,24;8,22 1,12;8,20 3,32;8,07 1,80;8,05 1,98;7,83 1,82;7,81 2,45;7,77 6,29;7,74 1,88;7,72 2,72;7,70 1,17;7,64 6,33;7,33 6,14;6,31 0,96;6,29 1,38;6,27 1,01;4,52 1,15;4,50 3,19;4,48 3,03;4,46 1,01;4,39 4,80;4,37 4,71;3,40 0,69;3,33 198,12;3,30 3,13;2,68 0,68;2,67 0,87;2,67 0,62;2,54 3,26;2,51 105,26;2,50 137,72;2,50 95,59;2,33 0,66;2,33 0,91;2,32 0,65;2,22 2,19;2,20 6,35;2,18 6,43;2,16 2,13;2,08 0,48;2,04 0,35;2,03 0,44;1,99 1,14;1,24 0,98;1,22 4,30;1.20 9,25;1,18 4,05;1,18 1,05;1,09 0,32;1,07 7,67;1,05 16,00;1,03 7,23;1,02 0,47;1,01 0,91;0,99 1,88;0,97 1,21;0,95 0,51;0,01 2,66;0,00 63,20;-0,01 2,15 |
| Verbindung Nr. 26 vgl. Synthesebeispiel 3 |
| Verbindung Nr. 27 [CD₃CN] 8,22 1,05;8,17 0,39;8,14 4,49;8,14 4,53;8,05 0,39;8,02 1,35;8,00 3,01;7,98 2,01;7,94 1,16;7,93 1,22;7,92 1,46;7,91 1,13;7,75 2,74;7,74 2,60;7,73 3,39;7,72 3,16;7,60 1,16;7,53 3,98;7,51 3,12;7,47 0,44;7,46 1,87;7,45 0,51;7,43 2,29;7,41 1,74;7,27 7,45;7,25 0,59;7,05 0,42;7,02 1,42;6,18 0,42;6,16 1,52;6,14 1,99;6,12 1,45;6,09 0,53;6,05 0,36;4,55 1,84;4,53 5,87;4,52 5,78;4,50 1,87;4,38 0,49;4,36 1,39;4,34 1,26;4,32 0,39;4,07 0,57;4,05 0,50;2,88 1,24;2,88 1,55;2,87 2,19;2,86 2,08;2,85 1,53;2,84 1,05;2,83 0,49;2,77 0,49;2,55 0,39;2,45 0,44;2,39 0,36;2,33 0,48;2,28 0,86;2,27 0,96;2,25 1,58;2,15 225,44;2,11 5,65;2,11 5,08;2,10 3,74;2,05 0,99;1,97 9,62;1,96 27,51;1,96 28,81;1,95 161,86;1,95 296,23;1,94 411,76;1,93 278,85;1,93 141,11;1,78 0,92;1,77 1,80;1,77 2,35;1,76 1,53;1,76 0,72;1,54 0,39;1,42 0,37;1,39 1,46;1,37 2,90;1,35 1,49;1,34 1,19;1,32 7,27;1,30 16,00;1,28 9,53;1,27 8,80;1,22 0,97;1,20 1,61;1,19 0,85;1,16 0,45;1,09 0,56;0,93 0,41;0,92 1,06;0,90 1,20;0,88 1,70;0,86 0,86;0,84 0,53;0,83 0,51;0,82 0,36;0,78 1,28;0,77 3,56;0,76 4,51;0,75 4,64;0,74 3,49;0,73 1,66;0,71 0,37;0,69 0,43;0,66 0,43;0,63 1,80;0,62 4,18;0,61 3,92;0,61 3,55;0,60 3,30;0,59 1,12;0,15 0,69;0,08 0,70;0,05 0,64;0,01 6,88;0,00 170,47;-0,01 6,32;-0,15 0,66 |
| Verbindung Nr. 28 [CD₃CN] 8,18 0,39;8,16 0,37;7,98 1,20;7,91 0,62;7,89 0,74;7,82 3,51;7,79 0,59;7,77 0,86;7,70 0,70;7,68 1,03;7,66 0,41;7,60 2,59;7,37 0,77;7,22 2,44;7,22 2,59;6,16 0,48;6,14 0,65;6,12 0,49;4,50 0,58;4,48 1,92;4,46 1,96;4,45 0,61;3,70 16,00;3,69 0,71;2,19 28,42;2,19 49,69;2,18 49,25;2,18 64,19;2,18 80,87;2,11 0,38;1,97 3,24;1,96 2,10;1,96 22,00;1,95 43,08;1,94 60,93;1,94 41,83;1,93 21,51;1,77 0,35;1,55 0,97;1,54 2,62;1,53 2,76;1,52 1,17;1,30 2,30;1,28 5,38;1,27 2,31;1,25 1,23;1,24 2,72;1,24 2,67;1,22 0,97;0,00 3,98 |
| Verbindung Nr. 29 [CD₃CN] 8,24 1,00;8,22 1,00;7,98 3,32;7,92 1,72;7,90 2,07;7,79 1,60;7,77 2,37;7,70 10,21;7,70 2,27;7,68 2,96;7,66 1,19;7,55 7,36;7,18 7,14;7,18 7,22;7,07 1,89;6,18 0,38;6,16 1,36;6,14 1,83;6,11 1,39;6,09 0,43;4,48 1,68;4,46 5,59;4,45 5,69;4,43 1,77;2,19 95,85;2,18 90,53;2,18 90,68;2,12 0,45;2,11 0,60;2,10 0,46;1,97 0,49;1,97 3,31;1,96 3,09;1,96 33,15;1,95 62,98;1,94 87,69;1,94 60,24;1,93 31,13;1,92 0,68;1,78 0,37;1,77 0,53;1,77 0,37;1,46 0,59;1,45 1,24;1,44 1,39;1,43 1,04;1,43 2,50;1,42 0,82;1,41 1,36;1,40 1,32;1,39 0,68;1,31 0,32;1,29 6,89;1,28 16,00;1,27 2,61;1,26 6,71;0,88 0,33;0,80 2,01;0,79 5,40;0,78 5,95;0,77 3,09;0,73 0,42;0,71 0,43;0,67 3,18;0,66 5,99;0,65 5,60;0,64 2,14;0,46 1,38;0,45 3,99;0,44 4,30;0,44 1,85;0,43 2,09;0,43 4,24;0,42 3,95;0,41 1,87;0,28 1,96;0,27 4,50;0,26 4,72;0,26 4,31;0,25 4,62;0,24 1,39;0,00 5,98 |
| Verbindung Nr. 30 [CD₃CN] 8,15 1,21;8,13 1,16;7,97 3,52;7,91 1,83;7,89 2,20;7,79 1,66;7,77 2,49;7,71 9,31;7,69 2,11;7,67 2,98;7,66 1,20;7,57 7,16;7,19 7,26;7,02 1,93;6,18 0,38;6,16 1,41;6,14 1,91;6,11 1,44;6,09 0,44;4,49 1,71;4,47 5,49;4,46 5,61;4,44 1,83;2,18 175,31;2,18 238,15;2,17 224,02;2,12 0,68;2,12 0,92;2,11 1.17:2.10 0,89;2,10 0,50;1,97 6,51;1,96 7,95;1,96 63,19;1,95 119,53;1,94 166,08;1,94 115,93;1,93 62,19;1,78 0,40;1,78 0,72;1,77 1,00;1,76 0,72;1,76 0,40;1,71 1,75;1,69 5,75;1,67 6,14;1,66 2,20;1,30 6,80;1,28 14,82;1,26 6,61;1,05 7,68;1,03 16,00;1,01 7,14;0,98 0,33;0,82 1,86;0,80 5,68;0,80 6,10;0,79 2,93;0,75 0,38;0,72 0,40;0,68 3,15;0,67 6,41;0,67 6,28;0,65 2,31;0,00 7,26;-0,01 0,41 |
| Verbindung Nr. 31 [CD₃CN] 8,10 1,23;8,07 1,27;7,97 3,27;7,90 1,70;7,88 2,06;7,79 1,74;7,78 8,88;7,77 2,61;7,70 1,88;7,68 2,84;7,66 1,38;7,64 2,13;7,61 6,78;7,22 6,69;6,18 0,36;6,16 1,31;6,14 1,80;6,12 1,35;6,09 0,40;4,51 1,53;4,49 4,96;4,47 5,06;4,45 1,68;3,73 2,19;3,71 7,11;3,70 7,26;3,68 2.40:2.23 0,51:2,23 0,52;2,22 0,56;2,18 648,78;2,17 518.21;2.12 1.13;2.12 1.53;2.11 1.99;2.10 1,57;2,10 0,88;1,97 11.38;1.96 13,56;1,95 111.69;1.95 210,38;1,94 293,25;1,94 204,62;1,93 109,92;1,78 0,70;1,78 1.23;1.77 1,72;1,76 1.25;1.76 0,68;1,44 6,16;1,30 6,30;1,29 13,52;1,27 6,58;1,17 7,81;1,15 16,00;1,13 7,65;1,11 1,82;1,09 4,12;1,09 5,24;1,07 3.28;1.04 0,73;1,03 0,74;1,00 3,10;0,99 5,17;0,99 4,28;0,97 1,92;0,88 0,32;0,01 0,46;0,00 13,02;-0,01 0,67 |
| Verbindung Nr. 32 [CD3CN] 8,1153 1,33; 8,0916 1,34; 7,9638 3,77; 7,8920 1,97; 7,8727 2,40; 7,7862 1,77; 7,7666 2,75; 7,7574 10,19; 7,6920 2,20; 7,6724 3,28; 7,6528 1,31; 7,6028 8,02; 7,3257 2,33; 7,2191 8,01; 6,1732 0,43; 6,1521 1,53; 6,1302 2,11; 6,1082 1,59; 6,0866 0,48; 4,5010 1,89; 4,4832 6,03; 4,4654 6,13; 4,4477 1,99; 2,4235 10,57; 2,1921 679,25; 2,1866 1166,03; 2,1206 1,65; 2,1144 2,02; 2,1081 2,56; 2,1020 1,83; 2,0959 1,16; 2,0874 0,63; 2,0266 0,32; 1,9723 1,39; 1,9651 12,24; 1,9590 13,12; 1,9532 108,76; 1,9470 204,00; 1,9408 285,97; 1,9346 201,93; 1,9284 107,88; 1,7816 0,73; 1,7754 1,29; 1,7692 1,77; 1,7631 1,25; 1,7569 0,71; 1,3401 0,78; 1,2988 7,42; 1,2811 16,00; 1,2712 2,44; 1,2631 7,98; 1,2456 4,52; 1,2385 6,39; 1,2298 4,80; 1,2039 0,81; 1,1957 4,78; 1,1866 6,22; 1,1799 4,51; 1,1639 1,94; 0,8823 0,43; -0,0002 1,43 |
| Verbindung Nr. 33 [CD3CN] 8,2991 1,30; 8,2763 1,22; 8,0004 4,10; 7,9518 2,13; 7,9322 2,52; 7,8159 2,03; 7,8045 10,53; 7,7967 3,25; 7,7241 2,34; 7,7045 3,45; 7,6850 1,38; 7,6041 8,01; 7,3015 2,45; 7,2284 8,32; 6,2051 0,46; 6,1838 1,62; 6,1621 2,24; 6,1398 1,68; 6,1185 0,51; 4,5195 1,89; 4,5018 6,08; 4,4840 6,22; 4,4663 2,06; 2,7331 9,82; 2,5678 0,64; 2,5540 5,18; 2,5406 3,87; 2,5343 10,14; 2,5158 4,82; 2,4853 0,49; 2,2520 143,31; 2,2459 247,47; 2,1724 0,52; 2,1526 0,84; 2,1440 0,85; 2,1371 0,50; 2,1314 1,21; 2,1244 2,02; 2,1126 1,27; 2,1036 2,67; 2,0978 1,36; 2,0924 1,62; 2,0837 2,15; 2,0773 2,39; 2,0711 1,19; 2,0606 1,32; 2,0564 1,38; 2,0492 0,89; 2,0400 0,68; 2,0322 0,40; 2,0282 0,46; 2,0008 0,63; 1,9941 0,85; 1,9877 1,04; 1,9822 13,07; 1,9760 25,24; 1,9698 36,59; 1,9637 25,98; 1,9575 14,01; 1,4639 1,63; 1,3282 7,27; 1,3105 16,00; 1,2927 7,23 |
| Verbindung Nr. 34 [CD3CN] 8,2615 1,06; 8,2383 1,07; 7,9739 3,19; 7,9178 1,67; 7,8984 2,02; 7,8036 8,10; 7,7891 1,63; 7,7691 2,24; 7,6951 1,82; 7,6755 2,70; 7,6559 1,09; 7,5778 6,44; 7,3743 2,32; 7,2042 6,38; 6,1764 0,34; 6,1550 1,26; 6,1330 1,74; 6,1110 1,30; 6,0897 0,38; 4,4911 1,46; 4,4734 4,72; 4,4556 4,82; 4,4379 1,56; 4,1794 2,38; 4,1616 7,49; 4,1438 7,62; 4,1261 2,52; 2,7272 1,66; 2,2079 105,08; 2,2051 173,86; 2,1998 226,53; 2,1211 0,34; 2,1148 0,38; 2,1087 0,43; 2,1025 0,34; 1,9656 2,26; 1,9595 2,36; 1,9537 20,05; 1,9475 37,63; 1,9413 52,81; 1,9351 37,22; 1,9289 19,88; 1,7697 0,33; 1,5411 2,16; 1,5290 6,01; 1,5208 6,43; 1,5098 2,69; 1,3399 0,43; 1,3001 5,46; 1,2824 12,39; 1,2730 8,84; 1,2646 5,87; 1,2552 16,00; 1,2444 3,31; 1,2374 8,66; 1,2335 7,07; 1,2252 6,35; 1,2131 2,32; -0,0002 0,47 |
| Verbindung Nr. 36 [CD3CN] 8,1771 1,48; 8,1536 1,50; 7,9698 3,98; 7,8988 2,47; 7,8799 2,51; 7,8698 10,29; 7,7895 1,90; 7,7700 2,83: 7,6960 2,32; 7,6846 0,73; 7,6764 3,47; 7,6567 1,42; 7,6415 7,92; 7,5847 2,31; 7,5540 0,56; 7,2632 0,42; 7,2436 7,70; 6,1794 0,44; 6,1583 1,61; 6,1363 2,23; 6,1141 1,67; 6,0929 0,50; 4,5137 1,96; 4,4959 6,14; 4,4781 6,22; 4,4603 2,00; 2,7968 1,43; 2,7905 0,77; 2,7821 1,77; 2,7752 2,20; 2,7711 1,45; 2,7686 1,58; 2,7637 2,24; 2,7609 2,26; 2,7535 1,53; 2,7494 2,69; 2,7430 2,31; 2,7359 0,95; 2,7285 2,02; 2,5332 1,65; 2,5261 0,76; 2,5139 2,44; 2,5103 3,11; 2,5013 1,78; 2,4907 2,48; 2,4817 2,58; 2,4776 2,09; 2,4647 0,90; 2,4583 1,74; 2,3550 0,54; 2,3029 1,34; 2,2273 1287,97; 2,1851 6,41; 2,1694 3,77; 2,1652 4,69; 2,1550 2,51; 2,1460 4,42; 2,1377 1,76; 2,1320 2,07; 2,1233 2,08; 2,1158 1,62; 2,1092 2,07; 2,1032 1,43; 2,0971 0,86; 2,0796 0,52; 1,9778 0,37; 1,9663 19,95; 1,9602 6,07; 1,9543 62,78; 1,9482 120,34; 1,9420 170,65; 1,9358 119,46; 1,9296 62,77; 1,7827 0,48; 1,7766 0,80; 1,7704 1,12; 1,7642 0,79; 1,7581 0,48; 1,3290 0,38; 1,3228 0,57; 1,3105 7,44; 1,2927 16,00; 1,2749 7,57; -0,0002 0,50 |
| Verbindung Nr. 37 [CD3CN] 8,6889 1,03; 8,6646 1,06; 7,8020 9,28; 7,7956 2,00; 7,7903 2,05; 7,7781 1,70; 7,7726 1,75; 7,7326 9,09; 7,6121 0,85; 7,6064 0,88; 7,6009 0,95; 7,5908 1,16; 7,5849 1,09; 7,5796 1,11; 7,5738 0,96; 7,3655 2,95; 7,3431 4,41; 7,3211 2,48; 6,9170 1,22; 6,0344 0,35; 6,0137 1,19; 5,9931 1,43: 5,9902 1,38; 5,9696 1,20; 5,9491 0,37; 4,6651 1,48; 4,6474 4,19; 4,6460 4,15; 4,6280 4,35; 4,6103 1,53; 3,8138 0,76; 2,8902 0,33; 2,8804 0,99; 2,8709 1,35; 2,8624 2,14; 2,8528 2,20; 2,8443 1,33; 2,8348 1,05; 2,8251 0,35; 2,1831 633,66; 2,1361 1,24; 2,1206 0,80; 2,1144 1,00; 2,1082 1,22; 2,1021 0,91; 2,0959 0,56; 1,9723 0,40; 1,9651 3,88; 1,9591 4,61; 1,9532 54,93; 1,9470 105,13; 1,9409 148,37; 1,9347 102,58; 1,9285 53,01; 1,9156 1,08; 1,7816 0,36; 1,7754 0,64; 1,7693 0,92; 1,7631 0,65; 1,7569 0,35; 1,4369 16,00; 1,4054 6,74; 1,3875 15,23; 1,3695 6,56; 1,2699 1,44; 0,7976 1,17; 0,7852 3,35; 0,7798 4,56; 0,7673 4,79; 0,7619 3,29; 0,7497 1,64; 0,6233 1,57; 0,6133 3,57; 0,6116 3,68; 0,6060 3,86; 0,6017 3,43; 0,5964 3,51; 0,5842 1,11; -0,0002 1,95 |
| Verbindung Nr. 38 [CD3CN] 8,9188 1,19; 8,8949 1,19; 8,0219 1,76; 8,0051 1,74; 7,9656 0,90; 7,9551 1,08; 7,9460 1,25; 7,9328 1,12; 7,8106 9,49; 7,7983 0,44; 7,7393 9,63; 7,7388 9,63; 7,4584 1,98; 7,4340 2,24; 7,4112 1,65; 7,3980 0,52; 7,3761 0,33; 7,0326 1,41; 6,1454 0,38; 6,1251 1,31; 6,1044 1,62; 6,0811 1,32; 6,0604 0,42; 5,4517 5,94; 4,6619 1,63; 4,6442 4,51; 4,6248 4,65; 4,6072 1,67; 4,0691 0,69: 4,0513 0,70; 3,7405 0,88; 2,8914 0,43; 2,8814 1,04; 2,8719 1,45; 2,8634 2,28; 2,8538 2,35; 2,8453 1,43; 2,8358 1,13; 2,8260 0,39; 2,4848 1,89; 2,4800 3,77; 2,4752 5,38; 2,4705 3,92; 2,4658 2,08; 2,2778 2202,44; 2,1965 3,08; 2,1225 1,17; 2,1164 1,75; 2,1102 2,26; 2,1040 1,68; 2,0978 1,09; 1,9735 3,75; 1,9671 8,20; 1,9610 9,45; 1,9552 111,09; 1,9490 213,30; 1,9428 303,85; 1,9366 212,15; 1,9304 110,51; 1,9176 2,53; 1,7836 0,78; 1,7774 1,37; 1,7712 1,95; 1,7651 1,36; 1,7589 0,78; 1,5433 0,33; 1,4367 0,85; 1,4029 7,09; 1,3850 16,00; 1,3671 7,10; 1,3516 0,68; 1,3399 0,56; 1,3341 0,40; 1,2850 1,06; 1,2763 2,37; 1,2702 4,09; 1,2536 0,41; 1,2221 1,08; 1,2042 1,91; 1,1864 0,98; 0,8813 0,57; 0,8633 0,32; 0,7968 1,27; 0,7845 3,59; 0,7791 4,90; 0,7666 5,12; 0,7612 3,59; 0,7491 1,76; 0,6237 1,70; 0,6120 4,02; 0,6063 4,22; 0,6021 3,77; 0,5967 3,80: 0,5845 1,19; -0,0002 1,57 |
| Verbindung Nr. 39 [DMSO] 9,7457 1,86; 9,7216 1,91; 9,2863 4,17; 7,8934 3,63; 7,8414 13,84; 7,7099 1,59; 7,6938 1,81; 7,5332 0,68; 7,5284 0,64; 7,5140 7,38; 7,5084 2,45; 7,4981 3,17; 7,4780 0,76; 7,3880 5,50; 6,1853 0,81; 6,1730 0,58; 6,1632 1,20; 6,1525 0,56; 6,1409 0,86; 4,5444 1,01; 4,5271 3,02; 4,5093 3,01; 4,4916 0,99; 3,3212 35,22; 3,2975 4,16; 2,6708 0,42; 2,5238 1,18; 2,5105 24,10; 2,5061 48,56; 2,5017 64,11; 2,4972 46,11; 2,4929 22,30; 2,3327 0,32; 2,3285 0.42; 2,3236 0,33; 1,5997 1,71; 1,5856 4,32; 1,5786 4,64; 1,5656 1,96; 1,3972 16,00; 1,2880 1,97; 1,2747 4,22; 1,2681 4,40; 1,2535 1,61; 1,2280 4,23; 1,2107 9,21; 1,1930 4,13; 0,0079 1,64; -0,0002 44,65; - 0,0084 1,64 |
| Verbindung Nr. 40 [DMSO] 9,7064 3,95; 9,6822 4,05; 8,3926 4,38; 8,3815 4,43; 8,0263 6,39; 7,7860 11,15; 7,7567 15,77; 7,7306 3,83; 7,6602 3,18; 7,6577 3,05; 7,6401 3,70; 7,6376 3,77; 7,4515 4,55; 7,4318 7,49; 7,4120 3,51; 7,3754 10,81; 7,3631 0,69; 6,1910 0,41; 6,1699 1,60; 6,1477 2,41; 6,1252 1,74; 6,1027 0.48; 4,5370 1,77; 4,5196 5,40; 4,5019 5,40; 4,4845 1,76; 4,0556 0,40; 4,0379 1,23; 4,0201 1,23; 4,0023 0,43; 3,3202 93,01; 3,2965 0,67; 2,8659 0,42; 2,8560 1,19; 2,8464 1,63; 2,8378 2,60; 2,8276 2,63; 2,8194 1,65; 2,8094 1,25; 2,7993 0,47; 2,6748 0,73; 2,6705 1,00; 2,6658 0,72; 2,5405 0,53; 2,5237 2,39; 2,5102 54,27; 2,5058 109,67; 2,5013 144,43; 2,4968 103,66; 2,4925 49,60; 2,3326 0,80; 2,3281 1,03; 2,3236 0,77; 1,9888 5,45; 1,4003 0,98; 1,3357 1,00; 1,2985 0,50; 1,2583 0,93; 1,2493 1,42; 1,2341 0,83; 1,2199 7,32; 1,2025 16,00; 1,1922 3,38; 1,1849 7,16; 1,1745 3,45; 1,1567 1,57; 0,7181 1,73; 0,7052 4,85; 0,7000 6,78; 0,6880 6,25; 0,6820 5,30; 0,6708 2,20; 0,5562 2,30; 0,5456 6,83; 0,5393 6,13; 0,5300 5,47; 0,5178 1,68; -0,0002 1,81 |
| Verbindung Nr. 41 [DMSO] 9,7421 2,31; 9,7180 2,36; 9,2859 5,24; 8,0247 3,68; 7,8417 16,00; 7,7495 1,92; 7,7303 2,17; 7,6619 1,82; 7,6594 1,70; 7,6438 2,03; 7,6418 2,11; 7,6392 2,15; 7,4534 2,60; 7,4337 4,31; 7,4139 2,02; 7,3858 6,36; 6,1751 0,92; 6,1528 1,39; 6,1301 0,99; 4,5426 1,03; 4,5254 3,02; 4,5078 2,99; 4,4903 1,00; 3,3206 67,32; 2,8904 2,49; 2,7313 2,01; 2,6750 0,49; 2,6706 0,63; 2,6661 0,46; 2,5407 0,43; 2,5103 35,69; 2,5059 70,33; 2,5015 91,34; 2,4969 64,57; 2,4925 30,39; 2,3326 0,48; 2,3282 0,63; 2,3238 0,45; 1,5992 1,77; 1,5850 4,33; 1,5782 4,63; 1,5652 1,98; 1,2981 0,48; 1,2876 2,14; 1,2742 4,39; 1,2675 4,64; 1,2585 1,05; 1,2529 1,76; 1,2274 4,20; 1,2100 9,01; 1,1923 4,01; -0,0002 0,78 |
| Verbindung Nr. 42 [DMSO] 9,7154 2,28; 9,6912 2,36; 9,2860 5,27; 8,1984 1,75; 8,1936 1,85; 8,1818 1,81; 8,1771 1,79; 7,8428 16,00; 7,8271 1,11; 7,8174 1,21; 7,8117 1,13; 7,8053 1,08; 7,8003 0,95; 7,5212 2,33; 7,4995 4,33; 7,4778 2,10; 7,3757 6,40; 6,2220 0,91; 6,1995 1,38; 6,1772 0,97; 4,5414 1,00; 4,5241 3,01; 4,5064 3,01; 4,4887 0,98; 4,0380 0,86; 4,0202 0,87; 3,3202 49,11; 3,2967 0,34; 2,6751 0,46; 2,6707 0,61; 2,6663 0,45; 2,5239 1,46; 2,5105 32,58; 2,5061 66,20; 2,5016 87,59; 2,4971 62,89; 2,4927 30,01; 2,3329 0,44; 2,3284 0,61; 2,3241 0,43; 1,9890 3,79; 1,6000 1,77; 1,5858 4,34; 1,5790 4,67; 1,5660 1,95; 1,3973 6,45; 1,2982 0,63; 1,2870 2,17; 1,2736 4,45; 1,2669 4,71; 1,2587 1,25; 1,2525 1,82; 1,2282 4,30; 1,2108 9,23; 1,1928 5,00; 1,1747 2,20; 1,1569 1,07; -0,0002 0,64 |
| Verbindung Nr. 43 [DMSO] 601,6MHz9,7088 3,55; 9,6926 3,72; 9,2857 8,18; 7,9153 12,63; 7,9125 12,15; 7,8497 14,94; 7,8459 10,09; 7,7894 0,48; 7,7656 0,69; 7,7511 0,50; 7,7324 4,65; 7,7293 8,71; 7,7262 4,47; 7,3893 8,90; 7,3787 0,52; 6,2682 1,14; 6,2539 1,72; 6,2390 1,24; 6,2245 0,35; 4,5318 1,64; 4,5202 4,94; 4,5083 4,93; 4,4965 1,61; 3,3176 270,67; 3,3038 0,66; 3,2938 10,10; 2,6188 0,87; 2,6158 1,91; 2,6127 2,65; 2,6097 1,91; 2,6067 0,88; 2,5404 0,76; 2,5221 3,50; 2,5190 4,48; 2,5159 4,78; 2,5071 140,39; 2,5040 305,21; 2,5010 425,77; 2,4979 304,51; 2,4949 139,92; 2,3912 0,80; 2,3882 1,82; 2,3852 2,56; 2,3821 1,81; 2,3791 0,79; 1,5934 2,88; 1,5841 6,60; 1,5796 7,45; 1,5708 2,91; 1,2812 3,23; 1,2721 6,65; 1,2678 7,24; 1,2582 2,67; 1,2333 1,07; 1,2249 7,45; 1,2132 16,00; 1,2015 7,58; 1,1941 0,74; 0,6979 0,42; 0,6896 0,38; 0,6859 0,32; 0,5429 0,40; 0,5390 0,37; 0,5360 0,34; 0,5328 0,37; 0,0965 0,36; 0,0052 2,82; -0,0002 98,55; -0,0058 2,84; -0,1001 0,37 |
| Verbindung Nr. 44 [DMSO] 9,8069 1,75; 9,7827 1,77; 9,2977 3,88; 8,2115 2,69; 8,0711 1,46; 8,0516 1,59; 7,8521 6,19; 7,8339 1,37; 7,8069 5,19; 7,7378 1,38; 7,7183 2,12; 7,6987 0,88; 7,4165 4,75; 6,3179 0,73; 6,2956 1,10; 6,2733 0,77; 3,9506 16,00; 3,3229 57,78; 3,2999 0,87; 2,6710 0,46; 2,6666 0,33; 2,5063 53,36; 2,5020 67,56; 2,4976 49,02; 2,3287 0,46; 2,0749 0,36; 1,6006 1,31; 1,5865 3,43; 1,5797 3,66; 1,5666 1,50; 1,2929 1,62; 1,2795 3,44; 1,2727 3,62; 1,2583 1,27; 0,0077 0,86; -0,0002 20,79; -0,0083 0,89 |
| Verbindung Nr. 45 [CD3CN] 601,6MHz7,9352 0,45; 7,9190 0,46; 7,7552 3,80; 7,6635 1,36; 7,5683 2,77; 7,5542 0,61; 7,5435 0,70; 7,4802 0,36; 7,4705 1,77; 7,4671 3,39; 7,4637 1,27; 7,4573 1,42; 7,4440 0,32; 7,2106 2,58; 7,2095 2,58; 6,8092 0,42; 6,0289 0,52; 6,0145 0,66; 5,9996 0,50; 3,9539 0,88; 3,9331 16,00; 3,2798 1,74; 3,2707 1,66; 2,8640 0,43; 2,8576 0,55; 2,8520 0,94; 2,8456 0,97; 2,8399 0,56; 2,8336 0,45; 2,1445 0,57; 2,1403 0,52; 2,1284 469,72; 2,1154 0,61; 2,0970 1,26; 2,0952 1,43; 2,0935 1,21; 2,0578 1,12; 2,0536 2,17; 2,0495 3,37; 2,0455 2,29; 2,0414 1,11; 1,9673 0,35; 1,9632 18,26; 1,9551 13,85; 1,9510 16,92; 1,9472 206,75; 1,9431 406,53; 1,9390 590,12; 1,9349 383,37; 1,9307 193,46; 1,9261 5,03; 1,9219 2,17; 1,9170 0,58; 1,9128 0,49; 1,9087 0,36; 1,8325 1,11; 1,8283 2,13; 1,8242 3,32; 1,8201 2,27; 1,8160 1,10; 1,5417 1,26; 1,2705 0,49; 0,7789 0,52; 0,7702 1,37; 0,7673 1,90; 0,7588 1,77; 0,7555 1,43; 0,7472 0,61; 0,5986 0,60; 0,5919 1,32; 0,5905 1,39; 0,5875 1,41; 0,5840 1,37; 0,5811 1,38; 0,5726 0,47; 0,0053 0,56; -0,0002 20,93; -0,0058 0,54 |
| Verbindung Nr. 46 [DMSO] 9,7177 1,65; 9,6936 1,70; 9,3061 3,76; 7,9008 2,66; 7,8451 6,36; 7,8077 4,50; 7,7126 1,22; 7,7075 1,01; 7,6962 1,37; 7,5323 0,59; 7,5273 0,54; 7,5170 2,36; 7,5127 5,56; 7,5073 2,04; 7,4957 2,30; 7,4757 0,61; 7,4146 4,28; 6,1804 0,65; 6,1578 0,98; 6,1357 0,71; 3,9517 16,00; 3,3290 77,33; 3,0294 0,55; 2,7612 0,45; 2,6757 0,39; 2,6711 0,54; 2,6666 0,40; 2,5411 0,34; 2,5244 2,18; 2,5197 3,53; 2,5111 29,97; 2,5066 59,78; 2,5020 78,80; 2,4975 57,13; 2,4930 27,54; 2,3334 0,41; 2,3287 0,56; 2,3242 0,41; 2,0758 1,77; 1,6010 1,29; 1,5869 3,02; 1,5799 3,23; 1,5670 1,40; 1,2916 1,50; 1,2781 3,02; 1,2715 3,24; 1,2570 1,18; -0,0002 4,80 |
| Verbindung Nr. 47 [DMSO] 9,6807 1,52; 9,6565 1,59; 8,4128 1,74; 8,4017 1,77; 8,0338 2,48; 7,7598 6,87; 7,7535 5,80; 7,7333 1,48; 7,6594 1,24; 7,6568 1,16; 7,6412 1,35; 7,6394 1,42; 7,6367 1,47; 7,4490 1,92; 7,4293 3,17; 7,4095 1,77; 7,4010 4,22; 6,1651 0,61; 6,1427 0,92; 6,1200 0,67; 5,7594 0,46; 3,9558 0,73; 3,9433 16,00; 3,3282 84,57; 2,8574 0,49; 2,8475 0,63; 2,8392 1,04; 2,8289 1,04; 2,8207 0,63; 2,8107 0,51; 2,6752 0,37; 2,6708 0,51; 2,6663 0,38; 2,5409 0,36; 2,5241 1,87; 2,5193 2,86; 2,5107 27,89; 2,5063 55,35; 2,5017 72,54; 2,4972 51,80; 2,4927 24,17; 2,3331 0,36; 2,3285 0,50; 2,3238 0,35; 2,0756 7,64; 0,7193 0,75; 0,7067 1,95; 0,7013 2,70; 0,6894 2,48; 0,6832 2,08; 0,6721 0,90; 0,5614 0,97; 0,5509 2,72; 0,5448 2,32; 0,5408 2,17; 0,5352 2,06; 0,5230 0,65; 0,0078 0,42; -0,0002 11,44; -0,0085 0,34 |
| Verbindung Nr. 48 [DMSO] 9,7155 1,75; 9,6913 1,83; 9,3066 4,00; 8,0333 2,73; 7,8470 6,64; 7,8081 4,79; 7,7530 1,43; 7,7335 1,61; 7,6612 1,31; 7,6586 1,27; 7,6431 1,47; 7,6411 1,53; 7,6385 1,60; 7,4514 2,04; 7,4316 3,39; 7,4124 5,72; 6,1709 0,68; 6,1488 1,03; 6,1259 0,74; 3,9517 16,00; 3,3294 43,11; 2,6714 0,35; 2,5246 1,40; 2,5198 2,20; 2,5113 18,47; 2,5068 36,37; 2,5023 47,55; 2,4978 34,41; 2,4934 16,82; 2,3291 0,35; 2,0760 6,18; 1,6014 1,35; 1,5874 3,20; 1,5804 3,45; 1,5675 1,48; 1,2924 1,57; 1,2790 3,18; 1,2722 3,42; 1,2579 1,23; -0,0002 2,97 |
| Verbindung Nr. 49 [DMSO] 9,7478 1,71; 9,7236 1,77; 8,4075 1,90; 8,3966 1,90; 8,3062 1,33; 8,2918 1,30; 8,1819 0,75; 8,1755 0,82; 8,1680 0,94; 8,1609 0,85; 8,1554 0,82; 7,7986 0,34; 7,7682 6,24; 7,7555 4,83; 7,6808 1,15; 7,6556 1,51; 7,6326 1,03; 7,3905 4,74; 6,3533 0,74; 6,3314 1,11; 6,3089 0,78; 4,0573 0,36; 4,0396 1,07; 4,0218 1,08; 4,0040 0,39; 3,9571 1,23; 3,9451 16,00; 3,3256 19,43; 2,8610 0,55; 2,8515 0,78; 2,8428 1,19; 2,8327 1,18; 2,8244 0,79; 2,8146 0,56; 2,5081 17,65; 2,5037 22,63; 2,4993 16,52; 1,9903 4,64; 1,3969 7,24; 1,1937 1,24; 1,1759 2,45; 1,1581 1,20; 0,7221 0,76; 0,7093 2,22; 0,7042 2,99; 0,6922 2,79; 0,6862 2,40; 0,6751 0,99; 0,5651 1,03; 0,5546 3,10; 0,5482 2,87; 0,5390 2,54; 0,5268 0,81; 0,0078 0,41; -0,0002 8,76; -0,0082 0,42 |
| Verbindung Nr. 50 [DMSO] 9,7819 1,66; 9,7577 1,72; 9,3013 3,79; 8,3043 1,17; 8,2884 1,13; 8,1818 0,62; 8,1754 0,69; 8,1679 0,80; 8,1603 0,72; 8,1543 0,70; 7,8549 6,24; 7,8097 4,65; 7,6832 1,05; 7,6577 1,34; 7,6349 0,98; 7,4016 4,48; 6,3584 0,65; 6,3362 0,98; 6,3138 0,71; 4,0574 0,65; 4,0395 1,99; 4,0217 2,02; 4,0039 0,69; 3,9526 16,00; 3,3247 8,89; 2,5259 0,34; 2,5127 7,59; 2,5083 15,50; 2,5038 20,53; 2,4993 14,66; 2,4949 6,96; 1,9904 8,57; 1,6037 1,30; 1,5896 3,15; 1,5826 3,38; 1,5697 1,43; 1,3968 1,47; 1,2946 1,57; 1,2812 3,22; 1,2745 3,42; 1,2599 1,41; 1,1938 2,39; 1,1760 4,76; 1,1581 2,33; -0,0002 3,14 |
| Verbindung Nr. 51 [DMSO] 9,6543 1,61; 9,6300 1,66; 8,4134 1,78; 8,4024 1,78; 8,2082 1,25; 8,2032 1,29; 8,1918 1,27; 8,1868 1,23; 7,8429 0,63; 7,8377 0,66; 7,8312 0,72; 7,8223 0,84; 7,8160 0,79; 7,8098 0,76; 7,8050 0,68; 7,7615 6,44; 7,7549 4,66; 7,5173 1,68; 7,4956 3,10; 7,4739 1,51; 7,3895 4,35; 6,2129 0,64; 6,1909 0,96; 6,1681 0,69; 3,9558 0,72; 3,9429 16,00; 3,3285 48,82; 2,8579 0,49; 2,8483 0,66; 2,8398 1,05; 2,8295 1,06; 2,8213 0,65; 2,8113 0,50; 2,6714 0,35; 2,5246 1,01; 2,5112 19,18; 2,5068 38,58; 2,5022 51,16; 2,4977 37,41; 2,4933 18,10; 2,3289 0,37; 2,0761 6,37; 1,4756 0,43; 0,7201 0,71; 0,7074 1,90; 0,7021 2,72; 0,6901 2,47; 0,6840 2,13; 0,6728 0,88; 0,5617 0,94; 0,5512 2,75; 0,5450 2,42; 0,5412 2,28; 0,5356 2,16; 0,5234 0,67; 0,0080 0,36; -0,0002 10,08; -0,0085 0,36 |
| Verbindung Nr. 52 [DMSO] 9,6888 1,62; 9,6645 1,66; 9,3070 3,80; 8,2063 1,21; 8,2013 1,29; 8,1899 1,25; 8,1848 1,23; 7,8470 6,42; 7,8369 0,82; 7,8307 0,80; 7,8206 0,92; 7,8086 5,19; 7,5193 1,74; 7,4976 3,19; 7,4759 1,56; 7,4009 4,13; 7,3998 4,15; 6,2175 0,62; 6,1953 0,93; 6,1729 0,67; 3,9500 16,00; 3,3322 147,58; 2,6760 0,39; 2,6715 0,52; 2,6670 0,40; 2,5417 0,51; 2,5249 2,41; 2,5202 3,47; 2,5115 27,71; 2,5070 55,43; 2,5024 73,26; 2,4978 53,27; 2,4933 26,16; 2,3338 0,37; 2,3291 0,51; 2,3246 0,39; 2,0760 1,51; 1,6017 1,30; 1,5876 3,04; 1,5807 3,26; 1,5678 1,42; 1,2914 1,52; 1,2779 3,07; 1,2712 3,28; 1,2568 1,21; -0,0002 4,96 |
| Verbindung Nr. 53 [DMSO] 9,6580 1,62; 9,6337 1,66; 8,4145 1,84; 8,4035 1,87; 8,0974 1,29; 8,0927 1,33; 8,0798 1,33; 8,0750 1,29; 7,8131 0,66; 7,8079 0,71; 7,8014 0,85; 7,7926 1,06; 7,7863 0,89; 7,7805 0,87; 7,7751 0,82; 7,7616 6,63; 7,7554 4,91; 7,5605 1,64; 7,5379 2,63; 7,5156 1,41; 7,3914 4,60; 6,2176 0,66; 6,1958 1,00; 6,1735 0,72; 3,9572 0,76; 3,9442 16,00; 3,3294 27,25; 2,8585 0,52; 2,8492 0,71; 2,8402 1,12; 2,8301 1,12; 2,8217 0,71; 2,8120 0,54; 2,5248 0,92; 2,5111 15,08; 2,5070 29,42; 2,5026 38,48; 2,4981 28,16; 2,4938 13,84; 2,0766 11,26; 0,7205 0,81; 0,7077 2,14; 0,7026 2,90; 0,6905 2,68; 0,6845 2,28; 0,6734 0,95; 0,5622 0,99; 0,5517 2,92; 0,5455 2,60; 0,5360 2,32; 0,5239 0,70; -0,0002 5,57 |
| Verbindung Nr. 54 [DMSO] 9,6934 1,71; 9,6691 1,77; 9,3093 3,86; 8,0972 1,30; 8,0925 1,37; 8,0796 1,34; 8,0747 1,32; 7,8493 6,31; 7,8105 5,31; 7,7971 0,86; 7,7933 0,91; 7,7872 0,88; 7,7814 0,84; 7,7764 0,72; 7,5635 1,70; 7,5410 2,60; 7,5187 1,46; 7,4049 4,53; 6,2242 0,68; 6,2023 1,02; 6,1799 0,73; 3,9531 16,00; 3,3315 12,11; 2,5258 0,85; 2,5211 1,27; 2,5123 12,27; 2,5080 23,95; 2,5035 30,69; 2,4990 22,10; 2,4947 10,69; 2,0776 1,14; 1,6039 1,32; 1,5898 3,23; 1,5829 3,46; 1,5700 1,45; 1,2935 1,56; 1,2801 3,26; 1,2735 3,44; 1,2589 1,22; 0,0080 0,64; -0,0002 16,93; -0,0085 0,60 |
| Verbindung Nr. 57 vgl. Synthesebeispiel 6 |
| Verbindung Nr. 58 [DMSO] 9,7970 2,74; 9,7729 2,83; 9,3210 6,19; 7,8916 5,10; 7,8818 16,00; 7,7126 2,00; 7,6964 2,23; 7,5342 0,82; 7,5291 0,78; 7,5154 8,89; 7,5096 3,06; 7,4954 8,57; 7,4799 1,02; 6,1908 1,15; 6,1689 1,75; 6,1462 1,26; 6,1244 0,36; 5,7571 1,24; 5,4521 6,47; 3,3200 67,51; 3,2964 0,58; 3,2185 2,35; 3,2127 4,86; 3,2071 2,20; 2,6746 0,62; 2,6704 0,82; 2,6663 0,60; 2,5405 0,58; 2,5099 46,23; 2,5057 91,90; 2,5013 120,36; 2,4969 86,86; 2,3324 0,61; 2,3280 0,81; 2,3238 0,60; 1,6055 2,13; 1,5912 5,38; 1,5844 5,75; 1,5715 2,39; 1,2917 2,60; 1,2784 5,49; 1,2717 5,79; 1,2572 2,07; -0,0002 7,42 |
| Verbindung Nr. 59 [DMSO] 9,7587 1,84; 9,7345 1,89; 8,4255 2,13; 8,4145 2,11; 8,0259 3,08; 7,8262 5,35; 7,7973 6,91; 7,7533 1,65; 7,7337 1,83; 7,6614 1,55; 7,6591 1,50; 7,6414 1,80; 7,6390 1,83; 7,4845 5,09; 7,4536 2,14; 7,4339 3,53; 7,4141 1,63; 7,3630 0,61; 6,1763 0,77; 6,1544 1,16; 6,1319 0,84; 5,4453 4,32; 3,3199 32,28; 3,2047 1,58; 3,1988 3,42; 3,1930 1,50; 2,8601 0,58; 2,8509 0,81; 2,8421 1,25; 2,8318 1,27; 2,8230 0,81; 2,8138 0,61; 2,6744 0,34; 2,6703 0,44; 2,6658 0,35; 2,5234 1,11; 2,5098 24,06; 2,5056 48,23; 2,5012 63,50; 2,4967 46,01; 2,4926 22,53; 2,3325 0,36; 2,3280 0,47; 2,3238 0,35; 1,9887 0,61; 1,3973 16,00; 1,3358 0,66; 1,2985 0,37; 1,2584 0,53; 1,2494 0,79; 1,2350 0,42; 1,1744 0,38; 0,7234 0,86; 0,7102 2,38; 0,7053 3,28; 0,6932 3,03; 0,6873 2,61; 0,6761 1,06; 0,5582 1,12; 0,5476 3,30; 0,5414 3,01; 0,5321 2,72; 0,5197 0,82; -0,0002 0,77 |
| Verbindung Nr. 60 [DMSO] 9,7921 2,95; 9,7679 3,08; 9,3201 6,98; 8,0233 5,42; 7,8827 16,00; 7,7524 2,91; 7,7330 3,34; 7,6626 2,82; 7,6602 2,70; 7,6425 3,28; 7,6400 3,37; 7,4941 8,74; 7,4551 4,26; 7,4354 7,02; 7,4156 3,27; 6,2012 0,36; 6,1800 1,23; 6,1586 1,91; 6,1362 1,35; 6,1144 0,41; 5,4506 7,81; 3,3190 330,09; 3,2954 33,08; 3,2169 3,23; 3,2109 7,17; 3,2050 3,00; 2,6747 3,23; 2,6701 4,45; 2,6655 3,26; 2,6611 1,51; 2,5403 2,58; 2,5234 11,20; 2,5100 237,85; 2,5056 489,39; 2,5010 649,51; 2,4965 460,97; 2,4920 215,78; 2,3323 3,21; 2,3278 4,43; 2,3232 3,20; 2,0742 0,47; 1,6050 2,72; 1,5910 6,60; 1,5840 7,11; 1,5709 3,02; 1,2911 3,06; 1,2778 6,43; 1,2712 6,74; 1,2567 2,42; 0,1461 1,63; 0,0080 14,72; -0,0002 439,89; -0,0085 14,03; -0,1496 1,72 |
| Verbindung Nr. 61 [DMSO] 9,8666 1,29; 9,8425 1,32; 9,3231 2,97; 8,2961 0,94; 8,2818 0,92; 8,1794 0,51; 8,1729 0,56; 8,1650 0,65; 8,1577 0,58; 8,1521 0,57; 7,8935 5,03; 7,8863 3,74; 7,6886 0,83; 7,6631 1,09; 7,6402 0,76; 7,4810 3,57; 6,3693 0,51; 6,3475 0,78; 6,3253 0,56; 5,4475 3,01; 3,3248 9,07; 3,2176 1,16; 3,2117 2,40; 3,2060 1,08; 2,5120 5,62; 2,5079 11,09; 2,5034 14,46; 2,4990 10,43; 1,6091 1,00; 1,5949 2,47; 1,5881 2,66; 1,5751 1,10; 1,3968 16,00; 1,2943 1,20; 1,2809 2,51; 1,2742 2,65; 1,2597 0,95; -0,0002 2,04 |
| Verbindung Nr. 62 [DMSO] 9,7682 2,39; 9,7440 2,46; 9,3209 5,48; 8,1959 2,03; 8,1914 2,04; 8,1795 2,08; 8,1749 1,97; 7,8836 13,57; 7,8401 1,02; 7,8347 1,05; 7,8284 1,14; 7,8196 1,34; 7,8133 1,25; 7,8071 1,21; 7,5230 2,57; 7,5012 4,87; 7,4805 8,91; 6,2276 1,00; 6,2051 1,49; 6,1830 1,07; 5,4481 5,84; 3,3204 92,12; 3,2968 4,01; 3,2214 2,16; 3,2156 4,49; 3,2098 1,98; 2,6891 6,05; 2,6750 0,61; 2,6705 0,80; 2,6662 0,59; 2,5405 0,44; 2,5236 1,97; 2,5100 43,69; 2,5059 86,70; 2,5015 113,27; 2,4970 80,43; 2,3327 0,59; 2,3282 0,80; 2,3238 0,58; 1,6062 1,96; 1,5921 4,94; 1,5852 5,29; 1,5722 2,25; 1,3975 16,00; 1,2913 2,34; 1,2779 4,94; 1,2713 5,22; 1,2570 1,98; 0,0077 0,35; - 0,0002 9,89; -0,0084 0,35 |
| Verbindung Nr. 63 [CD3CN] 601,6MHz8,0370 1,89; 8,0214 1,92; 7,7787 16,00; 7,7718 3,16; 7,7638 2,91; 7,7603 2,94; 7,7470 0,41; 7,6428 11,45; 7,5938 1,57; 7,5899 1,63; 7,5864 1,69; 7,5826 1,70; 7,5797 1,81; 7,5756 1,74; 7,5721 1,76; 7,5684 1,60; 7,3544 4,83; 7,3396 7,48; 7,3248 4,30; 7,2831 11,01; 7,2822 10,83; 7,1990 0,32; 6,8340 2,03; 6,0523 0,52; 6,0383 1,85; 6,0240 2,58; 6,0094 1,88; 5,9953 0,56; 5,3438 0,35; 5,3301 15,28; 5,3260 15,29; 3,9267 1,21; 2,8758 0,56; 2,8693 1,65; 2,8631 2,28; 2,8573 3,61; 2,8509 3,70; 2,8451 2,32; 2,8389 1,78; 2,8325 0,63; 2,5204 4,23; 2,5163 9,64; 2,5122 4,16; 2,1549 0,47; 2,1303 172,30; 2,0974 0,95; 2,0578 0,93; 2,0537 1,70; 2,0496 2,51; 2,0455 1,72; 2,0414 0,88; 1,9633 9,94; 1,9552 10,32; 1,9511 13,24; 1,9473 155,24; 1,9431 294,24; 1,9390 434,91; 1,9349 295,37; 1,9308 148,09; 1,9263 4,86; 1,9220 2,19; 1,9168 0,79; 1,9128 0,51; 1,8325 0,84; 1,8284 1,62; 1,8243 2,40; 1,8202 1,65; 1,8161 0,82; 1,2702 1,00; 0,7838 2,12; 0,7751 5,63; 0,7721 7,57; 0,7635 7,41; 0,7603 5,95; 0,7520 2,67; 0,7377 0,32; 0,6258 0,35; 0,6193 0,34; 0,5994 2,56; 0,5925 5,65; 0,5913 6,11; 0,5882 6,31; 0,5848 5,96; 0,5818 6,01; 0,5733 1,98; -0,0002 3,80 |
| Verbindung Nr. 64 [DMSO] 601,6MHz9,7659 2,32; 9,7499 2,42; 9,3182 5,41; 8,0786 1,98; 8,0753 1,79; 8,0669 2,01; 8,0636 1,67; 7,8815 16,00; 7,8014 0,95; 7,7979 1,04; 7,7940 1,10; 7,7901 1,19; 7,7873 1,21; 7,7835 1,19; 7,7797 1,14; 7,5574 2,62; 7,5425 4,18; 7,5277 2,42; 7,4826 2,26; 7,4799 5,99; 6,2220 0,80; 6,2073 1,21; 6,1925 0,84; 5,4799 0,33; 5,4760 0,33; 5,4499 3,80; 5,4463 6,46; 5,4427 3,86; 4,0348 0,88; 4,0229 0,86; 3,3171 236,70; 3,2932 23,65; 3,2201 2,86; 3,2161 6,05; 3,2121 2,71; 2,6187 0,88; 2,6157 1,98; 2,6126 2,81; 2,6095 1,99; 2,6065 0,87; 2,5402 0,86; 2,5219 3,87; 2,5188 4,93; 2,5157 4,89; 2,5070 143,70; 2,5039 317,56; 2,5009 443,31; 2,4978 317,16; 2,4947 143,64; 2,3911 0,86; 2,3881 1,94; 2,3850 2,75; 2,3820 1,94; 2,3789 0,86; 1,9884 4,02; 1,5989 2,16; 1,5896 5,05; 1,5851 5,69; 1,5762 2,28; 1,3977 2,75; 1,3875 0,36; 1,3344 0,64; 1,2980 4,65; 1,2852 2,48; 1,2759 5,21; 1,2717 5,52; 1,2621 2,34; 1,2584 7,00; 1,2358 1,37; 1,1863 1,25; 1,1744 2,40; 1,1626 1,16; 0,0965 0,56; 0,0053 4,52; -0,0002 169,32; -0,0057 4,88; -0,1000 0,57 |
| Verbindung Nr. 66 [DMSO] 9,7689 3,60; 9,7446 3.74; 9,3232 8,54; 9,2848 0,39; 7,9157 14,13; 7,9119 13,66; 7,8924 16,00; 7,8849 11,58; 7,8587 0,51; 7,7347 4,60; 7,7301 8,13; 7,7255 4,11; 7,4929 10,32; 6,3049 0,44; 6,2837 1,50; 6,2620 2,18; 6,2397 1,58; 6,2179 0,47; 5,4952 0,35; 5,4892 0,36; 5,4456 9,05; 5,4023 0,33; 5,3959 0,33; 3,3204 162,89; 3,2968 9,09; 3,2183 3,49; 3,2124 7,51; 3,2065 3,24; 2,6750 0,89; 2,6705 1,23; 2,6659 0,89; 2,5407 0,66; 2,5237 2,91; 2,5103 63,65; 2,5059 130,33; 2,5014 173,33; 2,4968 123,03; 2,4924 57,79; 2,3327 0,89; 2,3282 1,21; 2,3236 0,88; 2,0745 7,43; 1,6069 3,07; 1,5927 7,55; 1,5858 8,12; 1,5729 3,77; 1,3312 0,33; 1,2914 3,54; 1,2779 7,45; 1,2713 8,01; 1,2568 2,79; 0,0080 1,36; -0,0002 39,71; -0,0085 1,29 |
| Verbindung Nr. 91 [CD3CN] 8,7486 2,37; 8,7240 2,39; 7,9765 6,87; 7,9050 3,61; 7,8853 4,58; 7,8792 5,02; 7,8560 0,65; 7,8365 13,00; 7,7970 16,00; 7,7786 4,95; 7,7657 4,34; 7,7009 3,71; 7,6812 5,48; 7,6620 2,19; 6,9523 0,78; 6,8957 2,35; 6,1485 0,72; 6,1281 2,46; 6,1070 3,16; 6,0842 2,44; 6,0634 0,76; 5,5563 0,37; 5,5164 14,05; 5,5116 14,36; 5,4476 0,88; 2,8899 0,68; 2,8801 1.76; 2,8706 2,65; 2,8622 3,53; 2,8526 3,49; 2,8442 2,24; 2,8347 1,47; 2,8250 0,49; 2,6591 4,32; 2,6528 8,90; 2,6466 4,01; 2,1421 256,79; 2,1202 1,75; 2,1139 1,84; 2,1078 2,36; 2,1016 1,42; 2,0955 0,75; 1,9646 11,57; 1,9585 16,50; 1,9527 107,06; 1,9466 198,34; 1,9404 270,76; 1,9342 185,68; 1,9280 94,72; 1,7812 0,75; 1,7750 1,27; 1,7688 1,70; 1,7627 1,17; 1,7565 0,63; 1,4368 0,39; 1,3721 5,45; 1,3405 1,37; 1,2852 2,06; 1,2766 6,85; 1,2214 0,35; 1,2037 0,49; 0,8815 0,54; 0,8571 0,63; 0,8360 0,54; 0,8170 0,34; 0,8064 0,75; 0,7981 2,06; 0,7937 2,20; 0,7858 6,62; 0,7804 7,35; 0,7679 8,40; 0,7627 5,55; 0,7507 2,55; 0,7289 0,43; 0,7109 0,38; 0,6601 0,32; 0,6503 0,33; 0,6269 0,94; 0,6200 2,90; 0,6096 8,04; 0,6028 8,03; 0,5937 5,98; 0,5812 1,81; 0,5118 0,33; 0,1461 0,66; -0,0002 157,86; -0,0086 6,00; -0,1494 0,68 |
| Verbindung Nr. 97 [CD3CN] 8,8074 1,10; 8,7869 1,11; 8,0314 2,91; 8,0149 2,81; 7,9835 1,51; 7,9729 1.73; 7,9635 2,03; 7,9555 1,77; 7,9510 1,84; 7,9086 0,68; 7,8864 0,45; 7,8666 15,78; 7,8661 16,00; 7,8276 15,88; 7,7962 0,73; 7,4899 2,84; 7.4648 3,51; 7,4420 2,57; 6,9337 2,17; 6,1687 0,37; 6,1484 1,36; 6,1280 1,99; 6,1075 1,34; 6,0881 0,34; 5,5934 0,94; 5,5871 0,95; 5,5485 8,87; 5,5419 10,86; 5,5401 11,03; 5,5335 8,71; 5,4949 0,88; 5,4885 0,88; 4,1157 0,48; 4,0980 1,45; 4,0802 1,45; 4,0624 0,51; 2,9195 2,26; 2,9109 1,77; 2,9015 2,42; 2,8930 3,77; 2,8834 3,81; 2,8748 2,33; 2,8654 1,74; 2,8558 0,62; 2,8013 1,57; 2,7010 0,32; 2,6920 4,27; 2,6857 9,83; 2,6794 4,24; 2,2823 0,48; 2,1905 1004,56; 2,1892 1061,22; 2,1495 1,97; 2,1433 2,38; 2,1371 2,87; 2,1310 2,02; 2,1248 1,17; 2,0016 7,46; 1,9940 7,47; 1,9879 10,13; 1,9821 140,92; 1,9759 271,11; 1,9697 384,92; 1,9636 264,86; 1,9574 135,48; 1,9445 1,98; 1,8105 0,80; 1,8044 1,54; 1,7982 2,23; 1,7920 1,55; 1,7858 0,79; 1,4667 8,22; 1,3063 0,37; 1,2513 1,90; 1,2335 3,79; 1,2157 1,88; 0,8289 1,99; 0,8165 5,84; 0,8112 7,79; 0,7986 8,29; 0,7933 5,65; 0,7811 2,75; 0,7595 0,39; 0,7417 0,38; 0,6808 0,33; 0,6508 2,72; 0,6407 6,37; 0,6391 6,57; 0,6334 6,78; 0,6292 6,09; 0,6239 6,02; 0,6116 1,89; 0,0294 0,58 |
| Verbindung Nr. 101 [CD3CN] 8,7086 1,49; 8,6854 1,47; 7,8774 0,42; 7,8570 0,39; 7,8336 11,35; 7,7974 13,62; 7,7858 2,67; 7,7805 2,46; 7,7660 0,46; 7,6161 1,34; 7,6104 1,42; 7,6050 1,51; 7,5950 1,79; 7,5889 1,84; 7,5839 1,74; 7,5782 1,37; 7,3680 3,48; 7,3457 5,57; 7,3235 2,89; 6,9284 1,96; 6,0399 0,52; 6,0195 1,75; 5,9986 2,19; 5,9754 1,70; 5,9551 0,53; 5,5636 0,39; 5,5572 0,40; 5,5185 7,36; 5,5131 10,44; 5,5079 7,02; 5,4691 0,38; 5,4629 0,38; 2,8902 0,41; 2,8807 1,20; 2,8713 1,79; 2,8627 2,63; 2,8531 2,67; 2,8445 1,72; 2,8351 1,19; 2,8255 0,40; 2,6693 3,14; 2,6631 6,40; 2,6569 2,91; 2,6017 0,59; 2,4732 0,34; 2,4692 0,67; 2,4646 0,94; 2,4600 0,67; 2,4551 0,36; 2,1690 418,23; 2,1206 1,13; 2,1144 1,40; 2,1083 1,65; 2,1022 1,22; 2,0960 0,73; 1,9652 9,20; 1,9587 14,22; 1,9532 85,29; 1,9471 156,52; 1,9410 213,28; 1,9348 146,60; 1,9286 75,33; 1,7816 0,57; 1,7755 0,99; 1,7693 1,34; 1,7632 0,95; 1,7570 0,52; 1,4366 16,00; 1,3717 0,84; 1,2765 1,13; 0,7982 1,52; 0,7856 4,60; 0,7805 5,92; 0,7679 6,20; 0,7627 4,55; 0,7505 2,06; 0,7289 0,35; 0,6205 2,05; 0,6089 5,58; 0,6032 5,70; 0,5992 5,22; 0,5939 4,82; 0,5814 1,45; 0,1460 0,52; -0,0002 122,15; -0,0084 5,33; -0,1497 0,58 |
| Verbindung Nr. 183 [DMSO] 9,7242 2,09; 9,6999 2,16; 9,1083 4,26; 8,2077 3,27; 8,0689 1,75; 8,0493 1,96; 7,8321 1,61; 7,8123 2,34; 7,7385 1,96; 7,7275 7,41; 7,7193 2,99; 7,6997 1,17; 7,4652 4,63; 7,3422 5,76; 6,3167 0,86; 6,2944 1,29; 6,2718 0,92; 4,5201 0,99; 4,5027 3,05; 4,4850 3,05; 4,4676 0,99; 4,0565 0,38; 4,0387 1,16; 4,0209 1,19; 4,0031 0,39; 3,3229 24,97; 3,2992 1,30; 2,6896 0,63; 2,5245 0,68; 2,5113 14,16; 2,5070 28,91; 2,5024 38,42; 2,4979 27,55; 2,4935 13,30; 2,4695 16,00; 1,9894 5,08; 1,5764 1,59; 1,5623 4,07; 1,5555 4,36; 1,5426 1,87; 1,2949 2,13; 1,2815 4,40; 1,2749 4,62; 1,2604 1,77; 1,2348 4,45; 1,2174 9,24; 1,1998 4,14; 1,1929 1,98; 1,1751 2,90; 1,1573 1,44; 0,0080 1,01; -0,0002 28,99; -0,0085 1,02 |
| Verbindung Nr. 184 [DMSO] 9,5877 2,32; 9,5633 2,38; 8,2463 2,47; 8,2354 2,47; 7,8998 3,74; 7,7110 1,65; 7,6951 1,86; 7,6282 7,08; 7,5924 0,38; 7,5293 0,74; 7,5244 0,73; 7,5102 7,29; 7,5045 2,67; 7,4947 3,12; 7,4747 0,72; 7,4143 4,60; 7,3337 6,39; 6,1758 0,96; 6,1537 1,41; 6,1308 1,00; 4,5181 1,03; 4,5008 3,06; 4,4830 3,05; 4,4657 1,02; 3,9041 10,55; 3,5076 0,32; 3,3383 337,04; 3,1744 1,28; 3,1614 1,22; 2,8593 0,70; 2,8496 1,03; 2,8411 1,57; 2,8310 1,62; 2,8230 1,09; 2,8129 0,89; 2,8028 0,34; 2,6759 0,82; 2,6715 1,09; 2,6671 0,83; 2,5414 0,96; 2,5244 4,39; 2,5112 67,80; 2,5069 131,36; 2,5024 169,10; 2,4979 124,48; 2,4936 63,19; 2,4449 16,00; 2,3337 0,75; 2,3292 1,01; 2,3248 0,75; 2,2886 0,33; 1,2579 0,45; 1,2302 4,88; 1,2127 8,90; 1,1951 3,99; 1,1252 0,47; 1,1038 0,36; 1,0847 0,57; 0,8536 0,47; 0,8347 0,34; 0,7075 1,00; 0,6946 2,83; 0,6896 3,97; 0,6776 3,57; 0,6716 3,16; 0,6606 1,30; 0,5540 1,46; 0,5435 4,01; 0,5374 3,49; 0,5337 3,41; 0,5278 3,10; 0,5157 0,94; 0,0078 0,84; -0,0002 20,49; -0,0084 0,93 |
| Verbindung Nr. 185 [DMSO] 9,6302 2,19; 9,6059 2,24; 9,1185 4,39; 7,8969 3,68; 7,7179 7,56; 7,7110 2,07; 7,6949 2,21; 7,5370 0,33; 7,5314 0,81; 7,5266 0,86; 7,5124 7,29; 7,5067 2,61; 7,4970 3,21; 7,4768 1,08; 7,4646 4,47; 7,3412 5,93; 6,1814 0,94; 6,1589 1,40; 6,1364 0,98; 4,5217 1,00; 4,5046 2,93; 4,4869 2,92; 4,4694 1,02; 3,9042 8,49; 3,5086 0,34; 3,4184 0,35; 3,3433 493,98; 3,2899 0,52; 3,1746 1,50; 3,1616 1,43; 2,6763 0,84; 2,6719 1,15; 2,6674 0,88; 2,6624 0,54; 2,6583 0,63; 2,5421 0,94; 2,5247 4,72; 2,5116 74,43; 2,5073 143,87; 2,5028 184,99; 2,4983 134,34; 2,4939 66,92; 2,4664 16,00; 2,3385 0,44; 2,3340 0,86; 2,3296 1,17; 2,3250 0,85; 1,5759 1,61; 1,5617 4,09; 1,5549 4,41; 1,5420 1,88; 1,2932 2,43; 1,2799 4,56; 1,2732 4,86; 1,2587 2,11; 1,2382 5,94; 1,2211 9,23; 1,2035 4,04; 1,1531 0,34; 1,1399 0,47; 1,1352 0,57; 1,1166 0,34; 1,1122 0,38; 1,0935 0,66; 1,0744 0,35; 0,8536 0,58; 0,8351 0,40; 0,0079 0,92; -0,0002 23,77; -0,0085 0,99 |
| Verbindung Nr. 188 [DMSO] 9,6601 2,22; 9,6358 2,27; 8,3016 1,62; 8,2873 1,58; 8,2437 2,40; 8,2329 2,41; 8,1790 0,88; 8,1726 0,97; 8,1633 1,11; 8,1577 1,00; 8,1519 0,96; 7,6821 1,45; 7,6568 1,87; 7,6344 8,33; 7,4171 4,58; 7,3168 6,31; 6,3494 0,89; 6,3271 1,34; 6,3052 0,96; 4,5131 1,00; 4,4957 3,01; 4,4780 3,02; 4,4605 1,03; 3,9044 6,77; 3,3818 0,82; 3,3377 384,21; 3,1744 1,57; 3,1617 1,51; 2,8588 0,67; 2,8491 0,96; 2,8406 1,50; 2,8305 1,54; 2,8222 0,96; 2,8125 0,77; 2,6761 0,87; 2,6718 1,18; 2,6674 0,88; 2,5421 0,89; 2,5248 3,97; 2,5114 75,20; 2,5072 146,53; 2,5027 189,17; 2,4982 138,90; 2,4940 70,08; 2,4454 16,00; 2,3339 0,88; 2,3294 1,19; 2,3251 0,89; 1,2491 0,37; 1,2280 4,18; 1,2105 8,78; 1,1929 3,94; 0,7081 0,95; 0,6952 2,71; 0,6901 3,83; 0,6781 3,45; 0,6722 3,07; 0,6611 1,26; 0,5537 1,32; 0,5431 3,89; 0,5370 3,46; 0,5332 3,34; 0,5274 3,10; 0,5153 0,95; 0,0080 0,70, -0,0002 19,39; -0,0085 0,85 |
| Verbindung Nr. 189 [DMSO] 9,7021 2,25; 9,6777 2,28; 9,1149 4,59; 8,2999 1,67; 8,2856 1,55; 8,1781 0,88; 8,1718 0,97; 8,1627 1,11; 8,1558 0,99; 8,1507 0,95; 7,7249 7,04; 7,6847 1,45; 7,6590 1,84; 7,6363 1,31; 7,4673 4,59; 7,3246 6,12; 6,3553 0,89; 6,3333 1,34; 6,3105 0,93; 4,5168 1,00; 4,4995 2,94; 4,4818 2,92; 4,4646 1,00; 3,9045 8,93; 3,3950 0,43; 3,3873 0,46; 3,3815 0,50; 3,3346 305,34; 3,1746 0,65; 3,1616 0,61; 2,6761 0,90; 2,6716 1,20; 2,6673 0,90; 2,5419 0,99; 2,5111 76,43; 2,5071 147,69; 2,5027 190,11; 2,4982 138,56; 2,4664 16,00; 2,3339 0,88; 2,3294 1,18; 2,3248 0,86; 1,5771 1,56; 1,5629 4,06; 1,5562 4,38; 1,5433 1,81; 1,2919 2,21; 1,2786 4,55; 1,2719 4,82; 1,2575 2,06; 1,2358 7,26; 1,2189 9,00; 1,2013 4,03; 1,1400 0,53; 0,8535 0,59; 0,8353 0,38; 0,0079 1,06; -0,0002 27,66; -0,0083 1,09 |
| Verbindung Nr. 190 [DMSO] 9,5580 2,34; 9,5337 2,38; 8,2449 2,54; 8,2339 2,56; 8,2055 1,82; 8,2007 1,90; 8,1890 1,81; 8,1840 1,81; 7,8406 0,90; 7,8355 0,97; 7,8294 1,06; 7,8198 1,21; 7,8141 1,14; 7,8078 1,09; 7,8026 0,99; 7,6298 7,11; 7,5180 2,31; 7,4962 4,26; 7,4745 2,06; 7,4152 4,62; 7,3212 6,28; 6,2119 0,92; 6,1897 1,37; 6,1672 0,98; 4,5156 1,02; 4,4982 3,06; 4,4806 3,07; 4,4633 1,00; 3,9043 11,54; 3,3801 0,46; 3,3338 318,57; 3,1742 0,50; 3,1613 0,48; 2,8590 0,69; 2,8491 0,99; 2,8408 1,50; 2,8307 1,53; 2,8221 0,98; 2,8125 0,75; 2,6757 0,89; 2,6713 1,19; 2,6669 0,91; 2,5415 0,85; 2,5110 74,65; 2,5068 146,15; 2,5023 189,80; 2,4978 140,81; 2,4935 72,44; 2,4445 16,00; 2,3336 0,87; 2,3290 1,19; 2,3245 0,88; 1,2581 0,39; 1,2304 4,64; 1,2129 8,81; 1,1952 3,99; 0,8529 0,42; 0,8345 0,34; 0,7075 0,94; 0,6946 2,72; 0,6895 3,84; 0,6775 3,44; 0,6716 3,09; 0,6606 1,27; 0,5536 1,31; 0,5431 3,90; 0,5370 3,47; 0,5335 3,42; 0,5275 3,14; 0,5153 0,94; 0,0079 0,99; -0,0002 25,82; -0,0084 1,28 |
| Verbindung Nr. 191 [DMSO] 9,5999 2,22; 9,5756 2,29; 9,1163 4,59; 8,2030 1,69; 8,1983 1,79; 8,1866 1,73; 8,1818 1,72; 7,8401 0,85; 7,8349 0,91; 7,8286 0,97; 7,8191 1,13; 7,8135 1,09; 7,8070 1,04; 7,8022 0,94; 7,7197 7,01; 7,5206 2,24; 7,4988 4,13; 7,4771 2,22; 7,4657 4,54; 7,3290 6,00; 6,2178 0,88; 6,1953 1,31; 6,1727 0,93; 4,5193 0,95; 4,5022 2,86; 4,4844 2,88; 4,4668 0,96; 3,9043 8,00; 3,3330 294,83; 3,1740 0,86; 3,1615 0,80; 2,6758 0,86; 2,6714 1,19; 2,6668 0,89; 2,5416 0,74; 2,5244 3,81; 2,5110 74,69; 2,5068 146,49; 2,5023 189,94; 2,4978 138,63; 2,4935 68,83; 2,4657 16,00; 2,3335 0,94; 2,3290 1,25; 2,3247 0,94; 2,1798 0,42; 1,5766 1,55; 1,5624 3,98; 1,5556 4,31; 1,5427 1,81; 1,2923 2,21; 1,2790 4,45; 1,2723 4,77; 1,2578 2,13; 1,2354 7,77; 1,2214 9,03; 1,2037 3,99; 0,8535 0,77; 0,8354 0,41; 0,0078 1,06; -0,0002 27,74; -0,0081 1,11 |
| Verbindung Nr. 192 [DMSO] 9,5619 2,27; 9,5376 2,33; 8,2464 2,40; 8,2355 2,40; 8,0944 1,73; 8,0892 1,83; 8,0767 1,78; 8,0715 1,74; 7,8103 0,88; 7,8050 0,94; 7,7990 1,02; 7,7895 1,17; 7,7834 1,14; 7,7776 1,10; 7,7722 0,96; 7,6288 7,18; 7,5605 2,33; 7,5380 3,52; 7,5158 2,01; 7,4154 4,52; 7,3220 6,24; 6,2157 0,90; 6,1937 1,35; 6,1710 0,95; 4,5161 1,02; 4,4991 3,02; 4,4813 3,03; 4,4636 1,03; 3,9041 11,27; 3,4103 0,48; 3,3409 535,29; 3,2843 0,35; 3,1743 0,64; 3,1617 0,55; 2,8591 0,69; 2,8493 0,98; 2,8409 1,50; 2,8307 1,54; 2,8227 0,98; 2,8126 0,76; 2,6807 0,44; 2,6764 0,86; 2,6718 1,16; 2,6673 0,86; 2,6627 0,42; 2,5420 1,19; 2,5248 5,09; 2,5116 75,01; 2,5073 145,71; 2,5027 188,12; 2,4982 136,59; 2,4937 67,93; 2,4445 16,00; 2,3340 0,89; 2,3295 1,19; 2,3249 0,88; 2,3205 0,44; 1,2580 0,40; 1,2308 4,62; 1,2133 9,00; 1,1957 3,99; 0,8537 0,40; 0,7080 1,01; 0,6952 2,74; 0,6901 3,89; 0,6781 3,48; 0,6721 3,05; 0,6610 1,30; 0,5537 1,39; 0,5431 3,94; 0,5371 3,42; 0,5333 3,30; 0,5275 3,08; 0,5154 0,97; 0,0080 1,04; -0,0002 27,14; -0,0085 1,14 |
| Verbindung Nr. 193 [DMSO] 9,6033 2,25; 9,5790 2,29; 9,1176 4,57; 8,0921 1,76; 8,0873 1,82; 8,0746 1,76; 8,0696 1,73; 7,8099 0,90; 7,8046 0,97; 7,7987 1,06; 7,7890 1,20; 7,7830 1,16; 7,7774 1,12; 7,7722 0,99; 7,7185 7,11; 7,5635 2,30; 7,5408 3,54; 7,5187 1,97; 7,4658 4,55; 7,3299 6,05; 6,2216 0,91; 6,1999 1,36; 6,1773 0,97; 4,5205 1,01; 4,5030 2,94; 4,4852 2,95; 4,4674 1,01; 3,9043 10,45; 3,3818 0,77; 3,3370 369,94; 3,1742 0,70; 3,1616 0,66; 2,6761 0,86; 2,6716 1,18; 2,6671 0,90; 2,6626 0,51; 2,5419 1,02; 2,5248 4,54; 2,5114 75,06; 2,5071 146,50; 2,5026 189,12; 2,4981 138,44; 2,4937 69,51; 2,4656 16,00; 2,3337 0,85; 2,3293 1,16; 2,3247 0,88; 1,5768 1,60; 1,5626 4,07; 1,5558 4,39; 1,5429 1,85; 1,2924 2,32; 1,2790 4,56; 1,2723 4,89; 1,2579 2,06; 1,2391 4,76; 1,2218 9,13; 1,2041 4,05; 1,1398 0,39; 0,8533 0,39; 0,8349 0,33; 0,0080 0,96; -0,0002 25,38; -0,0085 1,11 |
| Verbindung Nr. 195 [DMSO] 9,6007 2,28; 9,5762 2,36; 9,1189 4,76; 7,9208 7,64; 7,9168 8,09; 7,7330 3,35; 7,7284 11,36; 7,4684 4,60; 7,3430 6,15; 6,2729 0,88; 6,2506 1,30; 6,2282 0,95; 4,5188 0,98; 4,5017 2,93; 4,4841 2,93; 4,4667 0,96; 3,9044 6,44; 3,3344 307,19; 3,1744 0,96; 3,1614 0,94; 2,6757 0,82; 2,6714 1,12; 2,6669 0,86; 2,5412 0,73; 2,5068 139,98; 2,5024 182,48; 2,4980 135,92; 2,4667 16,00; 2,3335 0,84; 2,3291 1,13; 2,3247 0,86; 1,5774 1,54; 1,5630 4,03; 1,5563 4,39; 1,5434 1,83; 1,2925 2,12; 1,2791 4,49; 1,2725 4,80; 1,2580 1,97; 1,2411 4,44; 1,2239 8,86; 1,2062 4,02; 0,8527 0,38; 0,8344 0,32; 0,0075 0,92; -0,0002 24,40; -0,0081 1,28 |
| Verbindung Nr. 196 [DMSO] 9,6977 1,66; 9,6734 1,73; 9,1262 3,41; 8,2154 2,29; 8,0709 1,22; 8,0515 1.37; 7,8306 1,09; 7,8107 1,58; 7,7340 6,12; 7,7162 1,92; 7,6966 0,79; 7,4395 3,29; 7,3741 4,28; 6,3131 0,63; 6,2909 0,95; 6,2682 0,69; 3,9365 16,00; 3,3291 48,92; 2,5252 0,86; 2,5204 1,29; 2,5117 16,09; 2,5073 32,47; 2,5027 43,26; 2,4981 31,94; 2,4936 15,63; 2,4749 10,86; 1,5762 1,10; 1,5621 2,77; 1,5552 2,98; 1,5424 1,26; 1,2994 1,47; 1,2861 2,99; 1,2793 3,19; 1,2649 1,16; 0,0079 1,10; -0,0002 32,32; -0,0085 1,09 |
| Verbindung Nr. 209 [DMSO] 10,0423 0,40; 10,0183 0,41; 9,7798 2,09; 9.7557 2,20; 9,1526 4,85; 8,3182 0,51: 8,2081 3,05; 8,1472 0,58: 8,0721 1,61; 8,0526 1,81; 8,0334 0,40; 8,0142 0,44; 7,8599 1,33; 7,8330 1,74; 7,8134 2,54; 7,7555 6,59; 7,7404 2,01; 7,7208 2,99; 7,7014 1,30; 7,5136 4,58; 7,4533 5,47; 6,3267 0,89; 6,3042 1,32; 6,2817 0,95; 5,4091 4,45; 5,1217 0,47; 5,1153 0,51; 5,1087 0,51; 5,1032 0,45; 4,0556 0,39; 4,0377 1,12; 4,0199 1,15; 4,0022 0,53; 3,8259 0,85; 3,8204 0,85; 3,3313 66,80; 3,1717 1,85; 3,1657 4,27; 3,1597 1,77; 3,1287 0,36; 3,1228 0,82; 3,1168 0,33; 2,7225 0,36; 2,7158 0,82; 2,7090 0,34; 2,6762 0,52; 2,6716 0,72; 2,6671 0,52; 2,5250 1,84; 2,5203 2,88; 2,5116 39,16; 2,5071 80,18; 2,5025 106,71; 2,4979 78,78; 2,4935 38,88; 2,4735 16,00; 2,3472 0,65; 2,3385 0,36; 2,3339 0,60; 2,3293 0,80; 2,3247 0,64; 2,3205 0,35; 2,3009 0,58; 1,9895 4,95; 1,5827 1,64; 1,5682 4,29; 1,5617 4,29; 1,5488 1,89; 1,3364 1,77; 1,2996 2,13; 1,2862 4,60; 1,2796 4,41; 1,2651 1,66; 1,2591 0,58; 1,2496 2,24; 1,2350 0,56; 1,1929 1,39; 1,1751 2,73; 1,1573 1,34; 0,0080 1,26; -0,0002 41,83; -0,0085 1,51 |
| Verbindung Nr. 262 [CD3CN]8,1075 0,75; 8,0917 0,73; 7,9737 1,70; 7,9030 0,91; 7,8900 1,00; 7,8416 5,10; 7,7880 0,88; 7,7749 1,13; 7,6927 0,93; 7,6797 1,53; 7,6667 0,65; 7,6277 3,70; 7,2536 1,28; 7,2390 3,43; 7,2383 3,44; 6,1575 0,60; 6,1431 0,84; 6,1283 0,62; 4,5007 0,85; 4,4888 2,76; 4,4769 2,79; 4,4651 0,87; 2,1530 27,63; 1,9583 0,58; 1,9542 0,94; 1,9504 16,58; 1,9463 33,29; 1,9422 49,12; 1,9381 32,72; 1,9340 15,98; 1,9295 0,49; 1,7820 0,56; 1.7752 16,00; 1,4320 0,38; 1,4293 0,70; 1,4272 0,44; 1,4230 0,43; 1,4184 0,96; 1,4159 0,73; 1,4081 0,64; 1,4049 0,44; 1,3960 0,40; 1,3032 3,34; 1,2914 7,71; 1,2796 3,40; 1,2714 0,32; 1,2684 0,33; 0,6950 0,81; 0,6904 1,05; 0,6878 1,62; 0,6868 1,48; 0,6845 1,49; 0,6838 1,48; 0,6808 1,94; 0,6786 1,44; 0,6758 1,75; 0,6730 1,26; 0,6699 1,71; 0,6671 0,67; 0,6648 1,32; 0,6622 0,68; 0,6575 0,81; 0,6561 0,77; 0,6543 0,76; 0,6494 1,13; 0,6412 0,64; 0,6384 0,36; 0,0053 2,18; -0,0002 74,22; -0,0058 2,44 |
| Verbindung Nr. 263 [CD3CN] 8,1743 1,18; 8,1515 1,19; 7,9742 3,61; 7,8978 1,87; 7,8782 2,28; 7,8229 10,03; 7,7874 1,68; 7,7677 2,48; 7,6933 2,11; 7,6736 3,16; 7,6541 1,26; 7,6195 7,90; 7,2359 7,97; 7,1875 1,18; 6,1779 0,43; 6,1571 1,51; 6,1351 2,04; 6,1129 1,55; 6,0918 0,47; 4,5064 1,89; 4,4887 6,16; 4,4709 6.28; 4,4531 2,02; 3,6271 2,65; 3,6110 7,66; 3,5953 7,87; 3,5793 2,96; 2,7517 5,98; 2,7355 12,14; 2,7193 5,66; 2,4718 0,37; 2,4670 0,52; 2,4623 0,38; 2,1864 317,72; 2,1818 416,45; 2,1203 0,77; 2,1142 1,00; 2,1080 1,20; 2,1018 0,86; 2,0956 0,56; 1,9649 14,55; 1,9588 6,12; 1,9529 56,15; 1,9468 107,20; 1,9406 151,90; 1,9344 107,30; 1,9282 57,22; 1,7814 0,38; 1,7752 0,65; 1,7690 0,94; 1,7628 0,67; 1,7567 0,38; 1,3066 7,00; 1,2889 16,00; 1,2710 8,08; 0,0081 1,30; -0,0002 47,92; -0,0085 2,38 |
| Verbindung Nr. 263 [CD3CN] 8,1743 1,18; 8,1515 1,19; 7,9742 3,61; 7,8978 1,87; 7,8782 2,28; 7,8229 10,03; 7,7874 1,68; 7,7677 2,48; 7,6933 2,11; 7,6736 3,16; 7,6541 1,26; 7,6195 7,90; 7,2359 7,97; 7,1875 1,18; 6,1779 0,43; 6,1571 1,51; 6,1351 2,04; 6,1129 1,55; 6,0918 0,47; 4,5064 1,89; 4,4887 6,16; 4,4709 6,28; 4,4531 2,02; 3,6271 2,65; 3,6110 7,66; 3,5953 7,87; 3,5793 2,96; 2,7517 5,98; 2,7355 12,14; 2,7193 5,66; 2,4718 0,37; 2,4670 0,52; 2,4623 0,38; 2,1864 317,72; 2,1818 416,45; 2,1203 0,77; 2,1142 1,00; 2,1080 1,20; 2,1018 0,86: 2,0956 0,56; 1,9649 14,55; 1,9588 6,12; 1,9529 56,15; 1,9468 107,20; 1,9406 151,90; 1,9344 107,30; 1,9282 57,22; 1,7814 0,38; 1,7752 0,65; 1,7690 0,94; 1,7628 0,67; 1,7567 0,38; 1,3066 7,00; 1,2889 16,00; 1,2710 8,08; 0,0081 1,30; -0,0002 47.92; -0,0085 2,38 |
| Verbindung Nr. 264 [CD3CN] 8,2107 0,52; 8,1875 0,52; 7,9701 1,75; 7,9082 0,92; 7,8887 1,09; 7,8175 4,48; 7,7886 0,85; 7,7690 1,24; 7,6957 1,05; 7,6762 1,53; 7,6566 0,61; 7,6146 3,52; 7,2248 3,69; 7,0082 1,06; 6,1576 0,72; 6,1355 0,97; 6,1135 0,74; 4,5025 0,85; 4,4847 2,71; 4,4669 2,75; 4,4492 0,88; 2,2209 97,12; 2,1448 0,47; 2,1260 1,29; 2,1074 1,71; 2,0907 2,72; 2,0719 2,59; 2,0532 0,92; 2,0497 0,88; 2,0310 2,43; 2,0127 2,79; 1,9954 1,72; 1,9774 1,28; 1,9663 2,77; 1,9600 1,12; 1,9543 9,87; 1,9481 18,68; 1,9420 26,78; 1,9358 18,71; 1,9296 9,80; 1,3037 3,42; 1,2861 7,47: 1,2683 3,46; 1,0957 7,61; 1,0771 16,00; 1,0585 7,05; -0,0002 0,57 |
| Verbindung Nr. 266 [CD3CN] 8,1744 0,69; 8,1510 0,69; 7,9715 2,11; 7,9060 1,09; 7,8862 1,29; 7,7868 1,02; 7,7669 1,49; 7,7509 5,68; 7,6926 1,22; 7,6731 1,81; 7,6535 0,70; 7,5809 4,44; 7,2009 4,29; 6,8697 1.09; 6,1555 0,86; 6,1339 1,17; 6,1115 0,87; 4,4967 1,08; 4,4789 3,43; 4,4612 3,47; 4,4433 1,09; 2,1748 229,04; 2,1417 1,79; 2,1203 0,36; 2,1140 0,45; 2,1078 0,53; 2,1016 0.42; 1,9647 1,01; 1,9585 1,55; 1,9528 24,12; 1,9466 46,56; 1,9404 66,70; 1,9343 46,07; 1,9281 23,85; 1,7689 0,40; 1,4332 16,00; 1,2988 3,91; 1,2811 8,69; 1,2633 3,82; 1,1694 7,24; 1,1540 8,39; 0,9599 0,60; 0,9446 0,84; 0,9381 0,82; 0,9288 0,57; 0,9226 1,09; 0,9072 0,68; 0,7795 1,51; 0,7669 1,62; 0,7576 1,22; 0,7450 1,26; 0,4141 1,11; 0,4003 1,71; 0,3862 1,01; -0,0002 3,31 |
| Verbindung Nr. 267 [CD3CN] 10,0848 1,09; 8,2814 0,93; 8,1224 1,53; 8,0987 1,58; 7,9663 3,82; 7,9386 0,40; 7,8959 2,17; 7,8766 2,41; 7,7877 1,85; 7,7687 12,47; 7,7062 0,96; 7,6932 2,28; 7,6736 3,29; 7,6538 1,32; 7,6128 8,06; 7,4079 2,56; 7,2197 7,98; 6,1755 0,47; 6,1547 1,56; 6,1330 2,15; 6,1106 1,60; 6,0893 0,48; 4,5026 1,92; 4,4849 6,17; 4,4671 6,28; 4,4493 2,01; 4,3786 0,35; 4,3576 0,35; 2,1718 684,33; 2,1203 1,15; 2,1141 1,16; 2,1079 1,22; 2,1017 0,95; 2,0955 0,62; 2,0871 0,35; 1,9766 0,49; 1,9648 41,98; 1,9587 4,23; 1,9529 46,51; 1,9467 89,72; 1,9405 128,18; 1,9343 88,79; 1,9281 45,95; 1,7812 0,33; 1,7751 0,58; 1,7689 0,79; 1,7628 0,55; 1,3926 2,21; 1,3783 5,53; 1,3719 5,92; 1,3582 3,53; 1,3363 1,47; 1,3182 1,29; 1,3023 7,24; 1,2845 16,00; 1,2668 7,49; 1,2583 1,99; 1,2543 2,06; 1,2406 4,81; 1,2237 1,09; 1,2201 1,02; -0,0002 2,17 |
| Verbindung Nr. 268 [CD3CN] 10,0914 0,78; 8,3863 0,67; 8,1436 1,42; 8,1205 1,49; 7,9700 3,99; 7,8977 2,20; 7,8780 2,74; 7,8645 9,54; 7,7892 1,94; 7,7694 2,78; 7,7402 0,67; 7,6952 2,28; 7,6756 3,34; 7,6560 1,42; 7,6388 7,45; 7,6172 0,90; 7,5828 1,10; 7,5433 1,38; 7,5232 1,41; 7,2386 7,51; 7,1952 0,77; 6,1787 0,48; 6,1575 1,61; 6,1356 2,19; 6,1135 1,64; 6,0922 0,49; 5,1375 1,28; 5,1207 2,95; 5,1181 1,86; 5,1037 1,65; 5,1009 2,99; 5,0841 1,26; 4,5068 1,92; 4,4891 6,09; 4,4713 6,21; 4,4535 2,00; 3,6587 0,33; 3,0637 3,88; 2,9240 0,43; 2,9174 0,47; 2,9038 4,67; 2,8973 4,80; 2,8871 4,76; 2,8805 4,73; 2,7998 2,50; 2,5061 0,42; 2,5000 2,77; 2,4934 5,63; 2,4868 2,61; 2,1766 287,45; 2,1208 0,58; 2,1144 0,56; 2,1082 0,62; 2,1021 0,56; 2,0959 0,35; 1,9651 11,86; 1,9591 1,89; 1,9532 24,21; 1,9470 47,23; 1,9409 67,90; 1,9347 47,47; 1,9285 24,85; 1,7694 0,42; 1,3572 0,46; 1,3393 1,00; 1,3214 0,67; 1,3086 7,13; 1,2910 16,00; 1,2731 7,31; -0,0002 1,49 |
| Verbindung Nr. 269 [CD3CN] 10,0865 0,59; 10,0836 0,59; 8,3186 0,81; 8,1251 1,25; 8,1016 1,27; 7,9714 3,40; 7,9536 0,33; 7,8975 1,80; 7,8778 2,15; 7,7940 9,95; 7,7684 2,36; 7,6942 2,05; 7,6746 2,96; 7,6551 1,14; 7,6079 7,28; 7,2258 7,04; 7,2245 7,21; 6,9842 1,10; 6,9654 1,09; 6,1780 0,41; 6,1567 1,40; 6,1346 1,89; 6,1126 1,42; 6,0911 0,42; 4,5037 1,77; 4,4859 5,66; 4,4681 5,72; 4,4504 1,79; 4,3726 0,85; 4,3578 1,35; 4,3556 1,35; 4,3407 1,59; 4,3390 1,60; 4,3220 1,23; 4,3073 0,59; 3,2822 0,40; 3,2686 0,39; 2,8687 1,94; 2,8545 1,91; 2,8265 3,52; 2,8122 3,32; 2,7297 0,44; 2,7244 3,72; 2,7099 3,69; 2,6822 2,15; 2,6677 2,09; 2,1585 403,11; 2,1253 1,36; 2,1136 0,91; 2,1074 1,08; 2,1012 0,82; 2,0951 0,49; 1,9643 2,30; 1,9581 3,00; 1,9524 46,88; 1,9462 90,98; 1,9400 130,48; 1,9338 90,21; 1,9277 46,59; 1,7747 0,57; 1,7684 0,79; 1,7623 0,54; 1,3676 1,27; 1,3537 15,72; 1,3367 16,00; 1,3184 0,69; 1,3059 6,52; 1,2882 14,61; 1,2704 6,60; - 0,0002 2,78 |
| Verbindung Nr. 270 [CD3CN] 8,1925 0,96; 8,1732 0,97; 7,9763 3,71; 7,9008 1,94; 7,8813 2,32; 7,8389 10,04; 7,7888 1,76; 7,7690 2,57; 7,6946 2,18; 7,6750 3,19; 7,6553 1,26; 7,6321 7,89; 7,4234 1,05; 7,4085 1,00; 7,2373 7,69; 6,1806 0,44; 6,1593 1,50; 6,1376 2,07; 6,1153 1,54; 6,0940 0,46; 4,8560 2,70; 4,8386 3,05; 4,8354 3,17; 4,8179 2,72; 4,5075 1,80; 4,4897 5,83; 4,4719 5,91; 4,4541 1,88; 2,2284 0,87; 2,2110 3,10; 2,1912 139,92; 2,1604 2,87; 2,1435 0,87; 2,1145 0,32; 2,1082 0.41: 2,1021 0,34; 1,9652 4,58; 1,9590 1,25; 1,9532 18,39; 1,9471 35,49; 1,9409 50,97; 1,9347 35,38; 1,9285 18,38; 1,3071 6,84; 1,2894 15,23; 1,2716 6,82; 1,1469 15,99; 1,1301 15,54; 1,1124 16,00; 1,0955 15,48; -0,0002 1,02 |
| Verbindung Nr. 271 vgl. Synthesebeispiel 7 |
| Verbindung Nr. 272 [DMSO] 9,9368 2,72; 9,9127 2,80; 9,3005 6,36; 8,2007 4,24; 8,0693 2,30; 8,0496 2,58; 7,8966 7,87; 7,8633 0,45; 7,8452 11,17; 7,8358 2,69; 7,8165 3,05; 7,7411 2,36; 7,7216 3,59; 7,7021 1,50; 7,3469 8,07; 6,3325 0,32; 6,3110 1,14; 6,2892 1,72; 6,2667 1,23; 6,2443 0,35; 4,4962 0,79; 4,4788 0,90; 4,4598 2,21; 4,4423 2,26; 4,4315 2,24; 4,4137 2,21; 4,3950 0,86; 4,3776 0,79; 3,3425 94,06; 3,3372 93,06; 3,3357 91,86; 2,6725 0,36; 2,5256 1,07; 2,5120 20,13; 2,5078 40,51; 2,5034 53,71; 2,4990 39,45; 2,3341 0,38; 2,3303 0,45; 2,0764 16,00; 1,6020 2,25; 1,5878 5,45; 1,5809 6,01; 1,5681 2,55; 1,2891 2,64; 1,2757 5,50; 1,2690 5,85; 1,2546 2,13; 1,1130 0,47; 1,0985 0,94; 1,0943 0,93; 1,0810 1,44; 1,0677 0,92; 1,0631 1,02; 1,0493 0,55; 0,3510 0,46; 0,3448 0,83; 0,3384 1,03; 0,3313 0,72; 0,3231 1,87; 0,3186 2,12; 0,3024 2,09; 0,2887 2,11; 0,2826 2,39; 0,2767 2,29; 0,2700 2,86; 0,2577 1,95; 0,2529 2,01; 0,2402 0,71; 0,2361 0,67; 0,1993 0,95; 0,1943 1,08; 0,1807 1,83: 0,1674 1,65; 0,1535 0,71; 0,0080 0,95; -0,0002 27,45; -0,0085 1,05 |
| Verbindung Nr. 273 vgl. Synthesebeispiel 9 |
| Verbindung Nr. 274 [DMSO] 9,9336 3,68; 9,9095 3,77; 9,2953 8,64; 8,1942 5,55; 8,0637 3,06; 8,0445 3,44; 7,8888 10,25; 7,8556 1,00; 7,8400 16,00; 7,8154 4,12; 7,7854 0,37; 7,7406 3,18; 7,7210 4,77; 7,7013 1,99; 7,3433 10,64; 6,3036 1,51; 6,2815 2,25; 6,2587 1,60; 6,2369 0,44; 5,7528 0,57; 4,5866 0,36; 4,4927 1.14: 4,4753 1,26; 4,4562 3,00; 4,4388 3,11; 4,4285 3,02; 4,4107 2,99; 4,3921 1,21; 4,3746 1,13; 3,3628 2627,74; 2,6777 1,84; 2,6733 2,54; 2,6688 1,85; 2,5430 1,59; 2,5265 9,31; 2,5217 13,51; 2,5130 143,04; 2,5087 285,87; 2,5042 374,45; 2,4997 271,01; 2,4954 133,10; 2,3352 1,93; 2,3309 2,67; 2,3265 1,87; 2,0731 7,39; 1,5991 3,09; 1,5850 7,36; 1,5781 8,02; 1,5652 3,48; 1,5253 0,40; 1,3293 0,40; 1,2899 3,67; 1,2764 7,51; 1,2698 8,00; 1,2553 3,03; 1,2369 0,93; 1,0918 1,23; 1,0790 1,85; 1,0604 1,34; 1,0476 0,82; 0,3453 1,14; 0,3385 1,30; 0,3190 2,75; 0,3031 2,63; 0,2888 2,66; 0,2775 3,42; 0,2694 2,96; 0,2565 2,63; 0,2447 1,89; 0,1916 1,40; 0,1786 2,50; 0,1655 2,17; -0,0002 2,56 |
| Verbindung Nr. 275 [DMSO] 9,7120 2,64; 9,6876 2,69; 9,2902 5,91; 8,1512 16,00; 7,8551 12,37; 7,8510 9,01; 7,3884 7,52; 6,3348 0,33; 6,3140 1,15; 6,2922 1,70; 6,2700 1,20; 6,2473 0,36; 4,5420 1,46; 4,5247 4,11; 4,5070 4,10; 4,4898 1,40; 4,0565 0,35; 4,0387 1,04; 4,0209 1,07; 4,0030 0,35; 3,3215 41,62; 3,2979 3,30; 2,6717 0,56; 2,6670 0,41; 2,5069 63,97; 2,5025 79,49; 2,4981 57,24; 2,3335 0,43; 2,3293 0,57; 1,9896 4,47; 1,6024 2,34; 1,5882 5,99; 1,5816 6,26; 1,5686 2,58; 1,3970 1,10; 1,2988 0,89; 1,2882 2,85; 1,2748 6,08; 1,2684 6,18; 1,2538 2,52; 1,2349 5,94; 1,2175 11,86; 1,1999 5,54; 1,1933 2,31; 1,1753 2,65; 1,1574 1,30; -0,0002 45,75; -0,0083 2,31 |
| Verbindung Nr. 276 [DMSO] 9,7201 3,68; 9,6959 3,80; 8,3922 4,13; 8,3812 4,17; 7,7866 11,03; 7,7533 14,99; 7,7054 0,33; 7,6806 0,34; 7,6168 7,38; 7,6000 4,80; 7,5452 4,09; 7,5247 7,01; 7,5164 0,62; 7,5127 0,63; 7,5043 3,26; 7,4514 4,08; 7,3740 10,69; 7,2668 11,16; 7,2524 2,73; 7,2476 2,80; 7,0822 4,24; 6,1876 0,41; 6,1666 1,55; 6,1444 2,32; 6,1220 1,65; 6,0997 0,47; 4,5407 1,74; 4,5233 5,13; 4,5055 5,11; 4,4878 1,72; 3,3216 34,66; 3,2980 1,01; 2,8662 0,42; 2,8562 1,20; 2,8466 1,65; 2,8381 2,64; 2,8278 2,70; 2,8193 1,74; 2,8097 1,35; 2,7994 0,57; 2,6704 0,44; 2,5104 25,15; 2,5060 50,96; 2,5015 67,18; 2,4970 47,95; 2,4927 22,78; 2,3328 0,39; 2,3282 0,49; 2,3238 0,41; 2,0748 0,88; 1,2427 0,52; 1,2220 7,34; 1,2046 16,00; 1,1870 7,11; 1,1207 0,36; 1,0636 0,44; 0,7183 1,79; 0,7056 4,90; 0,7003 6,91; 0,6883 6,34; 0,6823 5,37; 0,6711 2,22; 0,5572 2,40; 0,5468 6,90; 0,5405 6,05; 0,5311 5,42; 0,5189 1,64; 0,0080 1,48; -0,0002 41,80; -0,0085 1,34 |
| Verbindung Nr. 277 [DMSO] 9,7692 2,45; 9,7450 2,53; 9,3249 5,95; 8,1478 16,00; 7,8960 10,70; 7,8876 7,85; 7,4875 7,24; 6,3199 1,02; 6,2979 1,54; 6,2756 1,10; 5,4448 6,19; 5,4419 6,13; 3,3217 53,71; 3,2981 2,39; 3,2273 2,37; 3,2215 5,01; 3,2156 2,24; 3,0365 0,35; 2,6755 0,41; 2,6712 0,57; 2,6665 0,40; 2,5413 0,33; 2,5244 1,34; 2,5109 29,72; 2,5065 60,24; 2,5020 79,50; 2,4975 56,85; 2,4931 27,09; 2,3334 0,41; 2,3288 0,55; 2,3243 0,40; 1,6082 2,12; 1,5940 5,21; 1,5872 5,64; 1,5742 2,38; 1,2919 2,53; 1,2785 5,28; 1,2719 5,61; 1,2574 2,07; 1,2338 0,41; -0,0002 8,23 |
| Verbindung Nr. 278 [DMSO] 9,7680 4,02; 9,7438 4,12; 8,4241 4,69; 8,4130 4,67; 7,8696 0,37; 7,8249 12,00; 7,7932 16,00; 7,6197 6,77; 7,6148 6,39; 7,6057 5,47; 7,5469 4,53; 7,5401 0,65; 7,5264 7,68; 7,5162 0,86; 7,5060 3,67; 7,4869 11,33; 7,4512 4,45; 7,2666 11,88; 7,2532 2,83; 7,2485 2,72; 7,0820 4,67; 6,1927 0,43; 6,1713 1,63; 6,1494 2,47; 6,1269 1,75; 6,1047 0,49; 5,4506 9,39; 5,4466 9,33; 3,3199 95,53; 3,2964 1,70; 3,2062 3,57; 3,2002 7,80; 3,1944 3,33; 2,8695 0,45; 2,8594 1,28; 2,8499 1,73; 2,8412 2,78; 2,8310 2,80; 2,8227 1,75; 2,8128 1,33; 2,8026 0,49; 2,6746 0,75; 2,6701 1,03; 2,6655 0,74; 2,6608 0,35; 2,5404 0,51; 2,5234 2,41; 2,5099 52,90; 2,5055 108,20; 2,5010 143,74; 2,4965 102,43; 2,4921 48,28; 2,3324 0,81; 2,3277 1,09; 2,3233 0,78; 1,2339 0,38; 0,7230 1,87; 0,7101 5,06; 0,7050 7,22; 0,6929 6,63; 0,6869 5,69; 0,6757 2,34; 0,5580 2,42; 0,5475 7,19; 0,5413 6,39; 0,5318 5,83; 0,5196 1,80; -0,0002 4,95 |
| Verbindung Nr. 279 vgl. Synthesebeispiel 8 |
| Verbindung Nr. 281 [CD3CN] 8,8068 2,52; 8,7832 2,51; 7,9986 3,57; 7,9818 3,44; 7,9493 1,87; 7,9386 2,12; 7,9295 2,47; 7,9168 2,25; 7,8583 3,08; 7,8333 0,60; 7,8209 15,55; 7,8111 0,58; 7,8017 0,34; 7,7913 2,92; 7,7838 1,37; 7,7726 1,23; 7,7669 1,22; 7,7204 0,44; 7,6584 16,00; 7,6310 3,38; 7,6200 0,39; 7,5930 0,43; 7,5745 3,01; 7,4575 3,71; 7,4326 4,43; 7,4198 0,61; 7,4096 3,11; 7,3422 0,44; 7,3167 0,73; 7,2926 0,44; 7,2414 0,37; 7,2359 0,41; 7,1721 0,33; 7,1656 0,42; 6,9632 0,83: 6,9332 2,88; 6,1649 0,40; 6,1384 0,52; 6,1237 1,12; 6,1040 2,76; 6,0957 1,03; 6,0830 3,39; 6,0681 1,44; 6,0597 2,98; 6,0554 2,63; 6,0421 2,29; 6,0293 2,42; 6,0254 1,47; 6,0205 0,93; 6,0161 1,40; 6,0123 2,47; 5,9993 2,09; 5,9948 0,85; 5,9863 2,36; 5,9733 1,10; 5,8775 0,34; 5,8591 0,96; 5,8403 1,01; 5,8219 0,69; 5,8092 0,35; 5,7961 0,49; 5,7792 0,48; 5,7662 0,36; 5,7534 0,46; 5,4843 0,88; 5,4807 0,88; 5,4490 14,25; 5,4414 0,93; 5,4376 0,89; 5,3522 0,93; 5,3490 0,91; 5,3258 0,87; 5,3228 0,85; 5,2664 9,20; 5,2627 7,16; 5,2573 5,89; 5,2534 7,87; 5,2211 0,62; 5,1889 0,32; 5,1580 5,01; 5,1552 4,99; 5,1320 4,71; 5,1292 4,66; 5,1088 0,32; 5,0697 0,96; 5,0669 0,86; 5,0437 0,84; 5,0413 0,82; 5,0021 4,43; 4,9994 4,63; 4,9829 1,07; 4,9632 2,84; 4,9593 4,46; 4,9563 4,39; 4,9524 2,80; 4,9301 1,33; 4,9274 1,29; 4,9166 0,54; 4,8997 0,34; 4,8872 0,68; 4,8848 0,69; 4,8440 1,67; 4,8404 1,07; 4,8305 1,73; 4,8267 1,12; 2,8871 0,55; 2,8775 1,74; 2,8683 2,65; 2,8594 4,19; 2,8500 4,24; 2,8414 3,18; 2,8320 2,17; 2,8225 0,76; 2,4667 0,36; 2,1871 524,56; 2,1212 0,70; 2,1153 0,91; 2,1091 0,99; 2,1030 0,76; 2,0969 0,49; 1,9660 5,24; 1,9597 6,14; 1,9540 50,24; 1,9479 93,65; 1,9417 130,32; 1,9356 90,46; 1,9294 47,10; 1,7825 0,40; 1,7763 0,65; 1,7702 0,87; 1,7640 0,61; 1,7577 0,38; 1,4360 0,53; 1,3859 0,48; 1,3720 8,60; 1,3403 4,32; 1,2849 5,63; 1,2764 10,49; 1,2699 3,57; 1,2166 0,43; 0,8812 0,85; 0,8759 0,68; 0,8581 0,88; 0,8389 0,64; 0,8143 0,36; 0,7957 2,72; 0,7830 7,96; 0,7779 10,62; 0,7654 10,91; 0,7601 8,24; 0,7480 3,78; 0,7263 0,59; 0,7085 0,56; 0,6597 0,38; 0,6499 0,40; 0,6198 3,08; 0,6080 8,57; 0,6026 9,63; 0,5985 9,28; 0,5932 8,66, 0,5807 2,40; 0,0081 1,05; -0,0002 32,31; -0,0085 1,14 |
| Verbindung Nr. 282 [DMSO] 9,7255 1,68; 9,7012 1,73; 9,1573 3,54; 8,2097 2,53; 8,0695 1,34; 8,0500 1,50; 7,8333 1,23; 7,8137 1,78; 7,7387 1,33; 7,7193 2,05; 7,6997 0,92; 7,6854 5,33; 7,4638 3,87; 7,3404 4,50; 6,3203 0,69; 6,2980 1,06; 6,2749 0,75; 4,5364 0,73; 4,5192 2,21; 4,5015 2,24; 4,4842 0,74; 4,0554 1,21; 4,0376 3,74; 4,0198 3,79; 4,0020 1,27; 3,3267 27,82; 2,8915 0,93; 2,8729 2,78; 2,8542 2,83: 2,8357 1,00; 2,6752 0,35; 2,6710 0,50; 2,6665 0,40; 2,5104 27,73; 2,5063 55,55; 2,5019 75,05; 2,4975 58,23; 2,3332 0,37; 2,3287 0,51; 2,3243 0,41; 1,9893 16,00; 1,5789 1,19; 1,5646 3,06; 1,5580 3,43; 1,5452 1,41; 1,2831 1,64; 1,2697 3,28; 1,2630 3,50; 1,2487 1,39; 1,2350 3,15; 1,2177 6,58; 1,2052 4,94; 1,2004 4,06; 1,1923 5,92; 1,1867 8,90; 1,1745 9,49; 1,1681 4,46; 1,1567 4,55; 1,1211 0,36; 1,1027 0,33; 1,0839 0,38; 0,0080 0,58; -0,0002 14,36; -0,0083 0,77 |
| Verbindung Nr. 283 [DMSO] 9,8678 4,27; 9,8437 4,39; 9,3073 9,71; 8,1818 6,31; 8,0520 3,36; 8,0325 3,78; 7,8690 16,00; 7,8293 3,13: 7,8096 4,46; 7,7835 11,92; 7,7331 3,31; 7,7136 5,12; 7,6940 2,13; 7,4200 11,88; 6,3148 0,46; 6,2934 1,72; 6,2714 2,60; 6,2488 1,86; 6,2266 0,53; 5,9349 0,74; 5,9225 1,71; 5,9095 1,49; 5,8967 1,99; 5,8928 1,14; 5,8836 1,25; 5,8798 2,03; 5,8667 1,61; 5,8539 1,91; 5,8414 0,90; 5,1771 5,92; 5,1676 5,66; 4,9893 3,83; 4,9861 3,80; 4,9635 3,59; 4,9603 3,55; 4,7348 3,61; 4,7314 3,57; 4,6919 3,36; 4,6885 3,34; 3,3300 55,87; 2,6762 0,58; 2,6718 0,80; 2,6674 0,59; 2,5247 2,89; 2,5114 46,37; 2,5072 89,90; 2,5028 116,81; 2,4983 86,43; 2,4941 44,35; 2,3340 0,59; 2,3295 0,80; 2,3251 0,59; 2,0762 3,80; 1,6003 3,27; 1,5862 8,10; 1,5793 8,90; 1,5664 3,69; 1,3255 0,38; 1,2854 3,88; 1,2720 8,18; 1,2653 8,78; 1,2509 3,10; 0,0076 1,23; -0,0002 29,41; -0,0083 1,62 |
| Verbindung Nr. 284 [DMSO] 9,9380 1,18; 9,9137 1,22; 9,3023 2,75; 8,1638 1,86; 8,0237 1,01; 8,0049 1,12; 7,9539 2,38; 7,8736 7,51; 7,8287 0,95; 7,8096 1,27; 7,7228 0,92; 7,7031 1,41; 7,6834 0,60; 7,4147 3.23; 7,1682 2,68; 7,1584 3,75; 7,1520 3,90; 6,9908 1,71; 6,9819 1,98; 6,9729 1,64; 6,2880 0,51; 6,2657 0,75; 6,2432 0,54; 5,7843 2,86; 3,3309 43,45; 2,8909 16,00; 2,7318 13,98; 2,6716 0,39; 2,5066 46,43; 2,5024 55,22; 2,4983 40,55; 2,3291 0,37; 1,9896 0,80; 1,5928 0,89; 1,5786 2,34; 1,5721 2,51; 1,5590 1,06; 1,3974 0,83; 1,2715 1,10; 1,2583 2,63; 1,2517 2,59; 1,2372 0,92; 1,1747 0,46; -0,0002 12,25 |
| Verbindung Nr. 285 [DMSO] 9,8909 0,70; 9,8668 0,72; 9,3242 1,67; 8,2072 1,01; 8,0741 0,54; 8,0549 0.61; 7,9536 2,01; 7,8771 2,86; 7,8566 2,00; 7,8394 0,51; 7,8197 0,71; 7,7465 0,54; 7,7270 0,83; 7,7076 0,35; 7,4390 1,90; 6,3073 0,41; 5,3754 1,30; 5,3698 1,29; 3,3330 26,73; 2,8909 16,00; 2,7312 12,93; 2,5252 0,46; 2,5204 0,73; 2,5118 7,74; 2,5074 15,58; 2,5028 20,47; 2,4982 14,94; 2,4937 7,41; 1,6180 1,92; 1,6125 3,86; 1,6071 2,35; 1,5936 1,39; 1,5868 1,48; 1,5739 0,65; 1,2933 0,67; 1,2797 1,34; 1,2731 1,44; 1,2586 0,71; -0,0002 3,10 |
| Verbindung Nr. 286 [DMSO] 9,9937 2,01; 9,9695 2,11; 9,9154 0,63; 9,8911 0,61; 9,3368 6,19; 8,2236 3,00; 8,1982 0,83; 8,0784 1,62; 8,0583 2,03; 8,0345 0,43; 7,8931 16,00; 7,8869 3,08; 7,8707 1,78; 7,8385 1,52; 7,8186 2,15; 7,7906 0,53; 7,7286 1,55; 7,7091 2,56; 7,6889 1,24; 7,4640 1,28; 7,4428 0,43; 7,4243 6,17; 7,3227 0,38; 7,3199 0,42; 7,3049 0,46; 7,2980 1,07; 7,2802 1,34; 7,2765 0,97; 7,2717 0,65: 7,2609 0,40; 7,2454 0,39; 7,2337 0,43; 7,2291 0,50; 7,2169 1,14; 7,2143 1,30; 7,2044 2,28; 7,1999 7,39; 7,1952 3,89; 7,1899 1,21; 7,1853 2,09; 7,1815 4,37; 7,1744 0,90; 7,1713 0,87; 7,1670 1,42; 7,1596 1,86; 7,1407 0,71; 7,0751 3,50; 7,0716 3,83; 7,0566 2,83; 7,0518 2,65; 6,3374 0,84; 6,3159 1,32; 6,2935 0,98; 6,2715 0,33; 6,0699 1,70; 6,0604 0,67; 6,0426 0,90; 6,0157 4,31; 6,0083 4,32; 5,9815 0,80; 4,6898 0,44; 4,6844 0,54; 4,6730 0,33; 4,6659 0,36; 4,6485 1,21; 4,6410 1,19; 4,5421 0,88; 4,4114 0,39; 4,4067 0,39; 4,3513 1,81; 4,3298 2,77; 4,3222 4,32; 4,2829 4,12; 4,2537 1,80; 3,3301 51,21; 2,6756 0,38: 2,6714 0,56; 2,6668 0,42; 2,5244 1,88; 2,5112 31,82; 2,5068 63,42; 2,5023 83,54; 2,4977 61,33; 2,4932 30,60; 2,3335 0,41; 2,3289 0,57; 2,3246 0,41; 2,0759 1,12; 1,6078 2,10; 1,5936 5,01; 1,5868 5,49; 1,5739 2,36; 1,2917 2,27; 1,2783 4,82: 1,2717 5,12; 1,2571 1,86; 0,0080 0,92; -0,0002 23,96; -0,0085 1,09 |
| Verbindung Nr. 287 [DMSO] 9,9661 1,39; 9,9419 1,44; 9,3341 3,33; 8,1896 2,05; 8,0603 1,09; 8,0409 1,22; 7,9532 1,92; 7,8925 4,08; 7,8760 5,64; 7,8341 1,00; 7,8143 1,42; 7,7375 1,09; 7,7179 1,66; 7,6984 0.68; 7,3768 3,92; 6,3133 0,55; 6,2912 0,83; 6,2684 0,59; 5,9262 0,68; 5,8992 2,76; 5,8880 2,77; 5,8610 0,70; 3,3309 76,09; 3,2815 0,64; 3,2756 0,55; 3,2638 0,72; 3,2578 1,65; 3,2506 0,61; 3,2463 0,44; 3,2402 1,66; 3,2332 1,63; 3,2271 0,42; 3,2226 0,62; 3,2156 1,64; 3,2096 0,73; 3,1981 0,53; 3,1920 0,62; 2,8908 16,00; 2,7316 12,37; 2,7309 12,27; 2,6715 0,43; 2,5249 1,57; 2,5200 2,45; 2,5115 24,25; 2,5070 48,66; 2,5025 63,89; 2,4979 46,66; 2,4934 23,07; 2,3338 0,32; 2,3292 0,44; 2,3245 0,35; 1,6062 1,13; 1,5921 2,64; 1,5853 2,89; 1,5723 1,25; 1,3972 1,05; 1,2986 0,46; 1,2894 1,31; 1,2759 2,66; 1,2693 2,86; 1,2582 0,94; 1,2549 1,14; 1,2495 0,53; 0,9118 4,24; 0,8943 8,73; 0,8767 4,10; 0,0079 0,71; -0,0002 19,85; -0,0085 0,82 |
| Verbindung Nr. 288 [DMSO] 9,8833 3,58; 9,8591 3,71; 9,2967 8,30; 8,1963 5,23; 8,0650 2,78; 8,0457 3.15; 7,8642 9,90; 7,8432 13,73; 7,8321 2,88; 7,8122 3,64; 7,7363 2,74; 7,7167 4,24; 7,6972 1,77; 7,3547 9,86; 6,3407 0,38; 6,3194 1,40; 6,2972 2,14; 6,2747 1,54; 6,2522 0,44; 4,5062 0,63; 4,4883 1,92; 4,4713 3,50; 4,4605 3,57; 4,4433 1,93; 4,4259 0,63; 3,3293 92,63; 2,6759 0,68; 2,6716 0,95; 2,6671 0,70; 2,5414 0,50; 2,5248 3,12; 2,5112 52,91; 2,5070 104,07; 2,5025 136,24; 2,4980 100,67; 2,4937 50,98; 2,3337 0,70; 2,3292 0,95; 2,3249 0,72; 2,0760 0,72; 1,6461 0,52; 1,6281 2,21; 1,6100 4,50; 1,5993 4,09; 1,5914 5,78; 1,5854 7,97; 1,5782 8,54; 1,5655 3,54; 1,2856 3,21; 1,2722 6,73; 1,2655 7,27; 1,2511 2,59; 0,7254 7,49; 0,7071 16,00; 0,6885 6,89; 0,0078 1,35; -0,0002 35,98; -0,0083 1,77 |
| Verbindung Nr. 289 [DMSO] 9,5971 2,32; 9,5726 2,41; 9,1193 4,74; 8,1566 13,58; 7,7321 6,99; 7,4712 4,75; 7,3414 6,27; 6,3108 0,92; 6,2888 1,37; 6,2660 0,98; 4,5201 1,02; 4,5029 3,04; 4,4854 3,07; 4,4681 1,02; 4,0562 1,02; 4,0384 3,10; 4,0206 3,12; 4,0028 1,05; 3,3293 33,37; 2,6766 0,45; 2,6721 0,57; 2,6678 0,43; 2,5251 2,30; 2,5075 67,11; 2,5032 84,81; 2,4988 62,31; 2,4686 16,00; 2,3343 0,44; 2,3297 0,59; 2,3256 0,43; 1,9901 13,20; 1,5793 1,60; 1,5652 4,20; 1,5584 4,56; 1,5455 1,91; 1,2938 2,13; 1,2806 4,57; 1,2739 4,85; 1,2594 1,92; 1,2453 4,23; 1,2279 8,85; 1,2103 4,09; 1,1931 3,78; 1,1752 7,27; 1,1575 3,59; 0,0079 1,01; -0,0002 21,12; -0,0083 1,11 |
| Verbindung Nr. 290 [DMSO] 9,5708 2,32; 9,5462 2,40; 9,1192 4,75; 8,1202 6,40; 8,1043 6,41; 7,7312 7,34; 7,7089 0,33; 7,4703 4,65; 7,3393 6,33; 6,2929 0,91; 6,2713 1,34; 6,2483 0,97; 4,5214 0,99; 4,5042 3,01; 4,4865 3,03; 4,4691 0,99; 4,0563 0,68; 4,0384 2,07; 4,0206 2,10; 4,0028 0,70; 3,3304 26,99; 2,6723 0,41; 2,5256 1,39; 2,5122 23,18; 2,5079 46,13; 2,5033 60,59; 2,4988 44,61; 2,4943 22,50; 2,4683 16,00; 2,3301 0,42; 1,9902 8,97; 1,5795 1,66; 1,5653 4,24; 1,5585 4,60; 1,5457 1,96; 1,2939 2,37; 1,2804 4,63; 1,2737 4,89; 1,2593 1,98; 1,2461 4,25; 1,2287 9,20; 1,2110 4,15; 1,1934 2,55; 1,1756 4,90; 1,1577 2,53; 1,1380 0,55; 1,1184 0,57; 0,0079 0,70; -0,0002 18,27; - 0,0085 0,84 |
| Verbindung Nr. 291 [DMSO] 9,6243 2,33; 9,6001 2,38; 9,1166 4,72; 8,1124 4,48; 7,7833 0,41; 7,7639 10,97; 7,7392 0,42; 7,7202 7,24; 7,7001 0,39; 7,4653 4,62; 7,3347 6,14; 6,2374 0,96; 6,2156 1,42; 6,1927 0.99; 4,5196 1,01; 4,5026 3,01; 4,4849 2,99; 4,4675 0,99; 4,0557 0,62; 4,0380 1,88; 4,0201 1,91; 4,0023 0,65; 3,3287 55,40; 2,6759 0,45; 2,6716 0,63; 2,6672 0,46; 2,5245 2,71; 2,5114 36,79; 2,5070 71,49; 2,5025 93,01; 2,4980 68,22; 2,4936 34,13; 2,4670 16,00; 2,3339 0,48; 2,3293 0,65; 2,3249 0,47; 1,9895 8,13; 1,5768 1,67; 1,5624 4,24; 1,5557 4,56; 1,5428 1,91; 1,2933 2,39; 1,2800 4,55; 1,2733 4,79; 1,2589 1,83; 1,2397 4,28; 1,2224 9,03; 1,2047 4,05; 1,1929 2,37; 1,1752 4,29; 1,1573 2,21; 1,1369 0,49; 1,1202 0,46; 1,1017 0,55; 0,0076 0,97; -0,0002 22,55; -0,0084 0,97 |
| Verbindung Nr. 292 [DMSO] 9,5919 2,34; 9,5676 2,41; 9,1177 4,71; 9,0816 0,36; 7,9539 0,81; 7,9500 0,85; 7,9282 1,62; 7,9054 0,83; 7,9013 0,85; 7,7165 7,31; 7,6977 0,57; 7,6521 0,70; 7,6452 1,17; 7,6303 1,32; 7,6240 1,97; 7,5925 1,29; 7,5682 2,18; 7,5457 2,03; 7,5239 0,70; 7,4657 4,60; 7,3847 0,38; 7,3293 6,10; 6,2073 0,93; 6,1852 1,38; 6,1628 0,98; 4,5249 0,97; 4,5073 2,69; 4,4890 2,66; 4,4710 0,97; 4,0560 0,88; 4,0382 2,72; 4,0204 2,75; 4,0026 0,92; 3,3316 37,45; 2,6720 0,41; 2,5253 1,60; 2,5119 24,24; 2,5075 47,89; 2,5029 62,73; 2,4983 46,05; 2,4938 23,25; 2,4675 16,00; 2,3297 0,43; 1,9896 12,11; 1,5773 1,68; 1,5630 4,26; 1,5562 4,65; 1,5434 1,98; 1,2937 2,48; 1,2804 4,59; 1,2737 4,85; 1,2593 1,89; 1,2421 4,23; 1,2247 9,15; 1,2070 4,12; 1,1932 3,54; 1,1754 6,65; 1,1576 3,52; 1,1387 0,66; 1,1208 0,34; 1,1081 0,38; 1,0893 0,76; 1,0704 0,36; 0,0079 0,50; -0,0002 13,09; -0,0085 0,60 |
| Verbindung Nr. 293 [DMSO] 9,5953 2,24; 9,5708 2,31; 9,1176 4,61; 8,3151 1,32; 7,7969 3,73; 7,7240 8,26; 7,6945 1,56; 7,5663 1,41; 7,5612 2,03; 7,5560 1,29; 7,5446 1,48; 7,5396 2,04; 7,5342 1,17; 7,4651 4,54; 7,3432 6,10; 6,2654 0,86; 6,2435 1,29; 6,2210 0,92; 4,5232 0,98; 4,5058 2,88; 4,4880 2,85; 4,4705 0,94; 4,0380 0,73; 4,0202 0,72; 3,8314 1,16; 3,3301 194,48; 2,6810 0,39; 2,6764 0,83; 2,6719 1,16; 2,6673 0,85; 2,6628 0,40; 2,5421 0,60; 2,5252 3,91; 2,5204 6,16; 2,5119 63,14; 2,5074 127,12; 2,5028 167,45; 2,4982 121,38; 2,4937 59,13; 2,4684 16,00; 2,3385 0,38; 2,3341 0,82; 2,3296 1,13; 2,3249 0,82; 2,3203 0,40; 1,9891 3,24; 1,5759 1,61; 1,5617 3,99; 1,5549 4,35; 1,5421 1,85; 1,2934 2,21; 1,2800 4,43; 1,2733 4,77; 1,2588 1,92; 1,2446 4,22; 1,2272 9,25; 1,2095 4,06; 1,1935 1,05; 1,1757 1,86; 1,1579 0,93; 0,0080 1,35; -0,0002 40,16; -0,0085 1,55 |
| Verbindung Nr. 294 [DMSO] 9,9489 4,28; 9,9248 4,44; 9,3640 10,27; 8,2139 6,40; 8,0816 3,70; 8,0692 13,21; 7,9517 16,00; 7,8397 3,14; 7,8200 4,45; 7,7459 3,38; 7,7264 5,15; 7,7069 2,15; 7,6621 11,84; 6,3869 0,45; 6,3653 1,72; 6,3432 2,63; 6,3208 1,88; 6,2987 0,54; 5,7284 15,89; 3,3311 68,74; 2,6769 0,52; 2,6724 0,71; 2,6679 0,53; 2,5253 2,82; 2,5118 41,71; 2,5077 80,41; 2,5033 103,71; 2,4989 75,71; 2,3344 0,53; 2,3300 0,70; 2,3255 0,52; 2,0764 0,63; 1,6149 3,38; 1,6007 8,12; 1,5939 8,74; 1,5809 3,64; 1,5410 0,33; 1,3378 0,33; 1,2977 3,86; 1,2842 8,17; 1,2776 8,66; 1,2631 3,03; 0,0079 1,05; -0,0002 24,61; -0,0082 0,95 |
| Verbindung Nr. 295 [DMSO] 9,5502 2,30; 9,5259 2,37; 8,2465 2,41; 8,2356 2,44; 7,9567 0,84; 7,9531 0.90; 7,9313 1,63; 7,9086 0,85; 7,9040 0,86; 7,6525 0,77; 7,6452 1,21; 7,6400 0,99; 7,6266 8,82; 7,6034 0,34; 7,5902 1,40; 7,5678 1,91; 7,5654 2,00; 7,5432 2,02; 7,5215 0,70; 7,4153 4,55; 7,3825 0,42; 7,3330 0.64; 7,3213 6,23; 6,2019 0,96; 6,1797 1,41; 6,1573 1,00; 4,5205 1,03; 4,5031 2,85; 4,4847 2,81; 4,4668 1,02; 4,0559 0,48; 4,0381 1,46; 4,0203 1,46; 4,0025 0,48; 3,3302 54,56; 2,8599 0,73; 2,8504 0,99; 2,8417 1,62; 2,8316 1,61; 2,8234 1,19; 2,8134 0,84; 2,8034 0,42; 2,6762 0,42; 2,6716 0,58; 2,6671 0,48; 2,6583 1,12; 2,5251 2,20; 2,5202 3,44; 2,5117 31,46; 2,5073 62,57; 2,5027 81,82; 2,4982 59,46; 2,4937 29,41; 2,4457 16,00; 2,3340 0,43; 2,3293 0,57; 2,3251 0,43; 1,9896 6,49; 1,3365 1,08; 1,2591 0,52; 1,2497 1,58; 1,2418 1,27; 1,2332 4,60; 1,2158 8,88; 1,1981 4,01; 1,1932 2,64; 1,1752 3,51; 1,1573 1,75; 1,1451 0,35; 1,1277 0,65; 1,0978 0,38; 1,0792 0,77; 1,0601 0,37; 0,7082 1,04; 0,6955 2,87; 0,6902 4,07; 0,6783 3,66; 0,6722 3,20; 0,6611 1,35; 0,5622 0,42; 0,5547 1,56; 0,5442 4,08; 0,5381 3,43; 0,5343 3,32; 0,5285 3,08; 0,5163 0,96; 0,0079 0,58; -0,0002 15,02; -0,0085 0,66 |
| Verbindung Nr. 296 [DMSO] 9,5278 2,31; 9,5031 2,39; 8,3174 0,40; 8,2469 2,55; 8,2359 2,57; 8,1206 6,48; 8,1048 6,43; 8,0700 0,49; 8,0540 0,48; 7,6409 7,35; 7,6051 0,55; 7,4180 4,53; 7,3302 6,36; 6,2864 0,92; 6,2647 1,33; 6,2417 0,97; 5,7574 1,11; 4,5177 1,00; 4,5006 3,04; 4,4829 3,05; 4,4653 0,97; 4,0563 0,76; 4,0385 2,32; 4,0207 2,36; 4,0029 0,79; 3,3609 0,36; 3,3334 137,10; 2,8617 0,76; 2,8518 1,01; 2,8436 1,74; 2,8333 1,66; 2,8253 1,25; 2,8153 0,83; 2,8054 0,44; 2,6773 0,36; 2,6726 0,49; 2,6681 0,35; 2,5427 0,35; 2,5391 0,40; 2,5259 2,22; 2,5212 3,40; 2,5126 28,42; 2,5081 56,76; 2,5036 74,37; 2,4990 53,52; 2,4944 26,01; 2,4478 16,00; 2,3349 0,38; 2,3303 0,51; 2,3258 0,39; 1,9899 10,63; 1,2380 4,25; 1,2207 9,14; 1,2030 4,07; 1,1937 3,24; 1,1759 5,80; 1,1581 2,86; 1,1466 0,36; 1,1285 0,94; 1,1092 0,98; 1,0900 0,36; 0,7098 1,09; 0,6969 2,88; 0,6918 4,11; 0,6799 3,68; 0,6737 3,21; 0,6627 1,37; 0,5646 0,43; 0,5559 1,63; 0,5455 4,08; 0,5395 3,46; 0,5354 3,34; 0,5299 3,10; 0,5177 1,00; 0,0080 0,59; -0,0002 15,76; -0,0085 0,61 |
| Verbindung Nr. 297 [DMSO] 9,5835 2,22; 9,5591 2,29; 8,3183 0,36; 8,2457 2,46; 8,2347 2,37; 8,1150 4,71; 7,7901 0,32; 7,7633 11,41; 7,7613 10,75; 7,7419 0,63; 7,6302 7,15; 7,5969 0,44; 7,5909 0,45; 7,4354 0,35; 7,4150 4,53; 7,3267 6,26; 7,1036 0,39; 6,2324 0,96; 6,2101 1,43; 6,1872 1,01; 5,7585 0,58; 4,5157 1,04; 4,4984 3,15; 4,4807 3,17; 4,4632 1,04; 4,0556 0,63; 4,0378 1,94; 4,0200 1,98; 4,0022 0,65; 3,3293 69,56; 2,8922 3,79; 2,8596 0,76; 2,8498 1,08; 2,8415 1,61; 2,8313 1,70; 2,8233 1,08; 2,8132 0,92; 2,6761 0,61; 2,6715 0,85; 2,6669 0,66; 2,6621 0,42; 2,6557 0,78; 2,5385 0,60; 2,5247 3,42; 2,5199 5,38; 2,5114 46,39; 2,5070 91,51; 2,5024 119,56; 2,4979 86,94; 2,4934 43,03; 2,4644 1,48; 2,4456 16,00; 2,3337 0,63; 2,3292 0,84; 2,3246 0,62; 1,9895 8,55; 1,3460 0,61; 1,3360 1,56; 1,2493 2,15; 1,2312 4,90; 1,2136 8,93; 1,1957 4,20; 1,1931 4,21; 1,1751 4,73; 1,1572 2,32; 1,1264 0,51; 1,1103 0,46; 1,0921 0,60; 0,7079 1,07; 0,6950 2,98; 0,6899 4,21; 0,6780 3,80; 0,6718 3,33; 0,6608 1,40; 0,5547 1,57; 0,5442 4,19; 0,5381 3,62; 0,5343 3,46; 0,5285 3,21; 0,5163 1,00; 0,0080 0,86; -0,0002 22,57; -0,0084 0,94 |
| Verbindung Nr. 298 [DMSO] 9,5565 2,34; 9,5319 2,40; 8,2467 2,55; 8,2358 2,48; 7,8023 4,22; 7,7238 1,80; 7,7004 1,79; 7,6358 7,11; 7,5698 1,54; 7,5648 2,26; 7,5596 1,35; 7,5481 1,65; 7,5431 2,31; 7,5379 1,27; 7,4708 0,33; 7,4180 4,68; 7,3953 0,61; 7,3357 6,56; 6,2633 0,98; 6,2415 1,44; 6,2187 1,03; 4,5193 1,14; 4,5017 3,36; 4,4840 3,32; 4,4667 1,09; 4,0557 0,71; 4,0379 2,14; 4,0201 2,17; 4,0023 0,74; 3,3291 72,08; 2,9091 0,71; 2,9032 1,42; 2,8605 0,73; 2,8509 1,02; 2,8422 1,52; 2,8323 1,54; 2,8242 1,00; 2,8141 0,74; 2,6756 0,84; 2,6716 1,05; 2,6671 0,82; 2,6581 1,65; 2,5246 4,26; 2,5112 51,04; 2,5070 97,20; 2,5025 124,58; 2,4981 90,33; 2,4939 44,60; 2,4645 0,97; 2,4467 16,00; 2,3335 0,63; 2,3292 0,86; 2,3249 0,63; 1,9895 9,33; 1,3474 0,38; 1,3361 3,13; 1,2593 0,92; 1,2494 4,17; 1,2431 2,16; 1,2348 5,86: 1,2175 8,94; 1,1999 4,10; 1,1929 3,11; 1,1750 4,99; 1,1572 2,47; 0,7083 1,01; 0,6955 2,88; 0,6904 3,94; 0,6783 3,58; 0,6724 3,13; 0,6614 1,27; 0,5546 1,37; 0,5442 4,05; 0,5380 3,60; 0,5286 3,18; 0,5164 0,94; 0,0079 0,91; -0,0002 19,97; -0,0077 0,85 |
| Verbindung Nr. 299 [DMSO] 9,9273 3,75; 9,9032 3,91; 9,3024 8,90; 8,1830 5,51; 8,0574 2,97; 8,0381 3,30; 7,9526 0,86; 7,8756 10,33; 7,8375 16,00; 7,8102 3,87; 7,7327 2,97; 7,7132 4,53; 7,6937 1,90; 7,3185 10,82; 6,3272 0,42; 6,3065 1,49; 6,2835 2,24; 6,2610 1,59; 6,2391 0,46; 4,4126 0,93; 4,3939 1,09; 4,3770 2,72; 4,3585 3,16; 4,3525 3,23; 4,3333 2,77; 4,3178 1,09; 4,2985 0,97; 3,4037 0,39; 3,3326 880,92; 2,8905 6,85; 2,7307 5,55; 2,6756 2,67; 2,6711 3,67; 2,6666 2,73; 2,6621 1,35; 2,5244 14,21; 2,5111 213,88; 2,5067 428,19; 2,5021 560,62; 2,4976 408,56; 2,4931 204,67; 2,3378 1,21; 2,3333 2,58; 2,3289 3,56; 2,3243 2,66; 2,3200 1,31; 1,9273 0,72; 1,9105 1,44; 1,8934 1,82; 1,8765 1,48; 1,8590 0,76; 1,5975 3,04; 1,5834 7,27; 1,5766 7,97; 1,5637 3,43; 1,3355 2,93; 1,3232 0,46; 1,2978 1,91; 1,2825 3,64; 1,2690 7,50; 1,2623 8,31; 1,2488 6,33; 1,2354 1,14; 0,9990 0,32; 0,9821 0,32; 0,7562 0,35; 0,7381 0,74; 0,7173 13,64; 0,7007 13,29; 0,6666 13,48; 0,6500 13,15; 0,1460 0,99; 0,0079 8,68; -0,0002 234,15; -0,0085 10,63; -0,1498 1,03 |
| Verbindung Nr. 300 [DMSO] 10,4523 2,00; 10,4281 2,10; 8,4559 2,21; 8,4450 2,21; 8,3588 1,43; 8,3448 1,39; 8,2008 0,79; 8,1943 0,86; 8,1865 1,00; 8,1792 0,90; 8,1733 0,86; 7,9255 7,27; 7,7383 7,63; 7,6943 1,25; 7,6686 1,62; 7,6460 1,18; 6,3936 0,79; 6,3719 1,17; 6,3493 0,84; 4,0564 1,21; 4,0386 3,68; 4,0208 3,72; 4,0030 1,25; 3,5783 0,36; 3,5688 0,79; 3,5602 1,06; 3,5513 1,47; 3,5422 1,07; 3,5339 0,77; 3,5240 0,38; 3,3336 31,59; 2,8511 0,60; 2,8418 0,86; 2,8329 1,33; 2,8229 1,33; 2,8146 0,86; 2,8048 0,63; 2,5258 1,28; 2,5123 17,74; 2,5080 34,99; 2,5036 45,56; 2,4990 33,48; 2,4948 16,85; 2,3303 0,34; 1,9902 16,00; 1,3371 0,55; 1,2995 0,47; 1,2592 0,67; 1,2499 0,75; 1,2351 0,61; 1,1934 4,34; 1,1756 8,61; 1,1578 4,34; 1,1457 0,50; 1,1362 0,70; 1,1260 0,85; 1,1194 1,02; 1,1127 0,93; 1,1045 1,32; 1,0954 1,38; 1,0885 0,92; 1,0824 0,96; 1,0759 0,94; 1,0652 0,73; 1,0565 0,47; 0,8647 0,34; 0,8533 0,60; 0,8375 0,80; 0,8267 1,13; 0,8136 1,17; 0,8045 0,61; 0,7917 0,59; 0,7825 1,13; 0,7689 1,06; 0,7580 0,79; 0,7471 0,46; 0,7422 0,50; 0,7282 0,95; 0,7150 2,29; 0,7101 3,31; 0,6981 2,98; 0,6920 2,65; 0,6809 1,10; 0,5833 1,14; 0,5729 3,34; 0,5664 3,03; 0,5574 2,64; 0,5450 0,84; -0,0002 1,38 |
| Verbindung Nr. 301 [DMSO] 10,7140 2,01; 8,6376 1,15; 8,6264 1,16; 8,5370 4,38; 8,5259 4,41; 8,4752 3,01; 8,4630 2,87; 8,3178 0,93; 8,2604 1,86; 8,2550 2,00; 8,2483 2,29; 8,2378 2,47; 8,1843 13,71; 8,0901 3,43; 7,9851 3,51; 7,9495 0,64; 7,8515 14,05; 7,6658 2,87; 7,6401 3,72; 7,6172 2,73; 7,5925 0,37; 6,3961 1,26; 6,3760 1,76; 6,3569 1,22; 5,8181 2,18; 5,8048 16,00; 3,3276 362,33; 2,8863 0,50; 2,8761 1,38; 2,8663 1,92; 2,8580 2,79; 2,8478 2,85; 2,8394 1,76; 2,8297 1,28; 2,8193 0,50; 2,6757 2,66; 2,6712 3,57; 2,6668 2,69; 2,5412 3,95; 2,5384 3,34; 2,5244 16,00; 2,5109 206,69; 2,5066 402,55; 2,5022 521,64; 2,4977 377,76; 2,4934 185,73; 2,3334 2,61; 2,3289 3,52; 2,3245 2,64; 2,0753 2,61; 0,7538 0,54; 0,7402 2,76; 0,7361 2,29; 0,7223 7,71; 0,7100 6,19; 0,7042 5,41; 0,6930 2,06; 0,5716 2,42; 0,5608 7,71: 0,5550 7,31; 0,5510 6,82; 0,5460 5,91; 0,5335 1,73; 0,4661 0,37; 0,4578 0,33; 0,0079 0,58; -0,0002 15,23; -0,0083 0,68 |
| Verbindung Nr. 302 vgl. Synthesebeispiel 6 |
| Verbindung Nr. 303 [CD3CN] 8,5834 3,10; 7,7549 6,64; 7,7516 7,02; 7,7435 7,02; 7,7401 7,17; 7,7251 7,12; 7,6719 11,14; 7,5778 3,42; 7,5740 3,82; 7,5707 4,09; 7,5639 4,55; 7,5597 4,39; 7,5565 4,39; 7,5527 3,75; 7,3401 9,39; 7,3253 16,00; 7,3105 8,32; 6,0652 0,97; 6,0564 1,96; 6,0478 2,13; 6,0384 2,95; 6,0281 3,53; 6,0190 3,32; 6,0104 3,56; 6,0021 2,61; 5,9884 1,53; 5,9791 1,47; 5,9444 3,38; 5,4286 3,76; 5,3234 3,05; 5,2847 12,72; 5,2767 12,73; 5,1999 3,28; 5,1832 3,39; 5,1694 8,58; 5,1521 7,98; 5,1508 7,88; 5,1018 0,71; 5,0312 8,23; 5,0296 8,01; 5,0025 7,71; 5,0009 7,56; 4,1637 4,24; 4,0824 0,72; 4,0706 1,84; 4,0587 1,84; 4,0468 0,70; 3,6943 0,52; 2,8012 0,38; 2,7963 0,38; 2,6862 2,87; 2,1017 0,34; 2,0989 0,37; 2,0962 0,43; 2,0909 0,79; 2,0859 0,64; 2,0819 0,74; 2,0763 0,87; 2,0741 1,26; 2,0476 2813,50; 2,0318 4,75; 2,0227 1,39; 2,0197 1,45; 2,0164 1.47; 2,0146 1,48; 2,0006 0,41; 1,9979 0,33; 1,9947 0,32; 1,9921 0,33; 1,9842 0,35; 1,9634 7,77; 1,9568 0,48; 1,9491 4,04; 1,9409 5,40; 1,9330 105,39; 1,9289 201,71; 1,9248 295,53; 1,9207 203,36; 1,9166 103,84; 1,8179 0,59; 1,8140 1,14; 1,8098 1,67; 1,8057 1,15; 1,8016 0.61; 1,2780 1,65; 1,2150 1,91; 1,2031 3,77; 1,1913 1,86; 0,8815 0,50; 0,8700 0,55; 0,8479 0,75; 0,7825 0,74; 0,5714 5,77; 0,3783 5,80; 0,3706 5,71; -0,0001 3,12 |
| Verbindung Nr. 304 [DMSO] 9,6728 2,20; 9,6485 2,29; 8,4461 2,17; 8,4267 2,20; 8,3044 1,56; 8,2877 1,49; 8,1814 0,84; 8,1743 0,94; 8,1663 1,11; 8,1586 0,99; 8,1533 0,99; 7,6830 1,44; 7,6570 8,90; 7,6347 1,34; 7,6222 0,52; 7,4225 4,52; 7,3414 0,40; 7,3246 6,27; 6,3514 0,89; 6,3294 1,32; 6,3069 0,94; 4,5180 0,97; 4,5006 2,89; 4,4830 2,88; 4,4654 0,95; 4,4264 0,72; 4,4060 1,38; 4,3859 1,37; 4,3655 0,72; 4,0563 0,46; 4,0385 1,38; 4,0207 1,38; 4,0029 0,47; 3,4165 0,47; 3,3527 350,16; 2,6779 0,37; 2,6733 0,50; 2,6688 0,38; 2,5265 1,88; 2,5131 28,47; 2,5088 56,28; 2,5042 73,99; 2,4997 54,57; 2,4952 27,04; 2,4471 16,00; 2,3354 0,37; 2,3309 0,49; 2,3264 0,35; 2,2700 0,46; 2,2615 0,71; 2,2532 1,04; 2,2420 1,65; 2,2351 1,75; 2,2244 1,69; 2,2178 1,49; 2,2143 1,48; 2,1959 0,65; 2,0759 0,45; 2,0696 0,52; 2,0636 0,38; 2,0462 1,50; 2,0217 2,07; 2,0165 1,91; 1,9986 1,47; 1,9901 6,89; 1,9759 0,42; 1,9700 0,49; 1,7026 0,84; 1,6897 1,68; 1,6793 1,87; 1,6649 2,34; 1,6570 1,18; 1,6456 1,19; 1,6385 0,99; 1,6206 0,41; 1,2330 4,20; 1,2157 8,79; 1,1980 3,96; 1,1933 2,57; 1,1753 3,32; 1,1575 1,64; 1,1185 0,56; 1,1096 0,37; 1,0909 0,63; -0,0002 8,39 |
| Verbindung Nr. 305 [DMSO] 9,6686 1,63; 9,6442 1,69; 8,3031 1,15; 8,2866 1,11; 8,1794 0,65; 8,1735 0,70; 8,1643 0,80; 8,1573 0,75; 8,1521 0,73; 8,0646 1,56; 8,0450 1,59; 7,6825 1,05; 7,6566 1,35; 7,6339 1,17; 7,6254 5,49; 7,5800 0,33; 7,4173 3,42; 7,3191 4,76; 6,3495 0,66; 6,3276 0,99; 6,3051 0,71; 4,5173 0,72; 4,5000 2,19; 4,4823 2,20; 4,4646 0,72; 4,0864 0,66; 4,0698 0,99; 4,0508 0,99; 4,0380 0,75; 4,0344 0,70; 4,0204 0,54; 3,3587 329,02; 2,6734 0,34; 2,5263 1,06; 2,5133 18,93; 2,5089 38,28; 2,5044 50,99; 2,4998 37,20; 2,4954 18,07; 2,4524 12,62; 2,3313 0,35; 2,0754 0,68; 1,9899 1,79; 1,2319 3,15; 1,2145 6,70; 1,1968 3,08; 1,1749 2,29; 1,1680 16,00; 1,1516 15,70; 1,1352 0,44; 1,1172 0,46; 1,0860 0,50; 0,0079 0,68; -0,0002 19,51; -0,0085 0,74 |
| Verbindung Nr. 306 [DMSO] 9,6865 1,94; 9,6622 2,00; 8,5446 1,99; 8,5251 2,03; 8,3028 1,37; 8,2894 1,31; 8,2860 1,31; 8,1809 0,73; 8,1749 0,81; 8,1664 0,94; 8,1591 0,86; 8,1533 0,85; 7,7046 6,18; 7,6837 1,27; 7,6723 0,65; 7,6579 1,58; 7,6352 1,21; 7,4553 3,97; 7,3351 5,44; 6,3553 0,77; 6,3331 1,15; 6,3106 0,83; 5,7583 1,26; 4,6573 3,44; 4,6446 4,36; 4,5398 3,25; 4,5275 5,16; 4,5070 2,95; 4,4892 2,96; 4,4723 1,13; 4,4487 0,33; 4,0563 1,19; 4,0385 3,65; 4,0207 3,69; 4,0029 1,24; 3,6947 0,62; 3,3503 182,58; 3,3481 200,73; 2,6731 0,41; 2,6685 0,35; 2,5128 22,82; 2,5085 45,10; 2,5040 59,70; 2,4995 44,57; 2,4951 22,70; 2,4735 14,28; 2,3992 0,49; 2,3307 0,40; 2,0763 0,72; 1,9901 16,00; 1,2399 3,49; 1,2226 7,53; 1,2049 3,41; 1,1932 4,55; 1,1754 8,63; 1,1576 4,29; 1,1273 0,46; 1,0971 0,51; 0,0080 1,30; -0,0002 34,18; -0,0085 1,40 |
| Verbindung Nr. 307 [DMSO] 9,5471 2,18; 9,5228 2,28; 8,2449 2,37; 8,2340 2,40; 7,8891 3,66; 7,8864 3,80; 7,8217 0,37; 7,6364 1,66; 7,6329 1,74; 7,6219 7,32; 7,6158 2,71; 7,6121 2,45; 7,5933 0,71; 7,5432 0,51; 7,5251 5,34; 7,5041 3,72; 7,4139 4,33; 7,3315 0,55; 7,3189 6,00; 6,1999 0,86; 6,1779 1,27; 6,1551 0,92; 5,7580 6,51; 4,5196 0,88; 4,5019 2,40; 4,4830 2,34; 4,4648 0,87; 4,0559 1,17; 4,0381 3,60; 4,0203 3,66; 4,0025 1,22; 3,3276 76,24; 2,8595 0,69; 2,8500 0,90; 2,8413 1,56; 2,8310 1,52; 2,8232 1,13; 2,8129 0,79; 2,8031 0,40; 2,6760 0,53; 2,6716 0,71; 2,6670 0,52; 2,5249 1,78; 2,5201 2,90; 2,5116 37,27; 2,5071 74,63; 2,5025 97,70; 2,4979 70,56; 2,4934 33,50; 2,4444 15,20; 2,3338 0,50; 2,3292 0,67; 2,3246 0,50; 1,9895 16,00; 1,3530 1,24; 1,2335 4,48; 1,2161 8,61; 1,1983 3,93; 1,1930 5,14; 1,1752 8,98; 1,1574 4,40; 1,1465 0,38; 1,1288 0,72; 1,1111 0,35; 1,0973 0,43; 1,0787 0,89; 1,0599 0,41; 0,7078 1,01; 0,6952 2,74; 0,6899 3,89; 0,6779 3,49; 0,6718 3,01; 0,6608 1,27; 0,5616 0,44; 0,5537 1,54; 0,5433 3,86; 0,5372 3,18; 0,5332 3,06; 0,5276 2,84; 0,5154 0,91; 0,0080 2,60; -0,0002 76,07; -0,0085 2,39 |
| Verbindung Nr. 308 [DMSO] 9,5898 1,72; 9,5655 1,78; 9,1167 3,56; 9,0801 0,33; 7,8834 3,01; 7,7118 5,39; 7,6966 0,50; 7,6366 1,26; 7,6335 1,24; 7,6155 1,88; 7,6123 1,81; 7,5453 0,40; 7,5274 4,02; 7,5065 2,79; 7,4644 3,46; 7,3830 0,35; 7,3273 4,58; 6,2055 0,70; 6,1834 1,02; 6,1609 0,73; 5,7578 1,65; 4,5239 0,70; 4,5064 1,91; 4,4879 1,87; 4,4697 0,68; 4,0563 1,21; 4,0385 3,71; 4,0207 3,76; 4,0029 1,26; 3,3284 38,82; 2,6719 0,38; 2,5254 1,01; 2,5119 21,92; 2,5075 43,33; 2,5030 56,60; 2,4984 41,46; 2,4940 20,19; 2,4666 11,90; 2,3297 0,40; 1,9897 16,00; 1,5769 1,20; 1,5627 3,13; 1,5559 3,40; 1,5431 1,42; 1,4542 0,35; 1,4470 0,34; 1,3535 0,45; 1,2932 1,88; 1,2799 3,40; 1,2733 3,54; 1,2588 1,40; 1,2428 3,17; 1,2254 6,76; 1,2077 3,05; 1,1934 4,59; 1,1756 8,91; 1,1578 4,82; 1,1400 0,64; 1,1081 0,34; 1,0895 0,68; 1,0705 0,33; 0,0080 1,33; -0,0002 38,57; -0,0085 1,37 |
| Verbindung Nr. 309 [DMSO] 10,5019 1,42; 8,3280 7,72; 8,1088 4,37; 8,0893 4,81; 7,9759 15,51; 7,9628 1,98; 7,8699 16,00; 7,8587 1,28; 7,8273 4,22; 7,8076 5,83; 7,7805 1,62; 7,7194 3,92; 7,6999 6,23; 7,6804 2,71; 6,2912 2,04; 6,0479 0,77; 6,0347 1,83; 6,0216 1,76; 6,0088 2,37; 5,9918 3,07; 5,9890 2,85; 5,9779 2,90; 5,9657 2,94; 5,9631 3,20; 5,9523 1,77; 5,9457 2,64; 5,9323 1,66; 5,9198 2,11; 5,9057 0,97; 5,3285 4,19; 5,3249 4,31; 5,2854 4,34; 5,2817 4,57; 5,2515 5,70; 5,2145 4,87; 5,2112 4,70; 5,1888 4,25; 5,1855 4,10; 5,1247 5,18; 5,1221 5,31; 5,0986 4,90; 5,0962 5,23; 5,0713 0,37; 5,0031 4,37; 5,0001 4,17; 4,9602 4,00; 4,9572 3,85; 4,5640 0,35; 4,2802 0,72; 4,0558 0,81; 4,0380 1,95; 4,0201 2,04; 4,0024 1,10; 3,9730 0,73; 3,4961 0,34; 3,4817 0,45; 3,4280 0,49; 3,4158 0,54; 3,3267 127,16; 3,3031 2,43; 2,8094 0,37; 2,7994 0,52; 2,6760 1,11; 2,6715 1,55; 2,6669 1,32; 2,6620 1,17; 2,6507 1,60; 2,6429 2,17; 2,6340 2,83; 2,6252 2,23; 2,6171 1,64; 2,6073 0,84; 2,5418 0,90; 2,5248 3,45; 2,5113 72,48; 2,5070 149,29; 2,5024 201,19; 2,4979 149,02; 2,4936 73,09; 2,3381 0,48; 2,3337 1,03; 2,3292 1,39; 2,3247 1,03; 2,0963 1,20; 1,9896 7,03; 1,3514 0,40; 1,2337 0,97; 1,1925 1,95; 1,1747 3,84; 1,1569 1,89; 0,8541 0,46; 0,7395 1,34; 0,5507 5,70; 0,3608 4,53; 0,3455 4,37; 0,0080 2,08; -0,0002 63,47; -0,0085 2,22 |
| Verbindung Nr. 310 [DMSO] 9,8316 2,59; 9,8077 2,64; 9,3012 6,28; 8,3173 0,55; 8,2865 1,55; 8,2680 2,70; 8,2495 1,46; 7,8654 11,49; 7,8601 7,49; 7,4131 7,32; 6,3725 1,08; 6,3510 1,52; 6,3290 1,11; 6,3079 0,33; 4,5438 1,22; 4,5263 3,39; 4,5086 3,34; 4,4911 1,10; 3,3296 122,91; 2,6767 0,55; 2,6721 0,74; 2,6676 0,53; 2,5253 4,79; 2,5121 43,87; 2,5076 84,02; 2,5031 108,33; 2,4985 77,28; 2,4940 36,71; 2,3344 0,56; 2,3298 0,73; 2,3252 0,51; 1,9897 0,53; 1,6032 2,21; 1,5891 4,97; 1,5822 5,28; 1,5693 2,30; 1,3975 16,00; 1,3362 2,06; 1,2989 0,55; 1,2864 2,63; 1,2729 5,13; 1,2663 5,48; 1,2501 4,07; 1,2373 5,40; 1,2200 10,55; 1,2023 4,69; 1,1756 0,42; 0,0079 1,76; -0,0002 39,91; - 0,0085 1,41 |
| Verbindung Nr. 311 [DMSO] 9,6912 3,15; 9,6668 3,27; 9,2980 7,62; 8,1170 8,88; 8,1012 8,89; 7,8562 14,76; 7,8517 9,31; 7,3879 8,83; 6,2991 1,16; 6,2772 1,71; 6,2545 1,26; 6,2333 0,38; 4,5447 1,29; 4,5273 3,99; 4,5096 3,99; 4,4922 1,28; 4,0567 0,70; 4,0389 2,16; 4,0211 2,18; 4,0033 0,73; 3,3306 41,75; 2,6777 0,34; 2,6731 0,47; 2,6684 0,34; 2,5266 1,26; 2,5219 1,83; 2,5131 25,28; 2,5086 52,16; 2,5040 69,32; 2,4994 49,74; 2,4949 23,45; 2,3353 0,34; 2,3308 0,47; 2,3264 0,34; 1,9905 9,68; 1,6041 2,52; 1,5900 5,80; 1,5831 6,33; 1,5702 2,74; 1,3968 16,00; 1,3369 1,27; 1,3291 0,34; 1,2992 0,39; 1,2890 3,04; 1,2755 5,94; 1,2688 6,44; 1,2543 2,72; 1,2500 2,26; 1,2368 6,29; 1,2195 12,99; 1,2018 5,71; 1,1939 3,24; 1,1761 5,52; 1,1583 2,68; 0,0080 0,81; -0,0002 27,85; -0,0085 0,88 |
| Verbindung Nr. 312 vgl. Synthesebeispiel 5 |
| Verbindung Nr. 313 [DMSO] 10,4423 4,17; 10,4181 4,44; 9,6441 0,55; 9,6205 0,55; 8,4738 4,65; 8,4629 4,86; 8,3558 2,90; 8,3391 2,93; 8,3177 2,21; 8,1840 2,05; 8,1702 2,55; 8,0415 0,48; 8,0222 0,50; 7,8742 0,85; 7,8355 0,61; 7,8303 0,67; 7,8231 0,44; 7,8014 0,55; 7,7777 15,62; 7,7592 16,00; 7,7317 1,32; 7,7229 0,52; 7,6936 2,63; 7,6677 3,43; 7,6448 2,53; 7,1814 0,33; 6,4104 0,43; 6,3911 1,61; 6,3696 2,39; 6,3470 1,75; 6,3251 0,58; 6,2764 0,39; 4,0374 0,35; 4,0197 0,35; 3,5669 1,58; 3,5585 2,12; 3,5495 3,03; 3,5400 2,21; 3,5321 1,59; 3,5220 0,78; 3,3264 472,16; 2,8709 0,37; 2,8605 1,18; 2,8508 1,65; 2,8423 2,67; 2,8320 2,69; 2,8241 1,69; 2,8139 1,29; 2,8036 0,45; 2,6755 3,73; 2,6709 5,30; 2,6664 3,91; 2,5411 1,66; 2,5243 13,63; 2,5195 21,76; 2,5108 284,09; 2,5064 580,86; 2,5018 771,21; 2,4973 571,48; 2,4929 285,94; 2,4202 5,78; 2,3331 4,17; 2,3286 5,68; 2,3241 4,27; 1,9891 1,92; 1,6593 0,35; 1,6232 0,53; 1,3354 2,78; 1,2980 2,67; 1,2584 3,86; 1,2493 3,82; 1,2349 2,72; 1,1924 0,75; 1,1746 1,09; 1,1568 0,97; 1,1401 1,14; 1,1310 1,52; 1,1140 2,23; 1,0995 2,94; 1,0903 3,01; 1,0769 2,09; 1,0602 1,63; 1,0509 1,11; 1,0340 0,68; 0,8673 0,94; 0,8542 1,49; 0,8410 1,80; 0,8301 2,68; 0,8168 2,62; 0,8078 1,40; 0,7947 1,33; 0,7856 2,43; 0,7719 2,25; 0,7613 1,70; 0,7451 1,14; 0,7281 2,00; 0,7149 4,94; 0,7100 7,07; 0,6980 6,35; 0,6920 5,60; 0,6807 2,32; 0,6594 0,43; 0,6416 0,40; 0,6089 0,35; 0,5992 0,36; 0,5695 2,41; 0,5590 6,93; 0,5529 6,33; 0,5434 5,64; 0,5312 1,84; 0,4472 0,36; 0,1460 1,33; 0,0080 10,64; -0,0002 306,83; -0,0085 11,92; -0,0685 0,65; -0,1497 1,28 |
| Verbindung Nr. 314 [DMSO] 10,3348 1,85; 10,3106 1,92; 8,4744 1,99; 8,4635 2,02; 8,0662 2,84; 7,8300 0.35; 7,8275 0,34; 7,7744 6,88; 7,7653 1,78; 7,7554 7,00; 7,7458 1,80; 7,6771 1,37; 7,6746 1,32; 7,6570 1,59; 7,6544 1,64; 7,4608 2,01; 7,4411 3,31; 7,4213 1,57; 6,1951 0,70; 6,1732 1,04; 6,1507 0,76; 4,0553 1,19; 4,0376 3,60; 4,0198 3,64; 4,0020 1,22; 3,5726 0,34; 3,5634 0,69; 3,5549 0,93; 3,5460 1,30; 3,5368 0,94; 3,5285 0,68; 3,5189 0,35; 3,3285 66,38; 2,8608 0,53; 2,8511 0,73; 2,8427 1,15; 2,8325 1,16; 2,8243 0,73; 2,8145 0,57; 2,6756 0,36; 2,6709 0,48; 2,6663 0,36; 2,5243 1,63; 2,5109 26,54; 2,5065 52,26; 2,5020 68,41; 2,4974 50,41; 2,4930 25,21; 2,3332 0,34; 2,3287 0,47; 2,3241 0,35; 1,9892 16,00; 1,6767 0,36; 1,6594 0,35; 1,3358 0,61; 1,2983 0,75; 1,2584 1,08; 1,2493 0,78; 1,2340 0,56; 1,1925 4,35; 1,1747 8,62; 1,1569 4,26; 1,1162 0,65; 1,1016 1,79; 1,0875 1,84; 1,0747 0,74; 0,8519 0,55; 0,8419 0,46; 0,8305 0,62; 0,8218 1,47; 0,8124 1,17; 0,7997 1,10; 0,7901 1,38; 0,7805 0,56; 0,7691 0,39; 0,7636 0,36; 0,7592 0,41; 0,7274 0,76; 0,7143 2,08; 0,7094 2,95; 0,6973 2,70; 0,6913 2,34; 0,6801 1,00; 0,5704 1,00; 0,5598 2,90; 0,5536 2,64; 0,5500 2,52; 0,5443 2,38; 0,5320 0,77; 0,0080 1,33; -0,0002 34,44;-0,0084 1,44 |
| Verbindung Nr. 315 [DMSO] 9,7193 3,91; 9,6950 4,05; 9,2986 9,34; 7,9517 1,39; 7,9480 1,48; 7,9236 2,98; 7,9032 1,43; 7,8991 1,42; 7,8489 11,36; 7,8418 16,00; 7,6473 1,98; 7,6259 3,56; 7,5967 2,16; 7,5739 4,49; 7,5512 3,41; 7,5283 1,14; 7,3780 10,91; 6,2361 0,40; 6,2148 1,53; 6,1928 2,29; 6,1704 1,65; 6,1484 0.47; 4,5471 1,65; 4,5293 4,67; 4,5113 4,63; 4,4933 1,62; 4,0560 0,49; 4,0382 1,52; 4,0204 1,54; 4,0026 0,53; 3,3339 57,31; 2,6772 0,46; 2,6726 0,64; 2,6681 0,47; 2,5259 1,71; 2,5212 2,66; 2,5124 34,61; 2,5081 69,79; 2,5035 91,83; 2,4990 67,16; 2,4946 32,66; 2,3349 0,46; 2,3302 0,64; 2,3258 0,46; 1,9902 6,71; 1,6025 2,99; 1,5884 7,24; 1,5815 7,87; 1,5685 3,32; 1,3369 1,40; 1,2988 0,48; 1,2884 3,60; 1,2749 7,36; 1,2683 7,93; 1,2537 3,28; 1,2501 2,65; 1,2309 7,22; 1,2135 15,36; 1,1957 7,13; 1,1754 3,85; 1,1576 1,89; 0,0080 1,27; -0,0002 39,65; -0,0085 1,28 |
| Verbindung Nr. 316 [DMSO] 9,7524 2,29; 9,7282 2,38; 9,3010 5,43; 8,1132 4,28; 7,8490 16,00; 7,7870 0,46; 7,7669 10,52; 7,3860 6,28; 6,2483 0,88; 6,2264 1,33; 6,2039 0,96; 4,5448 0,93; 4,5276 2,82; 4,5099 2,83; 4,4925 0,94; 4,0570 0,67; 4,0392 2,05; 4,0214 2,08; 4,0036 0,70; 3,3370 23,15; 2,5271 0,61; 2,5223 0,98; 2,5137 11,94; 2,5093 23,97; 2,5048 31,51; 2,5003 23,22; 2,4959 11,43; 1,9912 9,04; 1,6043 1,70; 1,5901 4,10; 1,5833 4,47; 1,5703 1,89; 1,3960 5,72; 1,3378 0,94; 1,2908 2,03; 1,2773 4,17; 1,2706 4,47; 1,2561 1,77; 1,2498 1,43; 1,2315 4,16; 1,2140 8,61; 1,1944 4,89; 1,1763 4,99; 1,1584 2,46; 0,0080 0,43; -0,0002 12,94; -0,0085 0,47 |
| Verbindung Nr. 317 [DMSO] 20,0019 0,38; 10,4337 1,44; 10,4089 1,62; 8,5558 1,24; 8,5447 1,33; 8,4884 1,21; 8,4770 1,24; 8,3189 3,23; 8,1255 0,51; 8,1045 0,51; 8,0334 7,87; 8,0200 4,32; 7,9597 0,53; 7,9323 0,63; 7,9079 4,56; 7,9008 4,42; 7,8892 0,67; 7,8289 0,58; 7,8078 0,73; 7,7976 0,49; 7,7835 4,11; 7,7288 2,66; 7,7242 4,89; 7,7195 2,65; 7,6761 0,41; 7,6564 0,61; 7,6378 0,48; 7,5533 0,47; 7,5262 0,41; 7,5057 0,58; 7,4394 0,36; 7,4241 0,51; 7,4044 0,45; 7,3900 0,44; 6,5403 0,36; 6,2814 0,87; 6,2592 1,27; 6,2378 0,92; 4,7859 0,46; 4,7714 0,45; 4,7675 0,53; 4,7533 1,22; 4,7351 1,21; 4,7170 0,38; 4,6385 0,92; 4,6213 2,64; 4,6038 2,70; 4,0460 0,47; 4,0284 0,55; 3,5720 0,32; 3,3307 448,75; 3,2332 3,89; 3,0191 3,60; 2,8908 1,91; 2,8592 0,85; 2,8495 1,23; 2,8401 1,25; 2,8316 0,88; 2,8221 0,63; 2,7316 1,38; 2,6802 2,37; 2,6758 5,09; 2,6712 7,06; 2,6667 5,08; 2,5415 1,73; 2,5246 17,74; 2,5199 27,38; 2,5112 362,24; 2,5067 738,28; 2,5022 973,29; 2,4976 705,38; 2,4931 337,44; 2,3335 5,52; 2,3289 7,29; 2,3244 5,38; 2,2759 0,70; 2,2573 0,85; 2,2390 0,62; 1,6210 0,74; 1,6026 1,17; 1,5844 0,69; 1,5319 1,61; 1,5137 3,06; 1,4954 1,76; 1,4084 0,81; 1,4017 0,81; 1,3840 1,22; 1,3664 1,09; 1,3506 2,92; 1,3330 6,77; 1,3145 6,50; 1,2976 5,59; 1,2584 6,70; 1,2343 16,00; 1,1857 1,63; 1,1679 1,91; 1,1504 1,38; 1,1255 1,07; 0,8535 3,65; 0,8360 2,42; 0,7410 0,86; 0,7287 1,92; 0,7234 2,51; 0,7122 3,39; 0,7050 2,19; 0,6946 2,20; 0,6839 0,78; 0,5798 0,84; 0,5693 2,22; 0,5547 3,30; 0,5406 1,91; 0,5273 0,84; 0,1460 1,75; 0,0080 14,09; -0,0002 409,80; -0,0085 12,46; -0,1496 1,69 |
| Verbindung Nr. 318 [DMSO] 9,8544 4,12; 9,8302 4,28; 9,7781 4,66; 9,7581 4,72; 8,3185 3,23; 8,2056 6,31; 8,1508 0,36; 8,0700 3,39; 8,0506 3,69; 7,8975 15,98; 7,8601 11,43; 7,8464 1,00; 7,8347 3,09; 7,8146 4,65; 7,7851 0,86; 7,7381 3,28; 7,7189 5,00; 7,6992 2,14; 7,6232 0,32; 7,5946 6,91; 7,5763 9,89; 7,5132 3,33; 7,5099 4,80; 7,5053 2,04; 7,4924 10,59; 7,4884 4,79; 7,4735 6,54; 7,4516 4,62; 7,4397 1,72; 7,4337 5,15; 7,4267 1,39; 7,4154 1,54; 7,4118 1,19; 7,4017 11,52; 7,3767 0,33; 6,4289 5,72; 6,4089 5,73; 6,3441 0,58; 6,3233 1,80; 6,3014 2,52; 6,2784 1,78; 6,2572 0,48; 4,5435 1,95; 4,5265 5,38; 4,5086 5,23; 4,4914 1,76; 4,4705 0,36; 4,2313 0,34; 3,4006 0,38; 3,3307 830,52; 2,8185 0,34; 2,6758 5,42; 2,6712 7,32; 2,6667 5,41; 2,6624 2,61; 2,6330 0,46; 2,5415 4,03; 2,5246 21,80; 2,5198 33,33; 2,5111 370,43; 2,5067 740,94; 2,5022 970,57; 2,4976 705,98; 2,4932 339,25; 2,3380 2,38; 2,3335 5,03; 2,3289 6,85; 2,3244 4,88; 2,3200 2,26; 1,9894 0,57; 1,2587 0,54; 1,2262 7,63; 1,2088 16,00; 1,1912 7,37; 1,1748 0,66; 1,1357 0,40; 1,1190 0,82; 1,1018 0,40; 1,0883 0,38; 1,0673 0,68: 1,0485 0,39; 0,1459 1,32; 0,0080 11,06; -0,0002 331,07; -0,0085 11,56; -0,1496 1,39 |
| Verbindung Nr. 319 [DMSO] 9,7261 2,55; 9,7019 2,64; 9,1876 5,70; 8,2147 3,58; 8,0727 1,90; 8,0532 2,12; 7,8321 1,72; 7,8125 2,50; 7,7387 1,93; 7,7191 2,97; 7,6996 1,25; 7,6193 8,92; 7,5118 5,74; 7,3353 6,83; 6,3225 0,97; 6,3001 1,46; 6,2777 1,05; 4,5709 1,01; 4,5536 3,10; 4,5359 3,11; 4,5185 1,01; 4,0559 0,51; 4,0381 1,59; 4,0203 1,61; 4,0025 0,54; 3,4161 0,50; 3,3990 1,34; 3,3819 1,91; 3,3649 1,46; 3,3472 0,68; 3,3324 40,94; 2,6768 0,35; 2,6722 0,48; 2,6675 0,34; 2,5256 1,24; 2,5208 1,98; 2,5121 26,10; 2,5076 52,84; 2,5031 69,96; 2,4985 51,38; 2,4940 25,07; 2,3344 0,37; 2,3298 0,49; 2,3253 0,36; 1,9898 7,14; 1,5799 1,83; 1,5657 4,44; 1,5590 4,91; 1,5461 2,07; 1,3533 0,65; 1,2821 11,44; 1,2717 12,69; 1,2652 16,00; 1,2549 12,09; 1,2448 2,82; 1,2307 4,88; 1,2133 9,65; 1,1955 4,55; 1,1934 4,50; 1,1753 4,11; 1,1575 2,02; 0,0080 0,87; -0,0002 27,03; -0,0085 0,95 |
| Verbindung Nr. 321 [DMSO] 9,5524 1,51; 9,5281 1,57; 8,4084 1,48; 8,3878 1,51; 8,3200 0,33; 7,9558 0,59; 7,9344 1,06; 7,9072 0,56; 7,6742 4,44; 7,6564 0,52; 7,6488 0,76; 7,6342 0,93; 7,6279 1,25; 7,5940 0.79; 7,5696 1,30; 7,5471 1,26; 7,5254 0,44; 7,4371 3,14; 7,3352 3,72; 6,2074 0,62; 6,1855 0,91; 6,1630 0,64; 4,5273 0,64; 4,5096 1,74; 4,4911 1,74; 4,4737 0,65; 4,3756 0,70; 4,3585 2,94; 4,3481 2,46; 4,3387 0,73; 3,7921 0,50; 3,7815 0,51; 3,7768 0,68; 3,7663 0,64; 3,7616 0,52; 3,7511 0,49; 3,3337 87,26; 3,0339 0,60; 3,0158 0,62; 3,0023 1,35; 2,9840 1,27; 2,9588 1,31; 2,9417 1,38; 2,9273 0,68; 2,9100 0,58; 2,6762 0,83; 2,6718 1,11; 2,6673 0,88; 2,6446 16,00; 2,5822 0,49; 2,5607 0,42; 2,5402 1,16; 2,5249 3,44; 2,5071 116,69; 2,5027 151,65; 2,4984 112,68; 2,4698 10,91; 2,3338 0,75; 2,3294 1,04; 2,3252 0,77; 1,3048 5,13; 1,2881 5,08; 1,2395 2,77; 1,2223 5,74; 1,2046 2,67; 1,0444 14,60; 1,0291 14,52; 0,1463 0,42; 0,0080 3,58; -0,0002 93,99; -0,0085 4,69; -0,1491 0,42 |
| Verbindung Nr. 322 [DMSO] 8,4003 0,39; 8,3191 0,47; 8,1238 0,32; 8,0590 0,33; 7,9324 0,34; 7,9089 0,39; 7,8280 0,32; 7,8078 0,41; 7,7821 0,38; 7,6970 0,34; 7,3902 0,37; 7,3351 0,69; 7,3310 0,69; 7,1819 1,39; 7,1766 0,81; 3,8408 1,01; 3,3330 151,86; 3,3146 0,35; 2,6805 0,42; 2,6760 0,86; 2,6716 1,19; 2,6671 0,89; 2,6625 0,45; 2,5248 3,66; 2,5200 5,39; 2,5114 59,74; 2,5070 120,85; 2,5025 159,67; 2,4979 116,98; 2,4935 57,70; 2,3383 0,34; 2,3338 0,77; 2,3292 1,07; 2,3247 0,78; 2,3203 0,37; 1,3506 1,02; 1,3356 12,64; 1,2979 5,37; 1,2799 0,43; 1,2584 7,89; 1,2491 16,00; 1,2345 4,67; 1,1868 0,49; 0,8535 0,61; 0,5544 0,44; 0,5490 0,49; 0,5393 0,49; 0,3864 0,37; 0,3708 0,34; 0,3323 0,34; 0,3231 0,36; 0,0080 0,32; -0,0002 9,87; -0,0085 0,39 |
| Verbindung Nr. 323 [DMSO] 10,3305 3,35; 10,3169 3,02; 10,3060 3,55; 10,2925 2,69; 8,3192 1,55; 8,0683 8,71; 7,9544 11,87; 7,9130 8,79; 7,7838 3,16; 7,7643 7,07; 7,6334 4,13; 7,6139 5,61; 7,5344 2,16; 7,5188 2,86; 7,5141 4,02; 7,4989 3,91; 7,4946 2,93; 7,4791 1,92; 7,3136 2,09; 7,3082 2,24; 7,2923 3,69; 7,2870 3,83; 7,2708 1,78; 7,2655 1,77; 6,2099 0,61; 6,1891 2,22; 6,1671 3,31; 6,1448 2,35; 6,1229 0,67; 4,6398 1,61; 4,6225 5,26; 4,6049 6,87; 4,5879 4,01; 3,7663 0,65; 3,7491 0,89; 3,7328 0,97; 3,7149 0,87; 3,7069 0,61; 3,6882 1,16; 3,6708 1,48; 3,6541 1,69; 3,6367 1,40; 3,6194 0,49; 3,3832 0,75; 3,3656 2,02; 3,3327 315,73; 3,2721 0,89; 3,2548 1,02; 3,2375 0,93; 3,2201 0,73; 3,2046 0,44; 3,1894 0,78; 3,1718 1,34; 3,1533 1,81; 3,1399 1,96; 3,1228 1,70; 3,1057 0,88; 3,0271 1,25; 3,0091 1,93; 2,9917 1,90; 2,9768 1,42; 2,9597 0,87; 2,6763 2,61; 2,6718 3,57; 2,6673 2,69; 2,5422 33,92; 2,5249 12,51; 2,5114 186,54; 2,5072 368,37; 2,5027 489,26; 2,4982 370,51; 2,4940 191,66; 2,3339 2,55; 2,3295 3,49; 2,3250 2,61; 1,3565 3,29; 1,3387 7,19; 1,3304 7,00; 1,3128 11,58; 1,2949 5,26; 1,2342 1,25; 1,2142 6,97; 1,1967 16,00; 1,1831 12,70; 1,1657 5,15; 1,1501 0,72; 1,1327 0,36; 1,0257 2,97; 1,0086 8,66; 0,9931 10,38; 0,9759 6,76; 0,9582 1,77; 0,0079 0,63; -0,0002 18,08; -0,0083 0,93 |
| Verbindung Nr. 324 [DMSO] 11,7911 0,42; 11,6226 0,35; 9,9330 5,41; 9,9169 5,59; 9,4987 0,36; 9,4824 0,37; 9,4639 0,37; 9,3343 10,85; 8,3016 9,95; 8,2426 0,39; 8,1962 7,30; 8,1844 0,85; 8,0600 4,10; 8,0469 4,47; 7,8646 1,59; 7,8620 1,37; 7,8497 10,85; 7,8470 13,46; 7,8445 13,30; 7,8365 4,13; 7,8297 2,66; 7,8233 4,93; 7,7379 3,67; 7,7248 6,23; 7,7118 2,81; 7,5057 5,24; 7,4908 6,10; 7,4853 16,00; 7,4791 6,23; 7,4642 5,43; 7,4115 0,32; 7,3967 0,39; 7,3922 0,37; 6,8597 0,86; 6,3060 0,54; 6,2920 1,78; 6,2774 2,67; 6,2625 1,96; 6,2477 0,64; 5,4132 6,28; 5,4114 6,60; 5,3866 6,40; 5,3849 6,34; 5,2388 6,08; 5,2371 6,54; 5,2239 6,66; 5,2224 5,92; 4,0360 0,35; 4,0241 0,36; 3,3249 329,04; 2,6211 0,43; 2,6181 0,90; 2,6151 1,25; 2,6120 0,92; 2,6090 0,44; 2,5241 2,40; 2,5210 2,93; 2,5179 2,94; 2,5091 68,04; 2,5061 147,03; 2,5030 203,36; 2,5000 146,37; 2,4970 67,05; 2,3930 0,39; 2,3900 0,86; 2,3869 1,20; 2,3839 0,85; 2,3809 0,38; 1,9896 1,56; 1,9103 0,35; 1,6147 0,69; 1,6093 0,48; 1,6050 0,72; 1,6006 0,90; 1,5899 4,41; 1,5806 9,68; 1,5761 10,87; 1,5673 4,42; 1,5406 0,41; 1,3971 2,45; 1,3646 0,59; 1,3554 0,81; 1,3509 0,92; 1,3366 3,77; 1,3220 4,70; 1,3129 9,79; 1,3085 10,71; 1,2991 6,65; 1,2734 0,36; 1,2589 4,05; 1,2498 4,33; 1,2346 1,39; 1,2257 0,37; 1,1873 0,55; 1,1754 0,95; 1,1636 0,51; 0,0053 0,94; -0,0001 29,00; -0,0057 0,91 |
| Verbindung Nr. 325 [CD3CN] 7,9665 0,74; 7,8926 0,39; 7,8753 0,73; 7,8533 1,87; 7,7888 0,39; 7,7688 0,56; 7,6950 0,46; 7,6753 0,68: 7,6426 1,44; 7,2503 1,61; 7,2041 0,51; 6,1353 0,41; 4,5139 0,35; 4,4961 1,11; 4,4783 1,13; 4,4606 0,37; 3,9111 1,77; 3,8872 2,56; 3,7840 2,49; 3,7601 1,78; 3,4253 16,00; 2,1629 194,88; 2,1614 222,97; 2,1199 0,52; 2,1137 0,58; 2,1075 0,72; 2,1013 0,54; 1,9644 5,02; 1,9583 4,33; 1,9524 35,91; 1,9463 66,77; 1,9401 92,87; 1,9339 64,22; 1,9277 33.22; 1,9149 0,54; 1,7747 0,39; 1,7685 0,55; 1,7624 0,38; 1,3089 1,49; 1,2911 3,15; 1,2733 1,51; -0,0002 10,36 |
| Verbindung Nr. 326 [DMSO] 9,8219 1,59; 9,7977 1,65; 9,3268 3,91; 8,1999 2,28; 8,0626 1,22; 8,0429 1,36; 7,9311 4,44; 7,8860 6,43; 7,8273 1,11; 7,8076 1,59; 7,7315 1,20; 7,7120 1,86; 7,6924 0,77; 7,5722 4,40; 6,3012 0,62; 6,2790 0,93; 6,2563 0,68; 5,3453 5,56; 3,5942 16,00; 3,3290 61,17; 2,8906 0,33; 2,6892 0,41; 2,6757 0,34; 2,6712 0,46; 2,6667 0,34; 2,5245 1,48; 2,5112 26,22; 2,5068 52,24; 2,5022 68,57; 2,4976 49,87; 2,4932 24,69; 2,3334 0,35; 2,3289 0,46; 2,3244 0,34; 2,0756 6,18; 1,6051 1,23; 1,5909 2,94; 1,5841 3,21; 1,5712 1,37; 1,2913 1,43; 1,2778 2,95: 1,2712 3,17; 1,2567 1,15; 0,0080 0,70; -0,0002 18,53; -0,0085 0,76 |
| Verbindung Nr. 327 [DMSO] 9,9448 0,75; 9,6959 0,52; 9,6708 0,81; 9,6606 4,11; 9,6361 4,24; 8,4262 0,74; 8,4122 4,85; 8,4011 4,74; 8,3210 0,44; 8,2015 0,68; 8,1289 1,92; 8,1210 11,60; 8,1052 11,60; 8,0472 0,50; 8,0311 0,49; 8,0163 0,65; 7,7976 11,32; 7,7723 16,00; 7,7193 0,43; 7,6502 0,64; 7,6288 0,72; 7,4893 1,41; 7,4137 0,49; 7,3916 0,82; 7,3761 11,23; 7,3356 1,22; 7,3142 1,02; 7,2736 0,42; 7,2676 0,37; 7,2522 0,35; 6,8880 1,63; 6,7206 1,13; 6,3185 0,57; 6,2969 1,81; 6,2752 2,49; 6,2526 1,74; 6,2309 0,52; 4,5628 0,76; 4,5385 1,94; 4,5210 5,41; 4,5034 5,40; 4,4858 1,78; 3,9913 0,73; 3,5733 0,47; 3,3373 92,66; 3,0318 1,00; 2,8681 0,60; 2,8580 1,43; 2,8485 1,92; 2,8398 2,97; 2,8295 2,84; 2,8213 1,83; 2,8114 1,35; 2,8012 0,52; 2,7725 0,84; 2,7189 0,52; 2,6990 0,34; 2,6821 0,38; 2,6776 0,73; 2,6731 1,00; 2,6685 0,73; 2,5685 0,46; 2,5491 0,76; 2,5434 0,76; 2,5265 2,95; 2,5217 4,32; 2,5129 51,41; 2,5085 104,97; 2,5040 139,87; 2,4994 104,73; 2,4950 52,90; 2,3400 0,40; 2,3352 0,75; 2,3309 1,02; 2,3262 0,76; 2,1806 0,42; 1,3538 1,97; 1,3360 15,62; 1,2989 8,90; 1,2810 1,02; 1,2588 13,81; 1,2494 5,13; 1,2415 5,38; 1,2332 11,93; 1,2286 10,75; 1,2107 15,95; 1,1930 7,32; 1,1786 0,97; 1,1647 0,68; 1,1490 0,38; 1,0915 0,78; 1,0571 0,38; 0,8695 0,64; 0,8658 0,59; 0,8531 1,51; 0,8355 0,74; 0,7210 2,08; 0,7081 5,75; 0,7030 7,15; 0,6909 6,77; 0,6849 5,51; 0,6737 2,27; 0,5570 3,26; 0,5465 7,41; 0,5404 6,56; 0,5367 5,94; 0,5307 5,60; 0,5187 1,71; 0,0080 0,78; -0,0002 24,43; -0,0084 0,99 |
| Verbindung Nr. 328 [DMSO] 9,9556 1,11; 9,8212 0,42; 9,8051 4,00; 9,7812 4,19; 8,5288 0,32; 8,4167 4,66; 8,4056 4,63; 8,2949 2,49; 8,2764 4,60; 8,2579 2,54; 8,2040 1,03; 8,0618 0,33; 8,0169 0,98; 7,8078 11,56; 7,7837 15,80; 7,6593 0,38; 7,6375 0,44; 7,5099 0,85; 7,4172 0,62; 7,4035 11,68; 7,3434 0,62; 7,3220 0,56; 6,3929 0,63; 6,3729 1,92; 6,3516 2,64; 6,3294 1,90; 6,3085 0,57; 5,7611 6,64; 4,5635 0,45; 4,5389 1,95; 4,5214 5,58; 4,5037 5,59; 4,4862 1,91; 4,3621 0,41; 4,3435 0,42; 4,0565 0,44; 4,0387 1,38; 4,0208 1,40; 4,0031 0,47; 3,3369 57,37; 3,0325 0,71; 2,8683 0,56; 2,8583 1,41; 2,8489 1,94; 2,8401 2,98; 2,8299 2,92; 2,8214 1,88; 2,8118 1,38; 2,8015 0,51; 2,7739 0,54; 2,6779 0,47; 2,6734 0,66; 2,6689 0,50; 2,5267 1,80; 2,5131 36,45; 2,5088 73,85; 2,5044 98,26; 2,4999 74,32; 2,4958 38,99; 2,3354 0,53; 2,3311 0,76; 2,3268 0,55; 2,0784 2,48; 1,9910 6,11; 1,2903 0,58; 1,2726 1,28; 1,2612 1,00; 1,2529 1,09; 1,2439 1,87; 1,2304 7,68; 1,2131 16,00; 1,1949 8,13; 1,1761 3,45; 1,1583 1,68; 0,7217 2,28; 0,7088 5,89; 0,7037 7,49; 0,6916 6,90; 0,6858 5,75; 0,6746 2,35; 0,5571 3,17; 0,5465 7,60; 0,5403 6,75; 0,5369 6,40; 0,5308 5,92; 0,5188 1,83; 0,0080 0,93; -0,0002 27,97; -0,0081 1,41 |
| Verbindung Nr. 329 [DMSO] 9,6827 4,06; 9,6583 4,18; 8,4059 4,57; 8,3948 4,62; 8,3193 0,60; 7,9532 1,39; 7,9493 1,46; 7,9287 2,83; 7,9259 2,79; 7,9044 1,39; 7,9002 1,38; 7,7917 11,17; 7,7557 15,91; 7,6459 1,96; 7,6245 3,49; 7,5939 2,16; 7,5708 4,65; 7,5481 3,53; 7,5255 1,16; 7,3657 10,86; 7,3362 0,36; 7,3317 0,34; 7,1824 0,63; 7,1773 0,36; 6,2306 0,41; 6,2089 1,54; 6,1870 2,29; 6,1644 1,65; 6,1422 0,47; 5,7597 0,78; 4,5401 1,66; 4,5224 4,69; 4,5041 4,60; 4,4860 1,62; 3,3328 194,54; 3,3119 0,42; 2,8654 0,45; 2,8554 1,20; 2,8458 1,58; 2,8373 2,59; 2,8269 2,62; 2,8189 1,59; 2,8087 1,26; 2,7987 0,50; 2,6805 0,50; 2,6763 1,07; 2,6717 1,46; 2,6672 1,07; 2,6628 0,51; 2,5419 0,67; 2,5252 4,14; 2,5204 6,30; 2,5117 76,26; 2,5072 154,81; 2,5027 203,53; 2,4981 147,60; 2,4936 70,27; 2,3384 0.51; 2,3340 1,04; 2,3294 1,47; 2,3248 1,05; 2,3203 0,53; 1,3974 0,46; 1,3360 6,09; 1,2985 0,98; 1,2822 0,39; 1,2586 1,66; 1,2493 8,11; 1,2343 2,10; 1,2220 7,36; 1,2046 16,00; 1,1869 7,20; 0,7189 1,85; 0,7061 4,74; 0,7009 6,88; 0,6889 6,19; 0,6827 5,31; 0,6715 2,22; 0,5553 2,31; 0,5448 6,66; 0,5387 5,93; 0,5347 5,58; 0,5292 5,39; 0,5170 1,70; 0,0080 2,10; -0,0002 67,40; -0,0085 2,11 |
| Verbindung Nr. 330 [DMSO] 9,7163 2,35; 9,6922 2,46; 9,1309 5,11; 9,0990 0,68; 8,3053 1,35; 8,2867 2,46; 8,2682 1,36; 7,7399 7,36; 7,7160 0,96; 7,4800 4,74; 7,4004 0,69; 7,3680 6,42; 6,3796 0,93; 6,3585 1,38; 6,3363 1,09; 6,3153 0,42; 5,7611 0,49; 4,5219 0,99; 4,5046 2,98; 4,4868 3,00; 4,4693 1,02; 4,0565 0,52; 4,0387 1,60; 4,0209 1,62; 4,0031 0,55; 3,3373 28,73; 2,6904 0,67; 2,6735 0,33; 2,5268 1,05; 2,5132 18,11; 2,5089 36,45; 2,5044 48,54; 2,4998 36,71; 2,4955 18,84; 2,4762 3,44; 2,4675 16,00; 2,3311 0,34; 1,9909 7,16; 1,5819 1,72; 1,5676 4,43; 1,5609 4,89; 1,5481 2,07; 1,3145 0,34; 1,2941 2,67; 1,2807 4,67; 1,2740 4,83; 1,2595 2,03; 1,2482 4,23; 1,2308 9,13; 1,2132 4,09; 1,1939 2,03; 1,1761 3,84; 1,1583 2,19; 1,1467 0,81; 1,1381 1,22; 1,1279 1,42; 1,1203 0,67; 1,1090 0,64; 0,0080 0,76; -0,0002 22,55; -0,0084 0,98 |
| Verbindung Nr. 331 [DMSO] 9,6945 1,79; 9,6701 1,86; 9,1242 3,79; 8,1264 2,88; 8,0751 1,18; 8,0520 1,18; 7,8289 1,24; 7,8076 1,25; 7,7369 5,67; 7,4724 3,59; 7,3469 4,82; 6,4119 0,68; 6,3898 1,01; 6,3672 0,74; 4,5224 0,75; 4,5050 2,26; 4,4873 2,27; 4,4697 0,75; 4,0566 1,19; 4,0388 3,65; 4,0210 3,69; 4,0032 1,23; 3,3375 19,95; 2,5270 0,74; 2,5223 1,14; 2,5135 13,66; 2,5091 27,73; 2,5046 36,84; 2,5000 27,61; 2,4956 13,91; 2,4710 11,98; 1,9909 16,00; 1,5804 1,24; 1,5662 3,09; 1,5594 3,38; 1,5466 1,43; 1,2956 1,64; 1,2822 3,37; 1,2756 3,62; 1,2611 1,38; 1,2413 3,18; 1,2239 6,91; 1,2063 3,10; 1,1938 4,60; 1,1760 8,89; 1,1582 4,35; 0,0080 0,62; -0,0002 18,74; -0,0085 0,73 |
| Verbindung Nr. 332 [CD3CN] 10,5406 (1,19); 10,5161 (1,20); 8,4668 (1,47); 8,4555 (1,97); 8,4347 (0,95); 8,2445 (0,56); 8,2311 (0,69); 8,1627 (3,82); 7,7555 (4,17); 7,6680 (0,80); 7,6434 (1,04); 7,6195 (0,70); 6,3733 (0,54); 6,3511 (0,77); 6,3287 (0,54); 4,6394 (0,59); 4,6223 (1,71); 4,6045 (1,72); 4,5871 (0,57); 4,0562 (1,25); 4,0384 (3,75); 4,0206 (3,80); 4,0028 (1,28); 3,3353 (23,86); 2,8576 (0,37); 2,8486 (0,56); 2,8394 (0,83); 2,8295 (0,84); 2,8211 (0,58); 2,8115 (0,41); 2,6732 (0,32); 2,5083 (35,67); 2,5040 (46,25); 2,4997 (36,06); 1,9906 (16,00); 1,3430 (0,43); 1,3362 (0,64); 1,3260 (2,42); 1,3085 (4,74); 1,2993 (1,78); 1,2908 (2,36); 1,2590 (1,45); 1,2497 (0,76); 1,2334 (1,53); 1,1934 (4,37); 1,1756 (8,52); 1,1578 (4,37); 0,7301 (0,54); 0,7165 (1,53); 0,7122 (2,09); 0,7000 (1,89); 0,6943 (1,72); 0,6831 (0,69); 0,5800 (0,72); 0,5693 (2,10); 0,5630 (2,05); 0,5542 (1,75); 0,5416 (0,53); -0,0002 (1,92) |

| |
|---|
| a) Die Bestimmung des M⁺ mit der LC-MS im sauren Bereich erfolgt bei pH 2,7 Acetonitril (enthält 0,1% Ameisensäure) und Wasser als Eluenten; linearer Gradient von 10% Acetonitril bis 95% Acetonitril Gerät: Agilent 1100 LC-System, Agilent MSD System, HTS PAL. Die Bestimmung der in den vorangegangenen Tabellen und Herstellungsbeispielen angegebenen logP-Werte erfolgte gemäß EEC Directive 79/831 Annex V.A8 durch HPLC (High Performance Liquid Chromatography) an einer Phasenumkehrsäule (C18). Temperatur 43 °C. Die Eichung erfolgt mit unverzweigten Alkan-2-onen (mit 3 bis 16 Kohlenstoffatomen), deren logP Werte bekannt sind. b) Die Bestimmung der ¹H-NMR-Daten erfolgt mit einem Bruker Avance 400 ausgestattet mit einem Durchflussprobenkopf (60 µl Volumen), mit Tetramethylsilan als Referenz (0.0) und den Lösungsmitteln CD₃CN, CDCl₃, D₆-DMSO. Die NMR-Daten ausgewählter Beispiele werden entweder in klassischer Form (δ-Werte, Multiplettaufspaltung, Anzahl der H-Atome) oder als NMR-Peak-Listen aufgeführt. NMR-Peak-Listenverfahren: Wenn die ¹H-NMR-Daten ausgewählter Beispiele in Form von ¹H-NMR-Peaklisten notiert werden, wird zu jedem Signalpeak erst der δ-Wert in ppm und dann die Signalintensität durch ein Leerzeichen getrennt aufgeführt. Die δ-Wert - Signalintensitäts- Zahlenpaare von verschiedenen Signalpeaks werden durch Semikolons voneinander getrennt aufgelistet. Die Peakliste eines Beispieles hat daher die Form: δ₁ Intensität₁;δ₂ Intensität₂;........;δᵢ Intensitätᵢ;......; δₙ Intensitätₙ Das Lösungsmittel, in welchem das NMR-Spektrum aufgenommen wurde, wird in eckigen Klammern hinter der Nummer des Beispieles und vor der NMR-Peakliste bzw. der klassischen NMR-Interpretationsliste aufgeführt. |

### Anwendungsbeispiele

Die folgenden Beispiele zeigen die insektizide und akarizide Wirkung der erfindungsgemäßen Verbindungen. Dabei beziehen sich die genannten erfindungsgemäßen Verbindungen auf die in der Tabelle 1 mit den entsprechenden Referenzzeichen (Nr.) aufgeführten Verbindungen:

### Amblyomma hebaraeum -Test (AMBYHE)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Zeckennymphen (*Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank gelagert.

Nach 42 Tagen wird die Abtötung in % bestimmt. Dabei bedeutet 100 %, dass alle Zecken abgetötet wurden; 0 % bedeutet, dass keine Zecken abgetötet wurden.

Bei diesem Test zeigen z. B. die folgenden Verbindungen der Herstellungsbeispiele eine Wirkung von 100% bei einer Aufwandmenge von 100ppm: 14

### Boophilus microplus - Test (DIP)

Testtiere: adulte gesogene Weibchen von *Boophilus microplus* Stamm Parkhurst - (SP- resistent)

| | |
|---|---|
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 Zecken werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden in Glasröhrchen bis zum Larvenschlupf im Klimaschrank aufbewahrt. Eine Wirkung von 100 % bedeutet, dass keine Zecke fertile Eier gelegt hat.

Bei diesem Test zeigen z.B. die folgenden Verbindungen der Herstellungsbeispiele eine Wirkung von 100% bei einer Aufwandmenge von 100ppm: 7

Bei diesem Test zeigen z.B. die folgenden Verbindungen der Herstellungsbeispiele eine Wirkung von 80% bei einer Aufwandmenge von 100ppm: 18

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach 7 Tagen wird die Wirkung in % bestimmt. Die Wirkungskontrolle erfolgt auf Ablage fertiler Eier. Dabei bedeutet 100%, dass keine Zecke fertile Eier gelegt hat.

Bei diesem Test zeigen z.B. die folgenden Verbindungen der Herstellungsbeispiele eine Wirkung von 100% bei einer Aufwandmenge von 20µg/Tier: 2, 3, 6, 7, 8, 10, 11, 12, 13, 14, 15, 16, 18, 19, 20, 22, 27, 28, 32, 36, 37, 38, 39, 40, 41, 42, 48, 49, 50, 52, 54, 57, 61, 62, 63, 97, 188, 190, 191, 192, 195, 275, 276, 282, 283, 285, 288

### Ctenocephalides felis oral (CTECFE)

| | |
|---|---|
| Lösungsmittel: | 1 Gewichtsteil Dimethylsulfoxid |

Zwecks Herstellung einer zweckmäßigen Wirkstoffzubereitung vermischt man 10 mg Wirkstoff mit 0,5 ml Dimethylsulfoxid. Ein Teil des Konzentrats wird mit citriertem Rinderblut verdünnt und die gewünschte Konzentration hergestellt.

Ca. 20 nüchterne adulte Flöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass kein Floh abgetötet wurde.

Bei diesem Test zeigen z.B. die folgenden Verbindungen der Herstellungsbeispiele eine Wirkung von 100% bei einer Aufwandmenge von 100ppm: 3, 6, 7, 12, 13, 14, 15, 16, 18, 19, 20, 22, 32, 36, 37, 38, 39, 40, 41, 42, 48, 50, 52, 54, 57, 61, 62, 63, 188, 190, 191, 192, 195, 275, 276, 282, 283, 285,288

Bei diesem Test zeigen z.B. die folgenden Verbindungen der Herstellungsbeispiele eine Wirkung von 98% bei einer Aufwandmenge von 100ppm: 49

Bei diesem Test zeigen z.B. die folgenden Verbindungen der Herstellungsbeispiele eine Wirkung von 95% bei einer Aufwandmenge von 100ppm: 2, 8, 10, 28, 97

Bei diesem Test zeigen z.B. die folgenden Verbindungen der Herstellungsbeispiele eine Wirkung von 90% bei einer Aufwandmenge von 100ppm: 27

Bei diesem Test zeigen z.B. die folgenden Verbindungen der Herstellungsbeispiele eine Wirkung von 80% bei einer Aufwandmenge von 100ppm: 11

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit ca 20 *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: 2, 3, 6, 7, 8, 10, 11, 12, 13, 14, 15, 16, 18, 19, 20, 22, 27, 28, 32, 37, 38, 39, 40, 41, 42, 48, 49, 50, 52, 54, 57, 61, 62, 63, 97, 188, 190, 191, 192, 195, 275, 276, 282, 283, 285, 288

Bei diesem Test zeigen z.B. die folgenden Verbindungen der Herstellungsbeispiele eine Wirkung von 95% bei einer Aufwandmenge von 100ppm: 36

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domesticn-Adulten* besetzt.

Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele eine Wirkung von 100% bei einer Aufwandmenge von 100ppm: 3, 6, 7, 10, 12, 13, 14, 15, 16, 18, 19, 20, 22, 27, 37, 38, 42, 49, 50, 52, 54, 57, 61, 63, 97, 188, 190, 192, 195, 275, 276, 288

Bei diesem Test zeigen z.B. die folgenden Verbindungen der Herstellungsbeispiele eine Wirkung von 90% bei einer Aufwandmenge von 100ppm: 191, 282, 283, 285

Bei diesem Test zeigen z.B. die folgenden Verbindungen der Herstellungsbeispiele eine Wirkung von 80% bei einer Aufwandmenge von 100ppm: 62

### Aulacophora femoralis - Sprühtest (AUACFE)

| | |
|---|---|
| Lösungsmittel: | 3 Gewichtsteile Dimethylformamid |
| Emulgator: | 1 Gewichtsteil Polyoxyethylen Alkylphenylether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei Bedarf werden zusätzlich Ammoniumsalze oder Ammoniumsalze und Penetrationsförderer in einer Konzentration von 1000ppm hinzugefügt.

Junge Gurkenpflanzen (*Cucumis sativus*) im Kotyledonen-Blattstadium werden mit einer Wirkstoffzubereitung der gewünschten Konzentration gespritzt. Nach Trocknung wird das behandelte Pflanzenmaterial in Testgefäße gegeben und jeweils mit 5 L2-Larven des Gurkenblattkäfers (*Aulacophora femoralis*) infiziert.

Nach 6 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 20ppm: 6, 7

### Myzus persicae - Sprühtest (MYZUPE - OP/Carb-resistent)

| | |
|---|---|
| Lösungsmittel: | 3 Gewichtsteile Dimethylformamid |
| Emulgator: | 1 Gewichtsteil Polyoxyethylen Alkylphenylether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei Bedarf werden zusätzlich Ammoniumsalze oder Ammoniumsalze und Penetrationsförderer in einer Konzentration von 1000ppm hinzugefügt.

Auberginenpflanzen *.*(*Solanum melongena)* im 2-Blatt-Stadium, die mit einer gemischten Population der Grünen Pfirsichblattlaus (*Myzus persicae*) infiziert sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 98% bei einer Aufwandmenge von 100ppm: 7

Bei diesem Test zeigen z. B. die folgenden Verbindungen der Herstellungsbeispiele Wirkung von 90% bei einer Aufwandmenge von 100ppm: 6

### Spodoptera litura - Test (PRODLI)

| | |
|---|---|
| Lösungsmittel: | 3 Gewichtsteile Dimethylformamid |
| Emulgator: | 1 Gewichtsteil Polyoxyethylen Alkylphenylether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Der Boden eines PET-Gefäßes (7.5cm Durchmesser, 4cm tief) wird mit 2.3 g eines pulverförmigen Kunstfuttergemisches bedeckt. Nun werden 5ml der Wirkstoffzubereitung hinzugegeben und gleichmäßig mit dem Kunstfutter vermischt. Nach Gelieren werden jeweils 5 L3-Larven des Baumwollwurms (*Spodoptera litura*) in je ein Gefäß gesetzt.

Nach 6 Tagen wird die Wirkung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele Wirkung von 100% bei einer Aufwandmenge von 20ppm: 6, 7

### Gemeine Spinnmilbe - Test (TETRUR)

| | |
|---|---|
| Lösungsmittel: | 3 Gewichtsteile Dimethylformamid |
| Emulgator: | 1 Gewichtsteil Polyoxyethylen Alkylphenylether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei Bedarf werden zusätzlich Ammoniumsalze oder Ammoniumsalze und Penetrationsförderer in einer Konzentration von 1000ppm hinzugefügt.

Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 100ppm: 6, 7

### Thrips palmi - Sprühtest (THRIPL)

| | |
|---|---|
| Lösungsmittel: | 3 Gewichtsteile Dimethylformamid |
| Emulgator: | 1 Gewichtsteil Polyoxyethylen Alkylphenylether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei Bedarf werden zusätzlich Ammoniumsalze oder Ammoniumsalze und Penetrationsförderer in einer Konzentration von 1000ppm hinzugefügt.

Junge Gurkenpflanzen (*Cucumis sativus*) werden mit einer Wirkstoffzubereitung der gewünschten Konzentration gespritzt. Nach Trocknung werden Filterpapierscheiben mit etwa 100 Trips-Eiern (*Thrips palmi*) auf die behandelten Pflanzen gegeben und zur Wahrung von 100% Luftfeuchtigkeit mit Gehäusen abgedeckt.

Nach 6 Tagen wird die Wirkung in % Fraßschaden bestimmt. Dabei bedeutet 100 %, dass es keine Fraßschäden gibt; 0 % bedeutet, dass kein Unterschied zur unbehandelten Kontrolle zu sehen ist.

Bei diesem Test zeigen z. B. die folgenden Verbindungen der Herstellungsbeispiele Wirkung von 98% bei einer Aufwandmenge von 20ppm: 7

Bei diesem Test zeigen z. B. die folgenden Verbindungen der Herstellungsbeispiele Wirkung von 90% bei einer Aufwandmenge von 20ppm: 6

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500g/ha : 289, 290, 312

Bei diesem Test zeigen z. B. die folgenden Verbindungen der Herstellungsbeispiele Wirkung von 90% bei einer Aufwandmenge von 500g/ha : 5, 37, 38, 281, 301, 313

Bei diesem Test zeigen z. B. die folgenden Verbindungen der Herstellungsbeispiele Wirkung von 100% bei einer Aufwandmenge von 100g/ha : 38, 54, 97, 281, 300, 312

Bei diesem Test zeigen z. B. die folgenden Verbindungen der Herstellungsbeispiele Wirkung von 90% bei einer Aufwandmenge von 100g/ha : 7, 12, 40, 183, 191, 192, 193, 209, 276, 291, 295, 303, 311,314,315,316

Bei diesem Test zeigen z. B. die folgenden Verbindungen der Herstellungsbeispiele Wirkung von 80% bei einer Aufwandmenge von 100g/ha : 101, 282

### Phaedon -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis)* werden mit einer Wirkstoffzubereitung der gewünschten Konzentration gespritzt und nach dem Abtrocknen mit Larven des Meerrettichblattkäfers *(Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: 1, 4, 5, 13, 14, 37, 38, 97, 274, 281, 300, 301, 303, 312, 313, 317

Bei diesem Test zeigen z. B. die folgenden Verbindungen der Herstellungsbeispiele Wirkung von 83% bei einer Aufwandmenge von 500g/ha: 309

Bei diesem Test zeigen z. B. die folgenden Verbindungen der Herstellungsbeispiele Wirkung von 100% bei einer Aufwandmenge von 100g/ha: 2, 3, 6, 7, 8, 9,10, 11, 12, 15,16, 17, 18, 19, 20, 21, 22, 23, 24, 25, 26, 27, 28, 29, 30, 31, 32, 33, 34, 36, 39, 40, 42, 43, 44, 45, 46, 47, 48, 49, 50, 51, 52, 53, 54, 57, 58, 60, 61, 62, 63, 64, 66, 91, 101, 183, 184, 185, 188, 189, 190, 191, 192, 193, 195, 196, 209, 262, 263, 264, 266, 267, 268, 269, 270, 271, 272, 273, 275, 276, 277, 278, 279, 282, 283, 284, 285, 286, 287, 288, 289, 290, 291, 292, 293, 294, 295, 296, 297, 298, 299, 302, 304, 305, 306, 307, 308, 310, 311, 314, 315, 316, 318, 319, 320, 322, 325, 326

### Spodoptera frugiperda -Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays)* werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500g/ha: 1, 4, 5, 13, 14, 37, 38, 97, 274, 281, 300, 301, 303, 309, 312, 313, 317

Bei diesem Test zeigen z. B. die folgenden Verbindungen der Herstellungsbeispiele Wirkung von 100% bei einer Aufwandmenge von 100g/ha: 2, 3, 6, 7, 8, 9, 12, 15, 16, 17, 18, 20, 24, 25, 27, 31, 36, 39, 40, 41, 42, 43, 44, 45, 48, 49, 50, 51, 52, 53, 54, 57, 58, 59, 60, 61, 62, 63, 64, 65, 66, 91, 101, 183, 184, 185, 188, 189, 190, 191, 192, 193, 195, 196, 209, 262, 263, 267, 268, 272, 273, 275, 276, 277, 278, 279, 282, 283, 284, 285, 287, 288, 289, 290, 291, 292, 293, 295, 296, 297, 298, 299, 302, 304, 305, 307, 308, 310, 311, 314, 315, 316, 319, 320, 322

Bei diesem Test zeigen z. B. die folgenden Verbindungen der Herstellungsbeispiele Wirkung von 83% bei einer Aufwandmenge von 100g/ha: 10, 22, 28, 29, 30, 34, 306

### Tetranychus - test, OP-resistent (TETRUR Spritzbehandlung)

| | | |
|---|---|---|
| Lösungsmittel: | 78,0 | GewichtsteileAceton |
| | 1,5 | Gewichtsteile Dimethylformamid |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500g/ha: 2, 3, 4, 5, 14, 28, 37, 38, 97, 274, 281, 294, 300, 303,304,317

Bei diesem Test zeigen z. B. die folgenden Verbindungen der Herstellungsbeispiele eine Wirkung von 90% bei einer Aufwandmenge von 500g/ha: 301, 313

Bei diesem Test zeigen z. B. die folgenden Verbindungen der Herstellungsbeispiele eine Wirkung von 80% bei einer Aufwandmenge von 500g/ha: 13

Bei diesem Test zeigen z. B. die folgenden Verbindungen der Herstellungsbeispiele eine Wirkung von 100% bei einer Aufwandmenge von 100g/ha: 7, 12, 16, 32, 36, 39, 40, 41, 42, 43, 46, 48, 49, 50, 51, 52, 53, 54, 57, 58, 59, 60, 61, 63, 64, 65, 66, 183, 184, 185, 188, 189, 190, 191, 192, 193, 195, 209, 263, 268, 272, 273, 275, 279, 282, 283, 284, 285, 286, 287, 288, 289, 290, 291, 292, 293, 295, 296, 297, 298, 302, 305, 310, 311, 315, 316, 320

Bei diesem Test zeigen z. B. die folgenden Verbindungen der Herstellungsbeispiele eine Wirkung von 90% bei einer Aufwandmenge von 100g/ha: 9, 18, 47, 62, 276, 277, 278, 308, 314

Bei diesem Test zeigen z. B. die folgenden Verbindungen der Herstellungsbeispiele eine Wirkung von 80% bei einer Aufwandmenge von 100g/ha: 6, 20, 22, 101

## Patentansprüche

1. Verbindungen der allgemeinen Formel (I) sowie deren Diastereomere, Enantiomere, E/Z-Isomere, N-Oxide und Salze, 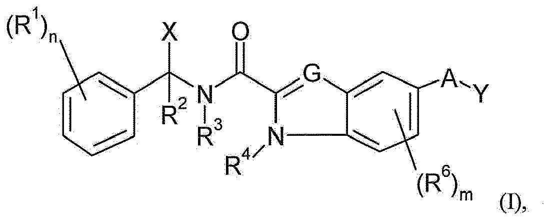 wobei
R¹ für Halogen, Nitro, Cyano, für gegebenenfalls einfach oder mehrfach durch Halogen substituiertes C₁-C₆-Alkyl, C₁-C₆-Alkoxy, C₁-C₆-Alkylthio, C₁-C₆-Alkylsulfinyl oder C₁-C₆-Alkylsulfonyl steht,
n für 1,2, 3, 4 oder 5 steht,
oder
R¹ für -OCF₂O-, -(CF₂)₂O-oder -O(CF₂)₂O- steht, und an zwei benachbarten Kohlenstoffatomen gebunden ist, wobei n dann für 1 steht,
R² für Wasserstoff, oder für gegebenenfalls einfach oder mehrfach, gleich oder verschieden substituiertes C₁-C₄-Alkyl steht,
wobei die Substituenten unabhängig voneinander ausgewählt sind aus Halogen oder C₁-C₄-Alkyl
R³ für Wasserstoff, für gegebenenfalls einfach oder mehrfach, gleich oder verschieden substituiertes C₁-C₄-Alkyl, C₁-C₄-Alkylcarbonyl, oder C₁-C₄-Alkoxycarbonyl steht,
wobei die Substituenten unabhängig voneinander ausgewählt sind aus Cyano, Halogen, C₁-C₄-Alkyl oder C₁-C₄-Alkoxy
R⁴ für gegebenenfalls einfach oder mehrfach, gleich oder verschieden substituiertes C₁-C₆-Alkyl, C₂-C₆-Alkenyl, C₃-C₆-Alkinyl, C₃-C₆-Cycloalkyl, C₃-C₆-Cycloalkyl-C₁-C₄-alkyl oder Aryl-C₁-C₄-alkyl steht,
wobei die Substituenten unabhängig voneinander ausgewählt sind aus Halogen, Cyano, C₁-C₄-Alkyl oder C₁-C₄-Alkoxy, C₁-C₄-Alkoxycarbonyl oder aus gegebenenfalls einfach oder mehrfach, gleich oder verschieden substituiertem Aryloxy oder Aryl-C₁-C₃-alkoxy
wobei die Substituenten unabhängig voneinander ausgewählt sind aus Halogen, Cyano, C₁-C₄-Alkyl, C₁-C₄-Alkoxy
G für C(R⁵) oder N steht,
R⁵ für Wasserstoff, Halogen oder Cyano steht,
R⁶ für Halogen, Nitro, Cyano, für gegebenenfalls einfach oder mehrfach, durch Halogen substituiertes C₁-C₆-Alkyl oder C₁-C₆-Alkoxy steht,
m für 0, 1, 2, 3 steht
X für C₁-C₆-Halogenalkyl steht, das gegebenenfalls zusätzlich einfach bis dreifach substituiert sein kann, wobei die Substituenten unabhängig voneinander ausgewählt sind aus Hydroxy, Cyano oder C₁-C₄-Alkoxy,
A für eine bivalente chemische Gruppierung steht, die ausgewählt ist aus den Gruppierungen -C(R¹¹)(R¹²)NR¹³C(=O)- oder -C(=O)NR¹³-, wobei die jeweils erstgenannte (linke) Anknüpfungsstelle am Ring und die jeweils zweitgenannte (rechte) Anknüpfungsstelle an Y anknüpft,
und wobei
R¹¹ und R¹² unabhängig voneinander für Wasserstoff oder für C₁-C₄-Alkyl steht,
R¹³ für Wasserstoff, für C₁-C₄-Alkyl, C₃-C₆-Cycloalkyl, C₁-C₄-Alkylcarbonyl, C₁-C₄-Alkoxycarbonyl oder C₂-C₄-Alkenyl, steht,
Y für gegebenenfalls ein- oder mehrfach, gleich oder verschieden substituiertes C₁-C₆-Alkyl, C₂-C₆-Alkenyl, C₃-C₆-Alkinyl, C₃-C₆-Cycloalkyl, Aryl, Aryl-C₁-C₄-alkyl, Hetaryl oder Hetaryl-C₁-C₄-alkyl steht,
wobei die Substituenten ausgewählt sind aus Halogen, Nitro, Cyano, Hydroxy, Aminothiocarbonyl, Aminocarbonyl, C₁-C₄-Alkylaminocarbonyl, Di-(C₁-C₄-Alkyl)-aminocarbonyl, Hydroxycarbonyl, C₁-C₄-Alkyl, C₁-C₄-Halogenalkyl, C₃-C₆-Cycloalkyl, C₁-C₄-Alkyl-C₃-C₄-Cycloalkyl, C₂-C₆-Alkenyl, C₂-C₆-Alkinyl, C₁-C₆-Alkoxy, C₁-C₆-Alkoxycarbonyl, C₁-C₆-Alkylcarbonyl, C₁-C₄-Alkoxyimino-C₁-C₄-Alkyl, C₁-C₆-Alkylthio, C₁-C₆-Alkylsulfinyl oder C₁-C₆-Alkylsulfonyl,
wobei
n für 2, 3, 4 oder 5 steht, wenn
mindestens ein Substituent R¹ für Trifluormethyl steht,
und gleichzeitig
Y für unsubstituiertes C₁-C₄-Alkyl, 2,2-Difluorethyl, unsubstituiertes C₂-C₆-Alkenyl, unsubstituiertes C₃-C₆-Alkinyl, für unsubstituiertes C₃-C₆-Cycloalkyl oder unsubstituiertes Hetaryl steht, und
A für -C(=O)NR¹³- steht,
und
G für C(R⁵) steht.

2. Verbindungen der allgemeinen Formel (I) sowie deren Diastereomere, Enantiomere, E/Z-Isomere, N-Oxide und Salze gemäß Anspruch 1, wobei
R¹ für Halogen, Nitro, Cyano, für gegebenenfalls einfach oder mehrfach durch Halogen substituiertes C₁-C₄-Alkyl, C₁-C₄-Alkoxy, C₁-C₄-Alkylthio, C₁-C₄-Alkylsulfinyl oder C₁-C₄-Alkylsulfonyl steht,
n für 1, 2, 3, 4 oder 5 steht
oder
R¹ für -OCF₂O- oder -O(CF₂)₂O- steht, und an zwei benachbarten Kohlenstoffatomen gebunden ist, wobei n dann für 1 steht,
R² für Wasserstoff oder für gegebenenfalls einfach bis dreifach substituiertes C₁-C₄-Alkyl steht,
wobei die Substituenten unabhängig voneinander ausgewählt sind aus Halogen oder C₁-C₄-Alkyl,
R³ für Wasserstoff, für gegebenenfalls einfach oder mehrfach, gleich oder verschieden substituiertes C₁-C₄-Alkyl, C₁-C₄-Alkylcarbonyl oder C₁-C₄-Alkoxycarbonyl steht,
wobei die Substituenten unabhängig voneinander ausgewählt sind aus Cyano, Halogen oder C₁-C₄-Alkoxy
R⁴ für gegebenenfalls einfach oder mehrfach, gleich oder verschieden substituiertes C₁-C₄-Alkyl, C₂-C₆-Alkenyl, C₃-C₆-Alkinyl, C₃-C₆-Cycloalkyl, C₃-C₆-Cycloalkyl-C₁-C₃-alkyl oder Aryl-C₁-C₄-alkyl steht,
wobei die Substituenten unabhängig voneinander ausgewählt sind aus Halogen, Cyano, C₁-C₄-Alkyl, C₁-C₄-Alkoxy, C₁-C₄-Alkoxycarbonyl oder aus gegebenenfalls einfach oder mehrfach, gleich oder verschieden substituiertem Aryloxy oder Aryl-C₁-C₃-alkoxy,
wobei die Substituenten unabhängig voneinander ausgewählt sind aus Halogen, Cyano, C₁-C₄-Alkyl, C₁-C₄-Alkoxy
G für C(R⁵) oder N steht,
R⁵ für Wasserstoff, Halogen oder Cyano steht,
R⁶ für Halogen, Nitro, Cyano, für gegebenenfalls einfach oder mehrfach, durch Halogen substituiertes C₁-C₄-Alkyl oder C₁-C₄-Alkoxy steht,
m für 0, 1, 2 steht
X für C₁-C₄-Halogenalkyl steht, das gegebenenfalls zusätzlich einfach bis dreifach durch Hydroxy, Cyano oder C₁-C₄-Alkoxy substituiert sein kann,
A für eine bivalente chemische Gruppierung steht, die ausgewählt ist aus den Gruppierungen -C(R¹¹)(R¹²)NR¹³C(=O)- oder -C(=O)NR¹³-, wobei die jeweils erstgenannte (linke) Anknüpfungsstelle am Ring und die jeweils zweitgenannte (rechte) Anknüpfungsstelle an Y anknüpft,
R¹¹ und R¹² unabhängig voneinander für Wasserstoff oder für C₁-C₄-Alkyl steht,
R¹³ für Wasserstoff, für C₁-C₄-Alkyl, C₃-C₆-Cycloalkyl, C₁-C₄-Alkylcarbonyl, C₁-C₄-Alkoxycarbonyl oder C₂-C₄-Alkenyl steht,
Y für gegebenenfalls ein- oder mehrfach, gleich oder verschieden substituiertes C₁-C₄-Alkyl, C₂-C₄-Alkenyl, C₂-C₄-Alkinyl, C₃-C₆-Cycloalkyl, Phenyl, Phenylmethyl, Pyridinyl, Pyridinylmethyl, Pyrimidinyl oder Pyrimidinylmethyl steht,
wobei die Substituenten ausgewählt sind aus Halogen, Nitro, Cyano, Hydroxy, Aminothiocarbonyl, Aminocarbonyl, C₁-C₄-Alkyl, C₁-C₄-Halogenalkyl C₃-C₅-Cycloalkyl, C₁-C₄-Alkyl-C₃-C₄-Cycloalkyl, C₂-C₄-Alkenyl, C₂-C₄-Alkinyl, C₁-C₄-Alkoxy, C₁-C₄-Alkylthio, C₁-C₄-Alkylsulfinyl, C₁-C₄-Alkylsulfonyl, C₁-C₄-Alkoxycarbonyl, C₁-C₆-Alkylcarbonyl oder C₁-C₄-Alkoxyimino-C₁-C₄-Alkyl.
wobei
n für 2, 3, 4 oder 5 steht, wenn mindestens ein Substituent R¹ für Trifluormethyl steht,
und gleichzeitig
Y für unsubstituiertes C₁-C₄-Alkyl, 2,2-Difluorethyl, unsubstituiertes C₂-C₆-Alkenyl, unsubstituiertes C₃-C₆-Alkinyl, für unsubstituiertes C₃-C₆-Cycloalkyl oder unsubstituiertes Hetaryl steht, und
A für -C(=O)NR¹³- steht,
und
G für C(R⁵) steht.

3. Verbindungen der allgemeinen Formel (I) sowie deren Diastereomere, Enantiomere, E/Z-Isomere, N-Oxide und Salze gemäß Anspruch 1 oder 2, wobei
R¹ für Halogen, Nitro, Cyano, für gegebenenfalls einfach oder mehrfach, durch Fluor oder Chlor substituiertes C₁-C₄-Alkyl, C₁-C₄-Alkoxy, C₁-C₄-Alkylthio, C₁-C₄-Alkylsulfinyl, C₁-C₄-Alkylsulfonyl steht,
n für 1, 2, 3, 4 oder 5 steht
oder
R¹ für -OCF₂O- steht, und an zwei benachbarten Kohlenstoffatomen gebunden ist, wobei n dann für 1 steht,
R² für Wasserstoff oder für Methyl steht,
R³ für Wasserstoff, Methyl, Ethyl, Methylcarbonyl, Ethylcarbonyl, Methoxycarbonyl oder Ethoxycarbonyl steht,
R⁴ für gegebenenfalls einfach bis dreifach substituiertes C₁-C₄-Alkyl, C₂-C₄-Alkenyl, C₃-C₄-Alkinyl, C₃-C₅-Cycloalkyl, C₃-C₅-Cycloalkyl-C₁-C₃-alkyl oder Phenylalkyl steht,
wobei die Substituenten unabhängig voneinander ausgewählt sind aus Fluor, Cyano, Methoxy, Ethoxy, Methyl, Ethyl, Methoxycarbonyl, Ethoxycarbonyl, Phenyloxy oder Phenyl-C₁-C₃-alkoxy.
G für C(R⁵) oder N steht,
R⁵ für Wasserstoff, Fluor, Chlor, Brom oder Cyano steht,
R⁶ für Halogen, Nitro, Cyano, oder für gegebenenfalls einfach bis dreifach durch Halogen substituiertes C₁-C₄-Alkyl oder C₁-C₄-Alkoxy steht,
m für 0, 1 oder 2 steht,
X für C₁-C₄-Halogenalkyl steht,
A für eine bivalente chemische Gruppierung steht, die ausgewählt ist aus den Gruppierungen -C(R¹¹)(R¹²)NR¹³C(-O)- oder -C(=O)NR¹³-, wobei die jeweils erstgenannte (linke) Anknüpfungsstelle am Ring und die jeweils zweitgenannte (rechte) Anknüpfungsstelle an Y anknüpft,
und wobei
R¹¹ und R¹² unabhängig voneinander für Wasserstoff oder Methyl stehen,
und wobei
R¹³ für Wasserstoff, Methyl, Ethyl, Cyclopropyl, Methylcarbonyl, Ethylcarbonyl, Methoxycarbonyl, Ethoxycarbonyl oder Prop-2-en-1-yl steht,
Y für gegebenenfalls ein- bis dreifach, gleich oder verschieden substituiertes C₁-C₄-Alkyl, C₂-C₄-Alkenyl, C₂-C₄-Alkinyl, C₃-C₆-Cycloalkyl, Phenyl, Phenylmethyl, Pyridin-2-yl, Pyridin-2-yl-methyl, 1,3-Pyrimidin-2-yl oder 1,3-Pyrimidin-2-yl-methyl steht,
wobei die Substituenten ausgewählt sind aus Fluor, Chlor, Nitro, Cyano, C₁-C₄-Alkyl, Trifluormethyl, C₃-C₄-Cycloalkyl, C₁-C₄-Alkyl-C₃-C₄-Cycloalkyl, C₂-C₄-Alkenyl, C₃-C₄-Alkinyl C₁-C₄-Alkoxy, C₁-C₄-Alkylthio, C₁-C₄-Alkylsulfinyl, C₁-C₆-Alkylsulfonyl, C₁-C₄-Alkoxycarbonyl oder Aminothiocarbonyl,
wobei
n für 2, 3, 4 oder 5 steht, wenn mindestens ein Substituent R¹ für Trifluormethyl steht,
und gleichzeitig
Y für unsubstituiertes C₁-C₄-Alkyl, 2,2-Difluorethyl, unsubstituiertes C₂-C₆-Alkenyl, unsubstituiertes C₃-C₆-Alkinyl, für unsubstituiertes C₃-C₆-Cycloalkyl oder unsubstituiertes Hetaryl steht, und
A für -C(=O)NR¹³- steht,
und
G für C(R⁵)steht.

4. Verbindungen der allgemeinen Formel (I) sowie deren Diastereomere, Enantiomere, E/Z-Isomere, N-Oxide und Salze gemäß Anspruch 1, 2 oder 3, wobei
R¹ für Cyano, Fluor, Chlor, Brom, Iod, Difluormethyl, Dichlorfluormethyl, Chlordifluormethyl, Trifluormethyl, Pentafluorethyl, Chlortetrafluorethyl, Difluormethoxy, Trifluomnethoxy, Trifluormethylthio, Trifluormethylsulfinyl, Trifluormethylsulfonyl, steht,
n für 1, 2, 3,4 oder 5 steht
oder
R¹ für -OCF₂O- steht, und an zwei benachbarten Kohlenstoffatomen gebunden ist, wobei n dann für 1 steht,
R² für Wasserstoff steht,
R³ für Wasserstoff steht,
R⁴ für Methyl, Ethyl, Prop-1-yl, Prop-2-en-1-yl, Prop-2-in-1-yl, Ethenyl, But-2-in-1-yl, Cyclopropyl, Cyclopropylmethyl, Cyclobutyl, Cyanomethyl, 2-Methylprop-1-yl, Ethoxymethyl, Methoxycarbonylmethyl, Phenylmethyl oder Benzyloxymethyl, steht,
G für C(R⁵) oder N steht,
R⁵ für Wasserstoff, Chlor, Brom oder Cyano steht,
R⁶ für Cyano, Fluor, Chlor, Brom, Methyl, Ethyl, Isopropyl oder Trifluormethyl steht,
m für 0 oder 1 steht,
X für Trifluormethyl steht,
A für eine bivalente chemische Gruppierung steht, die ausgewählt ist aus den Gruppierungen -CH₂NHC(=O)- oder -C(=O)NR¹³-, wobei die jeweils erstgenannte (linke) Anknüpfungsstelle am Ring und die jeweils zweitgenannte (rechte) Anknüpfungsstelle an Y anknüpft,
und wobei
R¹³ für Wasserstoff, Methyl, Ethyl oder Prop-2-en-1-yl steht,
Y für Methyl, Ethyl, Propan-1-yl, Propan-2-yl, Butan-1-yl, Butan-2-yl, 2-Methylpropan-1-yl, 2-Methylpropan-2-yl, Cyclopropyl, Cyclobutyl, Cyanomethyl, 1-Cyanoethyl, 2-Cyanoethyl, 1-Cyanoprop-1-yl, 2-Cyanoprop-1-yl, 3-Cyanoprop-1-yl, 1-Cyanoprop-2-yl, 2-Cyanoprop-2-yl, 1-Cyanocyclopropyl, 2-Cyanoprop-2-en-1-yl, 2-Cyanocyclopropyl, 1-Cyanocyclobutyl, 2-Cyanocyclobutyl, 3-Cyanocyclobutyl, 2-Fluorethyl, 2,2-Difluorethyl, 2,2,2-Trifluorethyl, 1-Fluorpropan-2-yl, 2,2-Difluorprop-1-yl, 1,3-Difluorpropan-2-yl, 1-Methylcyclopropyl, 2-Methylcyclopropyl, 1-Ethylcyclopropyl, 1-Ethinylcyclopropyl, 1-Ethinylcyclobutyl, 1-Methoxycyclopropyl, 1-Ethoxycyclopropyl, 1-Methoxycarbonylcyclopropyl, 1-Ethoxycarbonyl-cyclopropyl, 1,1'-Bi(Cyclopropyl)-1-yl, Cyclopropylmethyl, 1-Trifluormethylcyclopropyl, Pyridin-2-yl, 5-Chlorpyridin-2-yl, 5-Fluorpyridin-2-yl, 1-Cyano-1-phenylmethyl, 1,2-Dimethylcyclopropyl, 1-(Aminothiocarbonyl)cyclopropyl, 1-Cyano-2-methylpropan-1-yl, 1-Cyanobut-3-in-1-yl, 1-Cyano-2-methylpropan-1-yl, 1-Cyano-propan-2-yl, 1-Cyano-1-cyclopropylethyl, 1-Cyano-1-ethylprop-1-yl, 1-Cyano-1-methylcyclopropylmethyl, (2-R)-1-(Methylsulfinyl)propan-2-yl oder 1,3-Dimethoxy-2-cyanopropan-2-yl steht, wenn A für die Gruppierung -C(=O)NR¹³- steht,
oder
Y für Methyl, Ethyl, Propan-1-yl, Propan-2-yl, Butan-1-yl, 2-Fluorethyl, 2,2-Difluorethyl, 2,2,2-Trifluorethyl, Cyclopropyl, Cyclobutyl oder Cyclopropylmethyl steht, wenn A für die Gruppierung -CH₂NHC(=O)- steht,
wobei
n für 2, 3, 4 oder 5 steht, wenn
mindestens ein Substituent R¹ für Trifluormethyl steht,
und gleichzeitig
Y für unsubstituiertes C₁-C₄-Alkyl, 2,2-Difluorethyl, unsubstituiertes C₂-C₆-Alkenyl, unsubstituiertes C₃-C₆-Alkinyl, für unsubstituiertes C₃-C₆-Cycloalkyl oder unsubstituiertes Hetaryl steht, und
A für -C(=O)NH- steht,
und
G für C(R⁵) steht.

5. Verbindungen der allgemeinen Formel (I) sowie deren Diastereomere, Enantiomere, E/Z-Isomere, N-Oxide und Salze gemäß Anspruch 4, wobei G für CH und A für -C(=O)NH-steht.

6. Verfahren zur Herstellung von Verbindungen der allgemeinen Formel (I) nach Anspruch 1, bei dem man
a) Carbonsäuren der allgemeinen Formel (II) 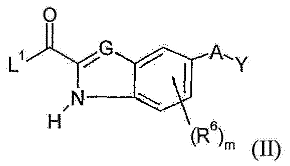 wobei
L¹ für Hydroxy oder Halogen steht,
mit Aminen der Formel (III) 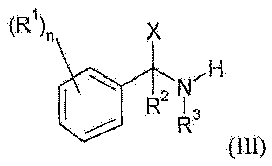 umsetzt,
wobei G, A, Y, R1, R2, R3, R6, n, m die in Anspruch 1 beschriebene Bedeutung haben, und
b) die so erhaltenen Verbindungen der Formel (IVa) 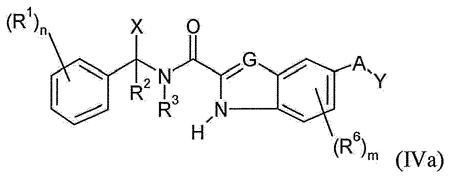 anschließend mit Alkylierungsmitteln der Formel (V)
R⁴-L² (V)
wobei
L² für Halogen, für die Mesyl- oder für die Tosylgruppe steht und R⁴ die in Anspruch 1 angegebene Bedeutung hat,
in Gegenwart einer Base zu Verbindungen der Formel (I) umsetzt.

7. Verfahren zur Herstellung von Schädlingsbekämpfungsmitteln, bei dem Verbindungen der 10 allgemeinen Formel (I) und / oder deren Salze gemäß Anspruch 1 mit Streckmitteln und / oder oberflächenaktiven Stoffen vermischt werden.

8. Verwendung von Verbindungen der allgemeinen Formel (I) und / oder deren Salzen gemäß Anspruch 1 zur Herstellung von Schädlingsbekämpfungsmitteln.

9. Schädlingsbekämpfungsmittel enthaltend Verbindungen der allgemeinen Formel (I) und / 15 oder deren Salze gemäß Anspruch 1 in biologisch wirksamen Gehalten von 0,00000001 bis zu 95 Gew.-%, bezogen auf das Gewicht des Schädlingsbekämpfungsmittels.

10. Schädlingsbekämpfungsmittel gemäß Anspruch 9 zusätzlich enthaltend einen weiteren agrochemischen Wirkstoff.

11. Verfahren zur Bekämpfung von tierischen Schädlingen, bei dem man Verbindungen der 20 allgemeinen Formel (I) und / oder deren Salze gemäß Anspruch 1 auf tierische Schädlinge und/oder ihren Lebensraum einwirken lässt, wobei Verfahren zur Behandlung des menschlichen oder tierischen Körpers ausgeschlossen sind.

12. Carbonsäuren der allgemeinen Formel (II-1aa) 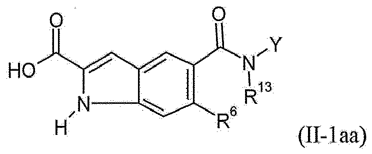 bei denen R⁶, Y und R¹³ die in Anspruch 4 angegebene Bedeutung haben.

13. Carbonsäuren der allgemeinen Formel (II-1ba) 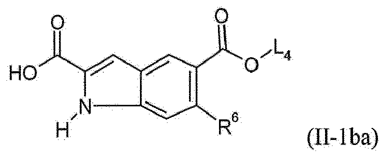 bei denen L⁴ für für C₁-C₄-Alkyl steht und R⁶ die in Anspruch 4 angegebene Bedeutung hat, wobei die Verbindung 6-Chlor-5-(ethoxycarbonyl)-1H-indol-2-carbonsäure ausgenommen ist.

14. Carbonsäuren der allgemeinen Formel (II-2aa) 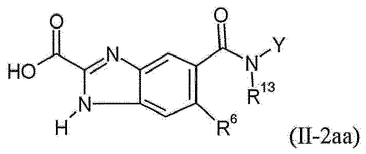 bei denen R⁶, Y und R¹³ die in Anspruch 4 angegebene Bedeutung haben.

15. Carbonsäuren der allgemeinen Formel (II-2ba) 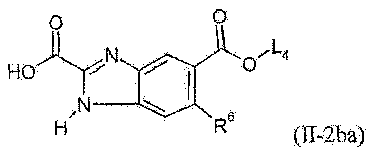 bei denen L⁴ für für C₁-C₄-Alkyl steht und R⁶ die in Anspruch 4 angegebene Bedeutung hat.

## Claims

1. Compounds of the general formula (I), and the diastereomers, enantiomers, E/Z isomers, N-oxides and salts thereof, 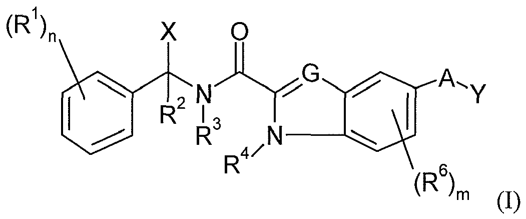 where
R¹ is halogen, nitro, cyano, optionally mono- or poly-halogen-substituted C₁-C₆-alkyl, C₁-C₆-alkoxy, C₁-C₆-alkylthio, C₁-C₆-alkylsulphinyl or C₁-C₆-alkylsulphonyl,
n is 1, 2, 3, 4 or 5,
or
R¹ is -OCF₂O-, -(CF₂)₂O- or -O(CF₂)₂O-, and is bonded to two adjacent carbon atoms, in which case n is 1,
R² is hydrogen, or optionally singly or multiply, identically or differently substituted C₁-C₄-alkyl,
where the substituents are each independently selected from halogen and C₁-C₄-alkyl,
R³ is hydrogen, optionally singly or multiply, identically or differently substituted C₁-C₄-alkyl, C₁-C₄-alkylcarbonyl, or C₁-C₄-alkoxycarbonyl,
where the substituents are each independently selected from cyano, halogen, C₁-C₄-alkyl and C₁-C₄-alkoxy,
R⁴ is optionally singly or multiply, identically or differently substituted C₁-C₆-alkyl, C₂-C₆-alkenyl, C₃-C₆-alkynyl, C₃-C₆-cycloalkyl, C₃-C₆-cycloalkyl-C₁-C₄-alkyl or aryl-C₁-C₄-alkyl,
where the substituents are each independently selected from halogen, cyano, C₁-C₄-alkyl and C₁-C₄-alkoxy, C₁-C₄-alkoxycarbonyl and from optionally singly or multiply, identically or differently substituted aryloxy and aryl-C₁-C₃-alkoxy,
where the substituents are each independently selected from halogen, cyano, C₁-C₄-alkyl, C₁-C₄-alkoxy,
G is C(R⁵) or N,
R⁵ is hydrogen, halogen or cyano,
R⁶ is halogen, nitro, cyano, or optionally mono- or poly-halogen-substituted C₁-C₆-alkyl or C₁-C₆-alkoxy,
m is 0, 1, 2, 3,
X is C₁-C₆-haloalkyl which may optionally additionally be mono- to trisubstituted, where the substituents are each independently selected from hydroxyl, cyano and C₁-C₄-alkoxy,
A is a bivalent chemical moiety which is selected from the-C(R¹¹)(R¹²)NR¹³C(=O)- and -C(=O)NR¹³- moieties, where the first (left-hand) connection site in each case connects to the ring and the second (right-hand) connection site in each case connects to Y,
and where
R¹¹ and R¹² are each independently hydrogen or C₁-C₄-alkyl,
R¹³ is hydrogen, C₁-C₄-alkyl, C₃-C₆-cycloalkyl, C₁-C₄-alkylcarbonyl, C₁-C₄-alkoxycarbonyl or C₂-C₄-alkenyl,
Y is optionally singly or multiply identically or differently substituted C₁-C₆-alkyl, C₂-C₆-alkenyl, C₃-C₆-alkynyl, C₃-C₆-cycloalkyl, aryl, aryl-C₁-C₄-alkyl, hetaryl or hetaryl-C₁-C₄-alkyl,
where the substituents are selected from halogen, nitro, cyano, hydroxyl, aminothiocarbonyl, aminocarbonyl, C₁-C₄-alkylaminocarbonyl, di-(C₁-C₄-alkyl)-aminocarbonyl, hydroxycarbonyl, C₁-C₄-alkyl, C₁-C₄-haloalkyl, C₃-C₆-cycloalkyl, C₁-C₄-alkyl-C₃-C₄-cycloalkyl, C₂-C₆-alkenyl, C₂-C₆-alkynyl, C₁-C₆-alkoxy, C₁-C₆-alkoxycarbonyl, C₁-C₆-alkylcarbonyl, C₁-C₄-alkoxyimino-C₁-C₄-alkyl, C₁-C₆-alkylthio, C₁-C₆-alkylsulphinyl and C₁-C₆-alkylsulphonyl,
where
n is 2, 3, 4 or 5 when
at least one R¹ substituent is trifluoromethyl,
and at the same time
Y is unsubstituted C₁-C₄-alkyl, 2,2-difluoroethyl, unsubstituted C₂-C₆-alkenyl, unsubstituted C₃-C₆-alkynyl, unsubstituted C₃-C₆-cycloalkyl or unsubstituted hetaryl, and
A is -C(=O)NR¹³-,
and
G is C(R⁵).

2. Compounds of the general formula (I), and the diastereomers, enantiomers, E/Z isomers, N-oxides and salts thereof, according to Claim 1, where
R¹ is halogen, nitro, cyano, optionally mono- or poly-halogen-substituted C₁-C₄-alkyl, C₁-C₄-alkoxy, C₁-C₄-alkylthio, C₁-C₄-alkylsulphinyl or C₁-C₄-alkylsulphonyl,
n is 1, 2, 3, 4 or 5,
or
R¹ is -OCF₂O- or -O(CF₂)₂O-, and is bonded to two adjacent carbon atoms, in which case n is 1,
R² is hydrogen or optionally mono- to trisubstituted C₁-C₄-alkyl,
where the substituents are each independently selected from halogen and C₁-C₄-alkyl,
R³ is hydrogen, optionally singly or multiply, identically or differently substituted C₁-C₄-alkyl, C₁-C₄-alkylcarbonyl, or C₁-C₄-alkoxycarbonyl,
where the substituents are each independently selected from cyano, halogen and C₁-C₄-alkoxy,
R⁴ is optionally singly or multiply, identically or differently substituted C₁-C₄-alkyl, C₂-C₆-alkenyl, C₃-C₆-alkynyl, C₃-C₆-cycloalkyl, C₃-C₆-cycloalkyl-C₁-C₃-alkyl or aryl-C₁-C₄-alkyl,
where the substituents are each independently selected from halogen, cyano, C₁-C₄-alkyl, C₁-C₄-alkoxy, C₁-C₄-alkoxycarbonyl and from optionally singly or multiply, identically or differently substituted aryloxy and aryl-C₁-C₃-alkoxy,
where the substituents are each independently selected from halogen, cyano, C₁-C₄-alkyl, C₁-C₄-alkoxy,
G is C(R⁵) or N,
R⁵ is hydrogen, halogen or cyano,
R⁶ is halogen, nitro, cyano, or optionally mono- or poly-halogen-substituted C₁-C₄-alkyl or C₁-C₄-alkoxy,
m is 0, 1, 2,
X is C₁-C₄-haloalkyl which may optionally be mono- to trisubstituted by hydroxyl, cyano or C₁-C₄-alkoxy,
A is a bivalent chemical moiety which is selected from the - C(R¹¹)(R¹²)NR¹³C(=O)- and -C(=O)NR¹³- moieties, where the first (left-hand) connection site in each case connects to the ring and the second (right-hand) connection site in each case connects to Y,
R¹¹ and R¹² are each independently hydrogen or C₁-C₄-alkyl,
R¹³ is hydrogen, C₁-C₄-alkyl, C₃-C₆-cycloalkyl, C₁-C₄-alkylcarbonyl, C₁-C₄-alkoxycarbonyl or C₂-C₄-alkenyl,
Y is optionally singly or multiply identically or differently substituted C₁-C₄-alkyl, C₂-C₄-alkenyl, C₂-C₄-alkynyl, C₃-C₆-cycloalkyl, phenyl, phenylmethyl, pyridinyl, pyridinylmethyl, pyrimidinyl or pyrimidinylmethyl,
where the substituents are selected from halogen, nitro, cyano, hydroxyl, aminothiocarbonyl, aminocarbonyl, C₁-C₄-alkyl, C₁-C₄-haloalkyl, C₃-C₅-cycloalkyl, C₁-C₄-alkyl-C₃-C₄-cycloalkyl, C₂-C₄-alkenyl, C₂-C₄-alkynyl, C₁-C₄-alkoxy, C₁-C₄-alkylthio, C₁-C₄-alkylsulphinyl, C₁-C₄-alkylsulphonyl, C₁-C₄-alkoxycarbonyl, C₁-C₆-alkylcarbonyl and C₁-C₄-alkoxyimino-C1-C4-alkyl,
where
n is 2, 3, 4 or 5 when
at least one R¹ substituent is trifluoromethyl,
and at the same time
Y is unsubstituted C₁-C₄-alkyl, 2,2-difluoroethyl, unsubstituted C₂-C₆-alkenyl, unsubstituted C₃-C₆-alkynyl, unsubstituted C₃-C₆-cycloalkyl or unsubstituted hetaryl, and
A is -C(=O)NR¹³-,
and
G is C(R⁵).

3. Compounds of the general formula (I), and the diastereomers, enantiomers, E/Z isomers, N-oxides and salts thereof, according to Claim 1 or 2, where
R¹ is halogen, nitro, cyano, optionally mono- or poly-fluorine- or -chlorine-substituted C₁-C₄-alkyl, C₁-C₄-alkoxy, C₁-C₄-alkylthio, C₁-C₄-alkylsulphinyl, C ₁-C₄-alkylsulphonyl,
n is 1, 2, 3, 4 or 5,
or
R¹ is -OCF₂O-, and is bonded to two adjacent carbon atoms, in which case n is 1,
R² is hydrogen or methyl,
R³ is hydrogen, methyl, ethyl, methylcarbonyl, ethylcarbonyl, methoxycarbonyl or ethoxycarbonyl,
R⁴ is optionally mono- to trisubstituted C₁-C₄-alkyl, C₂-C₄-alkenyl, C₃-C₄-alkynyl, C₃-C₅-cycloalkyl, C₃-C₅-cycloalkyl-C₁-C₃-alkyl or phenylalkyl,
where the substituents are each independently selected from fluorine, cyano, methoxy, ethoxy, methyl, ethyl, methoxycarbonyl, ethoxycarbonyl, phenyloxy and phenyl-C₁-C₃-alkoxy,
G is C(R⁵) or N,
R⁵ is hydrogen, fluorine, chlorine, bromine or cyano,
R⁶ is halogen, nitro, cyano, or optionally mono- to tri-halogen-substituted C₁-C₄-alkyl or C₁-C₄-alkoxy,
m is 0, 1 or 2,
X is C₁-C₄-haloalkyl,
A is a bivalent chemical moiety which is selected from the - C(R¹¹)(R¹²)NR¹³C(=O)- and -C(=O)NR¹³- moieties, where the first (left-hand) connection site in each case connects to the ring and the second (right-hand) connection site in each case connects to Y,
and where
R¹¹ and R¹² are each independently hydrogen or methyl,
and where
R¹³ für hydrogen, methyl, ethyl, cyclopropyl, methylcarbonyl, ethylcarbonyl, methoxycarbonyl, ethoxycarbonyl or prop-2-en-1-yl,
Y is optionally singly to triply, identically or differently substituted C₁-C₄-alkyl, C₂-C₄-alkenyl, C₂-C₄-alkynyl, C₃-C₆-cycloalkyl, phenyl, phenylmethyl, pyridin-2-yl, pyridin-2-ylmethyl, 1,3-pyrimidin-2-yl or 1,3-pyrimidin-2-ylmethyl, where the substituents are selected from fluorine, chlorine, nitro, cyano, C₁-C₄-alkyl, trifluoromethyl, C₃-C₄-cycloalkyl, C₁-C₄-alkyl-C₃-C₄-cycloalkyl, C₂-C₄-alkenyl, C₃-C₄-alkynyl, C₁-C₄-alkoxy, C₁-C₄-alkylthio, C₁-C₄-alkylsulphinyl, C₁-C₆-alkylsulphonyl, C₁-C₄-alkoxycarbonyl and aminothiocarbonyl,
where
n is 2, 3, 4 or 5 when
at least one R¹ substituent is trifluoromethyl,
and at the same time
Y is unsubstituted C₁-C₄-alkyl, 2,2-difluoroethyl, unsubstituted C₂-C₆-alkenyl, unsubstituted C₃-C₆-alkynyl, unsubstituted C₃-C₆-cycloalkyl or unsubstituted hetaryl, and
A is -C(=O)NR¹³-,
and
G is C(R⁵).

4. Compounds of the general formula (I), and the diastereomers, enantiomers, E/Z isomers, N-oxides and salts thereof, according to Claim 1, 2 or 3, where
R¹ is cyano, fluorine, chlorine, bromine, iodine, difluoromethyl, dichlorofluoromethyl, chlorodifluoromethyl, trifluoromethyl, pentafluoroethyl, chlorotetrafluoroethyl, difluoromethoxy, trifluoromethoxy, trifluoromethylthio, trifluoromethylsulphinyl, trifluoromethylsulphonyl,
n is 1, 2, 3, 4 or 5,
R¹ is -OCF₂O-, and is bonded to two adjacent carbon atoms, in which case n is 1,
R² is hydrogen,
R³ is hydrogen,
R⁴ is methyl, ethyl, prop-1-yl, prop-2-en-1-yl, prop-2-yn-1-yl, ethenyl, but-2-yn-1-yl, cyclopropyl, cyclopropylmethyl, cyclobutyl, cyanomethyl, 2-methylprop-1-yl, ethoxymethyl, methoxycarbonylmethyl, phenylmethyl or benzyloxymethyl,
G is C(R⁵) or N,
R⁵ is hydrogen, chlorine, bromine or cyano,
R⁶ is cyano, fluorine, chlorine, bromine, methyl, ethyl, isopropyl or trifluoromethyl,
m is 0 or 1,
X is trifluoromethyl,
A is a bivalent chemical moiety which is selected from the -CH₂NHC(=O)- and -C(=O)NR¹³- moieties, where the first (left-hand) connection site in each case connects to the ring and the second (right-hand) connection site in each case connects to Y,
and where
R¹³ is hydrogen, methyl, ethyl or prop-2-en-1-yl,
Y is methyl, ethyl, propan-1-yl, propan-2-yl, butan-1-yl, butan-2-yl, 2-methylpropan-1-yl, 2-methylpropan-2-yl, cyclopropyl, cyclobutyl, cyanomethyl, 1-cyanoethyl, 2-cyanoethyl, 1-cyanoprop-1-yl, 2-cyanoprop-1-yl, 3-cyanoprop-1-yl, 1-cyanoprop-2-yl, 2-cyanoprop-2-yl, 1-cyanocyclopropyl, 2-cyanoprop-2-en-1-yl, 2-cyanocyclopropyl, 1-cyanocyclobutyl, 2-cyanocyclobutyl, 3-cyanocyclobutyl, 2-fluoroethyl, 2,2-difluoroethyl, 2,2,2-trifluoroethyl, 1-fluoropropan-2-yl, 2,2-difluoroprop-1-yl, 1,3-difluoropropan-2-yl, 1-methylcyclopropyl, 2-methylcyclopropyl, 1-ethylcyclopropyl, 1-ethynylcyclopropyl, 1-ethynylcyclobutyl, 1-methoxycyclopropyl, 1-ethoxycyclopropyl, 1-methoxycarbonylcyclopropyl, 1-ethoxycarbonylcyclopropyl, 1,1'-bi(cyclopropyl)-1-yl, cyclopropylmethyl, 1-trifluoromethylcyclopropyl, pyridin-2-yl, 5-chloropyridin-2-yl, 5-fluoropyridin-2-yl, 1-cyano-1-phenylmethyl, 1,2-dimethylcyclopropyl, 1-(aminothiocarbonyl)cyclopropyl, 1-cyano-2-methylpropan-1-yl, 1-cyanobut-3-yn-1-yl, 1-cyano-2-methylpropan-1-yl, 1-cyanopropan-2-yl, 1-cyan-1-cyclopropylethyl, 1-cyano-1-ethylprop-1-yl, 1-cyano-1-methylcyclopropylmethyl, (2-R)-1-(methylsulphinyl)propan-2-yl or 1,3-dimethoxy-2-cyanopropan-2-yl when A is the -C(=O)NR¹³- moiety,
or
Y is methyl, ethyl, propan-1-yl, propan-2-yl, butan-1-yl, 2-fluoroethyl, 2,2-difluoroethyl, 2,2,2-trifluoroethyl, cyclopropyl, cyclobutyl or cyclopropylmethyl when A is the -CH₂NHC(=O)- moiety,
where
n is 2, 3, 4 or 5 when
at least one R¹ substituent is trifluoromethyl,
and at the same time
Y is unsubstituted C₁-C₄-alkyl, 2,2-difluoroethyl, unsubstituted C₂-C₆-alkenyl, unsubstituted C₃-C₆-alkynyl, unsubstituted C₃-C₆-cycloalkyl or unsubstituted hetaryl, and
A is -C(=O)NH-,
and
G is C(R⁵).

5. Compounds of the general formula (I), and the diastereomers, enantiomers, E/Z isomers, N-oxides and salts thereof, according to Claim 4, where G is CH and A is-C(=O)NH-.

6. Process for preparing compounds of the general formula (I) according to Claim 1, in which
a) carboxylic acids of the general formula (II) 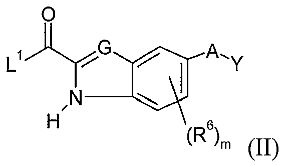 where
L¹ is hydroxyl or halogen,
are reacted with amines of the formula (III) 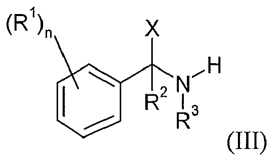 where G, A, Y, R¹, R², R³, R⁶, n, m are each defined as described in Claim 1, and
b) the compounds of the formula (IVa) thus obtained 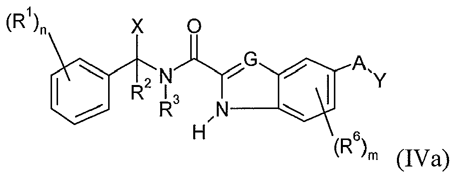 are then reacted with alkylating agents of the formula (V)
R⁴-L² (V)
where
L² is halogen, the mesyl group or the tosyl group and R⁴ is as defined in Claim 1, in the presence of a base to give compounds of the formula (I).

7. Process for producing pesticides, in which compounds of the general formula (I) and/or salts thereof according to Claim 1 are mixed with extenders and/or surface-active substances.

8. Use of compounds of the general formula (I) and/or salts thereof according to Claim 1 for production of pesticides.

9. Pesticide comprising compounds of the general formula (I) and/or salts thereof according to Claim 1 in biologically effective contents of 0.00000001 up to 95% by weight, based on the weight of the pesticide.

10. Pesticide according to Claim 9, additionally comprising a further active agrochemical ingredient.

11. Method for controlling animal pests, in which compounds of the general formula (I) and/or salts thereof according to Claim 1 are allowed to act on animal pests and/or their habitat, excluding methods for treatment of the human or animal body.

12. Carboxylic acids of the general formula (II-1aa) 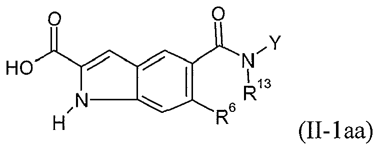 in which R⁶, Y and R¹³ are each as defined in Claim 4.

13. Carboxylic acids of the general formula (II-1ba) 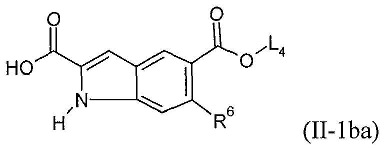 in which L⁴ is C₁-C₄-alkyl and R⁶ is as defined in Claim 4, excluding the compound 6-chloro-5-(ethoxycarbonyl)-1H-indole-2-carboxylic acid.

14. Carboxylic acids of the general formula (II-2aa) 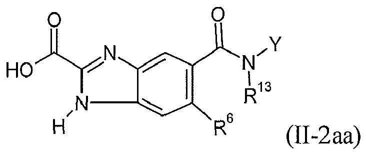 in which R⁶, Y and R¹³ are each as defined in Claim 4.

15. Carboxylic acids of the general formula (II-2ba) 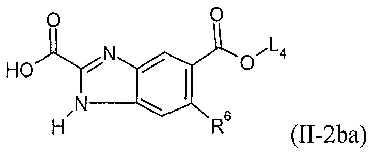 in which L⁴ is C₁-C₄-alkyl and R⁶ is as defined in Claim 4.

## Revendications

1. Composés de formule générale (I) ainsi que leurs diastéréoisomères, énantiomères, isomères E/Z, N-oxydes et sels, 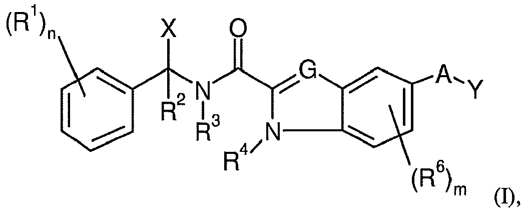 dans laquelle
R¹ représente un atome d'halogène, le groupe nitro, cyano, un groupe alkyle en C₁-C₆, alcoxy en C₁-C₆, alkyl(C₁-C₆) thio, alkyl(C₁-C₆)sulfinyle ou alkyl(C₁-C₆)sulfonyle éventuellement une ou plusieurs fois substitué par halogéno,
n représente 1, 2, 3, 4 ou 5,
ou
R¹ représente -OCF₂O-, -(CF₂)₂O- ou -O(CF₂)₂O-, et est lié à deux atomes de carbone contigus, n représentant alors 1,
R² représente un atome d'hydrogène ou un groupe alkyle en C₁-C₄ portant éventuellement un ou plusieurs substituants identiques ou différents,
les substituants étant choisis indépendamment les uns des autres parmi halogéno ou alkyle en C₁-C₄,
R³ représente un atome d'hydrogène, un groupe alkyle en C₁-C₄, alkyl (C₁-C₄)carbonyle ou alcoxy(C₁-C₄)carbonyle portant éventuellement un ou plusieurs substituants identiques ou différents,
les substituants étant choisis indépendamment les uns des autres parmi cyano, halogéno, alkyle en C₁-C₄ ou alcoxy en C₁-C₄
R⁴ représente un groupe alkyle en C₁-C₆, alcényle en C₂-C₆, alcynyle en C₃-C₆, cycloalkyle en C₃-C₆, cycloalkyl (C₃-C₆)-alkyle(C₁-C₄) ou aryl-alkyle(C₁-C₄) portant éventuellement un ou plusieurs substituants identiques ou différents,
les substituants étant choisis indépendamment les uns des autres parmi halogéno, cyano, alkyle en C₁-C₄ ou alcoxy en C₁-C₄, alcoxy(C₁-C₄)carbonyle ou parmi aryloxy ou aryl-alcoxy(C₁-C₃) portant éventuellement un ou plusieurs substituants identiques ou différents
les substituants étant choisis indépendamment les uns des autres parmi halogéno, cyano, alkyle en C₁-C₄, alcoxy en C₁-C₄
G représente C(R⁵) ou N,
R⁵ représente un atome d'hydrogène ou d'halogène ou le groupe cyano,
R⁶ représente un atome d'halogène, le groupe nitro, cyano, un groupe alkyle en C₁-C₆ ou alcoxy en C₁-C₆ portant éventuellement un ou plusieurs substituants halogéno,
m représente 0, 1, 2, 3
X représente un groupe halogénoalkyle(C₁-C₆) qui éventuellement peut en plus être une à trois fois substitué, les substituants étant choisis chaque fois indépendamment parmi hydroxy, cyano ou alcoxy en C₁-C₄,
A représente un groupement chimique divalent qui est choisi parmi les groupements -C(R¹¹)(R¹²)NR¹³C(=O)-ou -C(=O)NR¹³-, la position de liaison indiquée chaque fois en premier (gauche) étant attachée au cycle et la position de liaison indiquée chaque fois en second (droite) étant attachée à Y,
et dans laquelle
R¹¹ et R¹² représentent indépendamment l'un de l'autre un atome d'hydrogène ou un groupe alkyle en C₁-C₄,
R¹³ représente un atome d'hydrogène, un groupe alkyle en C₁-C₄, cycloalkyle en C₃-C₆, alkyl(C₁-C₄)-carbonyle, alcoxy(C₁-C₄)carbonyle ou alcényle en C₂-C₄,
Y représente un groupe alkyle en C₁-C₆, alcényle en C₂-C₆, alcynyle en C₃-C₆, cycloalkyle en C₃-C₆, aryle, aryl-alkyle(C₁-C₄), hétéroaryle ou hétéroaryl-alkyle(C₁-C₄) portant éventuellement un ou plusieurs substituants identiques ou différents,
les substituants étant choisis parmi halogéno, nitro, cyano, hydroxy, aminothiocarbonyle, aminocarbonyle, alkyl(C₁-C₄)aminocarbonyle, di-(alkyl(C₁-C₄))amino-carbonyle, hydroxycarbonyle, alkyle en C₁-C₄, halogénoalkyle(C₁-C₄), cycloalkyle en C₃-C₆, alkyl(C₁-C₄)-cycloalkyle(C₃-C₄), alcényle en C₂-C₆, alcynyle en C₂-C₆, alcoxy en C₁-C₆, alcoxy(C₁-C₆)-carbonyle, alkyl(C₁-C₆)carbonyle, alcoxy(C₁-C₄)imino-alkyle(C₁-C₄), alkyl(C₁-C₆)thio, alkyl(C₁-C₆)sulfinyle ou alkyl(C₁-C₆)sulfonyle,
n représentant 2, 3, 4 ou 5, lorsque
au moins un substituant R¹ représente trifluorométhyle,
et en même temps
Y représente un groupe alkyle en C₁-C₄ non substitué, 2,2-difluoroéthyle, alcényle en C₂-C₆ non substitué, alcynyle en C₃-C₆ non substitué, un groupe cycloalkyle en C₃-C₆ non substitué ou hétéroaryle non substitué, et
A représente -C(=O)NR¹³-,
et
G représente C(R⁵).

2. Composés de formule générale (I) ainsi que leurs diastéréoisomères, énantiomères, isomères E/Z, N-oxydes et sels selon la revendication 1, dans lesquels
R¹ représente un atome d'halogène, le groupe nitro, cyano, un groupe alkyle en C₁-C₄, alcoxy en C₁-C₄, alkyl(C₁-C₄)thio, alkyl(C₁-C₄)sulfinyle ou alkyl(C₁-C₄)sulfonyle éventuellement une ou plusieurs fois substitué par halogéno,
n représente 1, 2, 3, 4 ou 5,
ou
R¹ représente -OCF₂O- ou -O(CF₂)₂O-, et est lié à deux atomes de carbone contigus, n représentant alors 1,
R² représente un atome d'hydrogène ou un groupe alkyle en C₁-C₄ éventuellement une à trois fois substitué,
les substituants étant choisis chacun indépendamment parmi halogéno ou alkyle en C₁-C₄,
R³ représente un atome d'hydrogène, un groupe alkyle en C₁-C₄, alkyl (C₁-C₄) carbonyle ou alcoxy(C₁-C₄)carbonyle portant éventuellement un ou plusieurs substituants identiques ou différents,
les substituants étant choisis indépendamment les uns des autres parmi cyano, halogéno ou alcoxy en C₁-C₄
R⁴ représente un groupe alkyle en C₁-C₄, alcényle en C₂-C₆, alcynyle en C₃-C₆, cycloalkyle en C₃-C₆, cycloalkyl (C₃-C₆)-alkyle(C₁-C₃) ou aryl-alkyle(C₁-C₄) portant éventuellement un ou plusieurs substituants identiques ou différents,
les substituants étant choisis indépendamment les uns des autres parmi halogéno, cyano, alkyle en C₁-C₄, alcoxy en C₁-C₄, alcoxy(C₁-C₄)carbonyle ou parmi aryloxy ou aryl-alcoxy(C₁-C₃) portant éventuellement un ou plusieurs substituants identiques ou différents
les substituants étant choisis indépendamment les uns des autres parmi halogéno, cyano, alkyle en C₁-C₄, alcoxy en C₁-C₄
G représente C(R⁵) ou N,
R⁵ représente un atome d'hydrogène ou d'halogène ou le groupe cyano,
R⁶ représente un atome d'halogène, le groupe nitro, cyano, un groupe alkyle en C₁-C₄ ou alcoxy en C₁-C₄ portant éventuellement un ou plusieurs substituants halogéno,
m représente 0, 1, 2
X représente un groupe halogénoalkyle(C₁-C₄) qui éventuellement peut en plus être une à trois fois substitué par hydroxy, cyano ou alcoxy en C₁-C₄,
A représente un groupement chimique divalent qui est choisi parmi les groupements -C(R¹¹)(R¹²)NR¹³C(=O)-ou -C(=O)NR¹³-, la position de liaison indiquée chaque fois en premier (gauche) étant attachée au cycle et la position de liaison indiquée chaque fois en second (droite) étant attachée à Y,
R¹¹ et R¹² représentent indépendamment l'un de l'autre un atome d'hydrogène ou un groupe alkyle en C₁-C₄,
R¹³ représente un atome d'hydrogène, un groupe alkyle en C₁-C₄, cycloalkyle en C₃-C₆, alkyl(C₁-C₄)-carbonyle, alcoxy(C₁-C₄)carbonyle ou alcényle en C₂-C₄,
Y représente un groupe alkyle en C₁-C₄, alcényle en C₂-C₄, alcynyle en C₂-C₄, cycloalkyle en C₃-C₆, phényle, phénylméthyle, pyridinyle, pyridinyl-méthyle, pyrimidinyle ou pyrimidinylméthyle portant éventuellement un ou plusieurs substituants identiques ou différents,
les substituants étant choisis parmi halogéno, nitro, cyano, hydroxy, aminothiocarbonyle, aminocarbonyle, alkyle en C₁-C₄, halogénoalkyle(C₁-C₄), cycloalkyle en C₃-C₅, alkyl(C₁-C₄)-cycloalkyle(C₃-C₄), alcényle en C₂-C₄, alcynyle en C₂-C₄, alcoxy en C₁-C₄, alkyl(C₁-C₄)thio, alkyl(C₁-C₄) sulfinyle, alkyl(C₁-C₄)-sulfonyle, alcoxy(C₁-C₄)carbonyle, alkyl(C₁-C₆)carbonyle ou alcoxy(C₁-C₄)imino-alkyle(C₁-C₄),
n représentant 2, 3, 4 ou 5, lorsque
au moins un substituant R¹ représente trifluorométhyle, et en même temps
Y représente un groupe alkyle en C₁-C₄ non substitué, 2,2-difluoroéthyle, alcényle en C₂-C₆ non substitué, alcynyle en C₃-C₆ non substitué, un groupe cycloalkyle en C₃-C₆ non substitué ou hétéroaryle non substitué, et
A représente -C(=O)NR¹³-,
et
G représente C(R⁵).

3. Composés de formule générale (I) ainsi que leurs diastéréoisomères, énantiomères, isomères E/Z, N-oxydes et sels selon la revendication 1 ou 2, dans lesquels
R¹ représente un atome d'halogène, le groupe nitro, cyano, un groupe alkyle en C₁-C₄, alcoxy en C₁-C₄, alkyl(C₁-C₄)thio, alkyl (C₁-C₄) sulfinyle, alkyl(C₁-C₄)sulfonyle éventuellement une ou plusieurs fois substitué par fluoro ou chloro,
n représente 1, 2, 3, 4 ou 5,
ou
R¹ représente -OCF₂O-, et est lié à deux atomes de carbone contigus, n représentant alors 1,
R² représente un atome d'hydrogène ou le groupe méthyle,
R³ représente un atome d'hydrogène, le groupe méthyle, éthyle, méthylcarbonyle, éthylcarbonyle, méthoxycarbonyle ou éthoxycarbonyle,
R⁴ représente un groupe alkyle en C₁-C₄, alcényle en C₂-C₄, alcynyle en C₃-C₄, cycloalkyle en C₃-C₅, cycloalkyl(C₃-C₅)-alkyle(C₁-C₃) ou phénylalkyle portant éventuellement un à trois substituants,
les substituants étant choisis chacun indépendamment parmi fluoro, cyano, méthoxy, éthoxy, méthyle, éthyle, méthoxycarbonyle, éthoxycarbonyle, phényloxy ou phényl-alcoxy(C₁-C₃),
G représente C(R⁵) ou N,
R⁵ représente un atome d'hydrogène, de fluor, chlore, brome ou le groupe cyano,
R⁶ représente un atome d'halogène, le groupe nitro, cyano, ou un groupe alkyle en C₁-C₄ ou alcoxy en C₁-C₄ portant éventuellement un à trois substituants halogéno,
m représente 0, 1, 2
X représente un groupe halogénoalkyle(C₁-C₄),
A représente un groupement chimique divalent qui est choisi parmi les groupements -C(R¹¹)(R¹²)NR¹³C(=O)-ou -C(=O)NR¹³-, la position de liaison indiquée chaque fois en premier (gauche) étant attachée au cycle et la position de liaison indiquée chaque fois en second (droite) étant attachée à Y,
et
R¹¹ et R¹² représentant indépendamment l'un de l'autre un atome d'hydrogène ou le groupe méthyle,
et
R¹³ représentant un atome d'hydrogène, le groupe méthyle, éthyle, cyclopropyle, méthylcarbonyle, éthylcarbonyle, méthoxycarbonyle, éthoxycarbonyle ou prop-2-én-1-yle,
Y représente un groupe alkyle en C₁-C₄, alcényle en C₂-C₄, alcynyle en C₂-C₄, cycloalkyle en C₃-C₆, phényle, phénylméthyle, pyridin-2-yle, pyridin-2-yl-méthyle, 2,3-pyrimidin-2-yle ou 1,3-pyrimidin-2-yl-méthyle portant éventuellement un à trois substituants identiques ou différents,
les substituants étant choisis parmi fluoro, chloro, nitro, cyano, alkyle en C₁-C₄, trifluorométhyle, cycloalkyle en C₃-C₄, alkyl(C₁-C₄)-cycloalkyle(C₃-C₄). alcényle en C₂-C₄, alcynyle en C₃-C₄, alcoxy en C₁-C₄, alkyl(C₁-C₄)thio, alkyl(C₁-C₄)sulfinyle, alkyl(C₁-C₆)-sulfonyle, alcoxy(C₁-C₄)carbonyle ou aminothiocarbonyle,
n représentant 2, 3, 4 ou 5, lorsque
au moins un substituant R¹ représente trifluorométhyle,
et en même temps
Y représente un groupe alkyle en C₁-C₄ non substitué, 2,2-difluoroéthyle, alcényle en C₂-C₆ non substitué, alcynyle en C₃-C₆ non substitué, un groupe cycloalkyle en C₃-C₆ non substitué ou hétéroaryle non substitué, et
A représente -C(=O)NR¹³-,
et
G représente C(R⁵).

4. Composés de formule générale (I) ainsi que leurs diastéréoisomères, énantiomères, isomères E/Z, N-oxydes et sels selon la revendication 1, 2 ou 3, dans lesquels
R¹ représente le groupe cyano, un atome de fluor, chlore, brome, iode, le groupe difluorométhyle, dichlorofluorométhyle, chlorodifluorométhyle, trifluorométhyle, pentafluoroéthyle, chlorotétrafluoroéthyle, difluorométhoxy, trifluorométhoxy, trifluorométhylthio, trifluorométhylsulfinyle, trifluorométhylsulfonyle,
n représente 1, 2, 3, 4 ou 5,
ou
R¹ représente -OCF₂O-, et est lié à deux atomes de carbone contigus, n représentant alors 1,
R² représente un atome d'hydrogène,
R³ représente un atome d'hydrogène,
R⁴ représente le groupe méthyle, éthyle, prop-1-yle, prop-2-én-1-yle, prop-2-yn-1-yle, éthényle, but-2-yn-1-yle, cyclopropyle, cyclopropylméthyle, cyclobutyle, cyanométhyle, 2-méthylprop-1-yle, éthoxyméthyle, méthoxycarbonylméthyle, phénylméthyle ou benzyloxyméthyle,
G représente C(R⁵) ou N,
R⁵ représente un atome d'hydrogène, de chlore, de brome ou le groupe le groupe cyano,
R⁶ représente le groupe cyano, un atome de fluor, de chlore, de brome, le groupe méthyle, éthyle, isopropyle ou trifluorométhyle,
m représente 0 ou 1,
X représente le groupe trifluorométhyle,
A représente un groupement chimique divalent qui est choisi parmi les groupements -CH₂NHC(=O)- ou -C(=O)NR¹³-, la position de liaison indiquée chaque fois en premier (gauche) étant attachée au cycle et la position de liaison indiquée chaque fois en second (droite) étant attachée à Y,
et
R¹³ représentant un atome d'hydrogène, le groupe méthyle, éthyle ou prop-2-én-1-yle,
Y représente le groupe méthyle, éthyle, propan-1-yle, propan-2-yle, butan-1-yle, butan-2-yle, 2-méthylpropan-1-yle, 2-méthylpropan-2-yle, cyclopropyle, cyclobutyle, cyanométhyle, 1-cyano-éthyle, 2-cyanoéthyle, 1-cyanoprop-1-yle, 2-cyano-prop-1-yle, 3-cyanoprop-1-yle, 1-cyanoprop-2-yle, 2-cyanoprop-2-yle, 1-cyanocyclopropyle, 2-cyano-prop-2-én-1-yle, 2-cyanocyclopropyle, 1-cyanocyclobutyle, 2-cyanocyclobutyle, 3-cyanocyclobutyle, 2-fluoroéthyle, 2,2-difluoroéthyle, 2,2,2-trifluoroéthyle, 1-fluoropropan-2-yle, 2,2-difluoroprop-1-yle, 1,3-difluoropropan-2-yle, 1-méthylcyclopropyle, 2-méthylcyclopropyle, 1-éthylcyclopropyle, 1-éthynylcyclopropyle, 1-éthynylcyclobutyle, 1-méthoxycyclopropyle, 1-éthoxycyclopropyle, 1-méthoxycarbonyl-cyclopropyle, 1-éthoxycarbonyl-cyclopropyle, 1,1'-bi(cyclopropyl)-1-yle, cyclopropylméthyle, 1-trifluorméthylcyclopropyle, pyridin-2-yle, 5-chloropyridin-2-yle, 5-fluoropyridin-2-yle, 1-cyano-l-phenylméthyle, 1,2-diméthylcyclopropyle, 1-(aminothiocarbonyl)cyclopropyle, 1-cyano-2-méthylpropan-1-yle, 1-cyanobut-3-yn-1-yle, 1-cyano-2-méthylpropan-1-yle, 1-cyano-propan-2-yle, 1-cyano-l-cyclopropyléthyle, 1-cyano-1-éthylprop-1-yle, 1-cyano-1-méthylcyclopropyl-méthyle, (2-R)-1-(méthylsulfinyl)propan-2-yle ou 1,3-diméthoxy-2-cyanopropan-2-yle, lorsque A représente le groupement -C(=O)NR¹³-,
ou
Y représente le groupe méthyle, éthyle, propan-1-yle, propan-2-yle, butan-1-yle, 2-fluoroéthyle, 2,2-difluoroéthyle, 2,2,2-trifluoroéthyle, cyclopropyle, cyclobutyle ou cyclopropylméthyle, lorsque A représente le groupement -CH₂NHC(=O)-
n représentant 2, 3, 4 ou 5, lorsque
au moins un substituant R¹ représente trifluorométhyle,
et en même temps
Y représente un groupe alkyle en C₁-C₄ non substitué, 2,2-difluoroéthyle, alcényle en C₂-C₆ non substitué, alcynyle en C₃-C₆ non substitué, un groupe cycloalkyle en C₃-C₆ non substitué ou hétéroaryle non substitué, et
A représente -C(=O)NH-,
et
G représente C(R⁵).

5. Composés de formule générale (I) ainsi que leurs diastéréoisomères, énantiomères, isomères E/Z, N-oxydes et sels selon la revendication 4, dans lesquels G représente CH et A représente -C(=O)NH-.

6. Procédé pour la préparation de composés de formule générale (I) selon la revendication 1, dans lequel
a) on fait réagir des acides carboxyliques de formule générale (II) 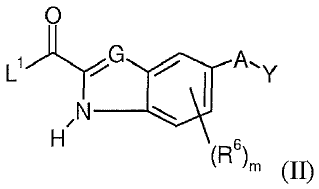 dans laquelle
L¹ représente le groupe hydroxy ou un atome d'halogène,
avec des amines de formule (III) 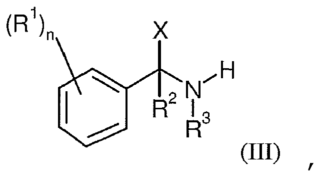 G, A, Y, R¹, R², R³, R⁶, n, m ayant la signification indiquée dans la revendication 1, et
b) on fait ensuite réagir les composés de formule (IVa) 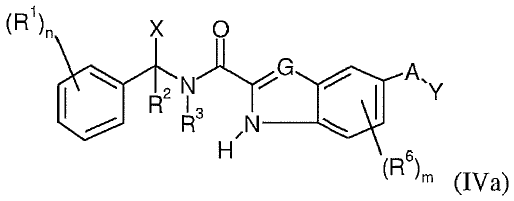 ainsi obtenus avec des agents d'alkylation de formule (V)
R⁴-L² (V)
dans laquelle
L² représente un atome d'halogène, le groupe mésyle ou le groupe tosyle et R⁴ a la signification indiquée dans la revendication 1,
en présence d'une base, pour aboutir aux composés de formule (I).

7. Procédé pour la fabrication de pesticides, dans lequel on mélange des composés de formule générale (I) et/ou leurs sels selon la revendication 1 avec des excipients et/ou des substances tensioactives.

8. Utilisation des composés de formule générale (I) et/ou de leurs sels selon la revendication 1, pour la fabrication de pesticides.

9. Pesticide contenant des composés de formule générale (I) et/ou leurs sels selon la revendication 1 en des teneurs biologiquement efficaces de 0,00000001 à 95 % en poids, par rapport au poids du pesticide.

10. Pesticide selon la revendication 9, contenant en plus une autre substance active agrochimique.

11. Procédé pour la lutte contre des ravageurs animaux, dans lequel on fait agir sur des ravageurs animaux et/ou leur habitat des composés de formule générale (I) et/ou leurs sels selon la revendication 1, les procédés pour le traitement du corps humain ou animal étant exclus.

12. Acides carboxyliques de formule générale (II-1aa) 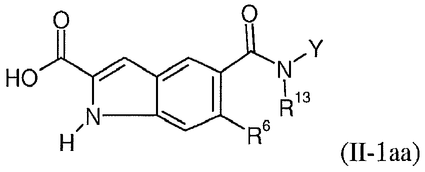 dans laquelle R⁶, Y et R¹³ ont la signification indiquée dans la revendication 4.

13. Acides carboxyliques de formule générale (II-1ba) 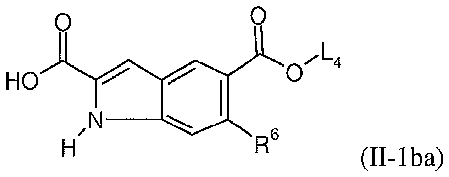 dans laquelle L⁴ représente un groupe alkyle en C₁-C₄ et R⁶ a la signification indiquée dans la revendication 4, le composé acide 6-chloro-5-(éthoxycarbonyl)-1H-indole-2-carboxylique étant exclu.

14. Acides carboxyliques de formule générale (II-2aa) 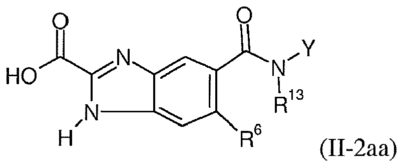 dans laquelle R⁶, Y et R¹³ ont la signification indiquée dans la revendication 4.

15. Acides carboxyliques de formule générale (II-2ba) 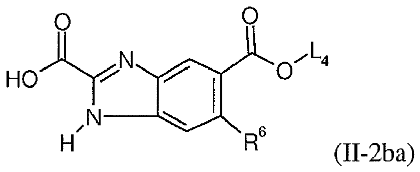 dans laquelle L⁴ représente un groupe alkyle en C₁-C₄ et R⁶ a la signification indiquée dans la revendication 4.
